(19) Europäisches Patentamt
European Patent Office
Office européen des brevets

(11) Numéro de publication: **0 446 133 B1**

(12) **FASCICULE DE BREVET EUROPEEN**

(45) Date de publication de fascicule du brevet: **14.09.94**

(21) Numéro de dépôt: **91400620.0**

(22) Date de dépôt: **07.03.91**

(51) Int. Cl.5: **C07D 495/14**, C07D 498/22, A61K 31/55, //(C07D495/14, 333:00,243:00,209:00), (C07D498/22,333:00,265:00, 243:00,209:00)

# (54) Nouveaux dérivés de la 4H-pyrrolo[1,2-a]thieno[3,2-f][1,4]-diazépine leur procédé de préparation et les compositions pharmaceutiques qui les contiennent.

(30) Priorité: **08.03.90 FR 9002933**

(43) Date de publication de la demande:
**11.09.91 Bulletin 91/37**

(45) Mention de la délivrance du brevet:
**14.09.94 Bulletin 94/37**

(84) Etats contractants désignés:
**AT BE CH DE DK ES FR GB GR IT LI LU NL SE**

(56) Documents cités:
**EP-A- 0 368 175**

**TETRAHEDRON LETTERS, no. 7, 1979, pages 643-644, Pergamon Press Ltd., Oxford,GB; S. RAULT et al.: "Pyrrolo[1,2-a]thieno[3,2-f]1,4-diazepines. Novel synthesis and X-ray analysis"**

(73) Titulaire: **ADIR ET COMPAGNIE**
**1 rue Carle Hébert**
**F-92415 Courbevoie Cédex (FR)**

(72) Inventeur: **Rault, Sylvain**
**route de St. Pierre sur Dives**
**F-14370 Moult (FR)**
Inventeur: **Boulouard, Michel**
**5 boulevard du Général Vanier**
**F-14000 Caen (FR)**
Inventeur: **Dallemagne, Patrick**
**Le Vieux Presbytère**
**F-14260 St-Georges D'Aunay (FR)**
Inventeur: **Robba, Max**
**4 rue Massillon**
**F-75004 Paris (FR)**
Inventeur: **Guardiola, Béatrice**
**6 rue Edouard Nortier**
**F-92200 Neuilly sur Seine (FR)**
Inventeur: **Devissaguet, Michelle**
**14 boulevard d'Inkermann**
**F-92200 Neuilly sur Seine (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

HETEROCYCLES, vol. 12, no. 8, 1979, pages 1009-1011, Sendai, JP; S. RAULT et al.:"Novel convenient synthesis of 1,4-diazepines; 6-alkoxy-5,6-dihydro-4H-pyrrolo[1,2-a]thieno[3,2--f]-1,4-diazepine-4-ones"

JOURNAL OF HETEROCYCLIC CHEMISTRY, vol. 14, no. 2, avril 1977, pages 235-240,Heterocorp., Utah, US; H. FUJIMORI et al.: "Diazepines. III. Synthesis of 4H-pyrrolo[1,2-a]thieno[3,2-f][1,4] diazepines"

## Description

La présente invention concerne des nouveaux dérivés de la 4H-pyrrolo [1,2-a] thiéno [3,2-f] [1,4]-diazépine, leur procédé de préparation et les compositions pharmaceutiques qui les contiennent.

Certains dérivés de la 5,6-dihydro 4-oxo 4H-pyrrolo [1,2-a] thiéno [3,2-f] [1,4]-diazépine sont décrits dans la littérature. (Heterocycles, 1979,,Vol. 12, N°8, pp.1009-1011 et Tetrahedron Letters, 1979,N°7, pp.643-644). Il est aussi connu que certains composés de la 6H-pyrrolo [1,2-a] thiéno [3,2-f] [1,4]-diazépine pourraient avoir des propriétés antinéoplastiques (C.R.Acad.Sc.Paris,1978,287,pp. 117-120). Des dérivés de la 4-phényl 6H-pyrrolo [1,2-a] thiéno [3,2-f] [1,4]-diazépine sont décrits dans J.Heter.Chem.,1977,14,N°2,pp.235-240.

Les pyrrolo [1,2-a] thiéno [3,2-f] [1,4]-diazépine de la demanderesse sont des composés de structure originale qui possèdent d'intéressantes propriétés pharmacologiques.

Ces composés sont dotés de propriétés antihypoxiques remarquables qui sont très intéressantes dans le traitement du vieillissement cérébrale, des accidents vasculaires cérébraux et des syndromes ischémiques de toute localisation aigue, transitoire ou progressive, en excerçant leurs propriétés pharmacologiques vis à vis de la défaillance d'oxygénation qui accompagne ces accidents.

Ce sont également de puissants antagonistes de la CCK (cholécystokinine). On a déjà décrit dans le brevet européen 167919 des diazépines, qui sont des antagonistes de la cholécystokinine et qui se lient spécifiquement aux récepteurs de celle-ci, ce qui permet d'envisager leur utilisation dans traitement de troubles du système nerveux central, de l'estomac, de l'intestin, du pancréas ou de la vésicule biliaire et d'autres troubles dépendant de la CCK. Les composés de l'invention, tout en présentant des activités égales à celles des composés du brevet européen précités les plus actifs, sont nettement moins toxiques de sorte que leur index thérapeutique est meilleur.

Les composés de la demanderesse sont également extrèmement intéressants de par leurs effets métaboliques, puisqu'ils possèdent d'importantes propriétés hypoglycémiantes, hypocholestérolémiantes, et hypotriglycéridémiantes en étant par exemple beaucoup plus actifs que le clofibrate.

Plus spécifiquement, l'invention concerne les composés de formule générale **(I) :**

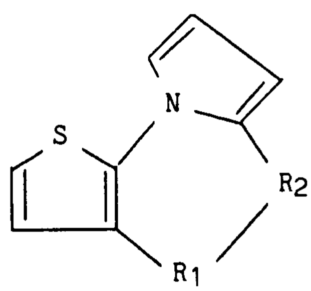

(I)

dans laquelle :

- $R_1$ représente un radical de formule générale **($Z_0$)**, **($Z_1$)**, **($Z_2$)**, **($Z_3$)** ou **($Z_4$)**

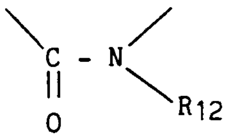

**($Z_0$)**

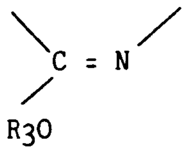

**($Z_1$)**

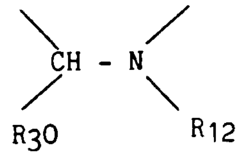

**($Z_2$)**

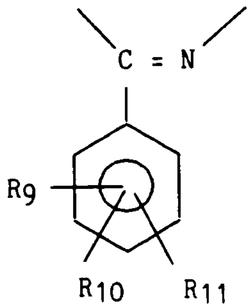

**($Z_3$)**

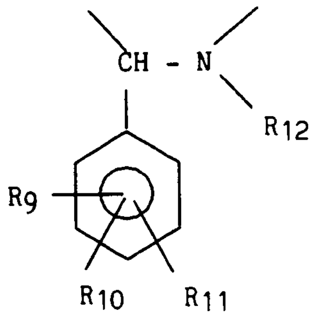

**($Z_4$)**

dans lesquelles :

- $R_3$ représente un atome d'hydrogène, un radical alkyle linéaire ou ramifié de 1 à 6 atomes de carbone saturé ou comportant une double liaison,
- $R_9$, $R_{10}$, $R_{11}$ identiques ou différents représentent chacun un atome d'hydrogène, un atome d'halogène ou un radical alkyle de 1 à 6 atomes de carbone,
- $R_{12}$ représente un atome d'hydrogène ou forme avec $R_2$ et l'atome d'azote auquel ils sont rattachés un radical de formule générale **(W) :**

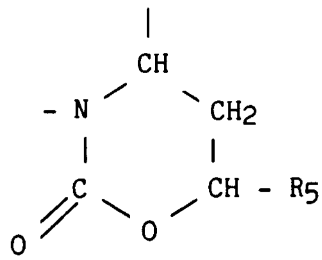

**(W)**

- $R_2$ représente un radical méthylène, un radical hydroxyméthylène, un radical carbonyle, un radical de formule générale **($Y_1$)**, **($Y_2$)**, **($Y_3$)** ou **($Y_4$) :**

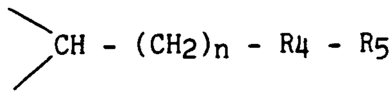

(Y1)

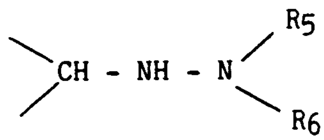

(Y2)

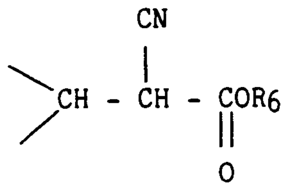

(Y3)

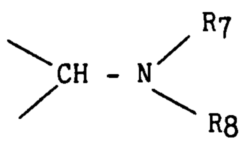

(Y4)

ou forme avec $R_{12}$ et l'atome d'azote auxquels ils sont rattachés un radical de formule générale **(W) :**

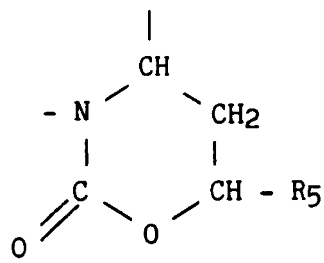

**(W)**

formules dans lesquelles :

- $R_4$ représente un atome d'oxygène, ou de soufre, un radical carbonyle, un radical de formule générale **($X_1$), ($X_2$), ($X_3$)** ou **($X_4$) :**

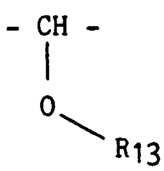

**($X_1$)**

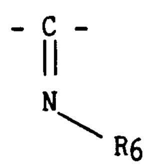

**($X_2$)**

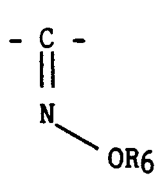

**($X_3$)**

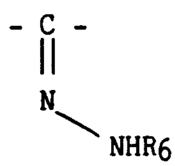

**($X_4$)**

- n est compris entre 0 et 4 inclusivement

- $R_5$ représente un atome d'hydrogène, une chaîne alkyle de 1 à 10 atomes de carbone, linéaire ou ramifiée, éventuellement interrompue par un ou plusieurs atomes d'oxygène, de soufre, un radical phényle, benzoyle ou aralkyle de 7 à 11 atomes de carbone (éventuellement substitués sur le noyau aromatique par un ou plusieurs atomes d'halogène, des radicaux alkyle de 1 à 6 atomes de carbone linéaires ou ramifiés, des radicaux nitro ou des radicaux alcoxy de 1 à 6 atomes de carbone linéaires ou ramifiés) un radical carboxyalkyle de 2 à 7 atomes de carbone linéaire ou ramifié, un radical alcoxycarbonylalkyle de 3 à 10 atomes de carbone linéaire ou ramifié, un radical alcoxycarbonyle de 2 à 7 atomes de carbone linéaire ou carbone, un système cyclique insaturé de 5 à 7 sommets comprenant au moins un hétéroatome choisi parmi azote, soufre, oxygène, un radical pyridinylcarbonyle, pyrimidylcarbonyle, un radical Clofibroyle ou un radical 6-hydroxy 2,5,7,8-tétraméthyle chromanne-2-carbonyle,
- $R_6$ représente un atome d'hydrogène ou un radical alkyle de 1 à 6 atomes de carbone linéaire ou ramifié,
- $R_7$, $R_8$ identiques ou différents représentent chacun un atome d'hydrogène, un radical alkyle linéaire ou ramifié de 1 à 6 atomes de carbone, un radical phényle ou phénylalkyle de 7 à 9 atomes de carbone (éventuellement substitués sur le noyau aromatique par un ou plusieurs atomes d'halogène ou groupements alkyle ou alcoxy de 1 à 6 atomes de carbone), ou forment ensemble avec l'atome d'azote auquel ils sont attachés un système cyclique de 5 à 7 sommets, saturé ou insaturé, comprenant de 1 à 2 hétéroatomes choisis parmi azote, oxygène et soufre éventuellement substitué par un groupement alkylcarbonyl ou alcoxy carbonyle de 2 à 5 atomes de carbone,
- $R_{13}$ représente un atome d'hydrogène, un radical alkyl carbonyl de 2 à 6 atomes de carbone linéaire ou ramifié, un radical benzoyle,

avec les réserves que :

- lorsque $R_1$ représente un radical de formule générale **($Z_3$)** alors $R_2$ ne peut représenter un radical méthylène,
- lorsque $R_1$ représente un radical de formule générale **($Z_0$)** et $R_{12}$ représente un atome d'hydrogène alors $R_2$ ne peut représenter les radicaux suivants :
  $>CH-O-CH_3$, $>CH-O-CH_2CH_3$, $>CH-O-CH_2CH_2CH_3$, $>CH-O-CH_2CH_2-O-CH_2CH_3$, $>CH-O-CH_2CH_2-O-CH_2CH_2CH_2CH_3$
- lorsque $R_1$ représente un radical de formule générale **($Z_0$)**, $R_{12}$ représente un atome d'hydrogène et $R_2$ représente un radical de formule générale **($Y_1$)** avec $n = 1$ et $R_4$ représentant un radical carbonyle alors $R_5$ ne peut représenter un radical méthyle, isopropyle, phényle,
- lorsque $R_1$ représente un radical de formule générale **($Z_0$)** et $R_{12}$ représente un atome d'hydrogène alors $R_2$ ne peut pas représenter un radical carbonyle.

L'invention concerne plus particulièrement les composés de formules générales :

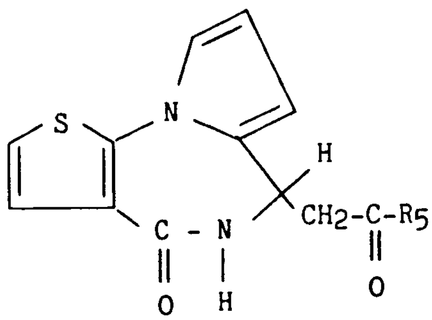

($I_A$)

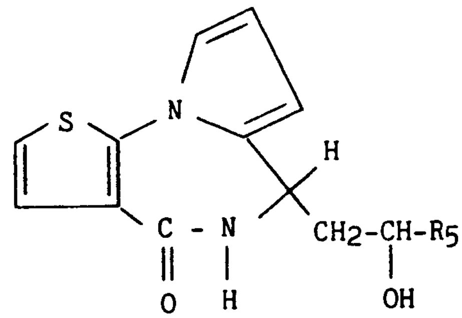

($I_B$)

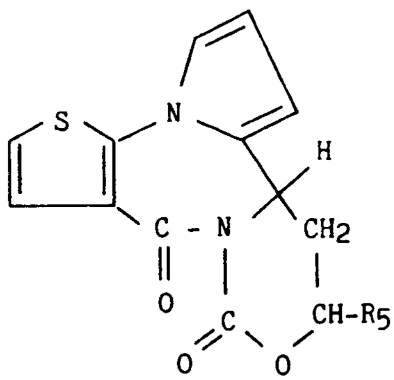

($I_C$)

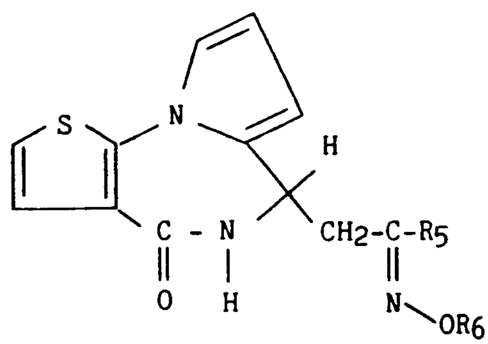

($I_D$)

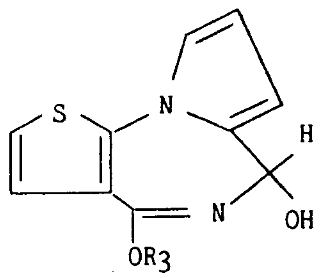

(I_E)

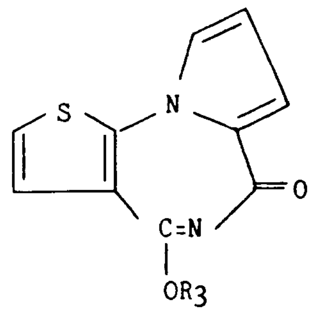

(I_F)

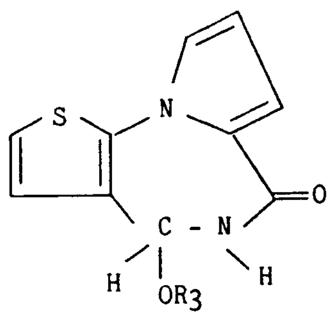

(I_G)

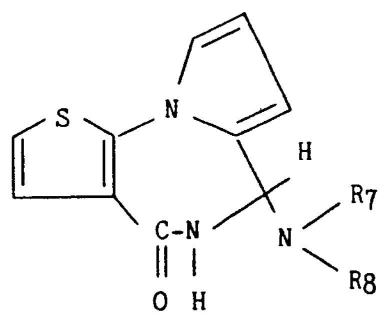

(I_H)

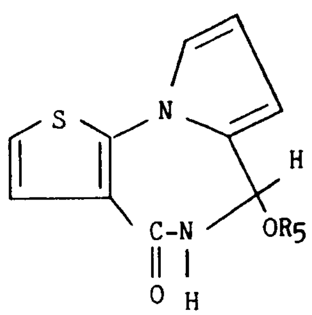

(I_J)

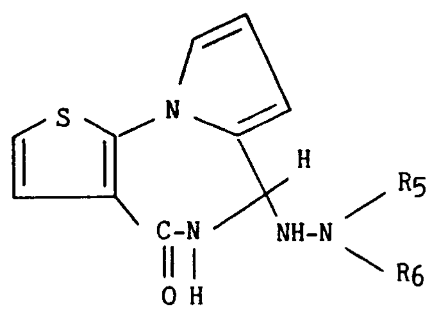

(I_K)

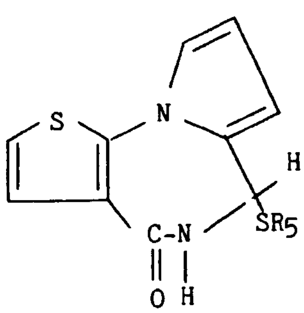

(I_L)

L'invention concerne encore plus particulièrement le 5,6-dihydro 6-(2-benzyloxy propyl) 4-oxo 4H-pyrrolo [1,2-a] thiéno [3,2-f] [1,4]-diazépine
et le 5,6-dihydro 6-(2-hydrazono-1-pentyl)4-oxo 4H-pyrrolo [1,2-a] thiéno [3,2-f] [1,4]-diazépine

La présente invention s'étend également au procédé d'obtention des composés de formule générale **(I)** caractérisé en ce que :

**soit**

- on fait réagir le 3-cyano 2-(2-formyl pyrrol-1-yl) thiophène, composé de formule **(II)** :

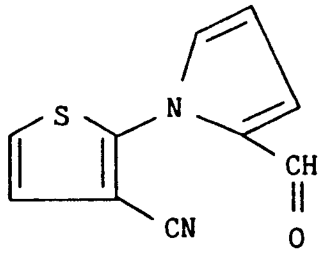

**(II)**

**ou**

- avec une méthylcétone de formule générale **(III)** :

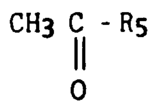

**(III)**

dans laquelle $R_5$ a la même signification que dans les dérivés de formule générale **(I)**, en présence d'une base minérale forte et de péroxyde d'hydrogène, de manière à obtenir les composés de formule générale **($I_A$)** :

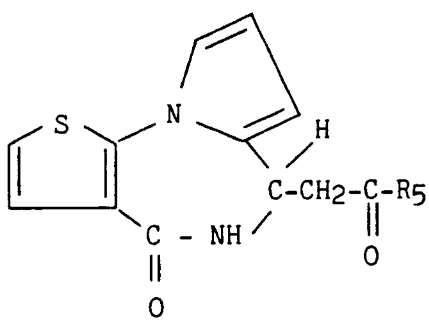

**($I_A$)**

dans laquelle $R_5$ a la même signification que dans les dérivés de formule générale **(I),**

lesquels ensuite :

soit

on soumet à l'action du borohydrure de sodium, en solution dans un solvant alcoolique de manière à obtenir les composés de formule générale **($I_B$) :**

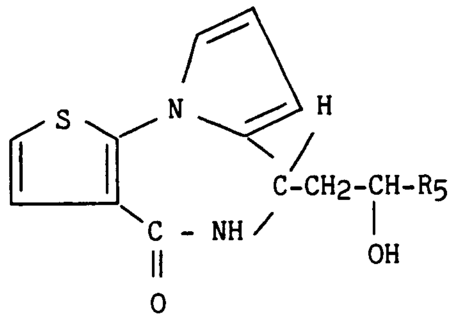

(IB)

dans laquelle $R_5$ a la même signification que dans les dérivés de formule générale **(I),** que l'on peut éventuellement soumettre à l'action du phosgène, dans un solvant organique aromatique, à chaud, pour former les composés de formule générale **($I_C$):**

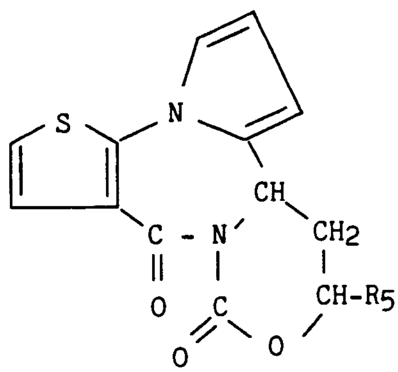

(IC)

dans laquelle $R_5$ a la même signification que dans les composés de formule générale **(I),**
ou faire réagir avec un composé de formule générale **(XI) :**

$R_{13}$ - Cl **(XI)**

dans laquelle $R_{13}$ a la même signification que dans les composés de formule générale **(I),** de manière à obtenir les composés de formule générale **($I_M$) :**

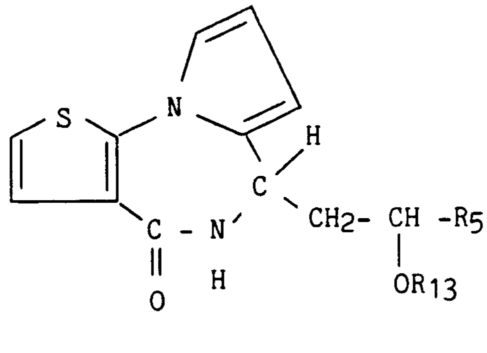

(IM)

dans laquelle $R_5$ et $R_{13}$ ont la même signification que dans les composés de formule générale **(I),**

soit

on condense avec un dérivé d'hydroxylamine de formule générale **(IV) :**

$H_2N$ - O - $R_6$ **(IV)**

dans laquelle $R_6$ a la même signification que dans les dérivés de formule générale **(I),** pour former les

composés de formule générale **($I_D$) :**

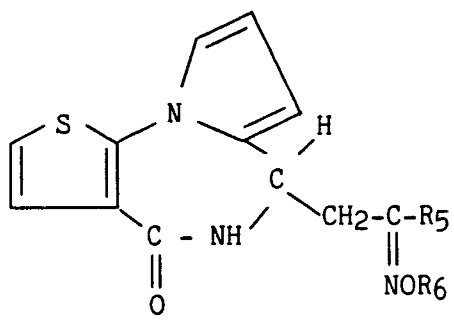

**($I_D$)**

dans laquelle $R_5$ et $R_6$ ont la même signification que dans les dérivés de formule générale **(I),**

soit

on condense avec un dérivé hydrazinique de formule générale **(XII) :**

$H_2N - NH - R_6$ **(XII)**

dans laquelle $R_6$ a la même signification que dans les composés de formule générale **(I),** de manière à obtenir les composés de formule générale **($I_N$) :**

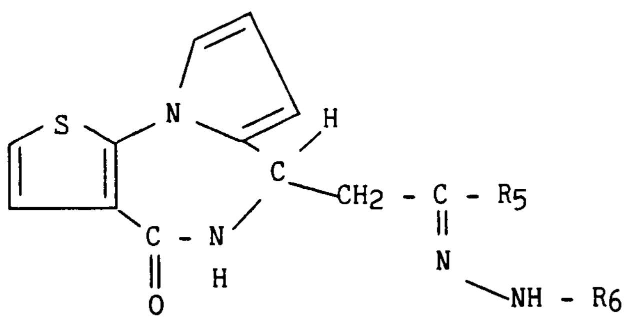

**($I_N$)**

dans laquelle $R_5$ et $R_6$ ont la même signification que dans les composés de formule générale **(I),**

**ou**

avec un alcool primaire de formule générale **(V) :**

$R_3OH$ **(V)**

dans laquelle $R_3$ a la même signification que dans les composés de formule générale **(I),**

en présence d'une base minérale forte et à une température comprise entre 30°-80°C, de manière à obtenir les composés de formule générale **($I_E$) :**

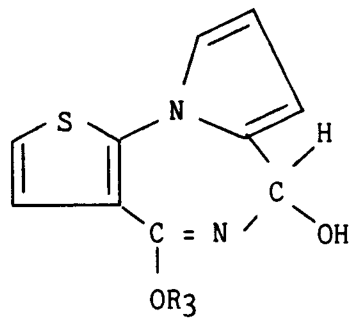

$(I_E)$

dans laquelle $R_3$ a la même signification que dans les composés de formule générale **(I),** que l'on soumet ensuite à l'action du permanganate de potassium, à température ambiante, pour former les composés de formule générale **($I_F$),**

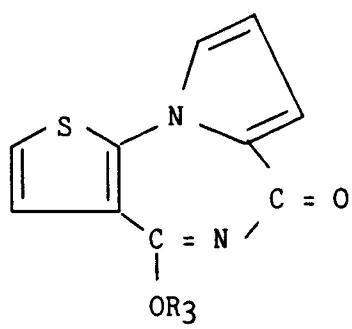

$(I_F)$

dans laquelle $R_3$ a la même signification que dans les composés de formule générale **(I),** que l'on peut éventuellement soumettre à l'action du borohydrure de sodium pour obtenir les composés de formule générale **($I_G$),**

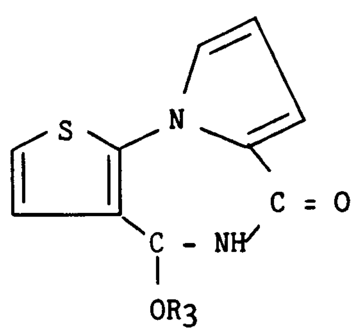

$(I_G)$

dans laquelle $R_3$ a la même signification que dans les composés de formule générale **(I),**

**soit**

on soumet le 2-(2-formyl pyrrol-1-yl)3-thiophène carboxamide, composé de formule **(VI) :**

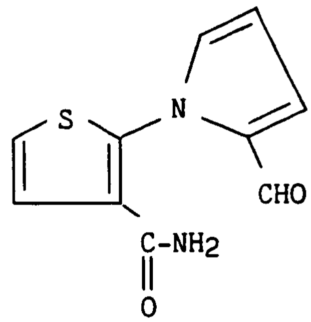

**(VI)**

**ou**

à l'action d'une amine de formule générale **(VII) :**

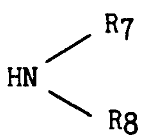

**(VII)**

dans laquelle $R_7$ et $R_8$ ont la même signification que dans les composés de formule générale **(I),** de manière à obtenir les composés de formule générale **($I_H$) :**

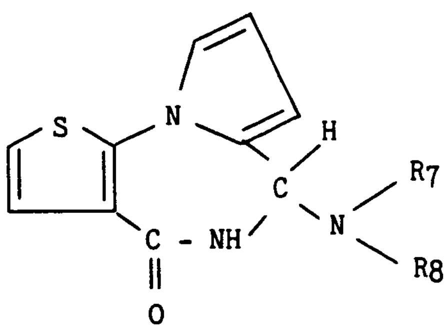

**($I_H$)**

dans laquelle $R_7$ et $R_8$ ont la même signification que dans les composés de formule générale **(I),** que l'on peut éventuellement soumettre, dans le cas où $R_7$ et $R_8$ forment ensemble avec l'atome d'azote auquel ils sont attachés un radical morpholino, à l'action du borohydrure de sodium, pour obtenir le composé de formule générale **(I)** dans laquelle $R_2$ représente un radical méthylène et $R_1$ un radical de formule générale **($Z_0$)** avec $R_{12}$ = H,

**ou**

à l'action de la triéthylamine en présence d'eau et à température ambiante pour former le composé de formule générale **(I)** dans laquelle $R_2$ représente un radical hydroxyméthylène et $R_1$ un radical de formule **($Z_0$)** avec $R_{12}$ = H,

lequel ensuite,

soit

on fait réagir avec une amine de formule générale **(VII),** pour former les composés de formule générale **($I_H$),**

soit

on condense avec un composé de formule générale **(VIII) :**

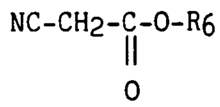

(VIII)

dans laquelle $R_6$ a la même signification que dans les composés de formule générale **(I),** en présence de triéthylamine, pour former les composés de formule générale **($I_I$) :**

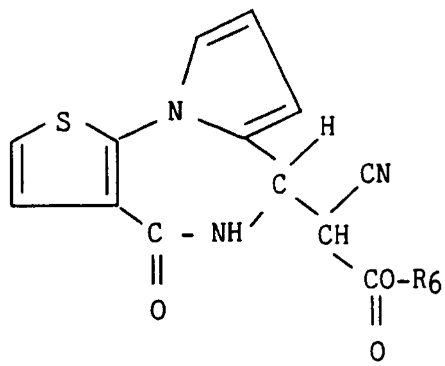

($I_I$)

dans laquelle $R_6$ a la même signification que dans les composés de formule générale **(I),**

soit

on fait réagir avec un alcool de formule générale **(IX),**

$R_5OH$ **(IX)**

dans laquelle $R_5$ a la même signification que dans les composés de formule générale **(I),** à chaud, pour obtenir les composés de formule générale **($I_J$) :**

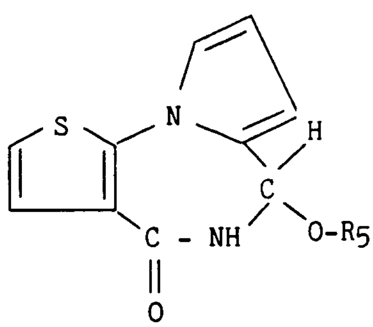

($I_J$)

dans laquelle $R_5$ a la même signification que dans les dérivés de formule générale **(I),**

**ou**

à l'action d'un composé de formule générale **(X) :**

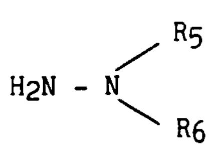

(X)

dans laquelle $R_5$ et $R_6$ ont la même signification que dans les composés de formule générale **(I),** pour former les composés de formule générale **($I_K$) :**

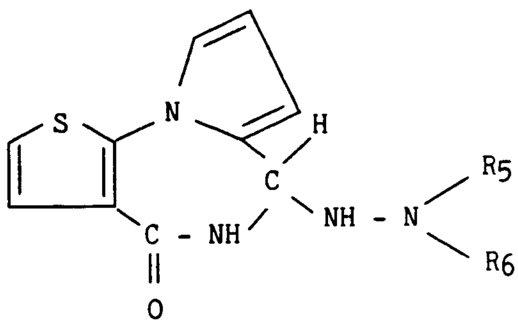

**($I_K$)**

dans laquelle $R_5$ et $R_6$ ont la même signification que dans les composés de formule générale **(I),**

**<u>ou</u>**

à l'action d'un alcool de formule générale **(IX)** pour former les composés de formule générale **($I_J$),**

**<u>ou</u>**

à l'action d'un thiol de formule générale **(XI) :**

$R_5SH$ **(XI)**

dans laquelle $R_5$ a la même signification que dans les composés de formule générale **(I),** à température ambiante, pour former les composés de formule générale **($I_L$),**

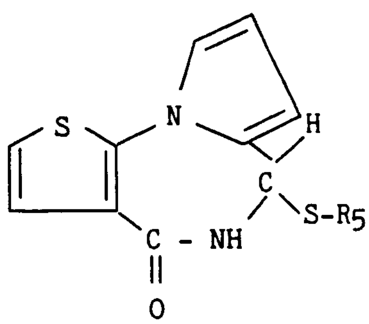

**($I_L$)**

dans laquelle $R_5$ a la même signification que dans les composés de formule générale **(I),** que l'on peut éventuellement soumettre, dans le cas où $R_5$ représente un radical carboxyalkyle, à l'action de la triéthylamine et d'un chloroformiate, puis à l'action d'ammoniac gazeux pour former les amides correspondants.

Les composés de formules générales **($I_A$)** à **($I_N$)** font partie de l'invention et font partie des composés de formule générale **(I).**

Le 3-cyano 2-(2-formyl pyrrol-1-yl) thiophène, composé de formule **(II)** est un composé déjà connu (Hétérocycles,1986,Vol <u>24</u>,(3),pp.575-578).

La condensation du composé de formule **(II)** avec les composés de formule générale **(III)** s'effectue dans un mélange hydroalcoolique, à chaud, en présence d'une base minérale forte et de péroxyde d'hydrogène.

La réduction des composés de formule générale **($I_A$),** par le borohydrure de sodium, qui conduit à l'obtention des composés de formule générale **($I_B$)** est réalisée dans un alcool primaire de petit poids

moléculaire, à température ambiante.

Pour obtenir les composés de formule génrérale **($I_C$)**, la cyclisation des composés de formule générale **($I_B$)** est réalisée à chaud, avec du phosgène, dans un solvant organique aromatique, comme par exemple le toluène.

Les composés de formule générale **($I_D$)** sont obtenus à partir des composés de formule génrérale **($I_A$)**. Ces derniers sont mis en solution dans un alcool de petit poids moléculaire. Puis, en présence d'acétate de sodium, on additionne les composés de formule générale **(IV)** et on chauffe à reflux.

Le 2-(2-formyl pyrrol-1-yl) 3-thiophène carboxamide, composé de formule **(VI)** a été préparé à partir du composé de formule **(II)** selon des méthodes classiques.

La réaction du composé de formule **(VI)** avec les amines de formule générale **(VII)** s'effectue dans l'eau à température ambiante lorsque ces dernières se trouvent en excès dans le milieu réactionnel (10 fois la quantité molaire nécessaire) ou à chaud, dans l'acétonitrile lorsque les quantités des composés **(VI)** et **(VII)** sont équimolaires.

Pour obtenir les composés de formule générale **($I_K$)**, la réaction des composés de formule générale **(X)** avec le composé de formule **(VI)** s'effectue dans l'eau à une température comprise entre 50°-70°C. La fin de la réaction est réalisée à température ambiante.

Les composés selon l'invention, ainsi que leurs sels, sont doués de propriétés pharmacologiques fort intéressantes. En effet, les essais pharmacologiques in vivo ont montré que les composés de la présente invention exercent un puissant effet antihypoxique chez l'animal.

Lors du vieillissement ou à la suite d'un accident vasculaire cérébral, la fragilité et la vulnérabilité cellulaire accrues sont des composantes physiopathologiques importantes pour la recherche de nouvelles thérapeutiques visant à protéger le cerveau mis en situation d'incapacité à répondre à toute nouvelle agression issue de son environnement.

Une telle agression peut être reproduite sous forme d'un défaut d'apport en oxygène et c'est pourquoi, par leurs conséquences, une analogie étroite existe entre l'hypoxie et le vieillissement cérébral.

Les composés de la présente invention ont été testés sur leur capacité à prolonger la survie du tissu cérébral lors de d'hypoxie aiguë chez la souris. Les résultats des essais ont prouvé que les composés de l'invention ont un effet protecteur antihypoxique très puissant et ont ainsi confirmé le grand intérêt de leur emploi en thérapeutique.

En s'opposant nettement à la mort cérébrale lors d'insuffisance d'apport en oxygène, les composés de la présente invention exercent un effet antihypoxique marqué et sont donc utiles dans les cas de syndromes ischémiques de toute localisation aiguë, transitoire ou progressive puisqu'ils exercent leurs propriétés pharmacologiques vis-à-vis de la défaillance d'oxygénation qui accompagne ces accidents. Leurs propriétés pharmacologiques permettent leur application dans la correction des désordres liés à l'hypoxémie par exemple lors du vieillissement cérébral.

L'affinité des composés de l'invention pour les récepteurs de la cholécystokinine a été étudiée en déterminant la concentration inhibitrice 50 ($CI_{50}$) pour la fixation de la cholécystokinine marqué à l'iode 125 aux récepteurs de membranes plasmatiques de pancréas de rat, et de membranes de cerveau de cobayes. Pour la majorité des composés de l'invention, il a été trouvé que le rapport de la $CI_{50}$ pour les récepteurs du cerveau à la $CI_{50}$ pour les récepteurs du pancréas est très élevée. Ceci démontre que les composés de l'invention présentent une meilleure sélectivité pour les récepteurs de la cholécystokinine périphériques, et permettent un meilleur traitement des troubles qui en dépendent, avec moins d'effets secondaires.

Les composés de l'invention apparaissent donc particulièrement utiles à la thérapeutique humaine ou vétérinaire dans le traitement des troubles de l'estomac, de l'intestin, du pancréas et de la vésicule biliaire dépendant de la cholécystokinine, comme par exemple les pancréatites, les troubles de la motricité gastrique ou vésiculaire, les ulcères et le syndrome du colon irritable. Ils trouvent aussi leur application dans le traitement de la douleur, éventuellement des troubles dépendant de l'intéraction de la cholécystokinine avec les neuromédiateurs du système nerveux central comme par exemple les désordres neuroleptiques, la maladie de Parkinson, la psychose, et la diskynésie tardive. Les composés de l'invention sont aussi utiles pour la régularisation de l'appétit.

Les composés de l'invention possèdent également de remarquables propriétés métaboliques en étant fortement hypolipémiants, hypocholestérolémiants, et hypotriglycéridémiants.

Donnés pendant 15 jours à des rats soumis à un régime hypercholestérolémiants, ils se sont révélés être beaucoup plus actifs (au moins 30 fois) que le Clofibrate sur l'abaissement des taux plasmatiques en triglycérides, cholestérol et HDL (high density lipoprotéines) - cholestérol.

La toxicité a été évaluée chez la souris mâle. La $DL_{50}$ des composés de l'invention est supérieure à 1500 mg/Kg (voie i.p.).

Les composés de formule générale **(I)** ainsi que leurs sels d'addition à un acide minéral ou organique pharmaceutiquement acceptable comme par exemple les acides chlorhydrique, méthanesulfonique, citrique, maléique peuvent être élaborés en préparations pharmaceutiques selon des procédés généralement connus comme par exemple en comprimés, gélules, dragées, solutions buvables, solutions injectables, suspension buvables, émulsions, suppositoires.

Outre les excipients inertes, non toxiques et pharmaceutiquement acceptables, tels par exemple l'eau distillées, le glucose, le lactose, l'amidon, le talc, les huiles végétales, l'éthylène glycol etc..., ces préparations peuvent également contenir des agents de préservation, stabilisants, mouillants, émulsifiants etc...

Les compositions ainsi obtenus se présentent généralement sous forme dosée et peuvent contenir selon les affections traitées, l'âge et le sexe du malade de 0,1 à 100 mg de principe actif. Elles peuvent selon le cas être administrées par voie orale, rectale, ou parentérale à la dose de 0,1 à 100 mg de une à plusieurs fois par jour.

Les exemples suivants illustrent l'invention

Les caractéristiques de spectrométrie RMN $^1$H sont regroupées dans les tableaux I à XIII.

**EXEMPLE 1**

**5,6-dihydro 4-oxo 6-(2-oxo pent-1-yl) 4H-pyrrolo [1,2-a] thiéno [3,2-f] [1,4]-diazépine**

Une solution de 12 g (0,059 mole) de 3-cyano 2-(2-formyl pyrrol-1-yl) thiophène dans un mélange de 200 ml de pentan-2-one, 200 ml d'hydroxyde de sodium 6N et 15 ml de péroxyde d'hydrogène à 33% est chauffée au reflux pendant une heure. L'éthanol et la pentan-2-one sont ensuite éliminés sous vide et le reste du mélange réactionnel est versé sur 200 ml d'eau froide. Le précipité formé est essoré, lavé à l'eau, séché et recristallisé.
Rendement : 68%
Point de fusion : 186 ° C (Ether éthylique - acétone) cristaux jaunes

Analyse élémentaire :

| | C% | H% | N% | S% |
|---|---|---|---|---|
| Théorie | 62,49 | 5,59 | 9,72 | 11,10 |
| Trouvé | 62,48 | 5,50 | 9,72 | 11,23 |

Spectre IR (KBr) : Bandes NH à 3275 et 3170 $cm^{-1}$,
Bandes CH à 3050, 2950, 2920 et 2870 $cm^{-1}$,
Bandes C=O à 1705 (cétone) et 1640 $cm^{-1}$ (lactame),
Bandes principales à 1470, 1440, 1325, 1150, 1095, 795, 705 et 685 $cm^{-1}$.

**EXEMPLE 2**

**6-(2-cyclopropyl 2-oxo éth-1-yl) 5,6-dihydro 4-oxo 4H-pyrrolo [1,2-a] thiéno [3,2-f] [1,4]-diazépine**

Ce composé a été préparé selon le procédé décrit dans l'exemple 1 mais en utilisant la cyclopropylméthylcétone au lieu de la pentan-2-one.
Rendement : 57%
Point de fusion : 200 ° C (Ether éthylique-acétone) cristaux blancs

Analyse élémentaire :

| | C% | H% | N% | S% |
|---|---|---|---|---|
| Théorie | 62,91 | 4,92 | 9,78 | 11,19 |
| Trouvé | 62,87 | 4,93 | 9,81 | 11,05 |

Spectre IR (KBr) : Bandes NH à 3270 et 3180 $cm^{-1}$,

Bandes CH à 3120, 3090, 3050 et 2890 $cm^{-1}$,
Bandes CO à 1685 et 1640 $cm^{-1}$,
Bandes principales à 1530, 1475, 1440, 1395, 1330, 1090, 910 et 735 $cm^{-1}$.

**EXEMPLE 3**

**5,6-dihydro 4-oxo 6-(2-oxo 2-phénéthyl éthyl) 4H-pyrrolo [1,2-a] thiéno [3,2-f] [1,4]-diazépine**

Ce composé a été préparé selon le procédé décrit dans l'exemple 1 mais en utilisant comme cétone la benzylacétone.
Rendement : 19%
Point de fusion : 172 °C (Ether éthylique) cristaux blancs

Analyse élémentaire :

| | C% | H% | N% | S% |
|---|---|---|---|---|
| Théorie | 68,55 | 5,18 | 7,99 | 9,15 |
| Trouvé | 68,22 | 5,36 | 7,90 | 8,85 |

Spectre IR (KBr) : Bandes NH à 3270 et 3170 $cm^{-1}$,
Bandes CH à 3050, 3020, 2920 et 2880 $cm^{-1}$,
Bandes CO à 1710 et 1645 $cm^{-1}$,
Bandes principales à 1485, 1440, 1330, 1190, 1100 et 705 $cm^{-1}$.

**EXEMPLE 4**

**5,6-dihydro 6-(4-méthyl phénacyl) 4-oxo 4H-pyrrolo [1,2-a] thiéno [3,2-f] [1,4]-diazépine**

Ce composé a été préparé selon le procédé décrit dans l'exemple 1 mais en utilisant la 4-méthyl acétophénone au lieu de la pentan-2-one.
Rendement : 44%
Point de fusion : 190 °C (Ether éthylique) cristaux blancs

Analyse élémentaire :

| | C% | H% | N% | S% |
|---|---|---|---|---|
| Théorie | 67,84 | 4,79 | 8,33 | 9,53 |
| Trouvé | 68,39 | 5,01 | 8,13 | 8,96 |

Spectre IR (KBr) : Bande NH à 3320 $cm^{-1}$,
Bandes CH à 3080, 3060, 3040 et 2920 $cm^{-1}$,
Bande CO à 1670 $cm^{-1}$,
Bandes principales à 1485, 1435, 1325, 810, 705 et 685 $cm^{-1}$.

**EXEMPLE 5**

**5,6-dihydro 6-(2-méthyl phénacyl) 4-oxo 4H-pyrrolo [1,2-a] thiéno [3,2-f] [1,4]-diazépine**

Ce composé a été préparé selon le procédé décrit dans l'exemple 1 mais en utilisant l'orthoacétotoluène.
Rendement : 44%
Point de fusion : 180 °C (Ether éthylique) cristaux gris

Analyse élémentaire :

| | C% | H% | N% | S% |
|---|---|---|---|---|
| Théorie | 67,84 | 4,79 | 8,33 | 9,53 |
| Trouvé | 67,72 | 4,82 | 8,44 | 9,43 |

Spectre IR (KBr) : Bandes NH à 3270 et 3160 $cm^{-1}$,
Bandes CH à 3040, 2920 et 2890 $cm^{-1}$,
Bandes CO à 1680 et 1650 $cm^{-1}$,
Bandes principales à 1530, 1480, 1435, 1330, 1210, 1145, 985, 810, 765 et 720 $cm^{-1}$.

**EXEMPLE 6**

**5,6-dihydro 6-(2,5-diméthoxy phénacyl) 4-oxo 4H-pyrrolo [1,2-a] thiéno [3,2-f] [1,4]-diazépine**

On opère comme dans l'exemple précédent mais en utilisant la 2,5-diméthoxy acétophénone.
Rendement : 19%
Point de Fusion : 180 ° C (acétonitrile) coton blanc

Analyse élémentaire :

| | C% | H% | N% |
|---|---|---|---|
| Théorie | 62,81 | 4,74 | 7,32 |
| Trouvé | 62,75 | 4,76 | 7,34 |

Spectre IR (KBr) : Bandes NH à 3260 et 3190 $cm^{-1}$,
Bandes CH à 3050, 2930, 2900 et 2830 $cm^{-1}$,
Bande C = O à 1675 $cm^{-1}$,
Bandes principales à 1490, 1410, 1220, 1035, 810, et 700 $cm^{-1}$.

**EXEMPLE 7**

**6-(4-chloro phénacyl) 5,6-dihydro 4-oxo 4H-pyrrolo [1,2-a] thiéno [3,2-f] [1,4]-diazépine**

Ce composé a été préparé selon le procédé décrit dans l'exemple 1 mais en utilisant la 4-chloro acétophénone.
Rendement : 24%
Point de Fusion : 224 ° C (Ethanol) cristaux beiges

Analyse élémentaire :

| | C% | H% | N% | Cl% |
|---|---|---|---|---|
| Théorie | 60,58 | 3,67 | 7,85 | 9,93 |
| Trouvé | 61,14 | 3,66 | 7,53 | 10,49 |

Spectre IR (KBr) : Bande NH à 3320 $cm^{-1}$,
Bandes CH à 3080 et 2910 $cm^{-1}$,
Bandes C = O à 1740 $cm^{-1}$ (acétone) et 1635 $cm^{-1}$ (lactame),
Bandes principales à 1580, 1480, 1430, 1320, 1210, 1090, 990 et 705 $cm^{-1}$.

**EXEMPLE 8**

**5,6-dihydro 4-oxo 6-(2-oxo 2-thién-2-yl éthyl) 4H-pyrrolo [1,2-a] thiéno [3,2-f] [1,4]-diazépine**

On opère comme dans l'exemple précédent mais en utilisant le 2-acétyl thiophène.
Rendement : 20%
Point de Fusion : 186 ° C (Isopropanol) cristaux beiges

Analyse élémentaire :

| | C% | H% | S% |
|---|---|---|---|
| Théorie | 58,52 | 3,68 | 19,52 |
| Trouvé | 57,91 | 3,91 | 18,90 |

Spectre IR (KBr) : Bande NH à 3320 $cm^{-1}$,
Bandes CH à 3080, 2960 et 2920 $cm^{-1}$,
Bandes CO à 1655 et 1640 $cm^{-1}$,
Bandes principales à 1530, 1485, 1440, 1325, 1220, 1140, 860, 720 et 710 $cm^{-1}$.

**EXEMPLE 9**

**5,6-dihydro 6-(4-fluoro phénacyl) 4-oxo 4H-pyrrolo [1,2-a] thiéno [3,2-f] [1,4]-diazépine**

Ce composé a été préparé selon le procédé décrit dans l'exemple 1 mais en utilisant la 4-fluoro acétophénone.
Rendement : 21%
Point de Fusion : 170 ° C (Ether éthylique) cristaux beiges

Analyse élémentaire :

| | C% | H% | S% | F% |
|---|---|---|---|---|
| Théorie | 63,51 | 3,84 | 9,41 | 5,58 |
| Trouvé | 63,61 | 3,66 | 8,88 | 5,40 |

Spectre IR (KBr) : Bande NH à 3320 $cm^{-1}$,
Bandes C=O à 1670 $cm^{-1}$ (cétone) et 1640 $cm^{-1}$ (lactame),
Bandes principales à 1595, 1485, 1440, 1330, 1215, 1000, 840 et 710 $cm^{-1}$.

**EXEMPLE 10**

**5.6-dihydro 6-(fur-2-yl 2-oxo éthyl) 4-oxo 4H-pyrrolo [1,2-a] thiéno [3,2-f] [1,4]-diazépine**

On opère comme dans l'exemple précédent mais en utilisant le 2-acétyl furane.
Rendement : 19,6%
Point de Fusion : 174 ° C (Ether éthylique) cristaux jaunes

Analyse élémentaire :

| | C% | H% | S% |
|---|---|---|---|
| Théorie | 61,53 | 3,87 | 10,26 |
| Trouvé | 61,29 | 3,69 | 10,41 |

Spectre IR (KBr) : Bande NH à 3180 $cm^{-1}$,
Bandes CH à 3050 et 2920 $cm^{-1}$,
Bandes C=O à 1670 $cm^{-1}$ (cétone) et 1640 $cm^{-1}$ (lactame),
Bandes principales à 1485, 1460, 1435, 1330, 1145, 995 et 725 $cm^{-1}$.

**EXEMPLE 11**

**5,6-dihydro 6-(3,3-diméthyl 2-oxo but-1-yl) 4-oxo 4H-pyrrolo [1,2-a] thiéno [3,2-f] [1,4]-diazépine**

Ce composé a été préparé selon le procédé décrit dans l'exemple 1 à partir de pinacolone.

Après élimination de l'éthanol, le reste du mélange réactionnel est versé sur 100 ml d'eau. Le précipité huileux formé est extrait avec de l'éther éthylique. La phase éthérée est lavée à l'eau, séchée sur sulfate de magnésium et éliminée sous vide. La pinacolone est alors éliminée du résidu par un lavage avec un mélange d'éther de pétrole-éther éthylique (50:50 V/V). Le solide insoluble est essoré, lavé à l'eau, séché et recristallisé.

Rendement : 26%

Point de Fusion : 170 °C (Ether éthylique) cristaux beiges

Analyse élémentaire :

| | C% | H% | N% | S% |
|---|---|---|---|---|
| Théorie | 63,55 | 5,99 | 9,26 | 10,60 |
| Trouvé | 62,20 | 5,72 | 8,80 | 10,41 |

Spectre IR (KBr) : Bandes NH à 3260 et 3170 $cm^{-1}$,
Bandes CH à 3110, 3060, 2980 et 2900 $cm^{-1}$,
Bandes C=O à 1700 $cm^{-1}$ (cétone) et 1735 $cm^{-1}$ (lactame),
Bandes principales à 1485, 1430, 1330, 1195, 1090, 830, 735 et 720 $cm^{-1}$.

**EXEMPLE 12**

**5,6-dihydro 6-(2-hydroxy propyl) 4-oxo 4H-pyrrolo [1,2-a] thiéno [3,2-f] [1,4]-diazépine**

A une solution de 2,5 g de 6-acétonyl 5,6-dihydro 4-oxo 4H-pyrrolo [1,2-a] thiéno [3,2-f] [1,4]-diazépine dans 200 ml de méthanol, on additionne par petites fractions 1,46 g de borohydrure de sodium puis le mélange réactionnel est agité à température ambiante pendant 3 heures. Le méthanol est ensuite éliminé sous vide et le solide résiduel trituré dans 200 ml d'eau. Le précipité obtenu est essoré, lavé à l'eau, séché et recristallisé.

Rendement : 80%

Point de Fusion : 171 °C (Ether éthylique) cristaux blancs

Analyse élémentaire :

| | C% | H% | N% | S% |
|---|---|---|---|---|
| Théorie | 59,47 | 5,34 | 10,67 | 12,20 |
| Trouvé | 59,42 | 5,28 | 10,72 | 12,08 |

Spectre IR (KBr) : Bande OH à 3450 $cm^{-1}$,
Bandes NH à 3260 et 3190 $cm^{-1}$,
Bandes CH à 3040, 2960, 2920 et 2880 $cm^{-1}$,
Bande CO à 1630 $cm^{-1}$,
Bandes principales à 1535, 1485, 1440, 1330, 1085, 885, 700 et 680 $cm^{-1}$.

**EXEMPLE 13**

**5,6-dihydro 4-oxo 6-(2-hydroxy pent-1-yl) 4H-pyrrolo [1,2-a] thiéno [3,2-f] [1,4]-diazépine**

Ce composé a été préparé à partir du composé décrit dans l'exemple 1 et selon le procédé décrit dans l'exemple 12.
Rendement : 80%
Point de Fusion : 188 ° C (Isopropanol) cristaux jaunes

Analyse élémentaire :

| | C% | H% | N% | S% |
|---|---|---|---|---|
| Théorie | 62,04 | 6,24 | 9,64 | 11,04 |
| Trouvé | 62,10 | 6,18 | 9,55 | 10,89 |

Spectre IR (KBr) : Bande OH à 3440 $cm^{-1}$,
Bandes NH à 3250 et 3170 $cm^{-1}$,
Bandes CH à 3030, 2950, 2930, 2900 et 2860 $cm^{-1}$,
Bande CO à 1630 $cm^{-1}$,
Bandes principales à 1530, 1480, 1440, 1335, 1090, 1025, 805, 705 et 690 $cm^{-1}$.

**EXEMPLE 14**

**6-(2-cyclopropyl 2-hydroxy éthyl) 5,6-dihydro 4-oxo 4H-pyrrolo [1,2-a] thiéno [3,2-f] [1,4]-diazépine**

Ce composé a été préparé à partir du composé de l'exemple 2 selon le procédé décrit dans l'exemple 12.
Rendement : 80%
Point de Fusion : 222 ° C (Isopropanol) cristaux blancs

Analyse élémentaire :

| | C% | H% | N% | S% |
|---|---|---|---|---|
| Théorie | 62,47 | 5,59 | 9,71 | 11,11 |
| Trouvé | 61,82 | 5,72 | 9,26 | 10,51 |

Spectre IR (KBr) : Bande OH à 3450 $cm^{-1}$,
Bandes NH à 3260 et 3180 $cm^{-1}$,
Bandes CH à 3080, 3000, 2950 et 2880 $cm^{-1}$,
Bande CO à 1625 $cm^{-1}$,
Bandes principales à 1530, 1485, 1440, 1330, 1300, 1095, 830, 800, 710 et 690 $cm^{-1}$.

**EXEMPLE 15**

**5,6-dihydro 6-(2-hydroxy phénéthyl) 4-oxo 4H-pyrrolo [1,2-a] thiéno [3,2-f] [1,4]-diazépine**

Ce composé a été préparé à partir de la 5,6-dihydro 4-oxo 6-phénacyl 4H-pyrrolo [1,2-a] thiéno [3,2-f] [1,4]-diazépine, (Tetrahedron Letters,1979,N ° 7,pp.643-644), selon le procédé décrit dans l'exemple 12.
Rendement : 79%
Point de Fusion : 190 ° C (Ether éthylique) cristaux blancs

Analyse élémentaire :

| | C% | H% | N% | S% |
|---|---|---|---|---|
| Théorie | 66,65 | 4,97 | 8,64 | 9,88 |
| Trouvé | 66,56 | 4,89 | 8,52 | 9,68 |

Spectre IR (KBr) : Bande OH à 3450 $cm^{-1}$,
Bandes NH à 3250 et 3180 $cm^{-1}$,
Bandes CH à 3030, 2955 et 2900 $cm^{-1}$,
Bande CO à 1635 $cm^{-1}$,
Bandes principales à 1485, 1440, 1335, 1060 et 710 $cm^{-1}$.

**EXEMPLE 16**

**5,6-dihydro 6-[2-hydroxy 2-(4-méthylphényl) éthyl] 4-oxo 4H-pyrrolo [1,2-a] thiéno [3,2-f] [1,4]-diazépine**

Ce composé a été préparé à partir du composé décrit dans l'exemple 4 et selon le procédé décrit dans l'exemple 12.
Rendement : 71%
Point de Fusion : 182 ° C (Ether éthylique) cristaux blancs

Analyse élémentaire :

| | C% | H% | N% | S% |
|---|---|---|---|---|
| Théorie | 67,43 | 5,36 | 8,27 | 9,47 |
| Trouvé | 67,30 | 5,44 | 8,16 | 9,33 |

Spectre IR (KBr) : Bande OH à 3420 $cm^{-1}$,
Bandes NH à 3250 et 3170 $cm^{-1}$,
Bandes CH à 3100, 3020, 2950 et 2920 $cm^{-1}$,
Bande CO à 1635 $cm^{-1}$,
Bandes principales à 1490, 1440, 1335, 1095, 1060, 830, 735 et 705 $cm^{-1}$.

**EXEMPLE 17**

**5,6-dihydro 6-(2-hydroxy 3-méthyl but-1-yl) 4-oxo 4H-pyrrolo [1,2-a] thiéno [3,2-f] [1,4]-diazépine**

Ce composé a été préparé à partir de la 5,6-dihydro 6-(2-oxo 3-méthyl but-1-yl) 4-oxo 4H-pyrrolo [1,2-a] thiéno [3,2-f] [1,4]-diazépine selon le procédé décrit dans l'exemple 12.
Rendement : 83%
Point de Fusion : 194 ° C (Acétone) cristaux beiges

Analyse élémentaire :

| | C% | H% | N% | S% |
|---|---|---|---|---|
| Théorie | 62,04 | 6,24 | 9,64 | 11,04 |
| Trouvé | 62,00 | 6,17 | 9,64 | 10,97 |

Spectre IR (KBr) : Bande OH à 3460 $cm^{-1}$,
Bandes NH à 3250 et 3170 $cm^{-1}$,

Bandes CH à 3020, 2950, 2930 et 2870 $cm^{-1}$,
Bande CO à 1625 $cm^{-1}$,
Bandes principales à 1530, 1490, 1445, 1330, 1100, 1055 et 710 $cm^{-1}$.

**EXEMPLE 18**

**5,6-dihydro 6-[2-hydroxy 2-(2,5-diméthoxy phényl) éthyl] 4-oxo 4H-pyrrolo [1,2-a] thiéno [3,2-f] [1,4]-diazépine**

Ce composé a été préparé à partir du composé de l'exemple 6 selon le procédé décrit dans l'exemple 12.
Rendement : 65%
Point de Fusion : 193 °C (Ether éthylique) cristaux blancs

Analyse élémentaire :

| | C% | H% | N% | S% |
|---|---|---|---|---|
| Théorie | 62,48 | 5,24 | 7,28 | 8,33 |
| Trouvé | 61,53 | 5,25 | 7,37 | 8,55 |

Spectre IR (KBr) :
Bande OH à 3420 $cm^{-1}$,
Bandes NH à 3260 et 3180 $cm^{-1}$,
Bandes CH à 3080, 3000, 2960, 2900 et 2820 $cm^{-1}$,
Bande CO à 1625 $cm^{-1}$,
Bandes principales à 1530, 1490, 1440, 1330, 1275, 1215, 1100, 1025, 800, 790 et 720 $cm^{-1}$.

**EXEMPLE 19**

**4,6-dioxo 8-méthyl 4,8,9,10-tétrahydro [1,3]-oxazino [4,3-c] pyrrolo [1,2-a] thiéno [3,2-f] [1,4]-diazépine**

A une solution de 0,7 g du composé obtenu dans l'exemple 12 dans 150 ml de toluène est ajouté 3 ml d'une solution à 20% de phosgène dans le toluène puis le mélange réactionnel est chauffé au reflux pendant une heure. Après refroidissement, l'excès de phosgène est éliminé en faisant barbotter un courant d'azote sous le milieu réactionnel. Le toluène est ensuite éliminé sous vide et le résidu trituré dans 200 ml d'eau. Le précipité est essoré, lavé à l'eau, séché et recristallisé.

**EXEMPLE 20**

**4,6-dioxo 8-propyl 4,8,9,10-tétrahydro [1,3]-oxazino [4,3-c] pyrrolo [1,2-a] thiéno [3,2-f] [1,4]-diazépine**

Ce composé a été obtenu à partir du composé de l'exemple 13 et selon le procédé décrit dans l'exemple précédent.
Rendement : 89%
Point de fusion: 162 °C (Ether éthylique) cristaux blancs

Spectre IR (KBr) :
Bandes CH à 3020, 2960 et 2880 $cm^{-1}$,
Bandes C=O à 1745 et 1665 $cm^{-1}$.
Bandes principales à 1385, 1280, 1195, 1160, 840 et 700 $cm^{-1}$.

**EXEMPLE 21**

**4,6-dioxo 8-(4-méthylphényl) 4,8,9,10-tétrahydro [1,3]-oxazino [4,3-c] pyrrolo [1,2-a] thiéno [3,2-f] [1,4]-diazépine**

Ce composé a été obtenu à partir du composé de l'exemple 16 et selon le procédé décrit ci-dessus.
Rendement : 78%
Point de fusion: 240 ° C (Ether éthylique) cristaux blancs

Analyse élémentaire :

| | C% | H% | N% | S% |
|---|---|---|---|---|
| Théorie | 65,89 | 4,39 | 7,68 | 8,78 |
| Trouvé | 65,72 | 4,33 | 7,61 | 8,72 |

Spectre IR (KBr) : Bandes CH à 3030 et 2920 $cm^{-1}$,
Bandes C=O à 1700 et 1625 $cm^{-1}$,
Bandes principales à 1490, 1440, 1330 et 715 $cm^{-1}$.

**EXEMPLE 22**

**5,6-dihydro 6-(2-hydroxy imino prop-1-yl) 4-oxo 4H-pyrrolo [1,2-a] thiéno [3,2-f] [1,4]-diazépine**

A une solution de 4 g de 6-acétonyl 5,6-dihydro 4-oxo 4H-pyrrolo [1,2-a] thiéno [3,2-f] [1,4]-diazépine dans 500 ml d'éthanol est additionnée une solution de 4,2 g de chlorhydrate d'hydroxylamine et de 4,92 g d'acétate de sodium dans 20 ml d'eau.

Après chauffage à reflux pendant une heure du mélange réactionnel, l'éthanol est éliminé sous vide. Le solide résiduel est trituré avec 300 ml d'eau, essoré, lavé à l'eau, séché et recristallisé.
Rendement : 85%
Point de fusion: 178 ° C (Ethanol) poudre blanche

Analyse élémentaire :

| | C% | H% | N% | S% |
|---|---|---|---|---|
| Théorie | 58,11 | 5,22 | 14,52 | 11,08 |
| Trouvé | 57,99 | 5,16 | 14,47 | 11,10 |

Spectre IR (KBr) : Bande OH à 3280 $cm^{-1}$,
Bande NH à 3200 $cm^{-1}$,
Bandes CH à 3100 et 2860 $cm^{-1}$,
Bande CO à 1625 $cm^{-1}$,
Bandes principales à 1540, 1495, 1440, 1325, 1090, 930, 890 et 715 $cm^{-1}$.

**EXEMPLE 23**

**5,6-dihydro 6-(2-hydroxy imino 3-méthyl butyl) 4-oxo 4H-pyrrolo [1,2-a] thiéno [3,2-f] [1,4] diazépine**

A une solution de 3 g de 5,6-dihydro 6-(3-méthyl 2-oxo but-1-yl) 4-oxo 4H-pyrrolo [1,2-a] thiéno [3,2-f] [1,4]-diazépine dans 300 ml d'éthanol on additionne une solution de 2,80 g de chlorhydrate d'hydroxylamine et de 3,3 g d'acétate de sodium dans 15 ml d'eau.

Le mélange réactionnel est chauffé au reflux pendant une heure, puis l'éthanol est éliminé sous vide. Le solide résiduel est trituré dans 250 ml d'eau, essoré, lavé à l'eau, séché et recristallisé.
Rendement : 92%
Point de fusion: 188 ° C (Isopropanol) cristaux blancs

Analyse élémentaire :

| | C% | H% | N% | S% |
|---|---|---|---|---|
| Théorie | 59,38 | 5,64 | 13,85 | 10,56 |
| Trouvé | 59,46 | 5,69 | 13,78 | 10,46 |

Spectre IR (KBr) : Bande OH
à 3270 - 3130 $cm^{-1}$,
Bande NH
Bandes CH à 3100, 2960 et 2870 $cm^{-1}$,
Bande CO à 1635 $cm^{-1}$,
Bandes principales à 1535, 1490, 1440, 1325, 1145, 1100, 1030, 950, 900, 785, 730, et 715 $cm^{-1}$.

**EXEMPLE 24**

**5,6-dihydro 6-(2-cyclopropyl 2-hydroxyimino éth-1-yl) 4-oxo 4H-pyrrolo [1,2-a] thiéno [3,2-f] [1,4]-diazépine**

A une solution de 5 g du composé de l'exemple 2 dans 500 ml d'éthanol on additionne une solution de 4,9 g de chlorhydrate d'hydroxylamine et de 5,8 g d'acétate de sodium dans 20 ml d'eau.

Après chauffage à reflux pendant une heure du mélange, l'éthanol est éliminé sous vide. Le solide résiduel est trituré avec 300 ml d'eau et le précipité formé est essoré, lavé à l'eau, séché et recristallisé.
Rendement : 87%
Point de fusion: 210 °C (Ether éthylique) cristaux blancs

Analyse élémentaire :

| | C% | H% | N% | S% |
|---|---|---|---|---|
| Théorie | 59,78 | 5,01 | 13,94 | 10,63 |
| Trouvé | 59,91 | 5,03 | 14,08 | 10,46 |

Spectre IR (KBr) : Bande OH
à 3280 - 3150 $cm^{-1}$,
Bande NH
Bandes CH à 3100, 3000 et 2970 $cm^{-1}$,
Bande CO à 1630 $cm^{-1}$,
Bandes principales à 1580, 1485, 1440, 1325, 1185, 1100, 935, 720 et 710 $cm^{-1}$.

**EXEMPLE 25**

**5,6-dihydro 6-(2-hydroxyimino pent-1-yl) 4-oxo 4H-pyrrolo [1,2-a] thiéno [3,2-f] [1,4]-diazépine**

Ce composé a été préparé selon le procédé décrit ci-dessus à partir du composé de l'exemple 1.
Rendement : 82%
Point de fusion: 110 °C (Ether éthylique)

Analyse élémentaire :

| | C% | H% | N% | S% |
|---|---|---|---|---|
| Théorie | 59,39 | 5,65 | 13,85 | 10,57 |
| Trouvé | 59,28 | 5,77 | 13,70 | 10,59 |

Spectre IR (KBr) : Bande OH
à 3280 - 3100 $cm^{-1}$,
Bande NH
Bandes CH à 3100, 2960, 2930 et 2880 $cm^{-1}$,
Bande CO à 1640 $cm^{-1}$,
Bandes principales à 1540, 1490, 1440, 1325, 1090, 955, 780 et 710 $cm^{-1}$.

**EXEMPLE 26**

**5,6-dihydro 6-(2-hydroxyimino phénéthyl) 4-oxo 4H-pyrrolo [1,2-a] thiéno [3,2-f] [1,4]-diazépine**

Ce composé a été préparé à partir de la 5,6-dihydro 6-(2-oxo phénéthyl) 4-oxo 4H-pyrrolo [1,2-a] thiéno [3,2-f] [1,4]-diazépine selon le procédé décrit ci-dessus.
Rendement : 82%
Point de fusion: 228 ° C (Ether éthylique) cristaux blancs

Analyse élémentaire :

| | C% | H% | N% |
|---|---|---|---|
| Théorie | 64,08 | 4,48 | 12,45 |
| Trouvé | 63,93 | 4,60 | 12,32 |

Spectre IR (KBr) : Bande OH
à 3300 $cm^{-1}$,
Bande NH
Bandes CH à 3100, 3000, 2830 $cm^{-1}$,
Bande CO à 1630 $cm^{-1}$,
Bandes principales à 1495, 1450, 1325, 1150, 960, 770, 720, et 695 $cm^{-1}$.

**EXEMPLE 27**

**5,6-dihydro 6-(2-hydroxyimino 2-(4-méthyl phényl) éth-2-yl) 4-oxo 4H-pyrrolo [1,2-a] thiéno [3,2-f] [1,4]-diazépine**

Ce composé a été préparé à partir du composé de l'exemple 4 selon le procédé décrit dans l'exemple 22.
Rendement : 74%
Point de fusion: 190 ° C (Ether éthylique) cristaux blancs

Analyse élémentaire :

| | C% | H% | N% | S% |
|---|---|---|---|---|
| Théorie | 64,95 | 4,84 | 11,96 | 9,12 |
| Trouvé | 65,61 | 5,12 | 11,68 | 8,81 |

Spectre IR (KBr) : Bande OH
à 3300-3140 $cm^{-1}$,
Bande NH
Bandes CH à 3100, 2970 et 2850 $cm^{-1}$,
Bande CO à 1625 $cm^{-1}$,
Bandes principales à 1535, 1490, 1445, 1325, 1185, 960, 820 et 720 $cm^{-1}$.

**EXEMPLE 28**

**6-(2-cyclopropyl 2-méthoxyimino éth-1-yl) 5,6-dihydro 4-oxo 4H-pyrrolo [1,2-a] thiéno [3,2-f] [1,4]-diazépine**

A une solution de 1 g du composé de l'exemple 2 dans 150 ml d'éthanol on additionne une solution de 1 g de chlorhydrate de méthoxylamine et de 1,15 g d'acétate de sodium dns 10 ml d'eau.

Après chauffage à reflux pendant une heure du mélange réactionnel, l'éthanol est éliminé sous vide. Après refroidissement, le solide résiduel est trituré dans 150 ml d'eau, essoré, séché et recristallisé.
Rendement : 82%
Point de fusion : 152 °C (Ether éthylique) cristaux beiges

Analyse élémentaire :

| | C% | H% | N% | S% |
|---|---|---|---|---|
| Théorie | 60,93 | 5,43 | 13,32 | 10,16 |
| Trouvé | 61,33 | 5,40 | 12,50 | 10,48 |

Spectre IR (KBr) : Bandes NH à 3270 et 3190 $cm^{-1}$,
Bandes CH à 3100, 2960 et 2930 $cm^{-1}$,
Bande CO à 1645 $cm^{-1}$,
Bandes principales à 1535, 1490, 1435, 1330, 1100, 1005 et 715 $cm^{-1}$.

**EXEMPLE 29**

**5,6-dihydro 6-(2-méthoxyimino prop-1-yl) 4-oxo 4H-pyrrolo [1,2-a] thiéno [3,2-f] [1,4]-diazépine**

Ce composé a été obtenu selon le procédé décrit dans l'exemple 22 mais en remplaçant le chlorhydrate d'hydroxylamine par le chlorhydrate de méthoxylamine.
Rendement : 93%
Point de fusion: 138 °C (Ether éthylique) coton blanc

Analyse élémentaire :

| | C% | H% | N% | S% |
|---|---|---|---|---|
| Théorie | 58,11 | 5,22 | 14,52 | 11,08 |
| Trouvé | 57,99 | 5,16 | 14,47 | 11,10 |

Spectre IR (KBr) : Bande NH à 3300 $cm^{-1}$,
Bandes CH à 3100, 2950, 2930, 2890 et 2815 $cm^{-1}$,
Bande CO à 1630 $cm^{-1}$,
Bandes principales à 1530, 1480, 1425, 1320, 1180, 1145, 1040, 710 et 690 $cm^{-1}$.

**EXEMPLE 30**

**6-hydroxy 4-méthoxy 6H-pyrrolo [1,2-a] thiéno [3,2-f] [1,4]-diazépine**

Une suspension de 10 g (0,049 mole) de 3-cyano 2-(2-formyl pyrrol-1-yl) thiophène dans 100 ml d'hydroxyde de sodium 6N et 250 ml de méthanol est chauffée lentement à 40 °C pendant une heure jusqu'à dissolution complète. Le méthanol est ensuite éliminé sous vide et le résidu liquide est versé sur 500 ml d'eau. Le précipité formé est essoré, lavé à l'eau, séché et recristallisé.
Rendement : 77%
Point de fusion: 200 °C (Acétone) cristaux blancs

Analyse élémentaire :

| | C% | H% | N% | S% |
|---|---|---|---|---|
| Théorie | 56,39 | 4,30 | 11,95 | 13,68 |
| Trouvé | 56,39 | 4,39 | 12,00 | 13,83 |

Spectre IR (KBr) : Bande OH à 3380 $cm^{-1}$,
Bandes CH à 3100, 2980 et 2940 $cm^{-1}$,
Bande C=N à 1640 $cm^{-1}$,
Bandes principales à 1540, 1480, 1330, 1260, 1100, 700 et 690 $cm^{-1}$.

**EXEMPLE 31**

**4-éthoxy 6-hydroxy 6H-pyrrolo [1,2-a] thiéno [3,2-f] [1,4]-diazépine**

Ce composé a été obtenu comme il est indiqué dans l'exemple 30 mais en remplaçant le méthanol par l'éthanol.
Rendement : 67%
Point de fusion: 140 °C (Ether éthylique) cristaux blancs

Analyse élémentaire :

| | C% | H% | N% | S% |
|---|---|---|---|---|
| Théorie | 58,04 | 4,87 | 11,28 | 12,91 |
| Trouvé | 58,46 | 5,16 | 10,92 | 12,53 |

Spectre IR (KBr) : Bande OH à 3400 cm-1,
Bandes CH à 3080, 2960, 2920 et 2850 $cm^{-1}$,
Bande C=N à 1620 $cm^{-1}$,
Bandes principales à 1545, 1470, 1325, 1270, 1100, 785 et 700 $cm^{-1}$.

**EXEMPLE 32**

**6-hydroxy 4-propoxy 6H-pyrrolo [1,2-a] thiéno [3,2-f] [1,4]-diazépine**

Ce composé a été obtenu comme il est indiqué dans l'exemple 30 mais en remplaçant le méthanol par le propan-1-ol.
Rendement : 62%
Point de fusion: 120 °C (Ether éthylique) cristaux blancs

Analyse élémentaire :

| | C% | H% | N% | S% |
|---|---|---|---|---|
| Théorie | 59,52 | 5,38 | 10,68 | 12,22 |
| Trouvé | 59,50 | 5,30 | 10,72 | 12,21 |

Spectre IR (KBr) : Bandes OH entre 3400 et 3300 cm-1,
Bandes CH à 3100 et 2960 $cm^{-1}$,
Bande C=N à 1630 $cm^{-1}$,
Bandes principales à 1540, 1480, 1335, 1260, 1100, 1005 et 690 $cm^{-1}$.

**EXEMPLE 33**

**4-allyloxy 6-hydroxy 6H-pyrrolo [1,2-a] thiéno [3,2-f] [1,4]-diazépine**

Ce composé a été préparé comme il est indiqué dans l'exemple 30 mais en utilisant comme alcool l'alcool allylique.
Rendement : 64%
Point de fusion: 122 ° C (Ether éthylique) coton blanc

Analyse élémentaire :

| | C% | H% | S% | |
|---|---|---|---|---|
| Théorie | 59,98 | 4,65 | 12,32 | |
| Trouvé | 60,15 | 4,68 | 12,20 | |

Spectre IR (KBr) : Bande OH à 3360 $cm^{-1}$,
Bandes CH à 3100, 3000, 2920 et 2860 $cm^{-1}$,
Bande C=N à 1630 $cm^{-1}$,
Bandes principales à 1535, 1480, 1320, 1245, 1085, 980 et 675 $cm^{-1}$.

**EXEMPLE 34**

**4-méthoxy 6-oxo 6H-pyrrolo [1,2-a] thiéno [3,2-f] [1,4]-diazépine**

Une solution de 10 g du composé de l'exemple 30 et de 20 g de permanganate de potassium dans 500 ml d'acétone est agitée à température ambiante pendant 4 heures. Les oxydes de manganèse précipités sont filtrés, l'acétone est éliminée sous vide et le résidu recristallisé.
Rendement : 50%
Point de fusion: 154 ° C (Ether éthylique) aiguilles blanches

Analyse élémentaire :

| | C% | H% | N% | S% |
|---|---|---|---|---|
| Théorie | 56,89 | 3,47 | 12,06 | 13,80 |
| Trouvé | 57,01 | 3,31 | 12,02 | 13,61 |

Spectre IR (KBr) : Bandes CH à 3110, 3075 et 2950 $cm^{-1}$,
Bande C=O à 1630 $cm^{-1}$,
Bande C=N à 1600 $cm^{-1}$,
Bandes principales à 1550, 1545, 1445, 1355, 1330, 1280, 1190, 1020, 770 et 725 $cm^{-1}$.

**EXEMPLE 35**

**4-éthoxy 6-oxo 6H-pyrrolo [1,2-a] thiéno [3,2-f] [1,4]-diazépine**

Ce composé a été préparé à partir du composé de l'exemple 31 selon le procédé indiqué ci-dessus.
Rendement : 55%
Point de fusion: 188 ° C (Ether éthylique) aiguilles blanches

Analyse élémentaire :

| | C% | H% | N% | S% |
|---|---|---|---|---|
| Théorie | 58,52 | 4,09 | 11,37 | 13,02 |
| Trouvé | 58,48 | 4,04 | 11,29 | 12,93 |

Spectre IR (KBr) : Bandes CH à 3120, 3070 et 2990 $cm^{-1}$,
Bande C=O à 1635 $cm^{-1}$,
Bande C=N à 1590 $cm^{-1}$,
Bandes principales à 1550, 1540, 1350, 1325, 1265, 1180, 1030, 765 et 720 $cm^{-1}$.

**EXEMPLE 36**

**6-oxo 4-propoxy 6H-pyrrolo [1,2-a] thiéno [3,2-f] [1,4]-diazépine**

Ce composé a été préparé à partir du composé décrit dans l'exemple 32 selon le procédé décrit dans l'exemple 34.
Rendement : 55%
Point de fusion: 130 °C (Ether éthylique) aiguilles blanches

Analyse élémentaire :

| | C% | H% | N% | S% |
|---|---|---|---|---|
| Théorie | 59,98 | 4,65 | 10,76 | 12,32 |
| Trouvé | 59,84 | 4,55 | 10,75 | 12,45 |

Spectre IR (KBr) : Bandes CH à 3310, 3075, 2985, 2940 et 2880 $cm^{-1}$,
Bande C=O à 1640 $cm^{-1}$,
Bande C=N à 1600 $cm^{-1}$,
Bandes principales à 1555, 1540, 1440, 1320, 1260, 1170, 980, 730 et 705 $cm^{-1}$.

**EXEMPLE 37**

**5,6-dihydro 4-méthoxy 6-oxo 4H-pyrrolo [1,2-a] thiéno [3,2-f] [1,4]-diazépine**

A une solution de 2 g du composé de l'exemple 34 dans 200 ml de méthanol, on additionne par petites fractions 1,30 g de borohydrure de sodium puis le mélange réactionnel est agité à température ambiante pendant 3 heures. Le méthanol est ensuite éliminé sous vide et le résidu solide est trituré dans 200 ml d'eau, essoré, lavé à l'eau, séché et recristallisé.
Rendement : 74%
Point de fusion: 205 °C (Ether éthylique) cristaux blancs

Analyse élémentaire :

| | C% | H% | N% | S% |
|---|---|---|---|---|
| Théorie | 56,40 | 4,30 | 11,96 | 13,68 |
| Trouvé | 56,39 | 4,21 | 11,99 | 13,52 |

Spectre IR (KBr) : Bande NH à 3260 $cm^{-1}$,
Bandes CH à 3100, 3000, 2950 et 2840 $cm^{-1}$,
Bande CO à 1650 $cm^{-1}$,

Bandes principales à 1590, 1470, 1400, 1320, 1075, 770 et 715 $cm^{-1}$.

**EXEMPLE 38**

**5,6-dihydro 4-éthoxy 4H-pyrrolo [1,2-a] thiéno [3,2-f] [1,4]-diazépine**

Ce composé a été préparé à partir du composé de l'exemple 35 selon le procédé décrit ci-dessus.
Rendement : 70%
Point de fusion: 170 °C (Ether éthylique) cristaux blancs

Analyse élémentaire :

| | C% | H% | N% | S% |
|---|---|---|---|---|
| Théorie | 58,05 | 4,87 | 11,28 | 12,91 |
| Trouvé | 57,88 | 4,74 | 11,35 | 12,77 |

Spectre IR (KBr) : Bande NH à 3260 $cm^{-1}$,
Bandes CH à 3120, 2695 et 2880 $cm^{-1}$,
Bande CO à 1640 $cm^{-1}$,
Bandes principales à 1590, 1540, 1460, 1390, 1060, 755 et 700 $cm^{-1}$.

**EXEMPLE 39**

**5,6-dihydro 6-oxo 4-propoxy 4H-pyrrolo [1,2-a] thiéno [3,2-f] [1,4]-diazépine**

Ce composé a été préparé à partir du composé de l'exemple 36 selon le procédé décrit dans l'exemple 37.
Rendement : 54%
Point de fusion: 160 °C (Ether éthylique) cristaux blancs

Analyse élémentaire :

| | C% | H% | N% | S% |
|---|---|---|---|---|
| Théorie | 59,52 | 5,38 | 10,68 | 12,22 |
| Trouvé | 59,27 | 5,25 | 10,67 | 12,06 |

Spectre IR (KBr) : Bande NH à 3295 $cm^{-1}$,
Bandes CH à 3105, 2930 et 2880 $cm^{-1}$,
Bande CO à 1650 $cm^{-1}$,
Bandes principales à 1595, 1545, 1460, 1390, 1305, 1075, 1030 et 760 $cm^{-1}$.

**EXEMPLE 40**

**5,6-dihydro 6-hydroxy 4-oxo 4H-pyrrolo [1,2-a] thiéno [3,2-f] [1,4]-diazépine**

A une suspension de 2 g de 2-(2-formyl pyrrol-1-yl) 3-thiophène carboxamide dans 60 ml d'eau, on additionne 0,5 ml de triéthylamine puis le mélange réactionnel est agité 12 heures à température ambiante. Le produit passe en solution progressivement puis reprécipite. Le précipité obtenu est essoré, lavé à l'eau, séché et recristallisé.
Rendement : 68%
Point de fusion: 166 °C (Eau) aiguilles beiges

Analyse élémentaire :

| | C% | H% | N% | S% |
|---|---|---|---|---|
| Théorie | 54,53 | 3,66 | 12,71 | 14,56 |
| Trouvé | 54,36 | 3,69 | 12,58 | 14,39 |

Spectre IR (KBr) : Bande OH à 3340 $cm^{-1}$,
Bande NH à 3220 $cm^{-1}$,
Bandes CH à 3120 et 3110 $cm^{-1}$,
Bande C=O à 1610 $cm^{-1}$,
Bandes principales à 1500, 1450, 1320, 1205, 1005, 830 et 720 $cm^{-1}$.

Spectre RMN du proton : (solvant DMSO-$d_6$) :

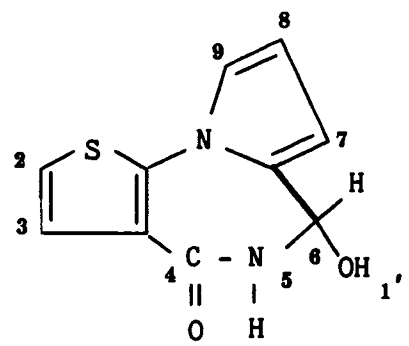

| $H_2$ ppm | $H_3$ ppm | $H_7$ ppm | $H_8$ ppm | $H_9$ ppm | NH ppm | $H_6$ ppm | Autres protons |
|---|---|---|---|---|---|---|---|
| 7,20 | 7,20 | 6,25 | 6,25 | 7,18 | 8,94 | 5,62 | OH : 6,33 |

**EXEMPLE 41**

**5,6-dihydro 6-méthylamino 4-oxo 4H-pyrrolo [1,2-a] thiéno [3,2-f] [1,4]-diazépine**

## Procédé A

A une suspension de 1,5 g du composé obtenu à l'exemple 40 dans 50 ml d'eau, on additionne 10 ml d'une solution aqueuse de méthylamine à 35%. Le précipité formé en fin de réaction est essoré, lavé à l'eau, séché et recristallisé.
Rendement : 88%

## Procédé B

A une solution de 1 g de 3-carboxamide 2-(2-formyl pyrrol-1-yl) thiophène dans 50 ml d'acétonitrile, on additionne 8 ml d'une solution aqueuse de méthylamine à 35% dans 50 ml d'eau. Le mélange réactionnel

est chauffé à reflux pendant environ 3 heures. L'acétonitrile est ensuite éliminé sous vide, et le résidu recristallisé dans l'éther éthylique.
Rendement : 84%
Point de fusion :168 °C (Ether éthylique) cristaux blancs

Analyse élémentaire :

| | C% | H% | N% | S% |
|---|---|---|---|---|
| Théorie | 56,63 | 4,75 | 18,01 | 13,74 |
| Trouvé | 56,55 | 4,73 | 17,88 | 13,69 |

Spectre IR (KBr) : Bandes NH à 3320, 3250 et 3170 $cm^{-1}$,
Bandes CH à 3100, 3040, 2980, 2930 et 2860 $cm^{-1}$,
Bande C=O à 1635 $cm^{-1}$,
Bandes principales à 1535, 1490, 1435, 1330, 1315, 1120, 1090, 820, 750 et 705 $cm^{-1}$.

**EXEMPLE 42**

**5,6-dihydro 6-éthylamino 4-oxo 4H-pyrrolo [1,2-a] thiéno [3,2-f] [1,4]-diazépine**

Procéder comme il est indiqué dans l'exemple 41 (Procédé A ou B) en utilisant au lieu d'une solution aqueuse de méthylamine une solution aqueuse d'éthylamine (70%).
Rendement : (Procédé A) = 89%
(Procédé B) = 80%
Point de fusion : 156° (Ether éthylique) cristaux blancs

Analyse élémentaire :

| | C% | H% | N% | S% |
|---|---|---|---|---|
| Théorie | 58,28 | 5,30 | 16,99 | 12,96 |
| Trouvé | 58,26 | 5,25 | 16,99 | 12,87 |

Spectre IR (KBr) : Bandes NH à 3300, 3240 et 3140 $cm^{-1}$,
Bandes CH à 3020, 2940 et 2850 $cm^{-1}$,
Bande C=O à 1630 $cm^{-1}$,
Bandes principales à 1525, 1475, 1450, 1420, 1315, 1115, 1070, 770, 705, 690 et 660 $cm^{-1}$.

**EXEMPLE 43**

**5,6-dihydro 4-oxo 6-propylamino 4H-pyrrolo[1,2-a] thiéno [3,2-f] [1,4]-diazépine**

Pour obtenir ce composé, on procède comme dans l'exemple 41 (Procédé A) en utilisant la propylamine au lieu de la méthylamine.
Rendement : 85%
Point de fusion : 130° (Ether éthylique) cristaux blancs

Analyse élémentaire :

| | C% | H% | N% | S% |
|---|---|---|---|---|
| Théorie | 59,75 | 5,78 | 16,08 | 12,87 |
| Trouvé | 59,63 | 5,82 | 15,95 | 12,13 |

Spectre IR (KBr) : Bandes NH à 3340, 3260 et 3170 $cm^{-1}$,
Bandes CH à 3100, 3040, 2960, 2930 et 2860 $cm^{-1}$,
Bande C=O à 1635 $cm^{-1}$,
Bandes principales à 1540, 1495, 1435, 1330, 1095, et 740 $cm^{-1}$.

**EXEMPLE 44**

**5,6-dihydro 6-isopropylamino 4-oxo 4H-pyrrolo [1,2-a] thiéno [3,2-f] [1,4]-diazépine**

On opère comme à l'exemple précédent en utilisant l'isopropylamine au lieu de la propylamine.
Rendement : 76%
Point de fusion : 152°C (Ether éthylique) cristaux jaunes

Analyse élémentaire :

| | C% | H% | N% | S% |
|---|---|---|---|---|
| Théorie | 59,75 | 5,78 | 16,08 | 12,27 |
| Trouvé | 59,67 | 5,88 | 16,18 | 12,12 |

Spectre IR (KBr) : Bandes NH à 3330, 3250 et 3170 $cm^{-1}$,
Bandes CH à 3040, 2970 et 2870 $cm^{-1}$,
Bande CO à 1625 $cm^{-1}$,
Bandes principales à 1485, 1430, 1320, 1180, 1090, 815, 790, 750, 690 et 670 $cm^{-1}$.

**EXEMPLE 45**

**5,6-dihydro 6-diméthylamino 4-oxo 4H-pyrrolo [1,2-a] thiéno [3,2-f] [1,4]-diazépine**

On opère comme à l'exemple 43 en utilisant une solution aqueuse de diméthylamine à 40%.
Rendement : 63%
Point de fusion : 180°C (Ether éthylique) cristaux blancs.

Analyse élémentaire :

| | C% | H% | N% | S% |
|---|---|---|---|---|
| Théorie | 58,28 | 5,30 | 16,99 | 12,96 |
| Trouvé | 58,12 | 5,22 | 16,91 | 13,09 |

Spectre IR (KBr) : Bandes NH à 3280 et 3180 $cm^{-1}$,
Bandes CH à 3060, 2950, 2940, 2820 et 2780 $cm^{-1}$,
Bande CO à 1650 $cm^{-1}$,
Bandes principales à 1540, 1490, 1440, 1325, 1300, 1215, 995, 810, 800, 715 et 695 $cm^{-1}$.

**EXEMPLE 46**

**5,6-dihydro 6-diéthylamino 4-oxo 4H-pyrrolo [1,2-a] thiéno [3,2-f] [1,4]-diazépine**

On opère comme dans l'exemple 45 à partir de 1 g du composé de l'exemple 40 et 3 ml de diéthylamine dans 50 ml d'eau.
Rendement : 65%
Point de fusion : 137°C (Ether éthylique) cristaux blancs

Analyse élémentaire :

| | C% | H% | N% | S% |
|---|---|---|---|---|
| Théorie | 61,06 | 6,22 | 15,26 | 11,64 |
| Trouvé | 60,99 | 6,21 | 15,31 | 11,86 |

Spectre IR (KBr) : Bandes NH à 3275 et 3185 $cm^{-1}$,
Bandes CH à 3050, 2960, 2930 et 2820 $cm^{-1}$,
Bande CO à 1645 $cm^{-1}$,
Bandes principales à 1545, 1500, 1445, 1325, 1215, 1090, 1025 et 705 $cm^{-1}$.

**EXEMPLE 47**

**5,6-dihydro 4-oxo 6-pyrrolidin-1-yl 4H-pyrrolo [1,2-a] thiéno [3,2-f] [1,4]-diazépine**

On opère comme dans l'exemple 45 en utilisant 8 g du composé de l'exemple 40 et 4 ml de pyrrolidine dans 100 ml d'eau.
Rendement : 89%
Point de fusion : 176 ° C (Ether éthylique) cristaux blancs

Analyse élémentaire :

| | C% | H% | N% | S% |
|---|---|---|---|---|
| Théorie | 61,52 | 5,53 | 15,37 | 11,73 |
| Trouvé | 61,65 | 5,58 | 15,46 | 11,80 |

Spectre IR (KBr) : Bandes NH à 3280 et 3180 $cm^{-1}$,
Bandes CH à 3060, 2950, 2930 et 2790 $cm^{-1}$,
Bande CO à 1645 $cm^{-1}$,
Bandes principales à 1540, 1500, 1445, 1325, 1310, 1215, 1125, 895, 825, 805, 720 et 705 $cm^{-1}$.

**EXEMPLE 48**

**5,6-dihydro 4-oxo 6-pipéridino 4H-pyrrolo [1,2-a] thiéno [3,2-f] [1,4]-diazépine**

On opère comme à l'exemple 45 en utilisant 1,5 g du composé de l'exemple 40 et 3 ml de pipéridine dans 60 ml d'eau.
Rendement : 86%
Point de fusion : 150 ° C (Ether éthylique) cristaux blancs

Analyse élémentaire :

| | C% | H% | N% | S% |
|---|---|---|---|---|
| Théorie | 62,69 | 5,96 | 14,62 | 11,16 |
| Trouvé | 61,43 | 5,87 | 14,50 | 10,90 |

Spectre IR (KBr) : Bandes NH à 3270 et 3190 $cm^{-1}$,
Bandes CH à 3060, 2940, 2860, 2800 et 2760 $cm^{-1}$,
Bande CO à 1650 $cm^{-1}$,
Bandes principales à 1540, 1495, 1445, 1325, 1310, 1230, 1100, 980, 880 et 710

$cm^{-1}$.

**EXEMPLE 49**

**5,6-dihydro 4-oxo 6-morpholin-4-yl 4H-pyrrolo [1,2-a] thiéno [3,2-f] [1,4]-diazépine**

On opère comme à l'exemple 48 en utilisant 3 ml de morpholine.
Rendement : 65%
Point de fusion : 180 °C (Ether éthylique) cristaux blancs

Analyse élémentaire :

| | C% | H% | N% | S% |
|---|---|---|---|---|
| Théorie | 58,11 | 5,22 | 14,52 | 11,08 |
| Trouvé | 58,13 | 5,23 | 14,64 | 10,94 |

Spectre IR (KBr) : Bandes NH à 3280 et 3200 $cm^{-1}$,
Bandes CH à 3110, 3060, 2960, 2910 et 2810 $cm^{-1}$,
Bande CO à 1645 $cm^{-1}$,
Bandes principales à 1545, 1495, 1440, 1330, 1110, 1000, 870, 790 et 700 $cm^{-1}$.

**EXEMPLE 50**

**5,6-dihydro 4-oxo 6-(4-éthoxycarbonyl piperazino) 4H-pyrrolo [1,2-a] thiéno [3,2-f] [1,4]-diazépine**

On procède comme pour l'exemple 45 à partir de 2 g de composé de l'exemple 40 et 5 ml de N-éthoxycarbonyl pipérazine dans 100 ml d'eau.
Rendement : 80%
Point de fusion : 200 °C (Ether éthylique) cristaux blancs

Analyse élémentaire :

| | C% | H% | N% | S% |
|---|---|---|---|---|
| Théorie | 56,65 | 5,59 | 15,54 | 8,89 |
| Trouvé | 56,23 | 5,41 | 15,34 | 8,45 |

Spectre IR (KBr) : Bandes NH à 3280 et 3180 $cm^{-1}$
Bandes CH à 3050, 2940, 2810 et 2775 $cm^{-1}$
Bandes CO à 1690 et 1650 $cm^{-1}$,
Bandes principales à 1435, 1250, 1230, 1120, 996 et 715 $cm^{-1}$.

**EXEMPLE 51**

**5,6-dihydro 4-oxo 6-anilino 4H-pyrrolo [1,2-a] thiéno [3,2-f] [1,4]-diazépine**

Une solution de 1 g de composé de l'exemple 1 dans 20 ml de dioxane est ajoutée en une seule fois à une émulsion de 5 ml d'aniline dans 10 $cm^3$ de soude 1N puis le milieu réactionnel est agité à température ambiante pendant 6 heures.

300 ml d'eau sont alors versés sur l'émulsion obtenue. Le précipité formé est essoré, lavé à l'eau, séché et recristallisé.
Rendement : 75%
Point de fusion : 160 °C (Ether éthylique) coton blanc

Analyse élémentaire :

| | C% | H% | N% | S% |
|---|---|---|---|---|
| Théorie | 65,07 | 4,44 | 14,23 | 10,85 |
| Trouvé | 64,67 | 4,56 | 14,01 | 11,10 |

Spectre IR (KBr) : Bandes NH à 3340, 3280 et 3190 $cm^{-1}$
Bandes CH à 3110 et 3060 $cm^{-1}$
Bandes CO à 1645 $cm^{-1}$,
Bandes principales à 1605, 1500, 1330, 1310, 1260, 755 et 700 $cm^{-1}$.

**EXEMPLE 52**

**5,6-dihydro 4-oxo 6-(N benzylamino) 4H-pyrrolo [1,2-a] thiéno [3,2-f] [1,4]-diazépine**

On procède comme pour l'exemple 45 à partir de 2 g de composé de l'exemple 40 et 7,5 ml de benzylamine dans 100 ml d'eau.
Rendement : 70%
Point de fusion : 148 ° C (Ether éthylique) coton blanc

Analyse élémentaire :

| | C% | H% | N% | S% |
|---|---|---|---|---|
| Théorie | 66,00 | 4,89 | 13,58 | 10,36 |
| Trouvé | 65,84 | 4,91 | 13,44 | 10,55 |

Spectre IR (KBr) : Bandes NH à 3340 et 3220 $cm^{-1}$
Bandes CH à 3090 et 2900 $cm^{-1}$
Bandes CO à 1635 $cm^{-1}$,
Bandes principales à 1500, 1465, 1330, 1230, 740, 715 et 700 $cm^{-1}$.

**EXEMPLE 53**

**5,6-dihydro 4-oxo 4H-pyrrolo [1,2-a] thiéno [3,2-f] [1,4]-diazépine**

A une solution de 1,5 g du composé obtenu dans l'exemple 49 dans 150 ml de méthanol, 0,62 g de borohydrure de sodium sont ajoutés par petites portions et le mélange réactionnel est agité à température ambiante pendant 15 minutes puis chauffé à reflux pendant 30 mn. Le méthanol est ensuite éliminé sous vide et le résidu solide trituré dans 200 ml d'eau. Le précipité formé est essoré, lavé à l'eau, séché et recristallisé.
Rendement : 86%

Analyse élémentaire :

| | C% | H% | N% | S% |
|---|---|---|---|---|
| Théorie | 58,81 | 3,95 | 13,72 | 15,70 |
| Trouvé | 59,02 | 3,87 | 13,78 | 15,65 |

Spectre IR (KBr) : Bandes NH à 3280 et 3170 $cm^{-1}$,
Bandes CH à 3090, 3040 et 2900 $cm^{-1}$,
Bande CO à 1660 $cm^{-1}$,
Bandes principales à 1540, 1495, 1460, 1225, 1095, 910, 750, 680 et 620 $cm^{-1}$.

Spectre de résonance magnétique nucléaire du proton : (solvant $CDCl_3$) :

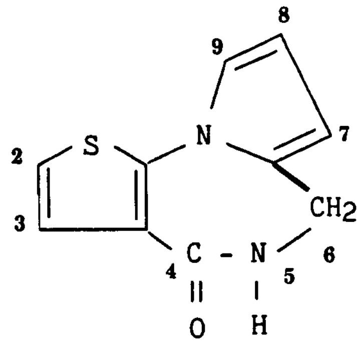

| H2 ppm | H3 ppm | H7 ppm | H8 ppm | H9 ppm | NH ppm | CH2 6 |
|---|---|---|---|---|---|---|
| 7,39 | 6,93 | 6,13 | 6,29 | 6,96 | 6,60 | 4,29 |

**EXEMPLE 54**

**(5,6-dihydro 4-oxo 4H-pyrrolo [1,2-a] thiéno [3,2-f] [1,4]-diazépin-6-yl) cyanacétate d'éthyle**

A une solution de 1 g du composé de l'exemple 40 dans 100 ml d'acétonitrile, on ajoute 2 ml de cyanoacétate d'éthyle et 0,5 ml de triéthylamine. Le mélange réactionnel est agité à température ambiante pendant 12 heures. L'acétonitrile est ensuite éliminé sous vide. Le résidu huileux est trituré dans 200 ml d'eau. Le précipité formé est essoré, lavé à l'eau, séché et recristallisé.
Rendement : 49%
Point de fusion : 208 ° C (Ether éthylique) cristaux jaunes

**Analyse élémentaire :**

| | C% | H% | N% | S% |
|---|---|---|---|---|
| Théorie | 57,13 | 4,16 | 13,33 | 10,17 |
| Trouvé | 56,99 | 4,17 | 13,15 | 10,03 |

Spectre IR (KBr) : Bandes NH à 3400 et 3220 $cm^{-1}$,
Bandes CH à 3050 et 3300 $cm^{-1}$,
Bande C≡N à 2270 $cm^{-1}$,
Bandes CO à 1720 (ester) et 1685 $cm^{-1}$ (lactame),
Bandes principales à 1590, 1470, 1425, 1240, 1095, 760 et 710 $cm^{-1}$.

Spectre de résonance magnétique nucléaire du proton : (solvant DMSO-$d_6$) :

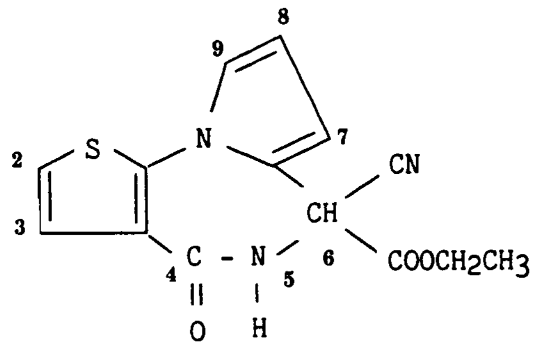

| H2 ppm | H3 ppm | H7 ppm | H8 ppm | H9 ppm | NH ppm | H6 ppm | Autres protons |
|---|---|---|---|---|---|---|---|
| 7,76 | 7,50 | 7,43 | 7,61 | 7,67 | 7,61 | 6,64 | $CH_2$ : 4,20<br>$CH_3$ : 1,22 |

**EXEMPLE 55**

**5,6-dihydro 6-(1-méthyl éthoxy) 4-oxo 4H-pyrrolo [1,2-a] thiéno [3,2-f] [1,4]-diazépine**

## Procédé A

Une solution de 1 g de 3-carboxamide 2-(2-formyl pyrrol-1-yl) thiophène dans 60 ml d'isopropanol est chauffée au reflux pendant une heure. L'isopropanol est ensuite éliminé sous vide et le solide résiduel recristallisé.
Rendement : 93%
Point de fusion : 165 ° C (Isopropanol) cristaux blancs

Analyse élémentaire :

| | C% | H% | N% | S% |
|---|---|---|---|---|
| Théorie | 59,52 | 5,37 | 10,67 | 12,22 |
| Trouvé | 59,71 | 5,36 | 10,84 | 12,18 |

Spectre IR (KBr) : Bandes
NH à 3270 et 3180 $cm^{-1}$, Bandes CH à 3050, 2960 et 2880 $cm^{-1}$,
Bande C=O à 1645 $cm^{-1}$,
Bandes principales à 1540, 1500, 1320, 1230, 1215, 1020, 730 et 705 $cm^{-1}$.

## Procédé B

Même protocole que pour le procédé A mais, on utilise 1 g du composé décrit dans l'exemple 40 au lieu du 3-carboxamide 2-(2-formyl pyrrol-1-yl) thiophène.
Rendement : 93%.

**EXEMPLE 56**

**6-benzyloxy 5,6-dihydro 4-oxo 4H-pyrrolo [1,2-a] thiéno [3,2-f] [1,4]-diazépine**

A une solution de 0,0045 mole du composé de l'exemple 40 dans 100 ml d'acétonitrile, on ajoute 0,0050 mole d'alcool benzylique puis le mélange réactionnel est chauffé au reflux pendant 90 mn. L'acétonitrile est ensuite éliminé sous vide. L'huile résiduelle qui cristallise par addition d'éther de pétrole est recristallisée.
Rendement : 86%
Point de fusion : 150 °C (Ether éthylique) cristaux blancs

Analyse élémentaire :

| | C% | H% | N% | S% |
|---|---|---|---|---|
| Théorie | 65,79 | 4,55 | 10,33 | 9,02 |
| Trouvé | 65,41 | 4,37 | 10,45 | 9,22 |

Spectre IR (KBr) : Bandes NH à 3260 et 3190 $cm^{-1}$,
Bandes CH à 3100, 3060, 2920 et 2860 $cm^{-1}$,
Bande C=O à 1635 $cm^{-1}$,
Bandes principales à 1540, 1490, 1440, 1310, 1025, 920, 710 et 685 $cm^{-1}$.

**EXEMPLE 57**

**5,6-dihydro 6-NN-diméthylhydrazino 4-oxo 4H-pyrrolo [1,2-a] thiéno [3,2-f] [1,4]-diazépine**

A une suspension de 1 g de 3-carboxamide 2-(2-formyl pyrrol-1-yl) thiophène dans 300 ml d'eau, on ajoute 2 ml de diméthylhydrazine. Le mélange réactionnel est chauffé à 50 °C pendant 30 mn puis agité à température ambiante pendant 12 heures. La solution orange obtenue est extraite avec 2 fois 100 ml d'éther éthylique. Les phases organiques sont rassemblées, lavées à l'eau, séchées sur sulfate de sodium et éliminées sous vide. L'huile résiduelle qui cristallise par addition d'éther de pétrole est recristallisée.
Rendement : 76%
Point de fusion : 116 °C (Ethanol) cristaux roses

Analyse élémentaire :

| | C% | H% | N% | S% |
|---|---|---|---|---|
| Théorie | 54,94 | 5,38 | 21,36 | 12,22 |
| Trouvé | 54,11 | 5,10 | 20,61 | 12,05 |

Spectre IR (KBr) : Bandes NH à 3280 et 3140 $cm^{-1}$,
Bandes CH à 3000, 2950, 2860 et 2790 $cm^{-1}$,
Bande CO à 1675 $cm^{-1}$,
Bandes principales à 1450, 1420, 1330, 1050, 860, 795 et 715 $cm^{-1}$.

**EXEMPLE 58**

**5,6-dihydro 1-oxo 6-(N-phénylhydrazino)4H-pyrrolo [1,2-a] thiéno [3,2-f] [1,4]-diazépine**

Ce composé a été préparé selon le procédé décrit dans l'exemple 57 mais en utilisant la phénylhydrazine.
Rendement : 71%
Point de fusion : 158 ° C (Ether éthylique) cristaux gris

Analyse élémentaire :

| | C% | H% | N% | S% |
|---|---|---|---|---|
| Théorie | 61,92 | 4,55 | 18,05 | 10,33 |
| Trouvé | 62,12 | 4,66 | 17,89 | 10,43 |

Spectre IR (KBr) : Bandes NH à 3460, 3320, 3280 et 3150 $cm^{-1}$,
Bandes CH à 3100 et 3040 $cm^{-1}$,
Bande CO à 1660 $cm^{-1}$,
Bandes principales à 1580, 1490, 1460, 1280, 1255, 1125, 750, 715 et 690 $cm^{-1}$.

**EXEMPLE 59**

**5,6-dihydro 6-(N-(2,5-dinitro phénylhydrazino)) 4-oxo 4H-pyrrolo [1,2-a] thiéno [3,2-f] [1,4]-diazépine**

Ce composé a été préparé selon le procédé décrit dans l'exemple 57 mais en utilisant la 2,5-dinitrophénylhydrazine.
Rendement : 65%
Point de fusion : 238 ° C (Ether éthylique - Acétone) cristaux rouges

Analyse élémentaire :

| | C% | H% | N% | S% |
|---|---|---|---|---|
| Théorie | 48,10 | 3,01 | 8,00 | 21,00 |
| Trouvé | 47,92 | 2,98 | 8,02 | 20,79 |

Spectre IR (KBr) : Bandes NH à 3270 et 3120 $cm^{-1}$,
Bandes CH à 3110 et 3090 $cm^{-1}$,
Bande C = O à 1650 $cm^{-1}$,
Bandes principales à 1615, 1500, 1420, 1340, 1320, 1135, 1090 et 715 $cm^{-1}$.

**EXEMPLE 60**

**5,6-dihydro 4-oxo 6-(méthoxycarbonyl-méthylthio) 4H-pyrrolo [1,2-a] thiéno [3,2-f] [1,4]-diazépine**

Une solution de 1 g de 3-carboxamide 2-(2-formyl pyrrol-1-yl) thiophène dans 100 ml d'acétonitrile est additionnée de 0,43 ml (0,005 mole) de thioglycolate de méthyle et le mélange réactionnel est agité à température ambiante pendant une nuit. L'acétonitrile est ensuite éliminé sous vide. L'huile résiduelle qui cristallise par addition l'éther de pétrole est recristallisée.
Rendement : 72%
Point de fusion : 158 ° C (Ether éthylique) cristaux blancs

Analyse élémentaire :

| | C% | H% | N% | S% |
|---|---|---|---|---|
| Théorie | 50,64 | 3,92 | 9,08 | 20,73 |
| Trouvé | 50,50 | 3,83 | 9,02 | 20,95 |

Spectre IR (KBr) : Bandes NH à 3360 et 3280 $cm^{-1}$,
Bandes CH à 3050 et 2950 $cm^{-1}$,
Bandes CO à 1730 (ester) et 1645 $cm^{-1}$ (lactame),
Bandes principales à 1535, 1495, 1440, 1310, 1240, 1165, 790, 735 et 705 $cm^{-1}$.

**EXEMPLE 61**

**5,6-dihydro 6-phénylthio 4-oxo 4H-pyrrolo [1,2-a] thiéno [3,2-f] [1,4]-diazépine**

Ce composé a été préparé selon le procédé décrit dans l'exemple 60 mais en utilisant le thiophénol au lieu du thioglycolate de méthyle.
Rendement : 78%
Point de fusion : 142 °C (Ether éthylique) cristaux blancs

Analyse élémentaire :

| | C% | H% | N% | S% |
|---|---|---|---|---|
| Théorie | 61,51 | 3,87 | 8,97 | 20,52 |
| Trouvé | 60,65 | 3,82 | 9,13 | 20,69 |

Spectre IR (KBr) : Bandes NH à 3370 et 3160 $cm^{-1}$,
Bandes CH à 3120, 3010 et 2930 $cm^{-1}$,
Bande CO à 1645 $cm^{-1}$,
Bandes principales à 1550, 1500, 1480, 1325, 1220, 1065, 775, 720 et 690 $cm^{-1}$.

**EXEMPLE 62**

**Acide 3-(5,6-dihydro 4-oxo 4H-pyrrolo [1,2-a] thiéno [3,2-f] [1,4]-diazépin-6-yl thio) propionique**

Ce composé a été préparé selon le procédé décrit dans l'exemple 60 mais en utilisant l'acide 3-mercaptopropionique au lieu du thioglycolate de méthyle.
Rendement : 50%
Point de fusion : 153 °C (Ether éthylique) cristaux blancs

Analyse élémentaire :

| | C% | H% | N% | S% |
|---|---|---|---|---|
| Théorie | 50,64 | 3,92 | 9,08 | 20,79 |
| Trouvé | 50,48 | 3,89 | 9,07 | 20,71 |

Spectre IR (KBr) : Bande OH à 3450 $cm^{-1}$,
Bandes NH à 3260 et 3200 $cm^{-1}$,
Bandes CH à 3060 et 2940 $cm^{-1}$,
Bandes C=O à 1700 (acide) et 1645 $cm^{-1}$ (lactame),
Bandes principales à 1550, 1500, 1430, 1245, 1190, 925 et 710 $cm^{-1}$.

**EXEMPLE 63**

**3-(5,6-dihydro 4-oxo 4H-pyrrolo [1,2-a] thiéno [3,2-f] [1,4]-diazépin-6-yl thio) propionamide**

A une suspension de 1 g du composé de l'exemple 62 dans 150 ml d'éther éthylique refroidie préalablement à 0 ° C est ajouté en une seule fois 0,50 ml (0,0035 mole) de triéthylamine puis le mélange réactionnel est agité 20 mn à la même température, et 0,30 ml de chloroformiate d'éthyle sont alors ajoutés goutte à goutte et l'agitation est continuée pendant 20 mn à 0 ° C. Le produit passe progressivement en solution et on observe l'apparition d'un précipité floconneux de chlorhydrate de triéthylamine. Ce dernier est filtré et on fait passer un courant d'ammoniac gazeux pendant 20 secondes dans la solution éthérée toujours maintenue à 0 ° C. Le précipité blanc obtenu est essoré, lavé à l'éther éthylique et recristallisé.
Rendement : 25%

Analyse élémentaire :

| | C% | H% | N% | S% |
|---|---|---|---|---|
| Théorie | 50,80 | 4,26 | 13,67 | 20,86 |
| Trouvé | 50,22 | 4,67 | 14,03 | 21,66 |

Spectre IR (KBr) : Bandes NH à 3460, 3340 et 3170 $cm^{-1}$,
Bandes CH à 3040, 2960 et 2920 $cm^{-1}$,
Bande C = O à 1660 $cm^{-1}$,
Bandes principales à 1490, 1430, 1410, 1425, 1090, 750 et 710 $cm^{-1}$.

**EXEMPLE 64**

**5,6-dihydro 6-(2-Benzoyloxy propyl) 4-oxo 4H-pyrrolo [1,2-a] thiéno [3,2-f] [1,4]-diazépine**

A une solution de 0,7 g de 5,6-dihydro 6-(2-hydroxy propyl) 4-oxo 4H-pyrrolo [1,2-a] thiéno [3,2-f][1,4]-diazépine (composé de l'exemple 12) dans 10 ml de pyridine, on ajoute 0,55 ml de chlorure de benzoyle.
Le milieu réactionnel est agité 24 heures à température ambiante puis la pyridine est éliminée sous vide.
Le résidu est repris par 100 ml d'eau et le produit extrait par 150 ml d'éther éthylique.
La phase organique est lavée à l'eau, séchée sur sulfate de magnésium puis concentrée.
L'huile résiduelle qui cristallise par addition l'éther de pétrole est recristallisée dans l'éther éthylique.
Rendement : 25%
Point de fusion : 95 ° C (Ether éthylique) cristaux blancs

Analyse élémentaire :

| | C% | H% | N% | S% |
|---|---|---|---|---|
| Théorie | 65,56 | 4,95 | 7,64 | 8,75 |
| Trouvé | 66,01 | 4,72 | 6,22 | 8,92 |

Spectre IR (KBr) : Bandes NH à 3260 et 3140 $cm^{-1}$,
Bandes CH à 2980, 2960 et 2890 $cm^{-1}$,
Bande CO à 1730 et 1675 $cm^{-1}$,
Bandes principales à 1550, 1500, 1290, 1245, 1100 et 720 $cm^{-1}$.

**EXEMPLE 65**

**5,6-dihydro 6-(2-hydrazono-1-pentyl) 4-oxo 4H-pyrrolo [1,2-a] thiéno [3,2-f] [1,4]-diazépine**

On additionne 0,6 ml d'hydrate d'hydrazine à une solution de 1 g de 5,6-dihydro 4-oxo 6-(2-oxo pent-1-yl) 4H-pyrrolo [1,2-a] thiéno [3,2-f] [1,4]-diazépine (exemple 1) dans 250 ml d'éthanol puis le mélange

réactionnel est chauffé au reflux pendant une heure. L'éthanol est ensuite éliminé sous vide et le résidu solide trituré dans 100ml d'eau.

Le précipité formé est essoré, lavé, séché et recristallisé
Rendement : 76%
Point de fusion : 158 ° C (Ether éthylique) cristaux blancs

Analyse élémentaire :

| | C% | H% | S% | N% |
|---|---|---|---|---|
| Théorie | 59,78 | 5,69 | 18,59 | 10,64 |
| Trouvé | 59,45 | 5,88 | 18,21 | 10,31 |

Spectre IR (KBr) : Bandes NH à 3410, 3370 et 3190 $cm^{-1}$,
Bandes CH à 3050, 2970 et 2880 $cm^{-1}$,
Bande CO à 1640 $cm^{-1}$,
Bandes principales à 1490, 1435, 1335, 1090 et 715 $cm^{-1}$.

TABLEAU I

Composés de formule $I_A$

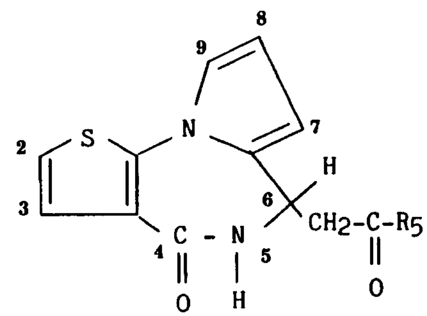

| Composés | Solvant | $R_5$ | $H_2$ (ppm) | $H_3$ (ppm) | $H_7$ (ppm) | $H_8$ (ppm) | $H_9$ (ppm) | NH (ppm) | $H_6$ (ppm) | Autres protons (ppm) |
|---|---|---|---|---|---|---|---|---|---|---|
| Exemple 1 | $CDCl_3$ | $-CH_2CH_2CH_3$ | 7,36 | 6,96 | 6,00 | 6,28 | 6,96 | 7,06 | 4,96 | $CH_2$ : 3,07<br>$CH_2$ : 2,46 $CH_3$ : 0,90<br>$CH_2$ : 1,60 |
| Exemple 2 | $CDCl_3$ | 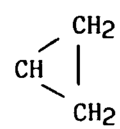 | 7,38 | 6,96 | 6,05 | 6,29 | 6,96 | 6,50 | 4,96 | $CH_2$ : 3,22<br>CH : 1,96<br>$2CH_2$ : 1,10 et 0,98 |

**TABLEAU I**
**Composés de formule $I_A$**
**(Suite 1)**

| Composés | Solvant | $R_5$ | $H_2$ (ppm) | $H_3$ (ppm) | $H_7$ (ppm) | $H_8$ (ppm) | $H_9$ (ppm) | NH (ppm) | $H_6$ (ppm) | Autres protons (ppm) |
|---|---|---|---|---|---|---|---|---|---|---|
| Exemple 3 | $CDCl_3$ | $-CH_2CH_2-$ 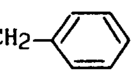 | 7,34 | 6,92 | 5,98 | 6,26 | 6,92 | 6,73 | 4,95 | $CH_2$ : 3,03<br>$CH_2$ : 2,86<br>$CH_2$ : 2,82 |
| Exemple 4 | $CDCl_3$ | 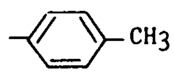 | 7,32 | 6,92 | 6,05 | 6,27 | 6,97 | 6,70 | 5,11 | $CH_2$ : 3,58<br>$CH_3$ : 2,42<br>$C_6H_4$ : 7,84 et 7,26 |
| Exemple 5 | $CDCl_3$ | 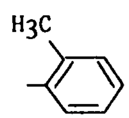 | 7,31 | 6,95 | 6,04 | 6,27 | 6,95 | 7,02 | 5,10 | $CH_2$ : 3,58<br>$CH_3$ : 2,48<br>$C_6H_4$ : 7,68, 7,40, 7,28 |
| Exemple 6 | $CDCl_3$ | 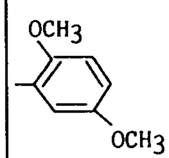 | 7,37 | 6,95 | 6,05 | 6,30 | 6,96 | 6,50 | 5,13 | $CH_2$ : 3,66<br>$C_6H_3$ : 7,29, 7,06 et 6,95<br>$2CH_3$ : 3,80 et 3,78 |
| Exemple 7 | DMSO-d6 | 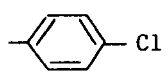 | 7,35 | 7,00 | 6,23 | 6,23 | 7,35 | 8,20 | 4,80 | $CH_2$ : 3,67<br>$C_6H_4Cl$ : 8,10, 8,0, 7,60 |

**TABLEAU I**
**Composés de formule $I_A$**
**(Suite 2)**

| Composés | Solvant | $R_5$ | $H_2$ (ppm) | $H_3$ (ppm) | $H_7$ (ppm) | $H_8$ (ppm) | $H_9$ (ppm) | NH (ppm) | $H_6$ (ppm) | Autres protons (ppm) |
|---|---|---|---|---|---|---|---|---|---|---|
| **Exemple 8** | $CDCl_3$ | 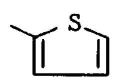 | 7,37 | 6,97 | 6,10 | 6,29 | 6,97 | 6,59 | 5,13 | $CH_2$ : 3,55<br>$C_4H_3S$ : 7,73 et 7,16 |
| **Exemple 9** | $CDCl_3$ | 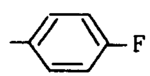 | 7,35 | 6,93 | 6,07 | 6,29 | 6,93 | 6,45 | 5,19 | $CH_2$ : 3,55<br>$C_6H_4F$ : 7,91, 6,93 et 7,15 |
| **Exemple 10** | $CDCl_3$ | 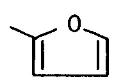 | 7,38 | 6,98 | 6,09 | 6,29 | 6,98 | 6,47 | 5,11 | $CH_2$ : 3,48<br>$C_4H_3O$ : 7,62, 7,38 et 6,51 |
| **Exemple 11** | $CDCl_3$ | 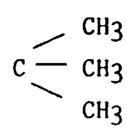 | 7,38 | 6,93 | 6,03 | 6,28 | 6,96 | 6,13 | 4,98 | $CH_2$ : 3,11<br>$3CH_3$ : 1,54 |

**TABLEAU II**

**Composés de formule $I_B$**

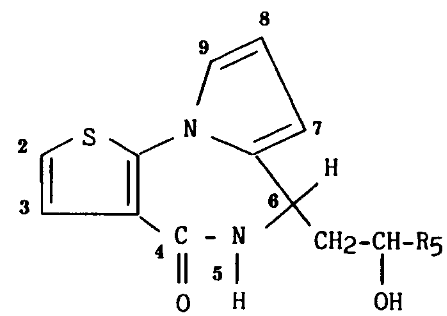

| Composés | Solvant | $R_5$ | $H_2$ (ppm) | $H_3$ (ppm) | $H_7$ (ppm) | $H_8$ (ppm) | $H_9$ (ppm) | NH (ppm) | $H_6$ (ppm) | Autres protons (ppm) |
|---|---|---|---|---|---|---|---|---|---|---|
| Exemple 13 | $CDCl_3$ | $-CH_2CH_2CH_3$ | 7,34 | 6,92 | 6,04 | 6,27 | 6,92 | 7,03 | 4,66 | CH : 4,04<br>OH : 2,40 $CH_3$ : 0,94<br>$CH_2$ : 2,10<br>$CH_2$ : 1,50 et 1,34 |
| Exemple 14 | $CDCl_3$ | 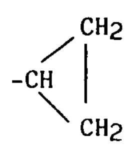 | 7,37 | 6,94 | 6,09 | 6,31 | 6,94 | 7,07 | 4,70 | CH : 3,29 OH : 2,40<br>$CH_2$ : 2,28<br>CH : 1,04<br>$CH_2$ : 0,58 et 0,31 |

**TABLEAU II**
**Composés de formule $I_B$**
**(Suite)**

| Composés | Solvant | R | $H_2$ (ppm) | $H_3$ (ppm) | $H_7$ (ppm) | $H_8$ (ppm) | $H_9$ (ppm) | NH (ppm) | $H_6$ (ppm) | Autres protons (ppm) |
|---|---|---|---|---|---|---|---|---|---|---|
| Exemple 15 | $CDCl_3$ | 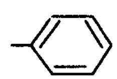 | 7,34 | 6,93 | 6,09 | 6,30 | 6,33 | 7,34 | 5,17 | $CH_2$ : 2,37<br>CH : 4,62<br>$C_6H_5$ : 7,34 |
| Exemple 17 | DMSO-d6 | 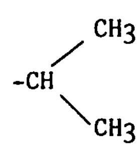 | 7,20 | 7,08 | 6,09 | 6,26 | 7,08 | 8,13 | 4,46 | OH : 4,52<br>CH : 3,46<br>$CH_2$ : 2,88 $2CH_3$ : 0,88<br>CH : 1,90 |
| Exemple 18 | $CDCl_3$ | 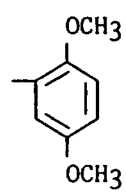 | 7,38 | 6,94 | 6,14 | 6,31 | 6,94 | 6,74 | 5,36 | OH : 4,70<br>OH : 2,56 $2CH_3$ : 3,81<br>$CH_2$ : 2,38<br>$C_6H_3$ : 7,07 et 6,81 |

**TABLEAU III**

**Composés de formule $I_C$**

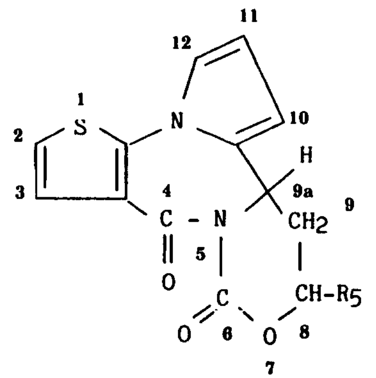

| Composés | Solvant | $R_5$ | $H_2$ (ppm) | $H_3$ (ppm) | $H_{10}$ (ppm) | $H_{11}$ (ppm) | $H_{12}$ (ppm) | $H_8$ (ppm) | $H_{9a}$ (ppm) | $CH_2'_9$ | Autres protons (ppm) |
|---|---|---|---|---|---|---|---|---|---|---|---|
| Exemple 19 | $CDCl_3$ | $-CH_3$ | 7,36 | 7,36 | 6,40 | 6,52 | 7,36 | 5,00 | 5,15 | CH : 2,54<br>CH : 2,31 | $CH_3$ : 1,42 |
| Exemple 20 | $CDCl_3$ | $-CH_2CH_2CH_3$ | 7,37 | 7,37 | 6,41 | 6,48 | 7,35 | 4,84 | 5,15 | CH : 2,71<br>CH : 2,35 | $CH_2$ : 1,68 et 1,45<br>$CH_3$ : 0,94 |
| Exemple 21 | $CDCl_3$ | 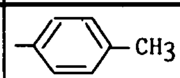 $CH_3$ | 7,26 | 7,26 | 6,22 | 6,32 | 7,26 | 5,30 | 4,50 | 2,52 | $CH_3$ : 2,32<br>$C_6H_4$ : 8,59, 7,40, 7,26 |

**TABLEAU IV**

**Composés de formule $I_D$**

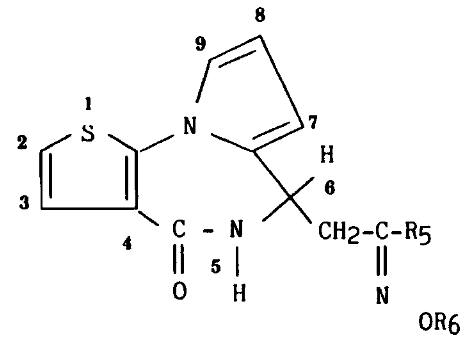

| Composés | Solvant | $R_6$ | $R_5$ | $H_2$ (ppm) | $H_3$ (ppm) | $H_7$ (ppm) | $H_8$ (ppm) | $H_9$ (ppm) | NH | $H_6$ | Autres protons (ppm) |
|---|---|---|---|---|---|---|---|---|---|---|---|
| Exemple 22 | $CDCl_3$ | H | $-CH_3$ | 7,40 | 7 | 6,37 | 6,13 | 7,0 | 7,89 | 4,57 | OH : 10,72<br>$CH_2$ : 2,89<br>$CH_3$ : 1,93 |
| Exemple 23 | $CDCl_3$ | H | 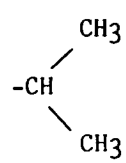 | 7,40 | 6,94 | 6,34 | 6,17 | 6,98 | 7,67 | 4,57 | OH : 9,63<br>$CH_2$ : 2,55<br>CH : 3,66<br>$2CH_3$ : 1,10 |

**TABLEAU IV**
**Composés de formule $I_D$**
**(Suite 1)**

| Composés | Solvant | R6 | R5 | H2 (ppm) | H3 (ppm) | H7 (ppm) | H8 (ppm) | H9 (ppm) | NH | H6 | Autres protons (ppm) |
|---|---|---|---|---|---|---|---|---|---|---|---|
| Exemple 24 | $CDCl_3$ | H | 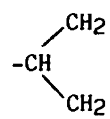 | 7,40 | 6,95 | 6,34 | 6,16 | 7,00 | 7,40 | 4,88 | OH : 4,88<br>CH : 1,49<br>$2CH_2$ : 0,75 |
| Exemple 25 | $CDCl_3$ | H | $CH_2CH_2CH_3$ | 7,40 | 6,95 | 6,34 | 6,14 | 6,97 | 7,50 | 4,6 | $CH_2$ : 2,9<br>$CH_2$ : 1,50 et 1,19<br>$CH_3$ : 0,90 |
| Exemple 26 | $CDCl_3$ | H | 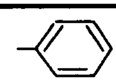 | 7,33 | 6,95 | 6,29 | 6,07 | 6,95 | 6,63 | 5,15 | $CH_2$ : 3,62<br>$C_6H_5$ : 7,95, 7,59, 7,47 |
| Exemple 27 | $CDCl_3$ | H | 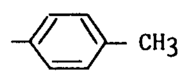 | 7,33 | 6,90 | 6,38 | 6,28 | 7,00 | 10,76 | 4,55 | $CH_2$ : 4,12 et 3,13<br>$CH_3$ : 2,33<br>$C_6H_4$ : 7,55 et 7,13 |

TABLEAU IV

Composés de formule $I_D$

(Suite 2)

| Composés | Solvant | R6 | R5 | $H_2$ (ppm) | $H_3$ (ppm) | $H_7$ (ppm) | $H_8$ (ppm) | $H_9$ (ppm) | NH | $H_6$ | Autres protons (ppm) |
|---|---|---|---|---|---|---|---|---|---|---|---|
| Exemple 28 | $CDCl_3$ | $CH_3$ | 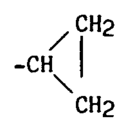 | 7,30 | 6,88 | 6,26 | 6,02 | 6,92 | 6,64 | 4,90 | CH : 1,24<br>$2CH_2$ : 0,92 et 0,88<br>$CH_3$ : 3,84 |
| Exemple 29 | DMSO-d6 | $CH_3$ | $-CH_3$ | 7,28 | 7,21 | 6,28 | 6,22 | 7,13 | 8,14 | 4,61 | $CH_3$ : 3,71<br>$CH_3$ : 1,75 |

**TABLEAU V**

**Composés de formule $I_E$**

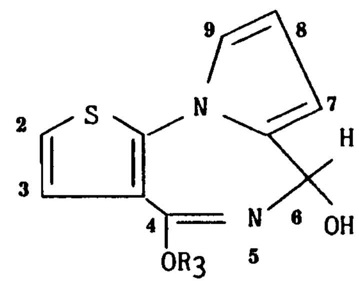

| Composés | Solvant | $R_3$ | $H_2$ (ppm) | $H_3$ (ppm) | $H_7$ (ppm) | $H_8$ (ppm) | $H_9$ (ppm) | $H_6$ (ppm) | OH (ppm) | Autres protons (ppm) |
|---|---|---|---|---|---|---|---|---|---|---|
| Exemple 30 | DMSO-d6 | $-CH_3$ | 7,30 | 7,14 | 6,34 | 6,34 | 7,10 | 5,60 | 6,18 | $CH_2$ : 3,70 |
| Exemple 31 | DMSO-d6 | $-CH_2CH_3$ | 7,31 | 7,14 | 6,2 | 6,2 | 7,11 | 5,60 | 6,20 | $CH_2$ : 4,16<br>$CH_3$ : 1,10 |
| Exemple 32 | DMSO-d6 | $-CH_2CH_2CH_3$ | 7,31 | 7,15 | 6,30 | 6,30 | 7,10 | 5,66 | 6,30 | $CH_2$ : 4,08<br>$CH_2$ : 1,63<br>$CH_3$ : 0,95 |
| Exemple 33 | DMSO-d6 | $-CH_2CH=CH_2$ | 7,33 | 7,16 | 6,20 | 6,20 | 7,10 | 5,63 | 6,20 | CH : 5,93<br>$CH_2$ : 4,63<br>$CH_2$ : 5,21 |

**TABLEAU VI**

**Composés de formule $I_F$**

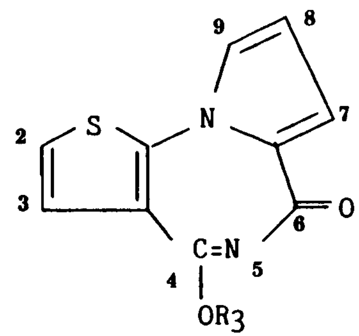

| Composés | Solvant | $R_3$ (ppm) | $H_2$ (ppm) | $H_3$ (ppm) | $H_7$ (ppm) | $H_8$ (ppm) | $H_9$ (ppm) | Autres protons (ppm) |
|---|---|---|---|---|---|---|---|---|
| **Exemple 34** | DMSO-d6 | $-CH_3$ | 7,43 | 7,30 | 7,08 | 6,63 | 7,70 | $CH_3$ : 3,93 |
| **Exemple 35** | DMSO-d6 | $-CH_2CH_3$ | 7,40 | 7,30 | 7,01 | 6,60 | 7,65 | $CH_2$ : 4,35<br>$CH_3$ : 1,34 |
| **Exemple 36** | DMSO-d6 | $-CH_2CH_2CH_3$ | 7,45 | 7,31 | 7,08 | 6,65 | 7,68 | $CH_2$ : 4,31<br>$CH_2$ : 1,78<br>$CH_3$ : 1,00 |

**TABLEAU VII**

**Composés de formule $I_G$**

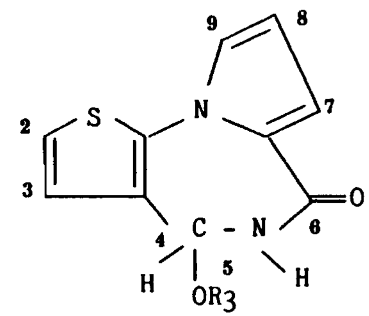

| Composés | Solvant | $R_3$ (ppm) | $H_2$ (ppm) | $H_3$ (ppm) | $H_7$ (ppm) | $H_8$ (ppm) | $H_9$ (ppm) | NH (ppm) | $H_4$ (ppm) | Autres protons (ppm) |
|---|---|---|---|---|---|---|---|---|---|---|
| Exemple 37 | $CDCl_3$ | $-CH_3$ | 7,26 | 6,93 | 6,93 | 6,40 | 7,17 | 7,31 | 5,25 | $CH_3$ : 3,35 |
| Exemple 38 | DMSO-d6 | $-CH_2CH_3$ | 7,25 | 7,11 | 6,97 | 6,37 | 7,37 | 9,05 | 5,32 | $CH_2$ : 3,48<br>$CH_3$ : 0,98 |
| Exemple 39 | DMSO-d6 | $-CH_2CH_2CH_3$ | 7,25 | 7,11 | 6,95 | 6,35 | 7,36 | 9,03 | 5,32 | $CH_2$ : 3,39<br>$CH_2$ : 1,36<br>$CH_2$ : 0,65 |

**TABLEAU VIII**

**Composés de formule $I_H$**

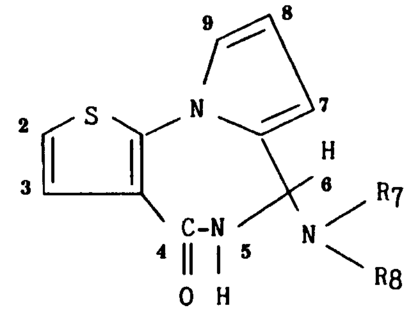

**Solvant utilisé $CDCl_3$ :**

| Composés | $R_7$ (ppm) | $R_8$ (ppm) | $H_2$ (ppm) | $H_3$ (ppm) | $H_7$ (ppm) | $H_8$ (ppm) | $H_9$ (ppm) | NH (ppm) | $H_6$ (ppm) | Autres protons (ppm) |
|---|---|---|---|---|---|---|---|---|---|---|
| **Exemple 41** | H | $-CH_3$ | 7,39 | 6,91 | 6,19 | 6,31 | 6,98 | 6,81 | 5,03 | $CH_3$ : 1,75 |
| **Exemple 42** | H | $-CH_2CH_3$ | 7,34 | 6,92 | 6,19 | 6,31 | 6,98 | 6,76 | 5,15 | $CH_2$ : 2,82<br>$CH_3$ : 1,11 |
| **Exemple 43** | H | $-CH_2CH_2CH_3$ | 7,39 | 6,91 | 6,18 | 6,31 | 6,98 | 6,52 | 5,13 | $CH_2$ : 2,73<br>$CH_2$ : 1,49<br>$CH_3$ : 0,88<br>NH : 4,82 |

**TABLEAU VIII**

**Composés de formule $I_H$**

**(Suite 1)**

| Composés | $R_7$ (ppm) | $R_8$ (ppm) | $H_2$ (ppm) | $H_3$ (ppm) | $H_7$ (ppm) | $H_8$ (ppm) | $H_9$ (ppm) | NH (ppm) | $H_6$ (ppm) | Autres protons (ppm) |
|---|---|---|---|---|---|---|---|---|---|---|
| **Exemple 44** | H | 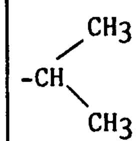 | 7,38 | 6,92 | 6,17 | 6,31 | 6,97 | 6,83 | 5,20 | CH : 3,08<br>$2CH_3$ : 1,07 |
| **Exemple 45** | $-CH_3$ | $-CH_3$ | 7,38 | 6,85 | 6,21 | 6,29 | 6,99 | 6,28 | 4,46 | $2CH_3$ : 2,20 |
| **Exemple 46** | $-CH_2CH_3$ | $-CH_2CH_3$ | 7,37 | 6,89 | 6,19 | 6,31 | 6,98 | 6,31 | 5,11 | $2CH_2$ : 2,66<br>$2CH_2$ : 0,98 |
| **Exemple 47** | 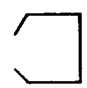 | | 7,37 | 6,83 | 6,19 | 6,27 | 6,97 | 6,71 | 4,64 | $CH_2$ : 2,64<br>$CH_2$ : 2,35<br>$2CH_2$ : 1,65 |
| **Exemple 48** | 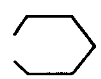 | | 7,35 | 6,85 | 6,18 | 6,28 | 6,97 | 6,62 | 4,65 | $CH_2$ : 2,63<br>$CH_2$ : 2,19<br>$3CH_2$ : 1,39 |
| **Exemple 49** | 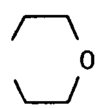 | | 7,35 | 6,86 | 6,22 | 6,29 | 7,00 | 6,93 | 4,53 | $2CH_2$ : 3,47<br>$CH_2$ : 2,50<br>$CH_2$ : 2,14 |

**TABLEAU VIII**
**Composés de formule $I_H$**
**(Suite 2)**

| Composés | $R_7$ (ppm) | $R_8$ (ppm) | $H_2$ (ppm) | $H_3$ (ppm) | $H_7$ (ppm) | $H_8$ (ppm) | $H_9$ (ppm) | NH (ppm) | $H_6$ (ppm) | Autres protons (ppm) |
|---|---|---|---|---|---|---|---|---|---|---|
| Exemple 50 | 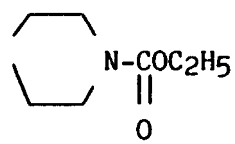 | | 7,35 | 6,85 | 6,18 | 6,28 | 6,97 | 6,67 | 4,65 | 2X $CH_2$ : 3,24<br>2X $CH_2$ : 2,48<br>$CH_2$ : 4,07<br>$CH_3$ : 1,21 |
| Exemple 51 | - H | 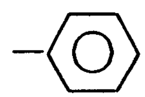 | 7,18 | 7,18 | 6,38 | 6,32 | 7,16 | 8,85 | 5,71 | CH ar: 7,19 / 7,16 / 6,78<br>NH : 6,62 |
| Exemple 52 | - H | 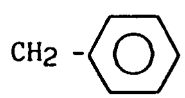 | 7,29 | 6,90 | 6,18 | 6,30 | 6,97 | 6,48 | 5,12 | $CH_2$ : 3,93<br>CH ar : 7,29<br>$CH_2$ : 1,6 |

**TABLEAU IX**

**Composés de formule $I_J$**

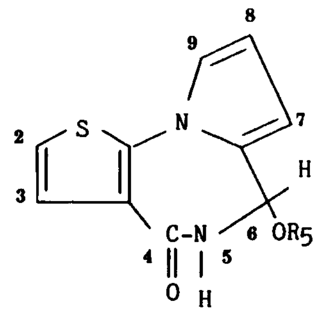

| Composés | Solvant | $R_5$ (ppm) | $H_2$ (ppm) | $H_3$ (ppm) | $H_7$ (ppm) | $H_8$ (ppm) | $H_9$ (ppm) | HH (ppm) | $H_6$ (ppm) | Autres protons (ppm) |
|---|---|---|---|---|---|---|---|---|---|---|
| Exemple 55 | $CDCl_3$ | 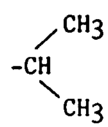 | 7,38 | 6,86 | 6,25 | 6,29 | 7,06 | 7,06 | 5,52 | CH : 3,79<br>$2CH_3$ : 1,71 |
| Exemple 56 | $CDCl_3$ | 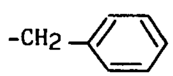 | 7,41 | 6,88 | 6,23 | 6,29 | 7,03 | 7,53 | 5,44 | $CH_2$ : 4,53<br>$C_6H_5$ : 7,20, 7,31, 7,26 |

**TABLEAU X**

**Composés de formule $I_K$**

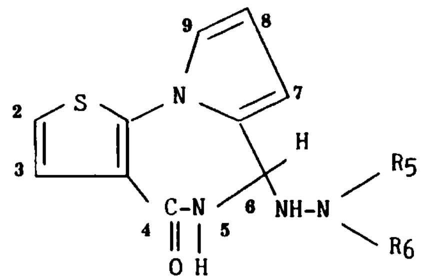

| Composés | Solvant | $R_5$ (ppm) | $R_6$ (ppm) | $H_2$ (ppm) | $H_3$ (ppm) | $H_7$ (ppm) | $H_8$ (ppm) | $H_9$ (ppm) | NH (ppm) | $H_6$ (ppm) | Autres protons (ppm) |
|---|---|---|---|---|---|---|---|---|---|---|---|
| **Exemple 57** | $CDCl_3$ | $-CH_3$ | $-CH_3$ | 7,48 | 7,21 | 6,53 | 6,79 | 6,94 | 5,52 | 5,15 | $2CH_3$ : 2,74<br>NH : 5,33 |
| **Exemple 58** | $CDCl_3$ | -H | 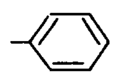 | 7,56 | 7,18 | 6,64 | 6,71 | 6,80 | 6,43 | 5,33 | NH : 5,33<br>NH : 6,38<br>$C_6H_5$ : 7,18, 7,12, 7,18, 6,70 |
| **Exemple 59** | DMSO | -H | 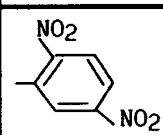 | 8,00 | 7,08 | 6,28 | 6,28 | 7,19 | 9,94 | 5,54 | NH : 5,85<br>NH : 8,79<br>$C_6H_3$ : 7,30 et 8,79 |

**TABLEAU XI**

**Composés de formule $I_L$**

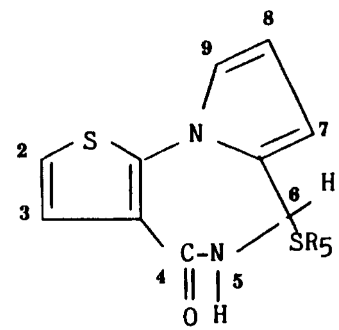

| Composés | Solvant | $R_5$ (ppm) | $H_2$ (ppm) | $H_3$ (ppm) | $H_7$ (ppm) | $H_8$ (ppm) | $H_9$ (ppm) | NH (ppm) | $H_6$ (ppm) | Autres protons (ppm) |
|---|---|---|---|---|---|---|---|---|---|---|
| **Exemple 60** | $CDCl_3$ | $CH_2CO_2CH_3$ | 7,40 | 6,92 | 6,28 | 6,28 | 7,04 | 6,92 | 5,94 | $CH_2$ : CH : 3,29 CH : 3,06<br>$CH_3$ : 3,76 |
| **Exemple 61** | $CDCl_3$ | $C_6H_5$ | 7,43 | 6,95 | 6,11 | 6,26 | 7,02 | 6,60 | 5,73 | $C_6H_5$ : 7,34 |
| **Exemple 62** | DMSO | $CH_2CH_2CO_2H$ | 7,21 | 7,21 | 6,31 | 6,31 | 7,21 | 9,11 | 5,73 | $CH_2$ : 2,43 et 2,60<br>OH : 12,21 |
| **Exemple 63** | DMSO | $CH_2CH_2CONH_2$ | 7,25 | 7,25 | 6,27 | 6,27 | 7,25 | 9,00 | 5,68 | $NH_2$ : 7,27 et 6,73<br>$CH_2$ : 2,56 et 2,24 |

## TABLEAU XII

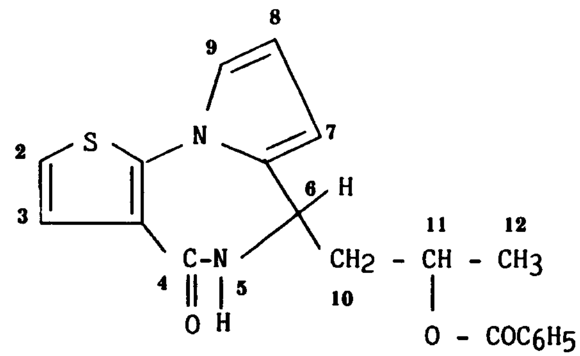

| Composés | $H_2$ (ppm) | $H_3$ (ppm) | $H_7$ (ppm) | $H_8$ (ppm) | $H_9$ (ppm) | NH (ppm) | $H_6$ (ppm) | $H_{10}$ (ppm) | $H_{11}$ (ppm) | $H_{12}$ (ppm) | Autres protons (ppm) |
|---|---|---|---|---|---|---|---|---|---|---|---|
| Exemple 64 | 7,35 | 6,93 | 6,10 | 6,16 | 6,93 | 7,40 | 5,15 | 2,27 | 4,44 | 1,32 | CH ar : 7,24<br>: 7,40<br>: 7,90 |

TABLEAU XIII

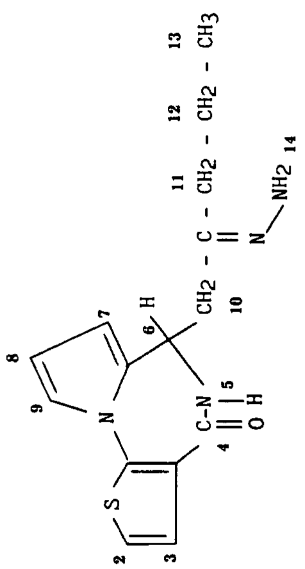

| Composés | H2 (ppm) | H3 (ppm) | H7 (ppm) | H8 (ppm) | H9 (ppm) | H5 (ppm) | H6 (ppm) | H10 (ppm) | H11 (ppm) | H12 (ppm) | H12 (ppm) | H12 (ppm) |
|---|---|---|---|---|---|---|---|---|---|---|---|---|
| Exemple 65 | 7,27 | 7,21 | 6,11 | 6,27 | 7,11 | 7,91 | 4,64 | 2,74 | 2,10 | 1,44 | 0,88 | 5,76 |

**EXEMPLE 66**

**Affinité pour les récepteurs de la cholécystokinine**

L'affinité des composés de formule (I) pour les récepteurs de la cholécystokinine a été étudiée en déterminant la concentration inhibitrice 50 ($CI_{50}$) pour la fixation de la cholécystokinine marquée à l'iode 125 aux récepteurs de membrances plasmiques de pancréas de rats (mâles SD, IFFA CREDO 200-225 g), et aux récepteurs de membranes de cerveau de cobayes (mâles, COB LABO, 325-350 g) selon le protocole décrit par INNIS R.B. et SNY DER S.M., Eur.J.Pharmacol.,65,123-124,1980.

Les $CI_{50}$ trouvées pour les récepteurs de membranes plasmatiques de pancréas de rats étaient pour la majorité des composés entre 0,01-60 nM/l et les CI50 trouvées pour les récepteurs de membranes de cerveau de cobayes entre 350-30.000 nM/l. Les rapports $CI_{50}$ relative aux récepteurs du pancréas / $CI_{50}$ relative aux récepteurs du cerveau ont été trouvés entre 3-20.000.

**EXEMPLE 67**

**Activité pour la cholécystokinine in vivo**

L'activité antagoniste des composés de formule (I) pour la CCK a été déterminée, chez la souris (SWISS mâle, 18-20 g) sur le modèle d'évacuation gastrique décrit par LOTTI V.J. et col. (LIFE SCIENCES,39; 1631-1638 ; 1986), en déterminant la dose efficace à 50% ($DE5_0$) qui protège les animaux de l'inhibition de l'évacuation gastrique provoquée par la CCK-8 sulfatée (80 µg/kg,sc). Les valeurs obtenues dans le test de l'évacuation gastrique étaient 0,032-0,290 mg/kg.

**EXEMPLE 68**

**Hypoxie aigüe chez la souris**

Des souris CD1 (Charles RIVER), de sexe mâle, ayant reçu 30 minutes auparavant par voie intrapéritonéale le composé à tester, sont soumises à une hypoxie aigüe de type hypobare. Pour cela, elles sont placées dans une enceinte où la pression atmosphérique peut être abaissée rapidement (en 30 secondes) à une valeur de 160 mbar, ce qui provoque la mort de tous les animaux, 15 secondes environ après l'obtention de cette pression hypoxique.

La survie du cerveau est appréciée par la mesure du temps d'apparition du dernier gasp respiratoire. Le temps de survie d'un lot traité est comparé à celui d'un lot témoin ne recevant que le solvant.

Les résultats de cette étude ont démontré que les composés de l'invention sont actifs à partir d'une dose de 3 mg/kg. Dans ce cas, on observe une augmentation de la survie des animaux d'environ 30-40%. Pour une dose de 100 mg/kg, certains composés de l'invention augmentent le temps de la survie des animaux jusqu'à 200%.

**EXEMPLE 69**

**Gélules dosées à 5 mg de 5,6-dihydro 6-[2-hydroxy 2-(4-méthylphényl) éthyl] 4-oxo 4H-pyrrolo [1,2-a] thiéno [3,2-f] [1,4]-diazépine (composé de l'exemple 16) :**

| | |
|---|---|
| Composé de l'exemple 16 | 5 mg |
| Amidon de maïs | 20 mg |
| Lactose | 30 mg |
| Talc | 10 mg |

Pour une gélule N°3.

**Revendications**

**Revendications pour les Etats contractants suivants : AT, BE, CH, DE, DK, FR, GB, IT, LI, LU, NL, SE**

**1.** Composés de formule générale **(I) :**

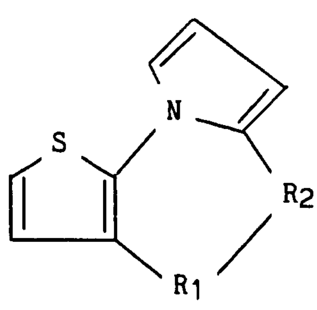

**(I)**

dans laquelle :

- $R_1$ représente un radical de formule générale **($Z_0$), ($Z_1$), ($Z_2$), ($Z_3$)** ou **($Z_4$)**

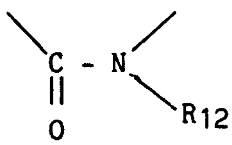
**($Z_0$)**
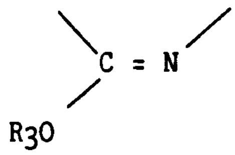
**($Z_1$)**
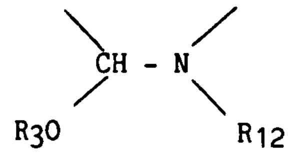
**($Z_2$)**

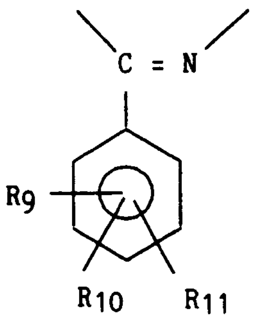
**($Z_3$)**
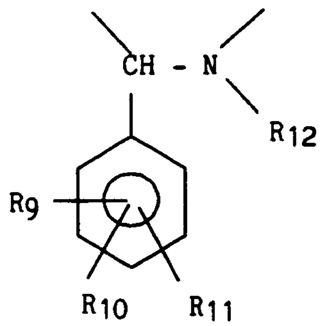
**($Z_4$)**

dans lesquelles :

- $R_3$ représente un atome d'hydrogène, un radical alkyle linéaire ou ramifié de 1 à 6 atomes de carbone saturé ou comportant une double liaison,
- $R_9$, $R_{10}$, $R_{11}$ identiques ou différents représentent chacun un atome d'hydrogène, un atome d'halogène ou un radical alkyle de 1 à 6 atomes de carbone,
- $R_{12}$ représente un atome d'hydrogène ou forme avec $R_2$ et l'atome d'azote auquel ils sont rattachés un radical de formule générale **(W) :**

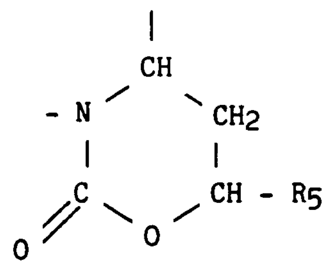
**(W)**

- $R_2$ représente un radical méthylène, un radical hydroxyméthylène, un radical carbonyle, un radical de formule générale **($Y_1$), ($Y_2$), ($Y_3$)** ou **($Y_4$) :**

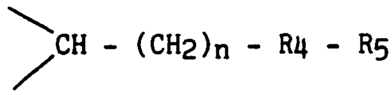

(Y1)

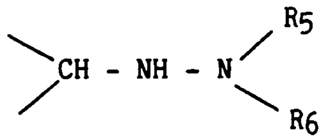

(Y2)

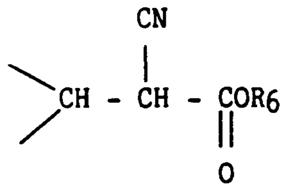

(Y3)

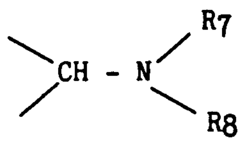

(Y4)

ou forme avec $R_{12}$ et l'atome d'azote auxquels ils sont rattachés un radical de formule générale **(W) :**

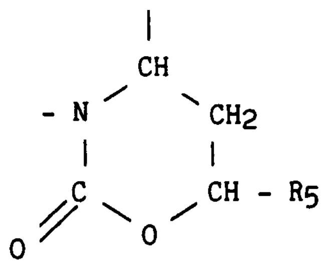

(W)

formules dans lesquelles :

- $R_4$ représente un atome d'oxygène, ou de soufre, un radical carbonyle, un radical de formule générale **($X_1$), ($X_2$), ($X_3$)** ou **($X_4$) :**

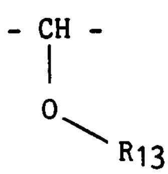

(X1)

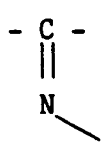

(X2)

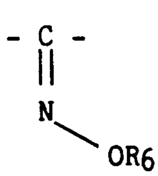

(X3)

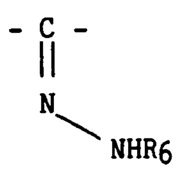

(X4)

- n est compris entre 0 et 4 inclusivement

- $R_5$ représente un atome d'hydrogène, une chaîne alkyle de 1 à 10 atomes de carbone, linéaire ou ramifiée, éventuellement interrompue par un ou plusieurs atomes d'oxygène, de soufre, un radical phényle, benzoyle ou aralkyle de 7 à 11 atomes de carbone (éventuellement substitués sur le noyau aromatique par un ou plusieurs atomes d'halogène, des radicaux alkyle de 1 à 6 atomes de carbone linéaires ou ramifiés, des radicaux nitro ou des radicaux alcoxy de 1 à 6 atomes de carbone linéaires ou ramifiés) un radical carboxyalkyle de 2 à 7 atomes de carbone linéaire ou ramifié, un radical alcoxycarbonylalkyle de 3 à 10 atomes de carbone linéaire ou ramifié, un radical alcoxycarbonyle de 2 à 7 atomes de carbone linéaire ou ramifié, un radical carbamoylalkyle de 2 à 7 atomes de carbone linéaire ou ramifié, un radical cycloalkyle de 3 à 7 atomes de carbone, un système cyclique insaturé de 5 à 7 sommets comprenant au moins un hétéroatome choisi parmi azote, soufre, oxygène, un radical pyridinylcarbonyle, pyrimidylcarbonyle, un radical Clofibroyle ou un radical 6-hydroxy 2,5,7,8-tétraméthyle chromanne-2-carbonyle,
- $R_6$ représente un atome d'hydrogène ou un radical alkyle de 1 à 6 atomes de carbone linéaire ou ramifié,
- $R_7$, $R_8$ identiques ou différents représentent chacun un atome d'hydrogène, un radical alkyle linéaire ou ramifié de 1 à 6 atomes de carbone, un radical phényle ou phénylalkyle de 7 à 9 atomes de carbone (éventuellement substitués sur le noyau aromatique par un ou plusieurs atomes d'halogène ou groupements alkyle ou alcoxy de 1 à 6 atomes de carbone), ou forment ensemble avec l'atome d'azote auquel ils sont attachés un système cyclique de 5 à 7 sommets, saturé ou insaturé, comprenant de 1 à 2 hétéroatomes choisis parmi azote, oxygène et soufre éventuellement substitué par un groupement alkylcarbonyl ou alcoxy carbonyle de 2 à 5 atomes de carbone,
- $R_{13}$ représente un atome d'hydrogène, un radical alkyl carbonyl de 2 à 6 atomes de carbone linéaire ou ramifié, un radical benzoyle,
- leurs isomères, diastéroisomères, énantiomères,
- leurs sels d'addition à un acide, minéral ou organique, pharmaceutiquement acceptable,

avec les réserves que :

- lorsque $R_1$ représente un radical de formule générale **($Z_3$)** alors $R_2$ ne peut représenter un radical méthylène,
- lorsque $R_1$ représente un radical de formule générale **($Z_0$)** et $R_{12}$ représente un atome d'hydrogène alors $R_2$ ne peut représenter les radicaux suivants :
  $>CH-O-CH_3$, $>CH-O-CH_2CH_3$, $>CH-O-CH_2CH_2CH_3$, $>CH-O-CH_2CH_2-O-CH_2CH_3$, $>CH-O-CH_2CH_2-O-CH_2CH_2CH_2CH_3$
- lorsque $R_1$ représente un radical de formule générale **($Z_0$)**, $R_{12}$ représente un atome d'hydrogène et $R_2$ représente un radical de formule générale **($Y_1$)** avec $n = 1$ et $R_4$ représentant un radical carbonyle alors $R_5$ ne peut représenter un radical méthyle, isopropyle, phényle,
- lorsque $R_1$ représente un radical de formule générale **($Z_0$)** et $R_{12}$ représente un atome d'hydrogène alors $R_2$ ne peut pas représenter un radical carbonyle.

**2.** Composés selon la revendication 1) de formule générale **($I_A$) :**

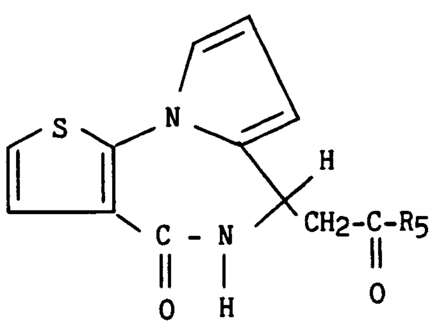

**($I_A$)**

dans laquelle $R_5$ a la signification définie dans la revendication 1),ainsi que leurs isomères, diastéréoisomères, énantiomères isolés ou sous forme de mélange.

**3.** Composés selon la revendication 1) de formule générale **($I_B$) :**

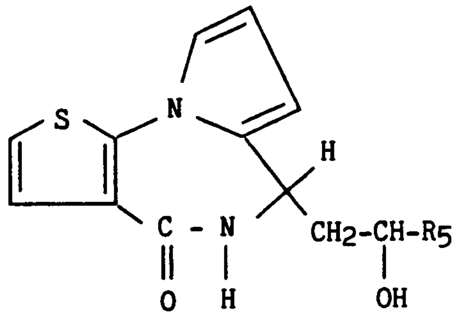

($I_B$)

dans laquelle $R_5$ a la signification définie dans la revendication 1), ainsi que leurs isomères, diastéréoisomères, énantiomères isolés ou sous forme de mélange.

**4.** Composés selon la revendication 1) de formule générale **($I_C$) :**

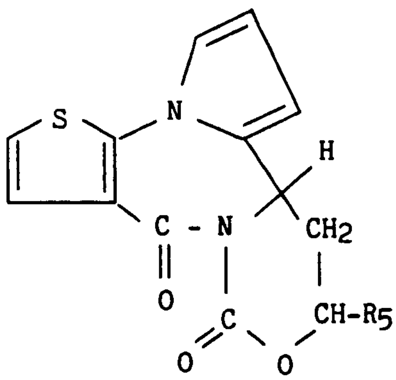

($I_C$)

dans laquelle $R_5$ a la signification définie dans la revendication 1), ainsi que leurs isomères, énantiomères, diastéréoisomères isolés ou sous forme de mélange.

**5.** Composés selon la revendication 1) de formule générale **($I_D$) :**

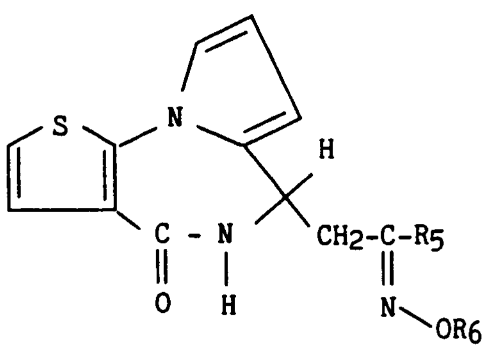

($I_D$)

dans laquelle $R_5$ et $R_6$ ont les significations définies dans la revendication 1), ainsi que leurs isomères, énantiomères, diastéréoisomères isolés ou sous forme de mélange.

**6.** Composés selon la revendication 1) de formule générale **($I_E$) :**

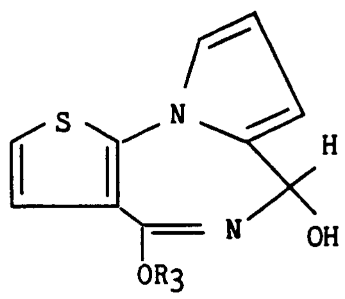

**($I_E$)**

dans laquelle $R_3$ a la signification définie dans la revendication 1), ainsi que leurs isomères, énantiomères, diastéréoisomères isolés ou sous forme de mélange.

**7.** Composés selon la revendication 1) de formule générale **($I_F$) :**

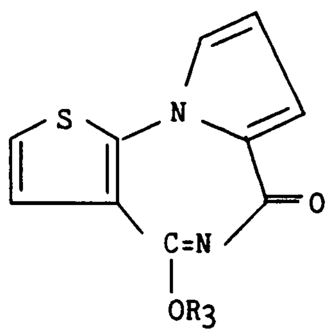

**($I_F$)**

dans laquelle $R_3$ a la signification définie dans la revendication 1), ainsi que leurs isomères, diastéréoisomères, énantiomères, isolés ou sous forme de mélange.

**8.** Composés selon la revendication 1) de formule générale **($I_G$) :**

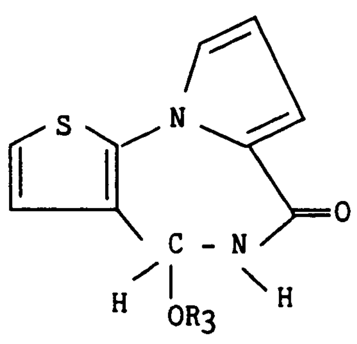

**($I_G$)**

dans laquelle $R_3$ a la signification définie dans la revendication 1), ainsi que leurs isomères, diastéréoisomères, énantiomères, isolés ou sous forme de mélange.

**9.** Composés selon la revendication 1) de formule générale **($I_H$) :**

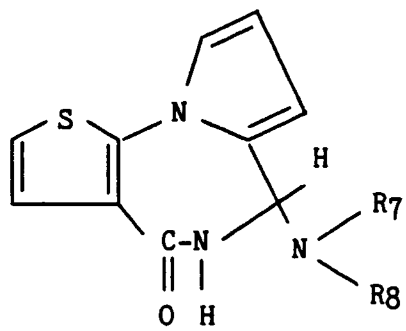

**($I_H$)**

dans laquelle $R_7$ et $R_8$ ont les significations définies dans la revendication 1), ainsi que leurs isomères, diastéréoisomères, énantiomères isolés ou sous forme de mélange et leurs éventuels sels d'addition à un acide minéral ou organique pharmaceutiquement acceptable.

**10.** Composés selon la revendication 1) de formule générale **($I_J$) :**

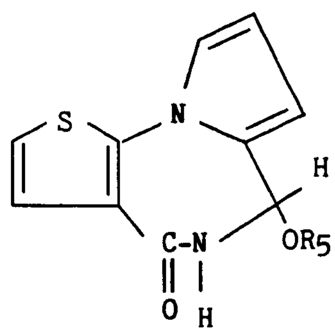

**($I_J$)**

dans laquelle $R_5$ a la signification définie dans la revendication 1), ainsi que leurs isomères, diastéréoisomères, énantiomères, isolés ou sous forme de mélange.

**11.** Composés selon la revendication 1) de formule générale **($I_K$) :**

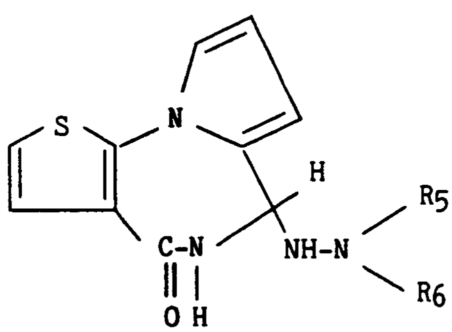

**($I_K$)**

dans laquelle $R_5$ et $R_6$ ont les significations définies dans la revendication 1),ainsi que leurs isomères, diastéréoisomères, énantiomères, isolés ou sous forme de mélange et leurs éventuels sels d'addition à un acide minéral ou organique pharmaceutiquement acceptable.

**12.** Composés selon la revendication 1) de formule générale **($I_L$) :**

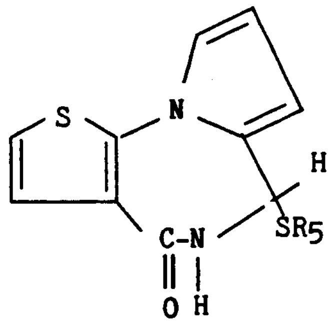

($I_L$)

dans laquelle $R_5$ a la signification définie dans la revendication 1),ainsi que leurs isomères, diastéréoisomères, énantiomères, isolés ou sous forme de mélange.

**13.** Composé selon la revendication 1) qui est le 5,6-dihydro 6-(2-benzyloxy propyl) 4-oxo 4H-pyrrolo [1,2-a] thiéno [3,2-f] [1,4]-diazépine ainsi que ses diastéréoisomères isolés ou sous forme de mélange.

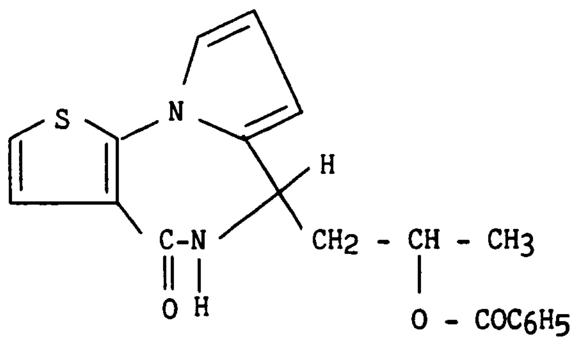

**14.** Composé selon la revendication 1) qui est le 5,6-dihydro 6-(2-hydrazono-1-pentyl)4-oxo 4H-pyrrolo [1,2-a] thiéno [3,2-f] [1,4]-diazépine ainsi que ses énantiomères isolés ou sous forme de mélange et ses sels d'addition à un acide minéral ou organique pharmaceutiquement acceptable.

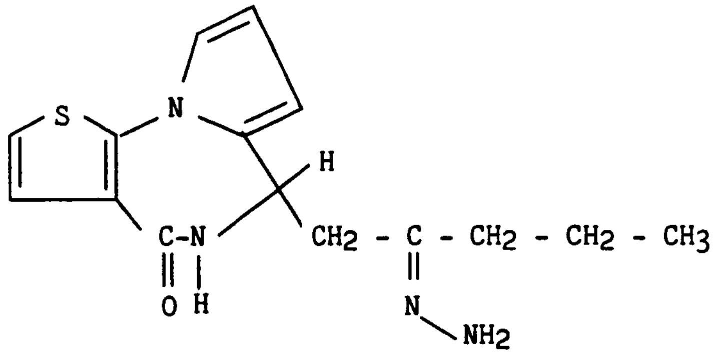

**15.** Composé selon les revendications 1) et 10) qui est le 5,6-dihydro 6-hydroxy 4-oxo 4H-pyrrolo [1,2-a] thiéno [3,2-f] [1,4]-diazépine ainsi que ses énantiomères isolés ou sous forme de mélange.

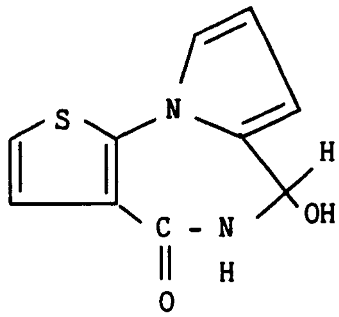

**16.** Composé selon la revendication 1) qui est le 5,6-dihydro 4-oxo 4H-pyrrolo [1,2-a] thiéno [3,2-f] [1,4]-diazépine

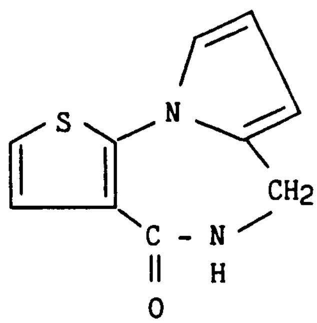

**17.** Procédé de préparation des composés de formule générale **(I),** caractérisé en ce que :

**soit**

- on fait réagir le 3-cyano 2-(2-formyl pyrrol-1-yl) thiophène, composé de formule **(II) :**

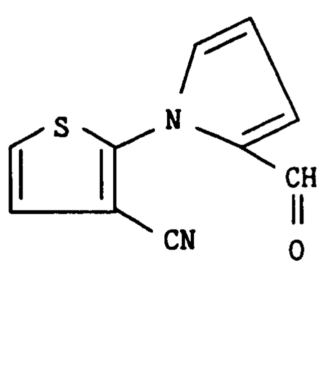

(II)

**ou**

- avec une méthylcétone de formule générale **(III) :**

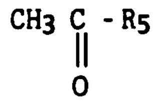

**(III)**

dans laquelle $R_5$ a la même signification que dans les dérivés de formule générale **(I),** en présence d'une base minérale forte et de péroxyde d'hydrogène, de manière à obtenir les composés de formule générale **($I_A$) :**

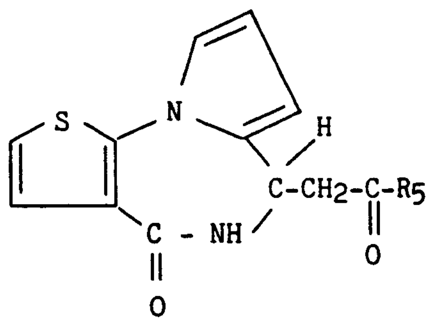

**($I_A$)**

dans laquelle $R_5$ a la même signification que dans les dérivés de formule générale **(I),**
lesquels ensuite :
soit
on soumet à l'action du borohydrure de sodium, en solution dans un solvant alcoolique de manière à obtenir les composés de formule générale **($I_B$) :**

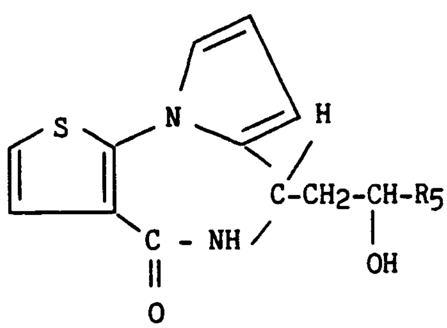

**($I_B$)**

dans laquelle $R_5$ a la même signification que dans les dérivés de formule générale **(I),** que l'on peut éventuellement soumettre à l'action du phosgène, dans un solvant organique aromatique, à chaud, pour former les composés de formule générale **($I_C$) :**

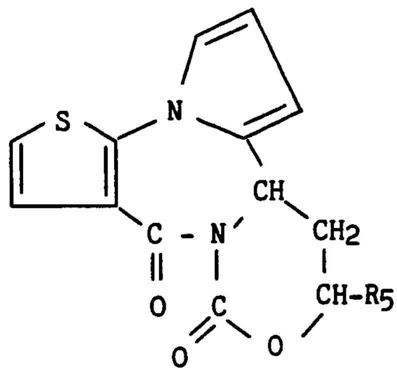

(IC)

dans laquelle $R_5$ a la même signification que dans les composés de formule générale **(I),**
ou faire réagir avec un composé de formule générale **(XI) :**

$R_{13}$ - Cl **(XI)**

dans laquelle $R_{13}$ a la même signification que dans les composés de formule générale **(I),** de manière à obtenir les composés de formule générale **($I_M$) :**

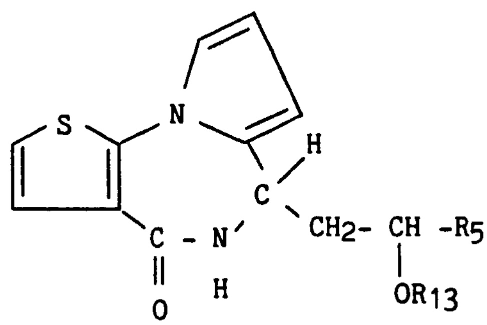

($I_M$)

dans laquelle $R_5$ et $R_{13}$ ont la même signification que dans les composés de formule générale **(I),**
soit
on condense avec un dérivé d'hydroxylamine de formule générale **(IV) :**

$H_2N$ - O - $R_6$ **(IV)**

dans laquelle $R_6$ a la même signification que dans les dérivés de formule générale **(I),** pour former les composés de formule générale **($I_D$) :**

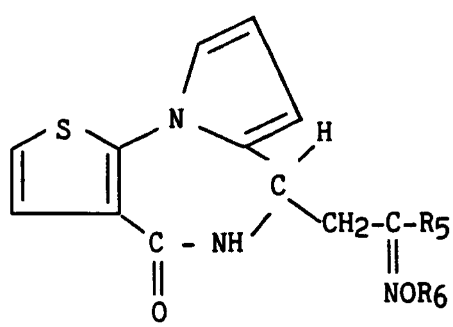

($I_D$)

dans laquelle $R_5$ et $R_6$ ont la même signification que dans les dérivés de formule générale **(I),**
soit

on condense avec un dérivé hydrazinique de formule générale **(XII) :**

$H_2N$ - NH - $R_6$ **(XII)**

dans laquelle $R_6$ a la même signification que dans les composés de formule générale **(I),** de manière à obtenir les composés de formule générale **($I_N$) :**

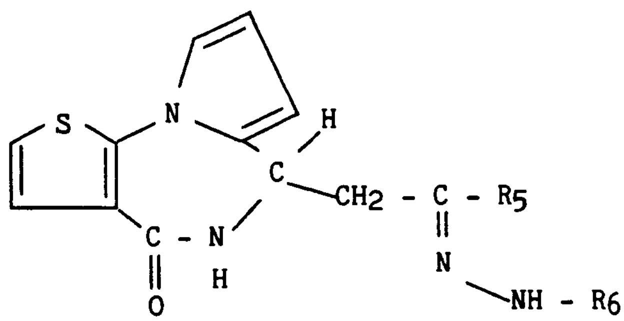

**($I_N$)**

dans laquelle $R_5$ et $R_6$ ont la même signification que dans les composés de formule générale **(I),**

**ou**

avec un alcool primaire de formule générale **(V) :**

$R_3OH$ **(V)**

dans laquelle $R_3$ a la même signification que dans les composés de formule générale **(I),**
en présence d'une base minérale forte et à une température comprise entre 30°-80°C, de manière à obtenir les composés de formule générale **($I_E$) :**

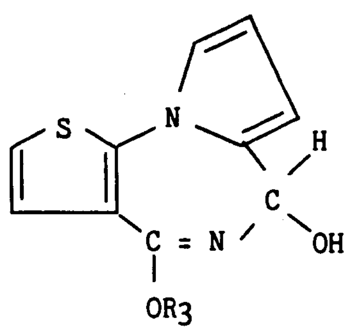

**($I_E$)**

dans laquelle $R_3$ a la même signification que dans les composés de formule générale **(I),** que l'on soumet ensuite à l'action du permanganate de potassium, à température ambiante, pour former les composés de formule générale **($I_F$),**

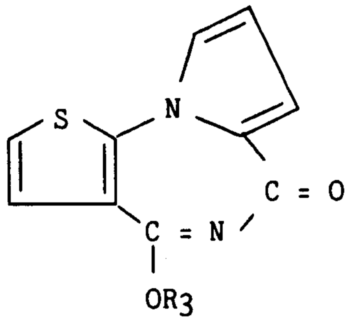

(I$_F$)

dans laquelle $R_3$ a la même signification que dans les composés de formule générale (I), que l'on peut éventuellement soumettre à l'action du borohydrure de sodium pour obtenir les composés de formule générale **(I$_G$),**

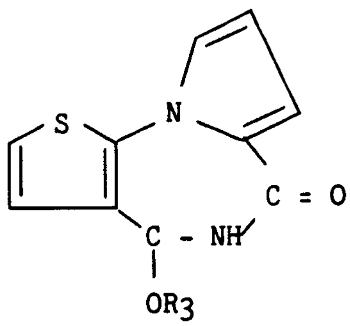

(I$_G$)

dans laquelle $R_3$ a la même signification que dans les composés de formule générale **(I),**

**soit**

on soumet le 2-(2-formyl pyrrol-1-yl) 3-thiophène carboxamide, composé de formule **(VI) :**

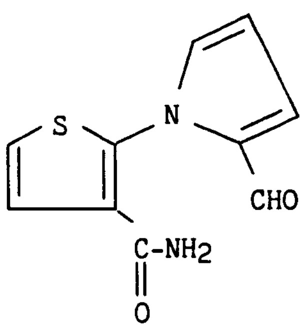

(VI)

**ou**

à l'action d'une amine de formule générale **(VII) :**

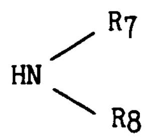

(VII)

dans laquelle $R_7$ et $R_8$ ont la même signification que dans les composés de formule générale **(I)**, de manière à obtenir les composés de formule générale **($I_H$) :**

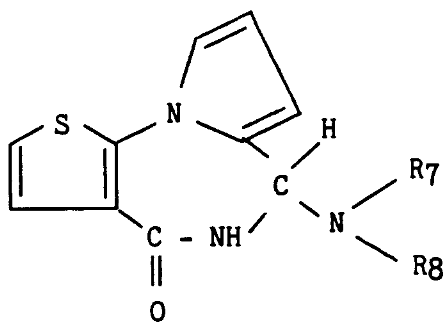

($I_H$)

dans laquelle $R_7$ et $R_8$ ont la même signification que dans les composés de formule générale **(I)**, que l'on peut éventuellement soumettre, dans le cas où $R_7$ et $R_8$ forment ensemble avec l'atome d'azote auquel ils sont attachés un radical morpholino, à l'action du borohydrure de sodium, pour obtenir le composé de formule générale **(I)** dans laquelle $R_2$ représente un radical méthylène et $R_1$ un radical de formule générale **($Z_0$)** avec $R_{12}$ = H,

**ou**

à l'action de la triéthylamine en présence d'eau et à température ambiante pour former le composé de formule générale **(I)** dans laquelle $R_2$ représente un radical hydroxyméthylène et $R_1$ un radical de formule **($Z_0$)** avec $R_{12}$ = H,

lequel ensuite,

soit

on fait réagir avec une amine de formule générale **(VII)**, pour former les composés de formule générale **($I_H$)**,

soit

on condense avec un composé de formule générale **(VIII) :**

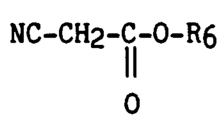

(VIII)

dans laquelle $R_6$ a la même signification que dans les composés de formule générale **(I)**, en présence de triéthylamine, pour former les composés de formule générale **($I_I$):**

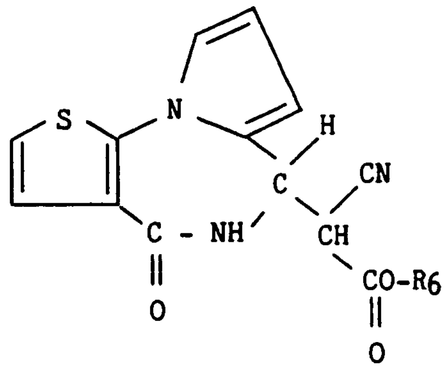

(I_I)

dans laquelle $R_6$ a la même signification que dans les composés de formule générale **(I),**
soit
on fait réagir avec un alcool de formule générale **(IX),**

$R_5OH$ **(IX)**

dans laquelle $R_5$ a la même signification que dans les composés de formule générale **(I),** à chaud, pour obtenir les composés de formule générale **($I_J$) :**

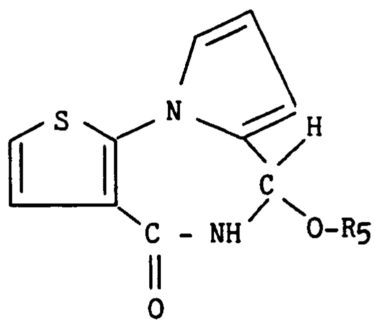

($I_J$)

dans laquelle $R_5$ a la même signification que dans les dérivés de formule générale **(I),**

**ou**

à l'action d'un composé de formule générale **(X) :**

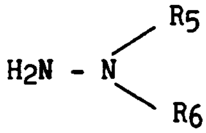

(X)

dans laquelle $R_5$ et $R_6$ ont la même signification que dans les composés de formule générale **(I),** pour former les composés de formule générale **($I_K$) :**

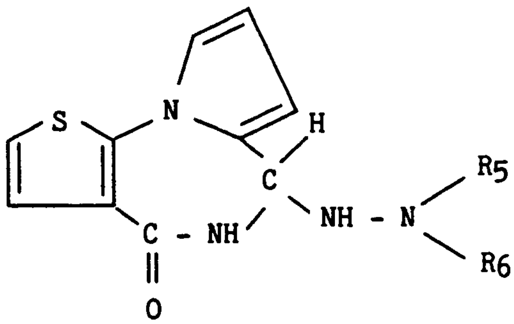

(I$_K$)

dans laquelle $R_5$ et $R_6$ ont la même signification que dans les composés de formule générale **(I),**

**ou**

à l'action d'un alcool de formule générale **(IX)** pour former les composés de formule générale **(I$_J$),**

**ou**

à l'action d'un thiol de formule générale **(XI) :**

$R_5SH$ **(XI)**

dans laquelle $R_5$ a la même signification que dans les composés de formule générale **(I),** à température ambiante, pour former les composés de formule générale **(I$_L$),**

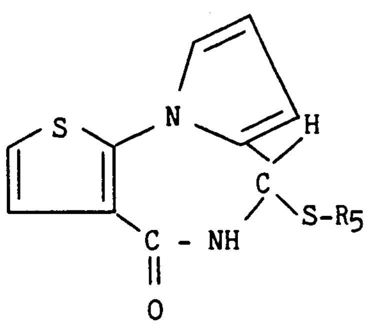

(I$_L$)

dans laquelle $R_5$ a la même signification que dans les composés de formule générale **(I),** que l'on peut éventuellement soumettre, dans le cas où $R_5$ représente un radical carboxyalkyle, à l'action de la triéthylamine et d'un chloroformiate, puis à l'action d'ammoniac gazeux pour former les amides correspondants,
étant entendu que les composés de formule générale ($I_A$), ($I_B$), ($I_C$), ($I_D$), ($I_E$), ($I_F$), ($I_G$), ($I_H$), ($I_I$), ($I_J$), ($I_K$), ($I_L$), ($I_M$) et ($I_N$) font partie de l'invention et appartiennent aux composés de formule générale **(I).**

**18.** Compositions pharmaceutiques contenant comme principe actif un composé selon les revendications 1) à 16) seul ou en combinaison avec un ou plusieurs excipients ou vehicules inertes non toxiques pharmaceutiquement acceptables.

**19.** Compositions pharmaceutiques selon la revendication 18) présentées sous une forme convenant notamment pour le traitement des accidents vasculaires cérébraux, du vieillissement cérébral normal ou pathologique et des syndromes ischémiques.

**20.** Compositions pharmaceutiques selon la revendication 18) présentées sous une forme convenant notamment pour le traitement des troubles gastro-intestinaux, du pancréas, de la vésicule biliaire, de l'appétit, du système nerveux ainsi que la douleur.

**21.** Compositions pharmaceutiques selon la revendication 18) présentées sous une forme convenant notamment pour le traitement de l'hyperlipidémie, de l'hypertriglycéridémie, de l'hypercholesterolémie, de l'hyperglycémie, de l'hypertension et des maladies qui leurs sont liées.

**Revendications pour l'Etat contractant suivant : ES**

**1.** Procédé de préparation des composés de formule générale **(I)** :

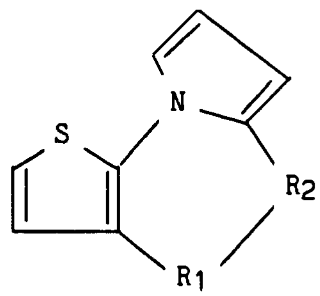

(I)

dans laquelle :

- $R_1$ représente un radical de formule générale **($Z_0$)**, **($Z_1$)**, **($Z_2$)**, **($Z_3$)** ou **($Z_4$)**

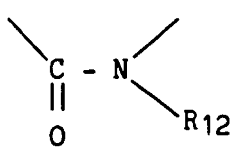

($Z_0$)

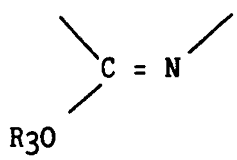

($Z_1$)

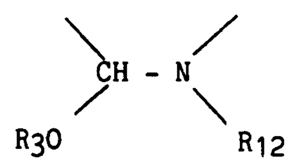

($Z_2$)

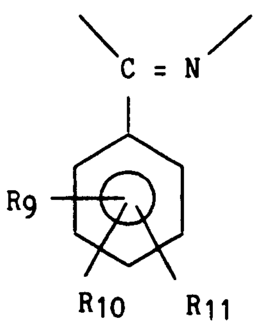

($Z_3$)

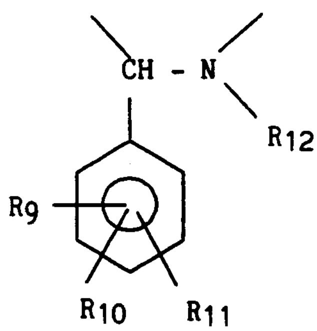

($Z_4$)

dans lesquelles :

- $R_3$ représente un atome d'hydrogène, un radical alkyle linéaire ou ramifié de 1 à 6 atomes de carbone saturé ou comportant une double liaison,
- $R_9$, $R_{10}$, $R_{11}$ identiques ou différents représentent chacun un atome d'hydrogène, un atome d'halogène ou un radical alkyle de 1 à 6 atomes de carbone,
- $R_{12}$ représente un atome d'hydrogène ou forme avec $R_2$ et l'atome d'azote auquel ils sont rattachés un radical de formule générale **(W)** :

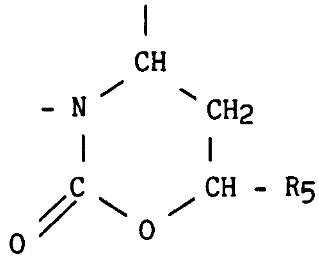

**(W)**

- $R_2$ représente un radical méthylène, un radical hydroxyméthylène, un radical carbonyle, un radical de formule générale **($Y_1$)**, **($Y_2$)**, **($Y_3$)** ou **($Y_4$) :**

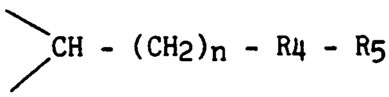

**($Y_1$)**

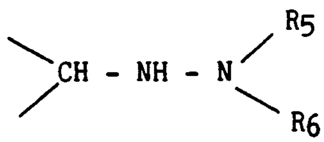

**($Y_2$)**

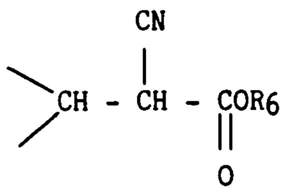

**($Y_3$)**

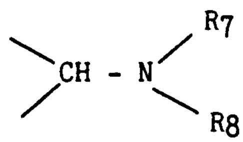

**($Y_4$)**

ou forme avec $R_{12}$ et l'atome d'azote auxquels ils sont rattachés un radical de formule générale **(W) :**

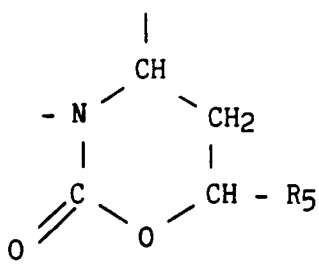

**(W)**

formules dans lesquelles:

- $R_4$ représente un atome d'oxygène, ou de soufre, un radical carbonyle, un radical de formule générale **($X_1$)**, **($X_2$)**, **($X_3$)** ou **($X_4$) :**

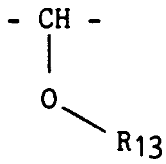 (X1)

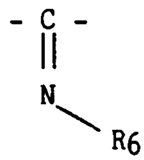 (X2)

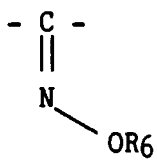 (X3)

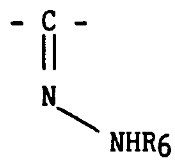 (X4)

- n est compris entre 0 et 4 inclusivement
- $R_5$ représente un atome d'hydrogène, une chaîne alkyle de 1 à 10 atomes de carbone, linéaire ou ramifiée, éventuellement interrompue par un ou plusieurs atomes d'oxygène, de soufre, un radical phényle, benzoyle ou aralkyle de 7 à 11 atomes de carbone (éventuellement substitués sur le noyau aromatique par un ou plusieurs atomes d'halogène, des radicaux alkyle de 1 à 6 atomes de carbone linéaires ou ramifiés, des radicaux nitro ou des radicaux alcoxy de 1 à 6 atomes de carbone linéaires ou ramifiés) un radical carboxyalkyle de 2 à 7 atomes de carbone linéaire ou ramifié, un radical alcoxycarbonylalkyle de 3 à 10 atomes de carbone linéaire ou ramifié, un radical alcoxycarbonyle de 2 à 7 atomes de carbone linéaire ou ramifié, un radical carbamoylalkyle de 2 à 7 atomes de carbone linéaire ou ramifié, un radical cycloalkyle de 3 à 7 atomes de carbone, un système cyclique insaturé de 5 à 7 sommets comprenant au moins un hétéroatome choisi parmi azote, soufre, oxygène, un radical pyridinylcarbonyle, pyrimidylcarbonyle, un radical Clofibroyle ou un radical 6-hydroxy 2,5,7,8-tétraméthyle chromanne-2-carbonyle,
- $R_6$ représente un atome d'hydrogène ou un radical alkyle de 1 à 6 atomes de carbone linéaire ou ramifié,
- $R_7$, $R_8$ identiques ou différents représentent chacun un atome d'hydrogène, un radical alkyle linéaire ou ramifié de 1 à 6 atomes de carbone, un radical phényle ou phénylalkyle de 7 à 9 atomes de carbone (éventuellement substitués sur le noyau aromatique par un ou plusieurs atomes d'halogène ou groupements alkyle ou alcoxy de 1 à 6 atomes de carbone), ou forment ensemble avec l'atome d'azote auquel ils sont attachés un système cyclique de 5 à 7 sommets, saturé ou insaturé, comprenant de 1 à 2 hétéroatomes choisis parmi azote, oxygène et soufre éventuellement substitué par un groupement alkylcarbonyl ou alcoxy carbonyle de 2 à 5 atomes de carbone,
- $R_{13}$ représente un atome d'hydrogène, un radical alkyl carbonyl de 2 à 6 atomes de carbone linéaire ou ramifié, un radical benzoyle,

ainsi que de leurs isomères, diastéroisomères, énantiomères et de leurs sels d'addition à un acide, minéral ou organique, pharmaceutiquement acceptable,

avec les réserves que :

- lorsque $R_1$ représente un radical de formule générale **($Z_3$)** alors $R_2$ ne peut représenter un radical méthylène,
- lorsque $R_1$ représente un radical de formule générale **($Z_0$)** et $R_{12}$ représente un atome d'hydrogène alors $R_2$ ne peut représenter les radicaux suivants :
  $>CH - O - CH_3$, $>CH - O - CH_2CH_3$, $>CH - O - CH_2CH_2CH_3$, $>CH - O - CH_2CH_2 - O - CH_2CH_3$, $>CH - O - CH_2CH_2 - O - CH_2CH_2CH_2CH_3$
- lorsque $R_1$ représente un radical de formule générale **($Z_0$)**, $R_{12}$ représente un atome d'hydrogène et $R_2$ représente un radical de formule générale **($Y_1$)** avec $n = 1$ et $R_4$ représentant un radical carbonyle alors $R_5$ ne peut représenter un radical méthyle, isopropyle, phényle,
- lorsque $R_1$ représente un radical de formule générale **($Z_0$)** et $R_{12}$ représente un atome d'hydrogène alors $R_2$ ne peut pas représenter un radical carbonyle,

caractérisé en ce que :

**soit**

- on fait réagir le 3-cyano 2-(2-formyl pyrrol-1-yl) thiophène, composé de formule **(II) :**

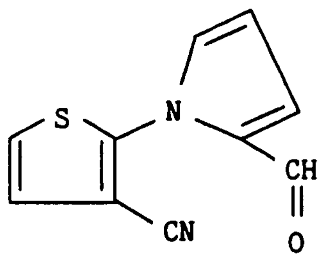

**(II)**

<u>**ou**</u>

- avec une méthylcétone de formule générale **(III) :**

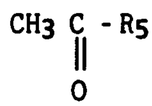

**(III)**

dans laquelle $R_5$ a la même signification que dans les dérivés de formule générale **(I),** en présence d'une base minérale forte et de péroxyde d'hydrogène, de manière à obtenir les composés de formule générale **($I_A$) :**

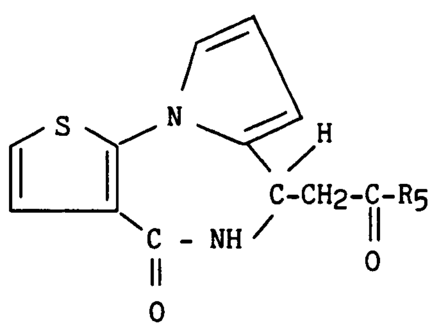

**($I_A$)**

dans laquelle $R_5$ a la même signification que dans les dérivés de formule générale **(I),**
<u>lesquels ensuite</u> :
<u>soit</u>
on soumet à l'action du borohydrure de sodium, en solution dans un solvant alcoolique de manière à obtenir les composés de formule générale **($I_B$) :**

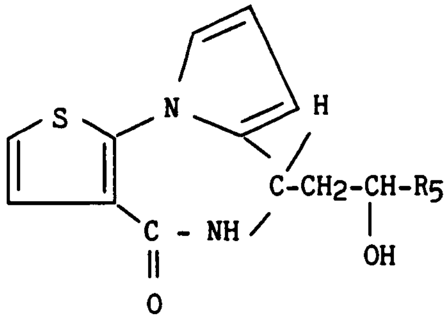

(IB)

dans laquelle $R_5$ a la même signification que dans les dérivés de formule générale **(I)**, que l'on peut éventuellement soumettre à l'action du phosgène, dans un solvant organique aromatique, à chaud, pour former les composés de formule générale **($I_C$)**:

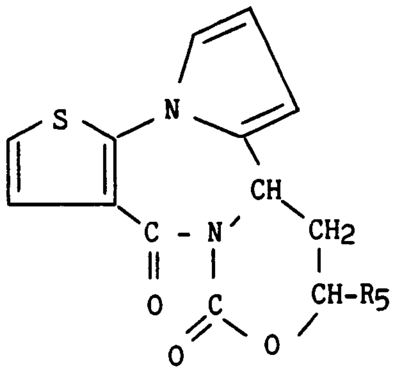

(IC)

dans laquelle $R_5$ a la même signification que dans les composés de formule générale **(I)**,
ou faire réagir avec un composé de formule générale **(XI)** :

$R_{13}$ - Cl **(XI)**

dans laquelle $R_{13}$ a la même signification que dans les composés de formule générale **(I)**, de manière à obtenir les composés de formule générale **($I_M$)** :

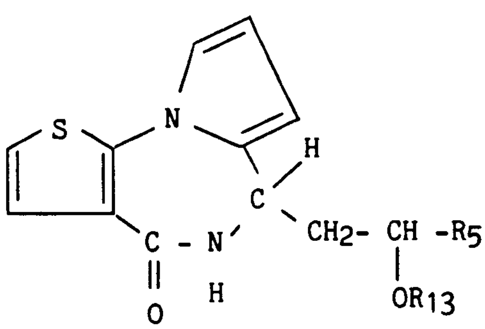

(IM)

dans laquelle $R_5$ et $R_{13}$ ont la même signification que dans les composés de formule générale **(I)**,

soit

on condense avec un dérivé d'hydroxylamine de formule générale **(IV)** :

$H_2N$ - O - $R_6$ **(IV)**

dans laquelle $R_6$ a la même signification que dans les dérivés de formule générale **(I),** pour former les composés de formule générale **($I_D$) :**

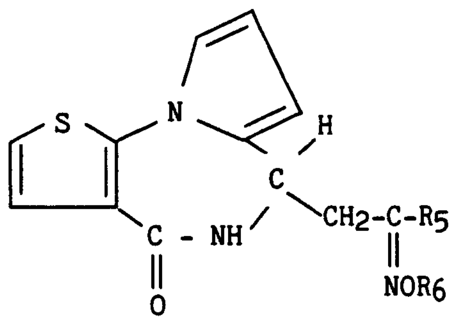

**($I_D$)**

dans laquelle $R_5$ et $R_6$ ont la même signification que dans les dérivés de formule générale **(I),**

soit

on condense avec un dérivé hydrazinique de formule générale **(XII) :**

$H_2N - NH - R_6$ **(XII)**

dans laquelle $R_6$ a la même signification que dans les composés de formule générale **(I),** de manière à obtenir les composés de formule générale **($I_N$) :**

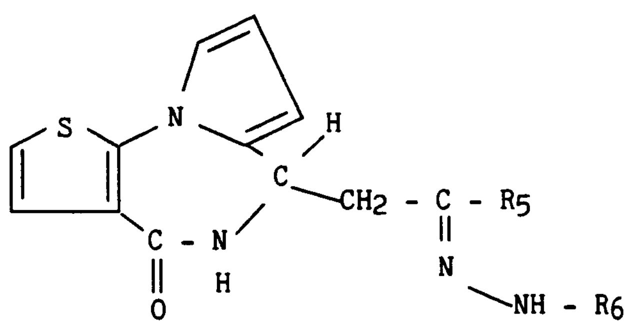

**($I_N$)**

dans laquelle $R_5$ et $R_6$ ont la même signification que dans les composés de formule générale **(I),**

**ou**

avec un alcool primaire de formule générale **(V) :**

$R_3OH$ **(V)**

dans laquelle $R_3$ a la même signification que dans les composés de formule générale **(I),**

en présence d'une base minérale forte et à une température comprise entre 30°-80°C, de manière à obtenir les composés de formule générale **($I_E$) :**

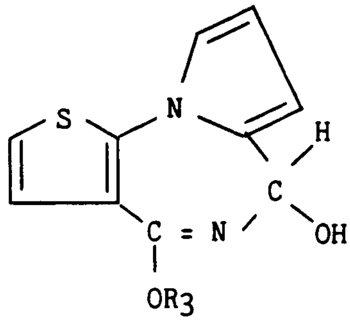

$(I_E)$

dans laquelle $R_3$ a la même signification que dans les composés de formule générale **(I),** que l'on soumet ensuite à l'action du permanganate de potassium, à température ambiante, pour former les composés de formule générale **($I_F$),**

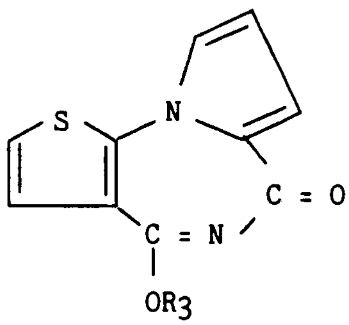

$(I_F)$

dans laquelle $R_3$ a la même signification que dans les composés de formule générale **(I),** que l'on peut éventuellement soumettre à l'action du borohydrure de sodium pour obtenir les composés de formule générale **($I_G$),**

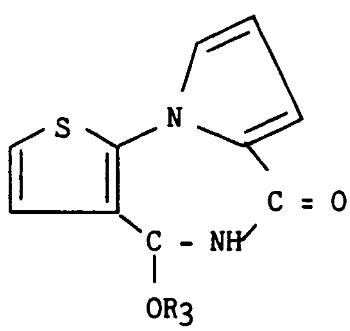

$(I_G)$

dans laquelle $R_3$ a la même signification que dans les composés de formule générale **(I),**

**soit**

on soumet le 2-(2-formyl pyrrol-1-yl) 3-thiophène carboxamide, composé de formule **(VI) :**

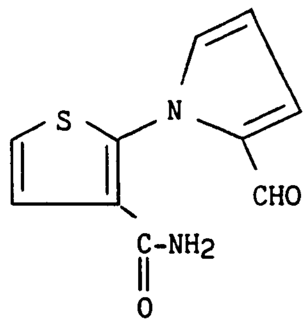

(VI)

ou

à l'action d'une amine de formule générale **(VII) :**

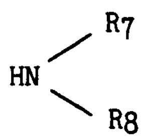

(VII)

dans laquelle $R_7$ et $R_8$ ont la même signification que dans les composés de formule générale **(I),** de manière à obtenir les composés de formule générale **($I_H$) :**

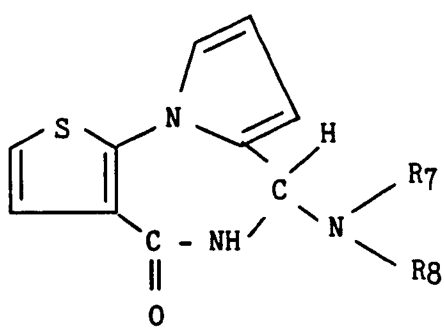

($I_H$)

dans laquelle $R_7$ et $R_8$ ont la même signification que dans les composés de formule générale **(I),** que l'on peut éventuellement soumettre, dans le cas où $R_7$ et $R_8$ forment ensemble avec l'atome d'azote auquel ils sont attachés un radical morpholino, à l'action du borohydrure de sodium, pour obtenir le composé de formule générale **(I)** dans laquelle $R_2$ représente un radical méthylène et $R_1$ un radical de formule générale **($Z_0$)** avec $R_{12}$ = H,

ou

à l'action de la triéthylamine en présence d'eau et à température ambiante pour former le composé de formule générale **(I)** dans laquelle $R_2$ représente un radical hydroxyméthylène et $R_1$ un radical de formule **($Z_0$)** avec $R_{12}$ = H,

lequel ensuite,

soit

on fait réagir avec une amine de formule générale **(VII),** pour former les composés de formule générale **($I_H$),**

soit

on condense avec un composé de formule générale **(VIII) :**

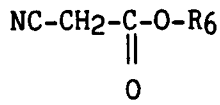

**(VIII)**

dans laquelle $R_6$ a la même signification que dans les composés de formule générale **(I)**, en présence de triéthylamine, pour former les composés de formule générale **($I_I$)** :

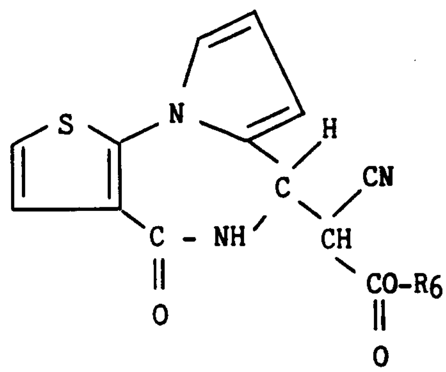

**($I_I$)**

dans laquelle $R_6$ a la même signification que dans les composés de formule générale **(I),**

soit

on fait réagir avec un alcool de formule générale **(IX),**

$R_5OH$ **(IX)**

dans laquelle $R_5$ a la même signification que dans les composés de formule générale **(I),** à chaud, pour obtenir les composés de formule générale **($I_J$)** :

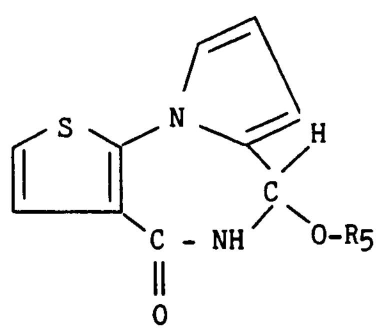

**($I_J$)**

dans laquelle $R_5$ a la même signification que dans les dérivés de formule générale **(I),**

**ou**

à l'action d'un composé de formule générale **(X)** :

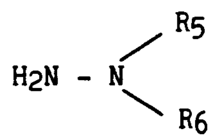

(X)

dans laquelle $R_5$ et $R_6$ ont la même signification que dans les composés de formule générale **(I)**, pour former les composés de formule générale **($I_K$) :**

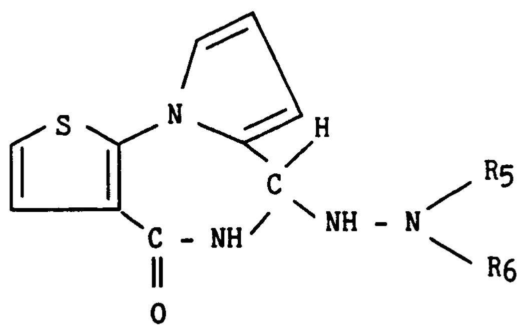

($I_K$)

dans laquelle $R_5$ et $R_6$ ont la même signification que dans les composés de formule générale **(I)**,

**<u>ou</u>**

à l'action d'un alcool de formule générale **(IX)** pour former les composés de formule générale **($I_J$)**,

**<u>ou</u>**

à l'action d'un thiol de formule générale **(XI) :**

$R_5SH$ **(XI)**

dans laquelle $R_5$ a la même signification que dans les composés de formule générale **(I)**, à température ambiante, pour former les composés de formule générale **($I_L$)**,

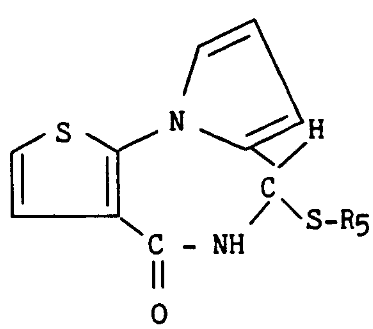

($I_L$)

dans laquelle $R_5$ a la même signification que dans les composés de formule générale **(I)**, que l'on peut éventuellement soumettre, dans le cas où $R_5$ représente un radical carboxyalkyle, à l'action de la triéthylamine et d'un chloroformiate, puis à l'action d'ammoniac gazeux pour former les amides correspondants.

**2.** Procédé de préparation selon la revendication 1) de composés de formule générale **($I_A$)** :

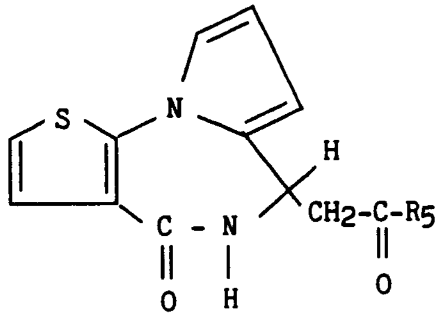

**($I_A$)**

dans laquelle $R_5$ a la signification définie dans la revendication 1),ainsi que de leurs isomères, diastéréoisomères, énantiomères isolés ou sous forme de mélange.

**3.** Procédé de préparation selon la revendication 1) des composés de formule générale **($I_B$)** :

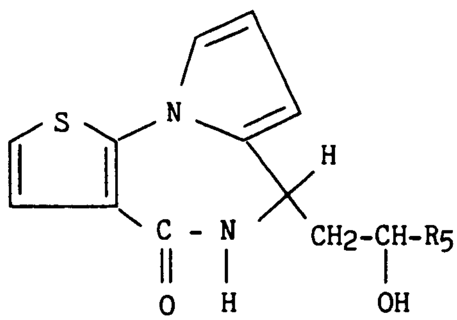

**($I_B$)**

dans laquelle $R_5$ a la signification définie dans la revendication 1), ainsi que de leurs isomères, diastéréoisomères, énantiomères isolés ou sous forme de mélange.

**4.** Procédé de préparation selon la revendication 1) des composés de formule générale **($I_C$)** :

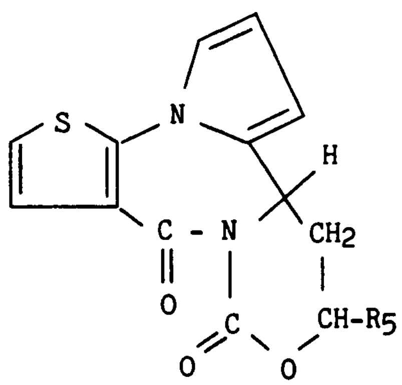

**($I_C$)**

dans laquelle $R_5$ a la signification définie dans la revendication 1), ainsi que de leurs isomères, énantiomères, diastéréoisomères isolés ou sous forme de mélange.

**5.** Procédé de préparation selon la revendication 1) des composés de formule générale **($I_D$) :**

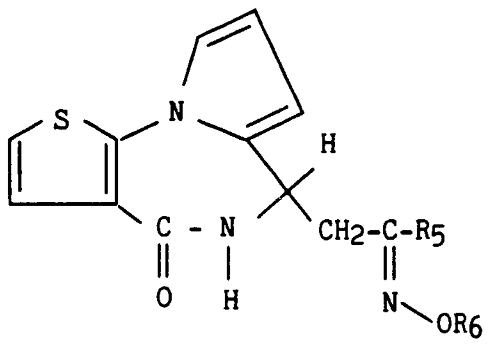

**($I_D$)**

dans laquelle $R_5$ et $R_6$ ont les significations définies dans la revendication 1), ainsi que de leurs isomères, énantiomères, diastéréoisomères isolés ou sous forme de mélange.

**6.** Procédé de préparation selon la revendication 1) des composés de formule générale **($I_E$) :**

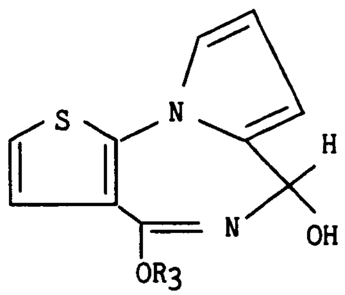

**($I_E$)**

dans laquelle $R_3$ a la signification définie dans la revendication 1), ainsi que de leurs isomères, énantiomères, diastéréoisomères isolés ou sous forme de mélange.

**7.** Procédé de préparation selon la revendication 1) des composés de formule générale **($I_F$) :**

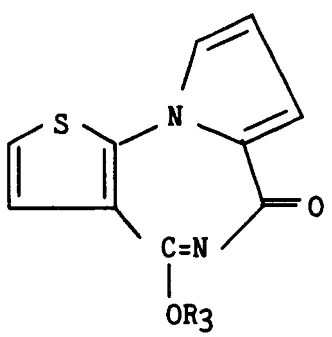

**($I_F$)**

dans laquelle $R_3$ a la signification définie dans la revendication 1), ainsi que de leurs isomères, diastéréoisomères, énantiomères, isolés ou sous forme de mélange.

**8.** Procédé de préparation selon la revendication 1) des composés de formule générale **($I_G$) :**

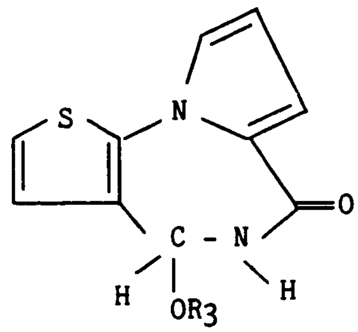

**($I_G$)**

dans laquelle $R_3$ a la signification définie dans la revendication 1), ainsi que de leurs isomères, diastéréoisomères, énantiomères, isolés ou sous forme de mélange.

**9.** Procédé de préparation selon la revendication 1) des composés de formule générale **($I_H$) :**

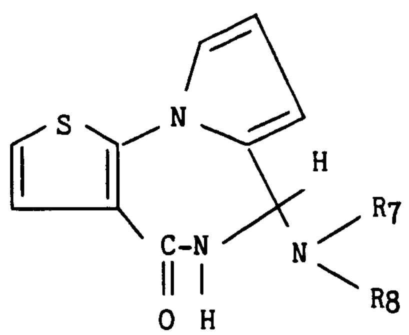

**($I_H$)**

dans laquelle $R_7$ et $R_8$ ont les significations définies dans la revendication 1), ainsi que de leurs isomères, diastéréoisomères, énantiomères isolés ou sous forme de mélange et de leurs éventuels sels d'addition à un acide minéral ou organique pharmaceutiquement acceptable.

**10.** Procédé de préparation selon la revendication 1) des composés de formule générale **($I_J$) :**

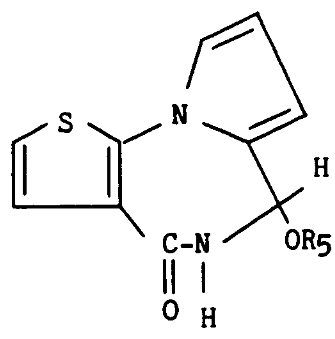

**($I_J$)**

dans laquelle $R_5$ a la signification définie dans la revendication 1), ainsi que de leurs isomères, diastéréoisomères, énantiomères, isolés ou sous forme de mélange.

**11.** Procédé de préparation selon la revendication 1) des composés de formule générale **($I_K$) :**

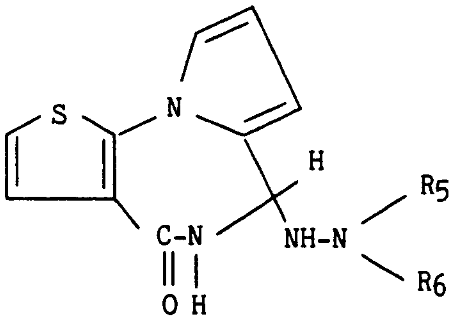

($I_K$)

dans laquelle $R_5$ et $R_6$ ont les significations définies dans la revendication 1),ainsi que de leurs isomères, diastéréoisomères, énantiomères, isolés ou sous forme de mélange et de leurs éventuels sels d'addition à un acide minéral ou organique pharmaceutiquement acceptable.

**12.** Procédé de préparation selon la revendication 1) des composés de formule générale **($I_L$) :**

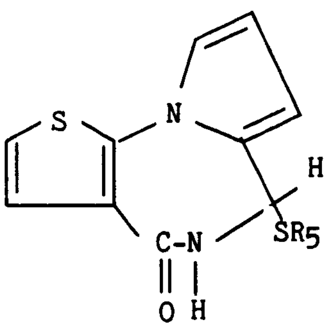

($I_L$)

dans laquelle $R_5$ a la signification définie dans la revendication 1),ainsi que de leurs isomères, diastéréoisomères, énantiomères, isolés ou sous forme de mélange.

**13.** Procédé de préparation selon la revendication 1) du composé qui est le 5,6-dihydro 6-(2-benzyloxy propyl) 4-oxo 4H-pyrrolo [1,2-a] thiéno [3,2-f] [1,4]-diazépine ainsi que de ses diastéréoisomères isolés ou sous forme de mélange.

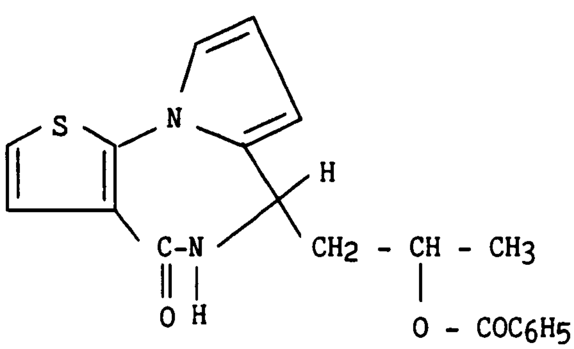

**14.** Procédé de préparation selon la revendication 1) du composé qui est le 5,6-dihydro 6-(2-hydrazono-1-pentyl)4-oxo 4H-pyrrolo [1,2-a] thiéno [3,2-f] [1,4]-diazépine ainsi que de ses énantiomères isolés ou sous forme de mélange et de ses sels d'addition à un acide minéral ou organique pharmaceutiquement acceptable.

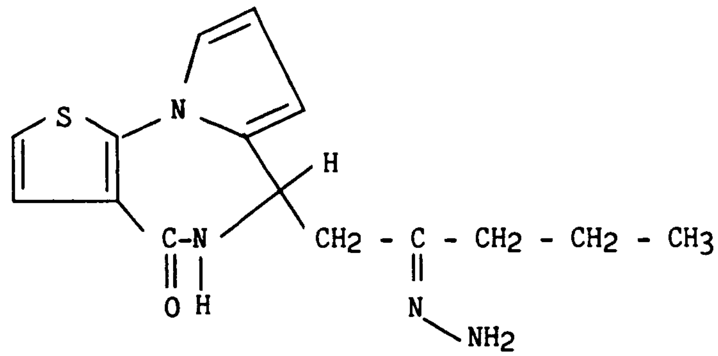

15. Procédé de préparation selon les revendications 1) et 10) du composé qui est le 5,6-dihydro 6-hydroxy 4-oxo 4H-pyrrolo [1,2-a] thiéno [3,2-f] [1,4]-diazépine ainsi que de ses énantiomères isolés ou sous forme de mélange.

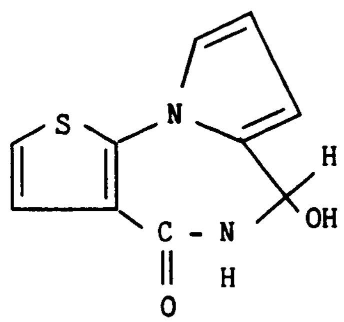

16. Procédé de préparation selon la revendication 1) du composé qui est le 5,6-dihydro 4-oxo 4H-pyrrolo [1,2-a] thiéno [3,2-f] [1,4]-diazépine

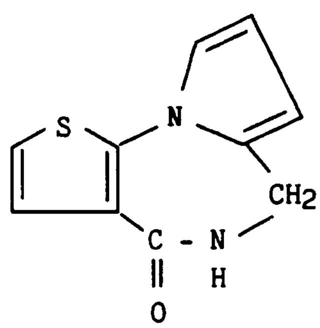

17. Procédé de préparation de compositions pharmaceutiques contenant comme principe actif un composé préparé selon les revendications 1) à 16) seul ou en combinaison avec un ou plusieurs excipients ou vehicules inertes non toxiques pharmaceutiquement acceptables.

18. Procédé de préparation selon la revendication 17) de compositions pharmaceutiques présentées sous une forme convenant notamment pour le traitement des accidents vasculaires cérébraux, du vieillissement cérébral normal ou pathologique et des syndromes ischémiques.

**19.** Procédé de préparation selon la revendication 17) de compositions pharmaceutiques présentées sous une forme convenant notamment pour le traitement des troubles gastro-intestinaux, du pancréas, de la vésicule biliaire, de l'appétit, du système nerveux ainsi que la douleur.

**20.** Procédé de préparation selon la revendication 17) de compositions pharmaceutiques présentées sous une forme convenant notamment pour le traitement de l'hyperlipidémie, de l'hypertriglycéridémie, de l'hypercholesterolémie, de l'hyperglycémie, de l'hypertension et des maladies qui leurs sont liées.

**Revendications pour l'Etat contractant suivant : GR**

**1.** Procédé de préparation des composés de formule générale **(I) :**

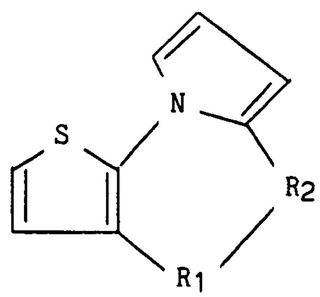

(I)

dans laquelle :

- $R_1$ représente un radical de formule générale **($Z_0$), ($Z_1$), ($Z_2$), ($Z_3$)** ou **($Z_4$)**

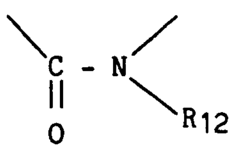

($Z_0$)

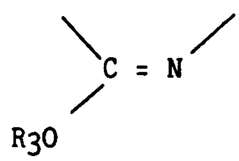

($Z_1$)

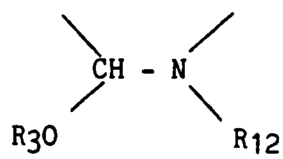

($Z_2$)

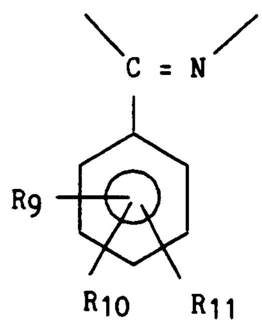

($Z_3$)

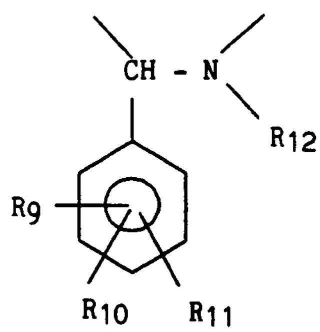

($Z_4$)

dans lesquelles :

- $R_3$ représente un atome d'hydrogène, un radical alkyle linéaire ou ramifié de 1 à 6 atomes de carbone saturé ou comportant une double liaison,
- $R_9$, $R_{10}$, $R_{11}$ identiques ou différents représentent chacun un atome d'hydrogène, un atome d'halogène ou un radical alkyle de 1 à 6 atomes de carbone,
- $R_{12}$ représente un atome d'hydrogène ou forme avec $R_2$ et l'atome d'azote auquel ils sont rattachés un radical de formule générale **(W) :**

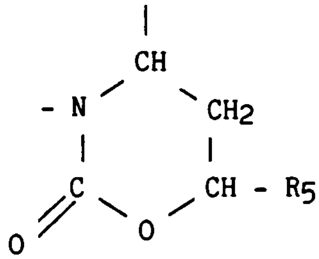

(W)

- $R_2$ représente un radical méthylène, un radical hydroxyméthylène, un radical carbonyle, un radical de formule générale **($Y_1$), ($Y_2$), ($Y_3$)** ou **($Y_4$) :**

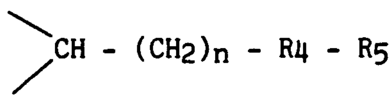

($Y_1$)

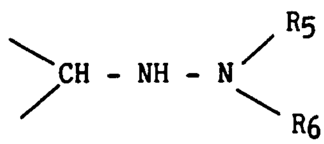

($Y_2$)

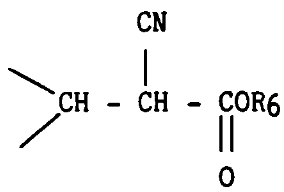

($Y_3$)

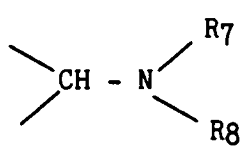

($Y_4$)

ou forme avec $R_{12}$ et l'atome d'azote auxquels ils sont rattachés un radical de formule générale **(W) :**

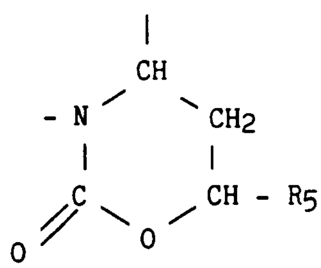

(W)

formules dans lesquelles :

- $R_4$ représente un atome d'oxygène, ou de soufre, un radical carbonyle, un radical de formule générale **($X_1$), ($X_2$), ($X_3$)** ou **($X_4$) :**

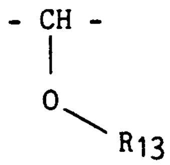 (X1)

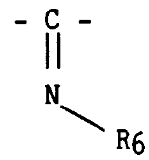 (X2)

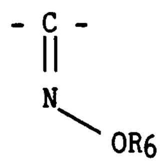 (X3)

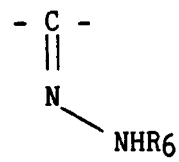 (X4)

- n est compris entre 0 et 4 inclusivement
- $R_5$ représente un atome d'hydrogène, une chaîne alkyle de 1 à 10 atomes de carbone, linéaire ou ramifiée, éventuellement interrompue par un ou plusieurs atomes d'oxygène, de soufre, un radical phényle, benzoyle ou aralkyle de 7 à 11 atomes de carbone (éventuellement substitués sur le noyau aromatique par un ou plusieurs atomes d'halogène, des radicaux alkyle de 1 à 6 atomes de carbone linéaires ou ramifiés, des radicaux nitro ou des radicaux alcoxy de 1 à 6 atomes de carbone linéaires ou ramifiés) un radical carboxyalkyle de 2 à 7 atomes de carbone linéaire ou ramifié, un radical alcoxycarbonylalkyle de 3 à 10 atomes de carbone linéaire ou ramifié, un radical alcoxycarbonyle de 2 à 7 atomes de carbone linéaire ou ramifié, un radical carbamoylalkyle de 2 à 7 atomes de carbone linéaire ou ramifié, un radical cycloalkyle de 3 à 7 atomes de carbone, un système cyclique insaturé de 5 à 7 sommets comprenant au moins un hétéroatome choisi parmi azote, soufre, oxygène, un radical pyridinylcarbonyle, pyrimidylcarbonyle, un radical Clofibroyle ou un radical 6-hydroxy 2,5,7,8-tétraméthyle chromanne-2-carbonyle,
- $R_6$ représente un atome d'hydrogène ou un radical alkyle de 1 à 6 atomes de carbone linéaire ou ramifié,
- $R_7$, $R_8$ identiques ou différents représentent chacun un atome d'hydrogène, un radical alkyle linéaire ou ramifié de 1 à 6 atomes de carbone, un radical phényle ou phénylalkyle de 7 à 9 atomes de carbone (éventuellement substitués sur le noyau aromatique par un ou plusieurs atomes d'halogène ou groupements alkyle ou alcoxy de 1 à 6 atomes de carbone), ou forment ensemble avec l'atome d'azote auquel ils sont attachés un système cyclique de 5 à 7 sommets, saturé ou insaturé, comprenant de 1 à 2 hétéroatomes choisis parmi azote, oxygène et soufre éventuellement substitué par un groupement alkylcarbonyl ou alcoxy carbonyle de 2 à 5 atomes de carbone,
- $R_{13}$ représente un atome d'hydrogène, un radical alkyl carbonyl de 2 à 6 atomes de carbone linéaire ou ramifié, un radical benzoyle,

ainsi que de leurs isomères, diastéroisomères, énantiomères et de leurs sels d'addition à un acide, minéral ou organique, pharmaceutiquement acceptable,

avec les réserves que :

- lorsque $R_1$ représente un radical de formule générale **($Z_3$)** alors $R_2$ ne peut représenter un radical méthylène,
- lorsque $R_1$ représente un radical de formule générale ($Z_0$) et $R_{12}$ représente un atome d'hydrogène alors $R_2$ ne peut représenter les radicaux suivants :
  $>CH - O - CH_3$, $>CH - O - CH_2CH_3$, $>CH - O - CH_2CH_2CH_3$, $>CH - O - CH_2CH_2 - O - CH_2CH_3$, $>CH - O - CH_2CH_2 - O - CH_2CH_2CH_2CH_3$
- lorsque $R_1$ représente un radical de formule générale ($Z_0$), $R_{12}$ représente un atome d'hydrogène et $R_2$ représente un radical de formule générale **($Y_1$)** avec n = 1 et $R_4$ représentant un radical carbonyle alors $R_5$ ne peut représenter un radical méthyle, isopropyle, phényle,
- lorsque $R_1$ représente un radical de formule générale **($Z_0$)** et $R_{12}$ représente un atome d'hydrogène alors $R_2$ ne peut pas représenter un radical carbonyle,

caractérisé en ce que :

**soit**

- on fait réagir le 3-cyano 2-(2-formyl pyrrol-1-yl) thiophène, composé de formule **(II)** :

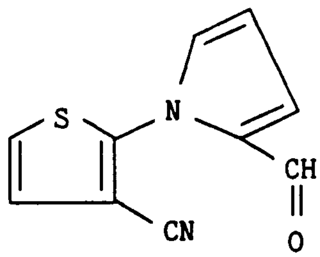

**(II)**

**ou**

- avec une méthylcétone de formule générale **(III)** :

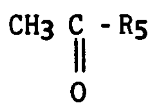

**(III)**

dans laquelle $R_5$ a la même signification que dans les dérivés de formule générale **(I)**, en présence d'une base minérale forte et de péroxyde d'hydrogène, de manière à obtenir les composés de formule générale **($I_A$)** :

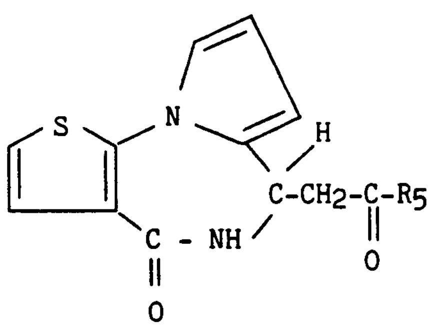

**($I_A$)**

dans laquelle $R_5$ a la même signification que dans les dérivés de formule générale **(I)**,

lesquels ensuite :

soit

on soumet à l'action du borohydrure de sodium, en solution dans un solvant alcoolique de manière à obtenir les composés de formule générale **($I_B$)** :

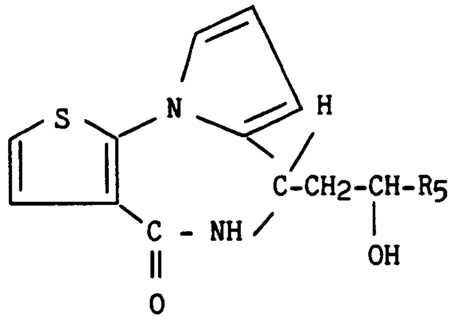

$(I_B)$

dans laquelle $R_5$ a la même signification que dans les dérivés de formule générale **(I),** que l'on peut éventuellement soumettre à l'action du phosgène, dans un solvant organique aromatique, à chaud, pour former les composés de formule générale **($I_C$):**

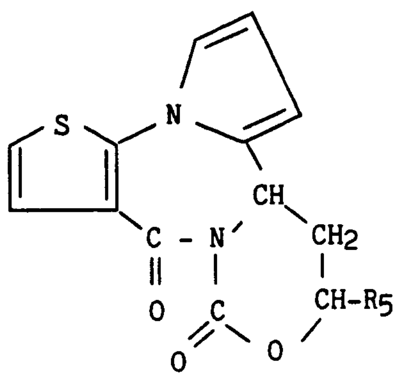

$(I_C)$

dans laquelle $R_5$ a la même signification que dans les composés de formule générale **(I),**
ou faire réagir avec un composé de formule générale **(XI) :**

$R_{13}$ - Cl (XI)

dans laquelle $R_{13}$ a la même signification que dans les composés de formule générale **(I),** de manière à obtenir les composés de formule générale **($I_M$) :**

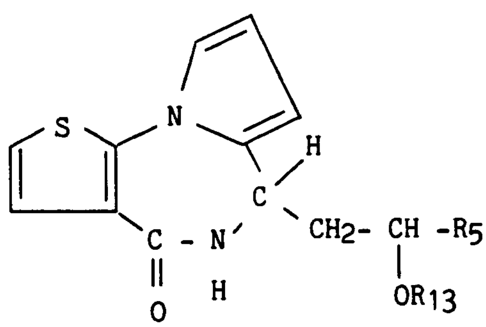

$(I_M)$

dans laquelle $R_5$ et $R_{13}$ ont la même signification que dans les composés de formule générale **(I),**
soit
on condense avec un dérivé d'hydroxylamine de formule générale **(IV) :**

$H_2N$ - O - $R_6$ (IV)

dans laquelle $R_6$ a la même signification que dans les dérivés de formule générale **(I),** pour former les composés de formule générale **($I_D$) :**

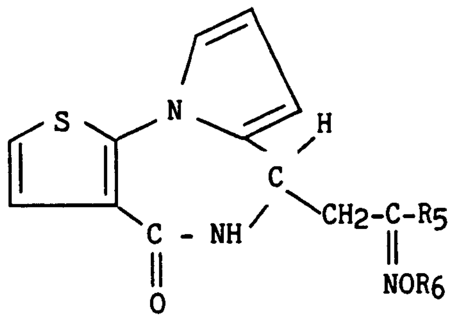

**($I_D$)**

dans laquelle $R_5$ et $R_6$ ont la même signification que dans les dérivés de formule générale **(I),**

soit

on condense avec un dérivé hydrazinique de formule générale **(XII) :**

$H_2N$ - NH - $R_6$ (XII)

dans laquelle $R_6$ a la même signification que dans les composés de formule générale **(I),** de manière à obtenir les composés de formule générale **($I_N$) :**

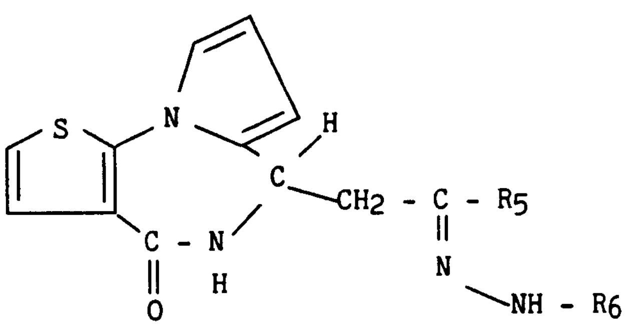

**($I_N$)**

dans laquelle $R_5$ et $R_6$ ont la même signification que dans les composés de formule générale **(I),**

**ou**

avec un alcool primaire de formule générale **(V) :**

$R_3OH$ (V)

dans laquelle $R_3$ a la même signification que dans les composés de formule générale **(I),**

en présence d'une base minérale forte et à une température comprise entre 30°-80°C, de manière à obtenir les composés de formule générale **($I_E$) :**

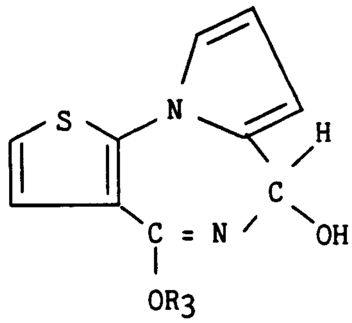

(IE)

dans laquelle $R_3$ a la même signification que dans les composés de formule générale **(I),** que l'on soumet ensuite à l'action du permanganate de potassium, à température ambiante, pour former les composés de formule générale **($I_F$),**

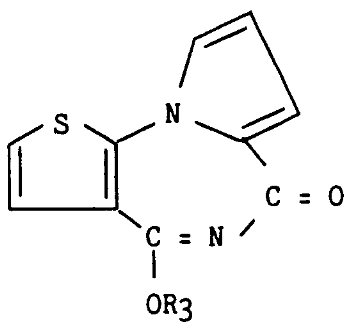

(IF)

dans laquelle $R_3$ a la même signification que dans les composés de formule générale **(I),** que l'on peut éventuellement soumettre à l'action du borohydrure de sodium pour obtenir les composés de formule générale **($I_G$),**

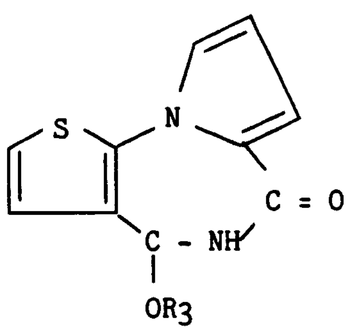

(IG)

dans laquelle $R_3$ a la même signification que dans les composés de formule générale **(I),**

**soit**

on soumet le 2-(2-formyl pyrrol-1-yl) 3-thiophène carboxamide, composé de formule **(VI) :**

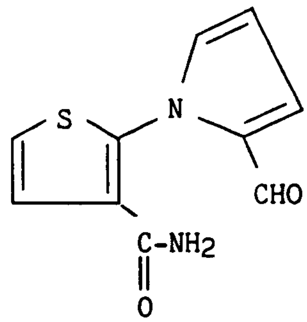

(VI)

ou

à l'action d'une amine de formule générale **(VII) :**

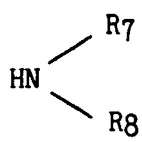

(VII)

dans laquelle $R_7$ et $R_8$ ont la même signification que dans les composés de formule générale **(I)**, de manière à obtenir les composés de formule générale **($I_H$) :**

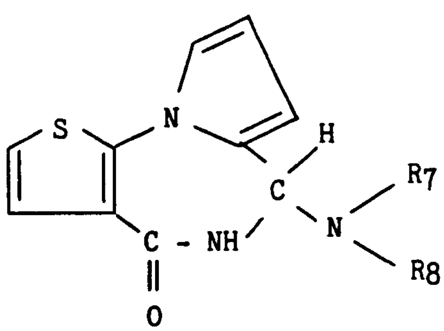

($I_H$)

dans laquelle $R_7$ et $R_8$ ont la même signification que dans les composés de formule générale **(I)**, que l'on peut éventuellement soumettre, dans le cas où $R_7$ et $R_8$ forment ensemble avec l'atome d'azote auquel ils sont attachés un radical morpholino, à l'action du borohydrure de sodium, pour obtenir le composé de formule générale **(I)** dans laquelle $R_2$ représente un radical méthylène et $R_1$ un radical de formule générale **($Z_0$)** avec $R_{12}$ = H,

**ou**

à l'action de la triéthylamine en présence d'eau et à température ambiante pour former le composé de formule générale **(I)** dans laquelle $R_2$représente un radical hydroxyméthylène et $R_1$ un radical de formule **($Z_0$)** avec $R_{12}$ = H,

lequel ensuite,

soit

on fait réagir avec une amine de formule générale **(VII)**, pour former les composés de formule générale **($I_H$)**,

soit

on condense avec un composé de formule générale **(VIII) :**

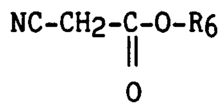

(VIII)

dans laquelle $R_6$ a la même signification que dans les composés de formule générale **(I),** en présence de triéthylamine, pour former les composés de formule générale **($I_I$):**

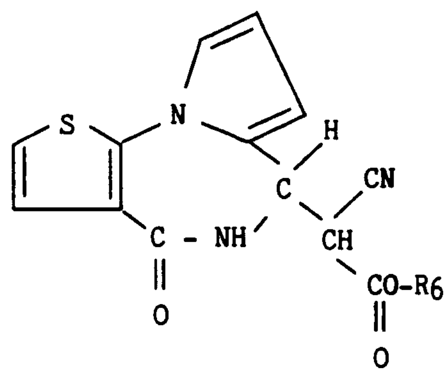

($I_I$)

dans laquelle $R_6$ a la même signification que dans les composés de formule générale **(I),**

soit

on fait réagir avec un alcool de formule générale **(IX),**

$R_5OH$ (IX)

dans laquelle $R_5$ a la même signification que dans les composés de formule générale **(I),** à chaud, pour obtenir les composés de formule générale **($I_J$) :**

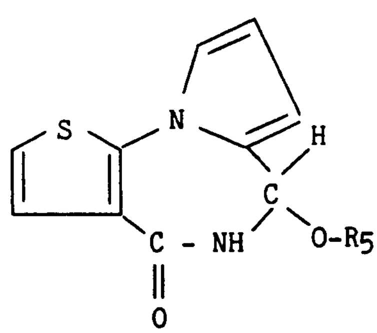

($I_J$)

dans laquelle $R_5$ a la même signification que dans les dérivés de formule générale **(I),**

**ou**

à l'action d'un composé de formule générale **(X) :**

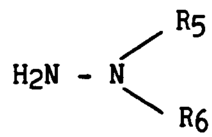

**(X)**

dans laquelle $R_5$ et $R_6$ ont la même signification que dans les composés de formule générale **(I),** pour former les composés de formule générale **($I_K$) :**

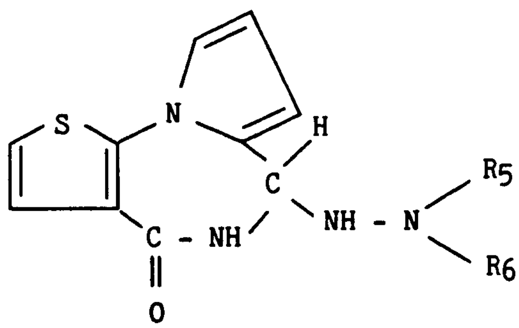

**($I_K$)**

dans laquelle $R_5$ et $R_6$ ont la même signification que dans les composés de formule générale **(I),**

**ou**

à l'action d'un alcool de formule générale **(IX)** pour former les composés de formule générale **($I_J$),**

**ou**

à l'action d'un thiol de formule générale **(XI) :**

$R_5SH$ (XI)

dans laquelle $R_5$ a la même signification que dans les composés de formule générale **(I),** à température ambiante, pour former les composés de formule générale **($I_L$),**

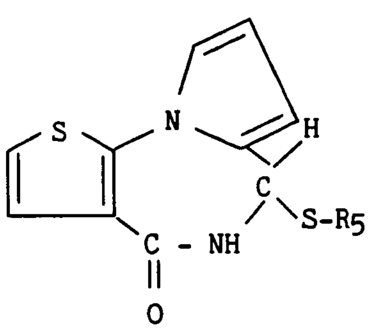

**($I_L$)**

dans laquelle $R_5$ a la même signification que dans les composés de formule générale **(I),** que l'on peut éventuellement soumettre, dans le cas où $R_5$ représente un radical carboxyalkyle, à l'action de la triéthylamine et d'un chloroformiate, puis à l'action d'ammoniac gazeux pour former les amides correspondants.

**2.** Procédé de préparation selon la revendication 1) de composés de formule générale **($I_A$) :**

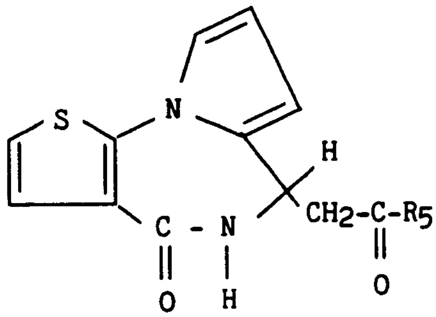

**($I_A$)**

dans laquelle $R_5$ a la signification définie dans la revendication 1),ainsi que de leurs isomères, diastéréoisomères, énantiomères isolés ou sous forme de mélange.

**3.** Procédé de préparation selon la revendication 1) des composés de formule générale **($I_B$) :**

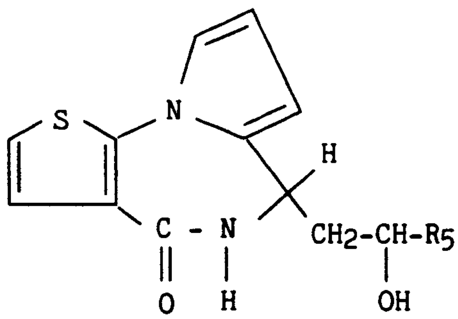

**($I_B$)**

dans laquelle $R_5$ a la signification définie dans la revendication 1), ainsi que de leurs isomères, diastéréoisomères, énantiomères isolés ou sous forme de mélange.

**4.** Procédé de préparation selon la revendication 1) des composés de formule générale **($I_C$) :**

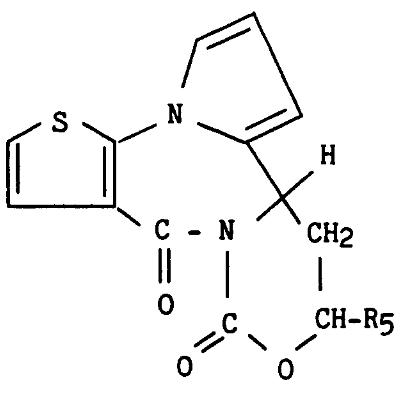

**($I_C$)**

dans laquelle $R_5$ a la signification définie dans la revendication 1), ainsi que de leurs isomères, énantiomères, diastéréoisomères isolés ou sous forme de mélange.

**5.** Procédé de préparation selon la revendication 1) des composés de formule générale **($I_D$) :**

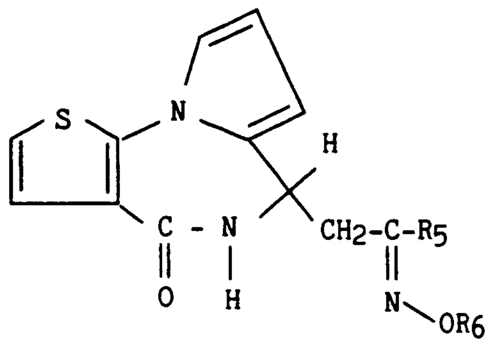

($I_D$)

dans laquelle $R_5$ et $R_6$ ont les significations définies dans la revendication 1), ainsi que de leurs isomères, énantiomères, diastéréoisomères isolés ou sous forme de mélange.

**6.** Procédé de préparation selon la revendication 1) des composés de formule générale **($I_E$) :**

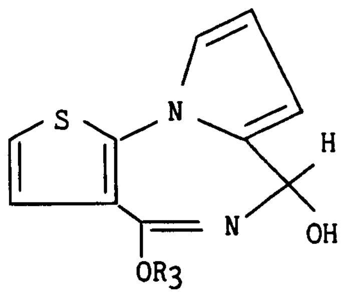

($I_E$)

dans laquelle $R_3$ a la signification définie dans la revendication 1), ainsi que de leurs isomères, énantiomères, diastéréoisomères isolés ou sous forme de mélange.

**7.** Procédé de préparation selon la revendication 1) des composés de formule générale **($I_F$) :**

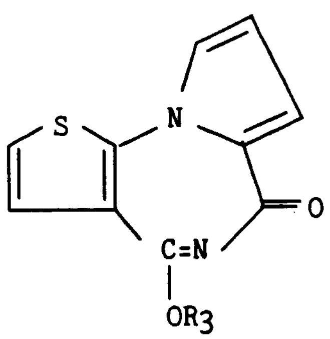

($I_F$)

dans laquelle $R_3$ a la signification définie dans la revendication 1), ainsi que de leurs isomères, diastéréoisomères, énantiomères, isolés ou sous forme de mélange.

**8.** Procédé de préparation selon la revendication 1) des composés de formule générale **($I_G$) :**

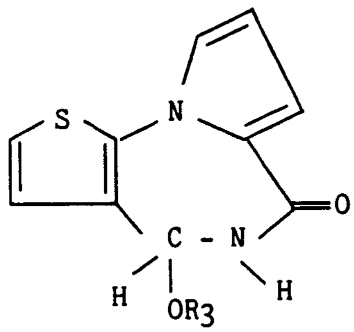

**($I_G$)**

dans laquelle $R_3$ a la signification définie dans la revendication 1), ainsi que de leurs isomères, diastéréoisomères, énantiomères, isolés ou sous forme de mélange.

**9.** Procédé de préparation selon la revendication 1) des composés de formule générale **($I_H$) :**

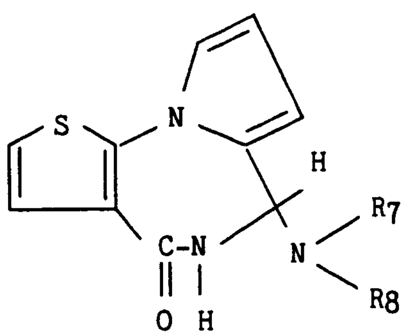

**($I_H$)**

dans laquelle $R_7$ et $R_8$ ont les significations définies dans la revendication 1), ainsi que de leurs isomères, diastéréoisomères, énantiomères isolés ou sous forme de mélange et de leurs éventuels sels d'addition à un acide minéral ou organique pharmaceutiquement acceptable.

**10.** Procédé de préparation selon la revendication 1) des composés de formule générale **($I_J$) :**

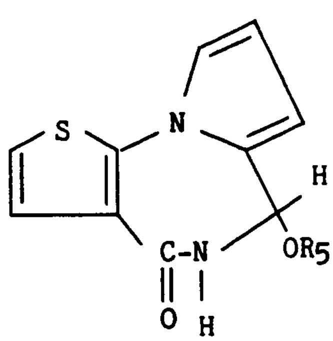

**($I_J$)**

dans laquelle $R_5$ a la signification définie dans la revendication 1), ainsi que de leurs isomères, diastéréoisomères, énantiomères, isolés ou sous forme de mélange.

**11.** Procédé de préparation selon la revendication 1) des composés de formule générale **($I_K$) :**

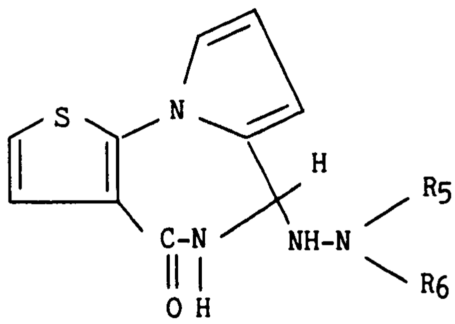

($I_K$)

dans laquelle $R_5$ et $R_6$ ont les significations définies dans la revendication 1),ainsi que de leurs isomères, diastéréoisomères, énantiomères, isolés ou sous forme de mélange et de leurs éventuels sels d'addition à un acide minéral ou organique pharmaceutiquement acceptable.

**12.** Procédé de préparation selon la revendication 1) des composés de formule générale **($I_L$) :**

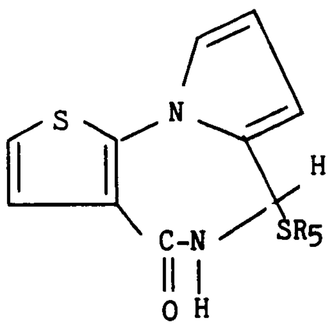

($I_L$)

dans laquelle $R_5$ a la signification définie dans la revendication 1),ainsi que de leurs isomères, diastéréoisomères, énantiomères, isolés ou sous forme de mélange.

**13.** Procédé de préparation selon la revendication 1) du composé qui est le 5,6-dihydro 6-(2-benzyloxy propyl) 4-oxo 4H-pyrrolo [1,2-a] thiéno [3,2-f] [1,4]-diazépine ainsi que de ses diastéréoisomères isolés ou sous forme de mélange.

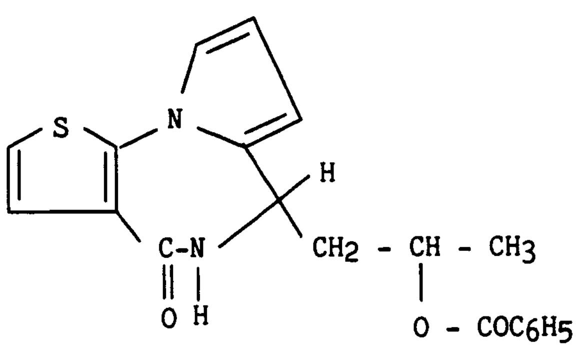

**14.** Procédé de préparation selon la revendication 1) du composé qui est le 5,6-dihydro 6-(2-hydrazono-1-pentyl)4-oxo 4H-pyrrolo [1,2-a] thiéno [3,2-f] [1,4]-diazépine ainsi que de ses énantiomères isolés ou sous forme de mélange et de ses sels d'addition à un acide minéral ou organique pharmaceutiquement acceptable.

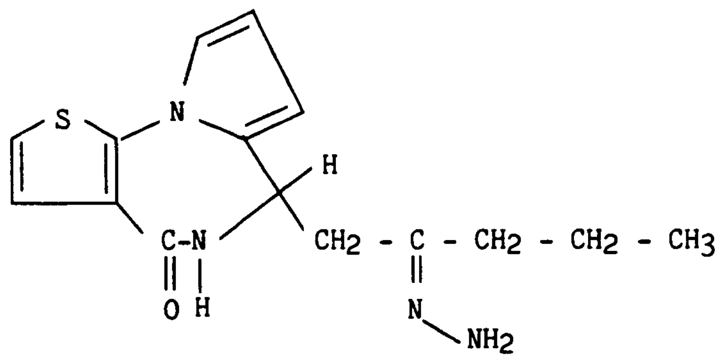

15. Procédé de préparation selon les revendications 1) et 10) du composé qui est le 5,6-dihydro 6-hydroxy 4-oxo 4H-pyrrolo [1,2-a] thiéno [3,2-f] [1,4]-diazépine ainsi que de ses énantiomères isolés ou sous forme de mélange.

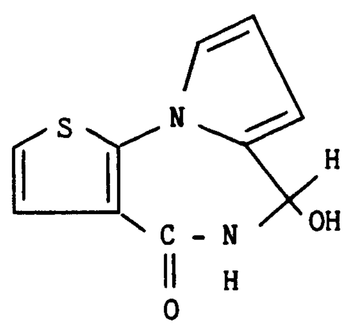

16. Procédé de préparation selon la revendication 1) du composé qui est le 5,6-dihydro 4-oxo 4H-pyrrolo [1,2-a] thiéno [3,2-f] [1,4]-diazépine

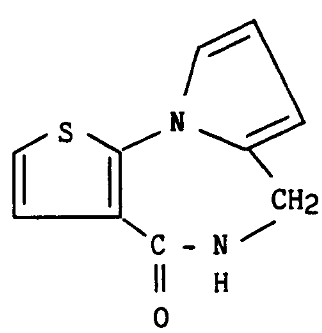

17. Procédé de préparation de compositions pharmaceutiques contenant comme principe actif un composé préparé selon les revendications 1) à 16) seul ou en combinaison avec un ou plusieurs excipients ou vehicules inertes non toxiques pharmaceutiquement acceptables.

18. Procédé de préparation selon la revendication 17) de compositions pharmaceutiques présentées sous une forme convenant notamment pour le traitement des accidents vasculaires cérébraux, du vieillissement cérébral normal ou pathologique et des syndromes ischémiques.

**19.** Procédé de préparation selon la revendication 17) de compositions pharmaceutiques présentées sous une forme convenant notamment pour le traitement des troubles gastro-intestinaux, du pancréas, de la vésicule biliaire, de l'appétit, du système nerveux ainsi que la douleur.

**20.** Procédé de préparation selon la revendication 17) de compositions pharmaceutiques présentées sous une forme convenant notamment pour le traitement de l'hyperlipidémie, de l'hypertriglycéridémie, de l'hypercholesterolémie, de l'hyperglycémie, de l'hypertension et des maladies qui leurs sont liées.

**Claims**

**Claims for the following Contracting States : AT, BE, CH, DE, DK, FR, GB, IT, LI, LU, NL, SE**

**1.** Compounds of the general formula (I) :

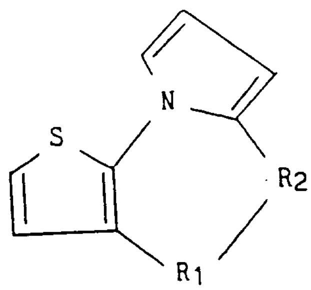

(I)

in which :

- $R_1$ represents a radical of the general formula **($Z_0$)**, **($Z_1$)**, **($Z_2$)**, **($Z_3$)** or **($Z_4$)**

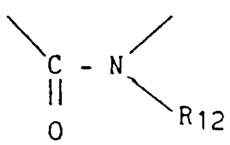

($Z_0$)

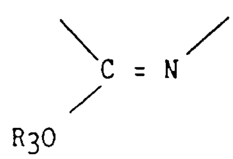

($Z_1$)

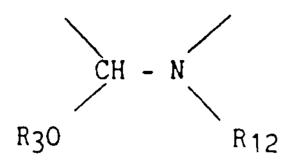

($Z_2$)

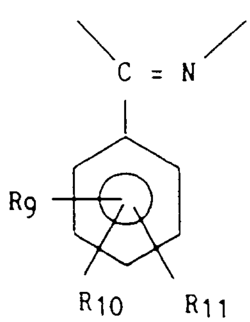

($Z_3$)

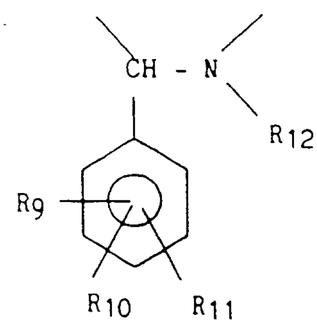

($Z_4$)

in which :

- $R_3$ represents a hydrogen atom, or a straight-chain or branched alkyl radical having from 1 to 6 carbon atoms which is saturated or contains a double bond,
- $R_9$, $R_{10}$ and $R_{11}$, which are the same or different, each represents a hydrogen atom, a halogen atom or an alkyl radical having from 1 to 6 carbon atoms,
- $R_{12}$ represents a hydrogen atom or, together with $R_2$ and the nitrogen atom to which they are attached, forms a radical of the general formula **(W)** :

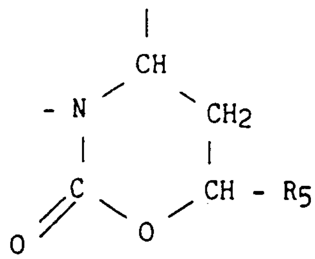

(W)

- $R_2$ represents a methylene radical, a hydroxymethylene radical, a carbonyl radical, a radical of the general formula **($Y_1$)**, **($Y_2$)**, **($Y_3$)** or **($Y_4$)** :

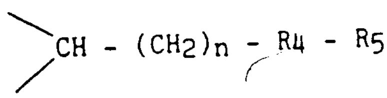

($Y_1$),

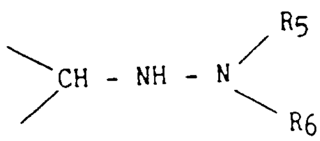

($Y_2$)

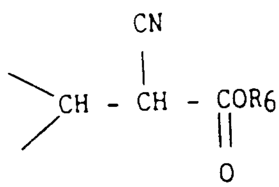

($Y_3$)

($Y_4$)

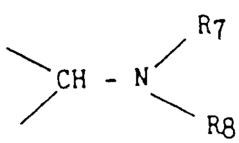

or, together with $R_{12}$ and the nitrogen atom to which they are attached, forms a radical of the general formula **(W)** :

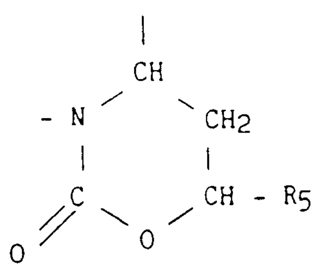

(W)

in which formulae :

- $R_4$ represents an oxygen atom, a sulphur atom, a carbonyl radical, a radical of the general formula **($X_1$)**, **($X_2$)**, **($X_3$)** or **($X_4$)** :

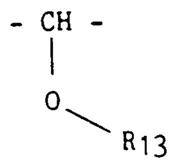 (X1)

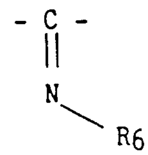 (X2)

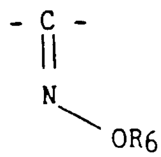 (X3)

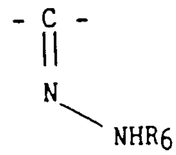 (X4)

- n is from 0 to 4 inclusive,
- $R_5$ represents a hydrogen atom, a straight or branched alkyl chain containing from 1 to 10 carbon atoms which is optionally interrupted by one or more oxygen or sulphur atoms, a phenyl, benzoyl or aralkyl radical containing from 7 to 11 carbon atoms (optionally substituted at the aromatic nucleus by one or more halogen atoms, straight-chain or branched alkyl radicals containing from 1 to 6 carbon atoms, nitro radicals or straight-chain or branched alkoxy radicals containing from 1 to 6 carbon atoms), a straight-chain or branched carboxyalkyl radical containing from 2 to 7 carbon atoms, a straight-chain or branched alkoxycarbonylalkyl radical containing from 3 to 10 carbon atoms, a straight-chain or branched alkoxycarbonyl radical containing from 2 to 7 carbon atoms, a straight-chain or branched carbamoylalkyl radical containing from 2 to 7 carbon atoms, a cycloalkyl radical containing from 3 to 7 carbon atoms, an unsaturated cyclic system having from 5 to 7 apices and containing at least one hetero atom selected from nitrogen, sulphur and oxygen, a pyridinylcarbonyl radical, a pyrimidylcarbonyl radical, a Clofibroyl radical or a 6-hydroxy-2,5,7,8-tetramethylchroman-2-carbonyl radical,
- $R_6$ represents a hydrogen atom or a straight-chain or branched alkyl radical containing from 1 to 6 carbon atoms,
- $R_7$ and $R_8$, which are the same or different, each represents a hydrogen atom, a straight-chain or branched alkyl radical containing from 1 to 6 carbon atoms, a phenyl or phenylalkyl radical containing from 7 to 9 carbon atoms (optionally substituted at the aromatic nucleus by one or more halogen atoms or alkyl or alkoxy groups containing from 1 to 6 carbon atoms) or, together with the nitrogen atom to which they are attached, form a cyclic system having from 5 to 7 apices, which is saturated or unsaturated, contains 1 or 2 hetero atoms selected from nitrogen, oxygen and sulphur and is optionally substituted by an alkylcarbonyl or alkoxycarbonyl group containing from 2 to 5 carbon atoms,
  and
- $R_{13}$ represents a hydrogen atom, a straight-chain or branched alkylcarbonyl radical having from 2 to 6 carbon atoms, or a benzoyl radical,
- their isomers, diastereoisomers and enantiomers, and
- their addition salts with a pharmaceutically acceptable mineral or organic acid,

with the provisos that :

- when $R_1$ represents a radical of the general formula ($Z_3$), $R_2$ cannot represent a methylene radical,
- when $R_1$ represents a radical of the general formula ($Z_0$) and $R_{12}$ represents a hydrogen atom, $R_2$ cannot represent the following radicals :
  $>CH-O-CH_3$, $>CH-O-CH_2CH_3$, $>CH-O-CH_2CH_2CH_3$, $>CH-O-CH_2CH_2-O-CH_2CH_3$, $>CH-O-CH_2CH_2-O-CH_2CH_2CH_2CH_3$
- when $R_1$ represents a radical of the general formula ($Z_0$), $R_{12}$ represents a hydrogen atom and $R_2$ represents a radical of the general formula **($Y_1$)** in which n = 1 and $R_4$ represents a carbonyl radical, $R_5$ cannot represent a methyl, isopropyl or phenyl radical,

- when $R_1$ represents a radical of the general formula **($Z_0$)** and $R_{12}$ represents a hydrogen atom, $R_2$ cannot represent a carbonyl radical.

**2.** Compounds according to claim 1) of the general formula **($I_A$)** :

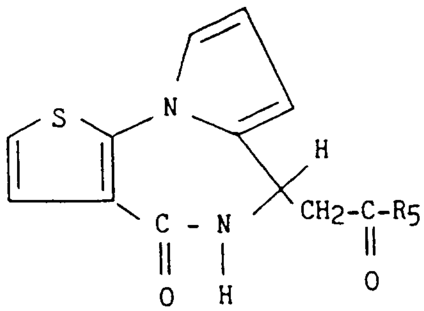

($I_A$)

in which $R_5$ is as defined in claim 1), as well as their isomers, diastereoisomers and enantiomers, isolated or in the form of a mixture.

**3.** Compounds according to claim 1) of the general formula **($I_B$)** :

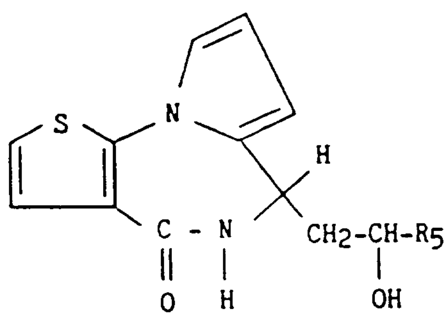

($I_B$)

in which $R_5$ is as defined in claim 1), as well as their isomers, diastereoisomers and enantiomers, isolated or in the form of a mixture.

**4.** Compounds according to claim 1) of the general formula **($I_C$)** :

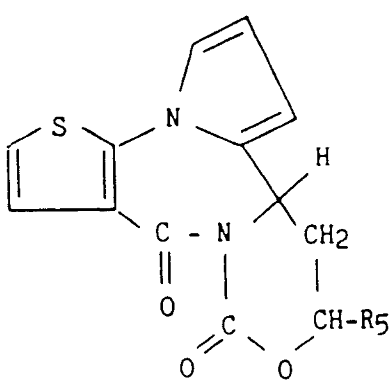

($I_C$)

in which $R_5$ is as defined in claim 1), as well as their isomers, enantiomers and diastereoisomers, isolated or in the form of a mixture.

**5.** Compounds according to claim 1) of the general formula **($I_D$)** :

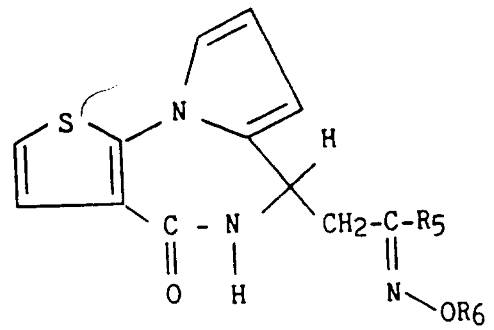

$(I_D)$

in which $R_5$ and $R_6$ are as defined in claim 1), as well as their isomers, enantiomers and diastereoisomers, isolated or in the form of a mixture.

**6.** Compounds according to claim 1) of the general formula **($I_E$)** :

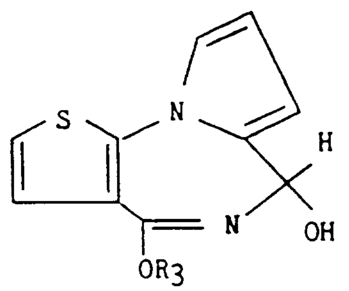

$(I_E)$

in which $R_3$ is as defined in claim 1), as well as their isomers, enantiomers and diastereoisomers, isolated or in the form of a mixture.

**7.** Compounds according to claim 1) of the general formula **($I_F$)** :

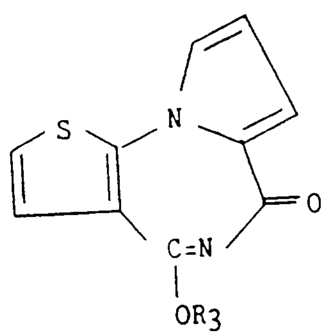

$(I_F)$

in which $R_3$ is as defined in claim 1), as well as their isomers, diastereoisomers and enantiomers, isolated or in the form of a mixture.

**8.** Compounds according to claim 1) of the general formula **($I_G$)** :

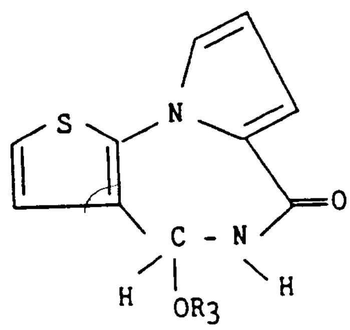

($I_G$)

in which $R_3$ is as defined in claim 1), as well as their isomers, diastereoisomers and enantiomers, isolated or in the form of a mixture.

**9.** Compounds according to claim 1) of the general formula **($I_H$)** :

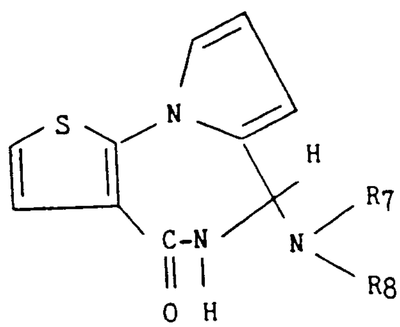

($I_H$)

in which $R_7$ and $R_8$ are as defined in claim 1), as well as their isomers, diastereoisomers and enantiomers, isolated or in the form of a mixture, and their possible addition salts with a pharmaceutically acceptable mineral or organic acid.

**10.** Compounds according to claim 1) of the general formula **($I_J$)** :

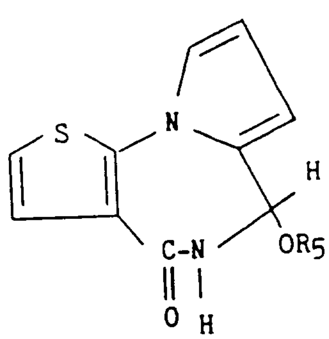

($I_J$)

in which $R_5$ is as defined in claim 1), as well as their isomers, diastereoisomers and enantiomers, isolated or in the form of a mixture.

**11.** Compounds according to claim 1) of the general formula **($I_K$)** :

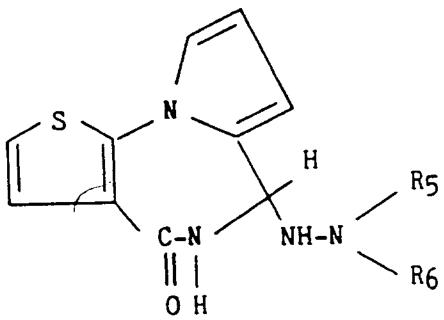

($I_K$)

in which $R_5$ and $R_6$ are as defined in claim 1), as well as their isomers, diastereoisomers and enantiomers, isolated or in the form of a mixture, and their possible addition salts with a pharmaceutically acceptable mineral or organic acid.

**12.** Compounds according to claim 1) of the general formula **($I_L$)** :

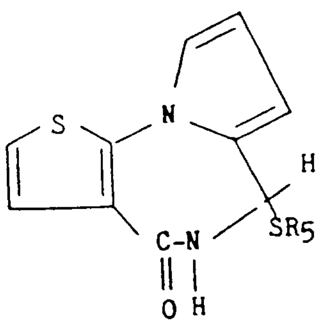

($I_L$)

in which $R_5$ is as defined in claim 1), as well as their isomers, diastereoisomers and enantiomers, isolated or in the form of a mixture.

**13.** Compound according to claim 1, which is 5,6-dihydro-6-(2-benzyloxypropyl)-4-oxo-4H-pyrrolo[1,2-a]-thieno[3,2-f][1,4]diazepine, as well as its diastereoisomers, isolated or in the form of a mixture.

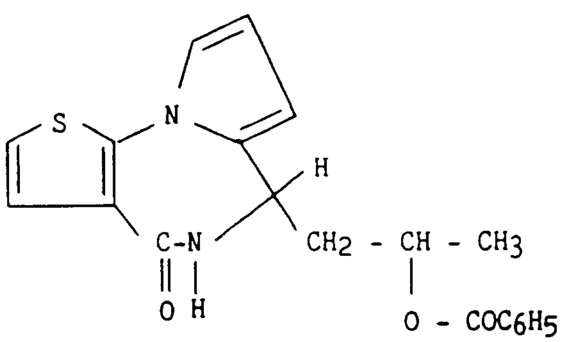

**14.** Compound according to claim 1, which is 5,6-dihydro-6-(2-hydrazono-1-pentyl)-4-oxo-4H-pyrrolo[1,2-a]-thieno[3,2-f][1,4]diazepine, as well as its enantiomers, isolated or in the form of a mixture, and its addition salts with a pharmaceutically acceptable mineral or organic acid

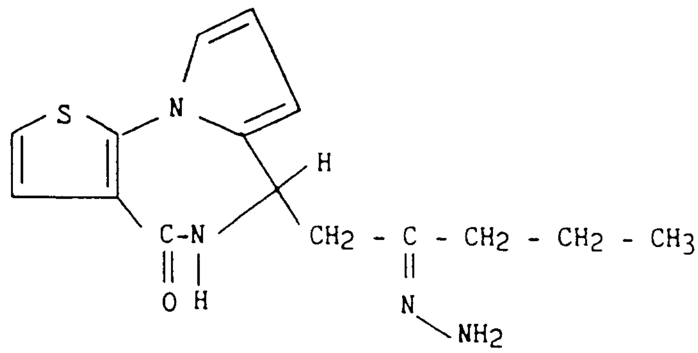

**15.** Compound according to claims 1) and 10), which is 5,6-dihydro-6-hydroxy-4-oxo-4H-pyrrolo[1,2-a]-thieno[3,2-f][1,4]diazepine, as well as its enantiomers isolated or in the form of a mixture.

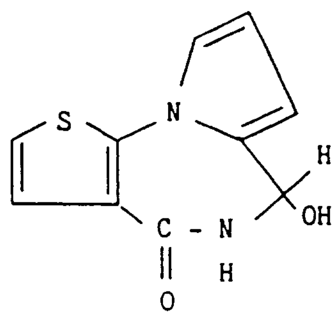

**16.** Compound according to claim 1, which is 5,6-dihydro-4-oxo-4H-pyrrolo[1,2-a]thieno[3,2-f][1,4]diazepine

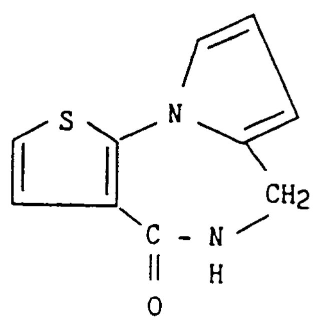

**17.** Process for the preparation of compounds of the general formula (I), characterised in that :

either

- 3-cyano-2-(2-formylpyrrol-1-yl)thiophene, a compound of formula (II) :

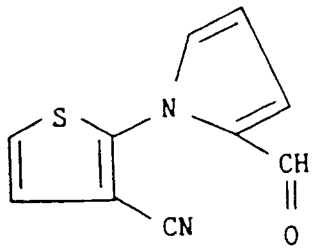

(II)

is reacted

**either**

- with a methylketone of the general formula (III) :

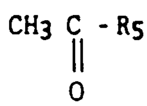

(III)

in which $R_5$ is as defined for compounds of the general formula (I), in the presence of a strong mineral base and hydrogen peroxide, to obtain compounds of the general formula **($I_A$)** :

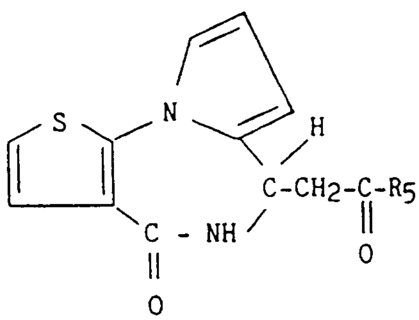

($I_A$)

in which $R_5$ is as defined for compounds of the general formula (I),

**which are then :**

**either**

subjected to the action of sodium borohydride, in solution in an alcoholic solvent, to yield compounds of the general formula **($I_B$)** :

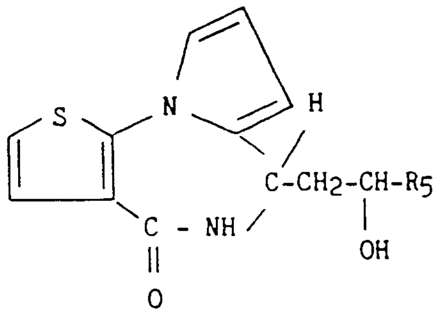

($I_B$)

in which $R_5$ is as defined for compounds of the general formula (I), which may optionally be subjected to the action of phosgene, in an aromatic organic solvent, with the application of heat, to form compounds of the general formula **($I_C$):**

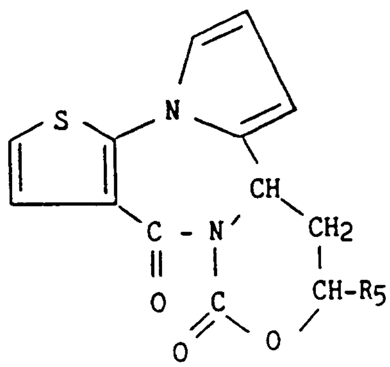

($I_C$)

in which $R_5$ is as defined for compounds of the general formula (I)
or reacted with a compound of the general formula (XI) :

$R_{13}$ - Cl (XI)

in which $R_{13}$ is as defined for compounds of the general formula (I), to obtain compounds of the general formula **($I_M$)** :

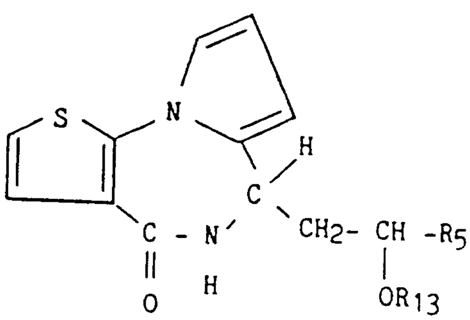

($I_M$)

in which $R_5$ and $R_{13}$ are as defined for compounds of the general formula (I),

<u>or</u>

condensed with a hydroxylamine compound of the general formula (IV) :

$H_2N$ - O - $R_6$ (IV)

in which $R_6$ is as defined for compounds of the general formula (I), to form compounds of the

general formula **($I_D$)** :

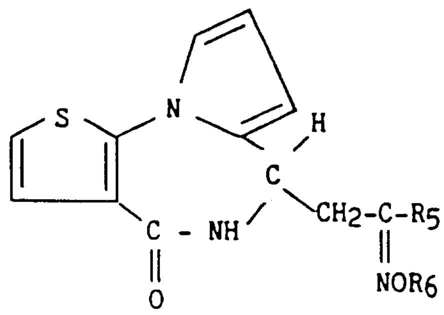

($I_D$)

in which $R_5$ and $R_6$ are as defined for compounds of the general formula (I),

<u>or</u>

condensed with a hydrazine compound of the general formula (XII) :

$H_2N - NH - R_6$ (XII)

in which $R_6$ is as defined for compounds of the general formula (I), to obtain compounds of the general formula **($I_N$)**:

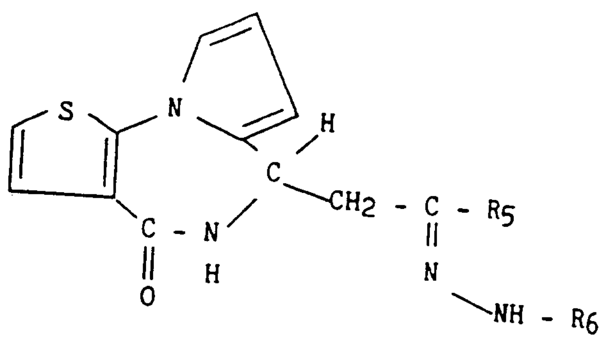

($I_N$)

in which $R_5$ and $R_6$ are as defined for compounds of the general formula (I),

**or**

with a primary alcohol of the general formula (V) :

$R_3OH$ (V)

in which $R_3$ is as defined for compounds of the general formula (I),

in the presence of a strong mineral base at a temperature of from 30° to 80°C, to obtain compounds of the general formula **($I_E$)** :

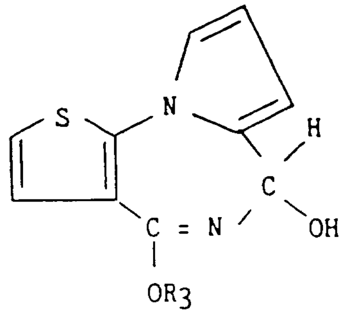

$(I_E)$

in which $R_3$ is as defined for compounds of the general formula (I), which are then subjected to the action of potassium permanganate at room temperature to form compounds of the general formula **($I_F$)**,

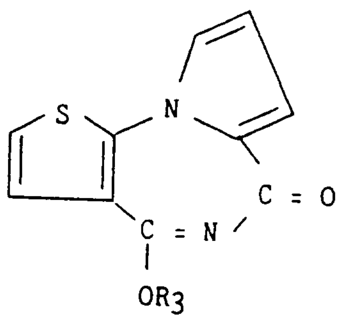

$(I_F)$

in which $R_3$ is as defined for compounds of the general formula (I), which may optionally be subjected to the action of sodium borohydride to obtain compounds of the general formula **($I_G$)**,

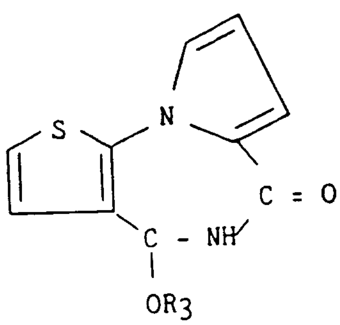

$(I_G)$

in which $R_3$ is as defined for compounds of the general formula (I),

or

2-(2-formylpyrrol-1-yl)-3-thiophene carboxamide, a compound of formula (VI) :

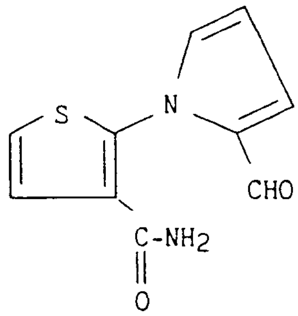

(VI)

is subjected

either

to the action of an amine of the general formula (VII) :

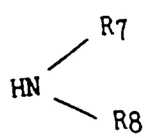

(VII)

in which $R_7$ and $R_8$ are as defined for compounds of the general formula (I), to obtain compounds of the general formula **($I_H$)** :

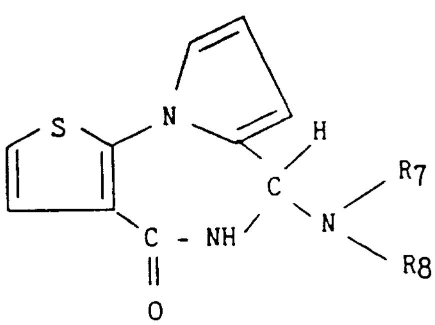

($I_H$)

in which $R_7$ and $R_8$ are as defined for compounds of the general formula (I), which may optionally be subjected, when $R_7$ and $R_8$ together with the nitrogen atom to which they are attached form a morpholino radical, to the action of sodium borohydride, to obtain the compound of the general formula (I) in which $R_2$ represents a methylene radical and $R_1$ represents a radical of the general formula **($Z_0$)** in which $R_{12}$ = H,

or

to the action of triethylamine, in the presence of water and at room temperature, to form the compound of the general formula (I) in which $R_2$ represents a hydroxymethylene radical and $R_1$ represents a radical of the formula **($Z_0$)** in which $R_{12}$ = H,

which is then,

either

reacted with an amine of the general formula (VII) to form a compound of the general formula **($I_H$)**

or

condensed with a compound of the general formula (VIII) :

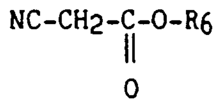

(VIII)

in which $R_6$ is as defined for compounds of the general formula (I), in the presence of triethylamine, to form a compound of the general formula **($I_I$)** :

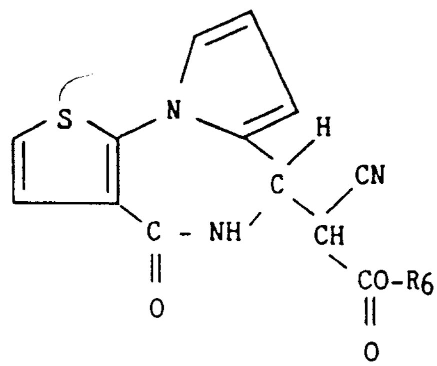

($I_I$)

in which $R_6$ is as defined for compounds of the general formula (I),

<u>or</u>

reacted with an alcohol of the general formula (IX)

$R_5OH$ (IX)

in which $R_5$ is as defined for compounds of the general formula (I), with the application of heat, to obtain a compound of the general formula **($I_J$)** :

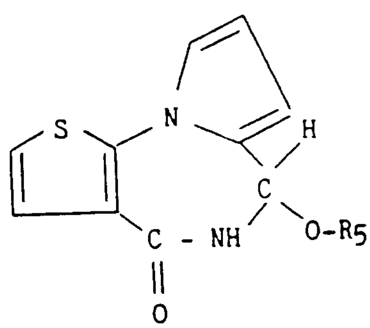

($I_J$)

in which $R_5$ is as defined for compounds of the general formula (I),

<u>or</u>

to the action of a compound of the general formula (X) :

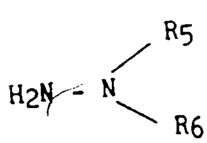

(X)

in which $R_5$ and $R_6$ are as defined for compounds of the general formula (I), to form a compound of the general formula **($I_K$)** :

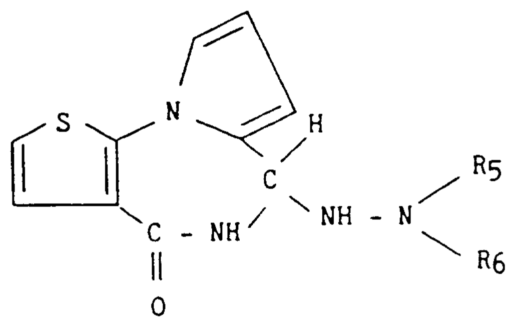

($I_K$)

in which $R_5$ and $R_6$ are as defined for compounds of the general formula (I),

or

to the action of an alcohol of the general formula (IX) to form a compound of the general formula **($I_J$)**,

or

to the action of a thiol of the general formula (XI) :

$R_5SH$ (XI)

in which $R_5$ is as defined for compounds of the general formula (I), at room temperature, to form a compound of the general formula **($I_L$)**,

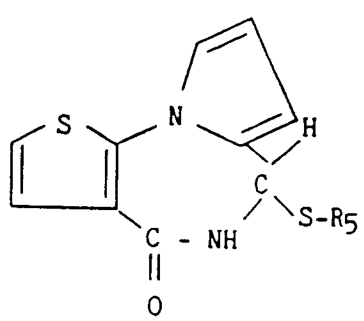

($I_L$)

in which $R_5$ is as defined for compounds of the general formula (I), which may optionally be subjected, when $R_5$ represents a carboxyalkyl radical, to the action of triethylamine and a chloroformate, then to the action of gaseous ammonia, to form the corresponding amide,
it being understood that the compounds of the general formulae ($I_A$), ($I_B$), ($I_C$), ($I_D$), ($I_E$), ($I_F$), ($I_G$), ($I_H$), ($I_I$), ($I_J$), ($I_K$), ($I_L$), ($I_M$) and ($I_N$) form part of the invention and belong to the compounds of the general formula (I).

**18.** Pharmaceutical compositions comprising as active ingredient a compound according to any one of claims 1) to 16), alone or in combination with one or more pharmaceutically acceptable, inert, non-toxic excipients or carriers.

**19.** Pharmaceutical compositions according to claim 18), presented in a form suitable especially for the treatment of cerebral vascular accidents, normal or pathological cerebral ageing, and ischaemic syndromes.

**20.** Pharmaceutical compositions according to claim 18), presented in a form suitable especially for the treatment of gastrointestinal disorders, disorders of the pancreas, of the gall bladder, of the appetite and of the nervous system, and pain.

**21.** Pharmaceutical compositions according to claim 18) presented in a form suitable especially for the treatment of hyperlipidaemia, hypertriglyceridaemia, hypercholesterolaemia, hyperglycaemia, hypertension, and associated disorders.

**Claims for the following Contracting State : ES**

**1.** Process for the preparation of compounds of the general formula (I) :

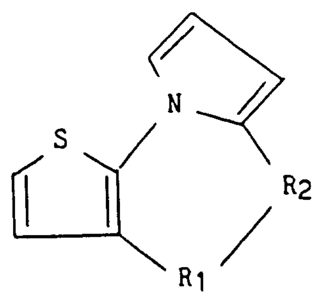

(I)

in which :

- $R_1$ represents a radical of the general formula **($Z_0$)**, **($Z_1$)**, **($Z_2$)**, **($Z_3$)** or **($Z_4$)**

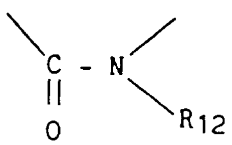

($Z_0$)

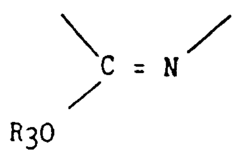

($Z_1$)

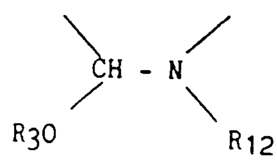

($Z_2$)

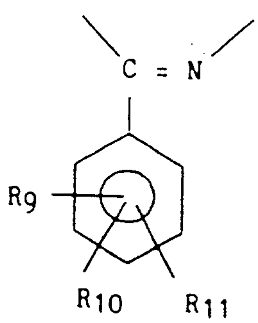

($Z_3$)

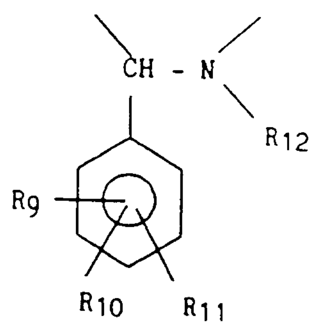

($Z_4$)

in which :

- $R_3$ represents a hydrogen atom, or a straight-chain or branched alkyl radical having from 1 to 6 carbon atoms which is saturated or contains a double bond,
- $R_9$, $R_{10}$ and $R_{11}$, which are the same or different, each represents a hydrogen atom, a halogen atom or an alkyl radical having from 1 to 6 carbon atoms,
- $R_{12}$ represents a hydrogen atom or, together with $R_2$ and the nitrogen atom to which they are attached, forms a radical of the general formula **(W)** :

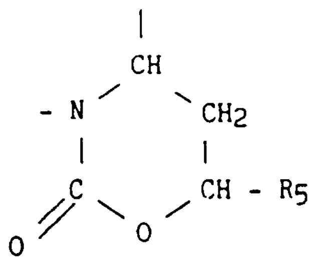

**(W)**

- $R_2$ represents a methylene radical, a hydroxymethylene radical, a carbonyl radical, a radical of the general formula **($Y_1$)**, **($Y_2$)**, **($Y_3$)** or **($Y_4$)** :

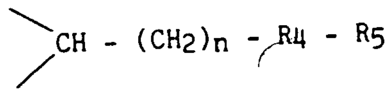

**($Y_1$)**,

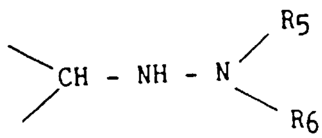

**($Y_2$)**

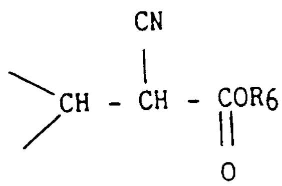

**($Y_3$)**

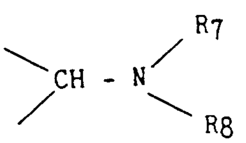

**($Y_4$)**

or, together with $R_{12}$ and the nitrogen atom to which they are attached, forms a radical of the general formula **(W)** :

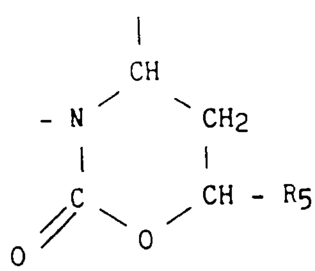

**(W)**

in which formulae :

- $R_4$ represents an oxygen atom, a sulphur atom, a carbonyl radical, a radical of the general formula **($X_1$)**, **($X_2$)**, **($X_3$)** or **($X_4$)** :

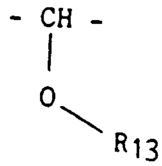 (X1) 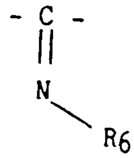 (X2)

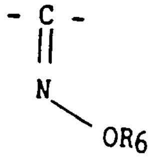 (X3) 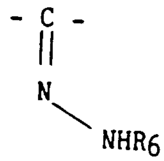 (X4)

- n is from 0 to 4 inclusive,
- $R_5$ represents a hydrogen atom, a straight or branched alkyl chain containing from 1 to 10 carbon atoms which is optionally interrupted by one or more oxygen or sulphur atoms, a phenyl, benzoyl or aralkyl radical containing from 7 to 11 carbon atoms (optionally substituted at the aromatic nucleus by one or more halogen atoms, straight-chain or branched alkyl radicals containing from 1 to 6 carbon atoms, nitro radicals or straight-chain or branched alkoxy radicals containing from 1 to 6 carbon atoms), a straight-chain or branched carboxyalkyl radical containing from 2 to 7 carbon atoms, a straight-chain or branched alkoxycarbonylalkyl radical containing from 3 to 10 carbon atoms, a straight-chain or branched alkoxycarbonyl radical containing from 2 to 7 carbon atoms, a straight-chain or branched carbamoylalkyl radical containing from 2 to 7 carbon atoms, a cycloalkyl radical containing from 3 to 7 carbon atoms, an unsaturated cyclic system having from 5 to 7 apices and containing at least one hetero atom selected from nitrogen, sulphur and oxygen, a pyridinylcarbonyl radical, a pyrimidylcarbonyl radical, a Clofibroyl radical or a 6-hydroxy-2,5,7,8-tetramethylchroman-2-carbonyl radical,
- $R_6$ represents a hydrogen atom or a straight-chain or branched alkyl radical containing from 1 to 6 carbon atoms,
- $R_7$ and $R_8$, which are the same or different, each represents a hydrogen atom, a straight-chain or branched alkyl radical containing from 1 to 6 carbon atoms, a phenyl or phenylalkyl radical containing from 7 to 9 carbon atoms (optionally substituted at the aromatic nucleus by one or more halogen atoms or alkyl or alkoxy groups containing from 1 to 6 carbon atoms) or, together with the nitrogen atom to which they are attached, form a cyclic system having from 5 to 7 apices, which is saturated or unsaturated, contains 1 or 2 hetero atoms selected from nitrogen, oxygen and sulphur and is optionally substituted by an alkylcarbonyl or alkoxycarbonyl group containing from 2 to 5 carbon atoms,
  and
- $R_{13}$ represents a hydrogen atom, a straight-chain or branched alkylcarbonyl radical having from 2 to 6 carbon atoms, or a benzoyl radical,

and also their isomers, diastereoisomers and enantiomers, and their addition salts with a pharmaceutically acceptable mineral or organic acid,
with the provisos that :

- when $R_1$ represents a radical of the general formula **($Z_3$)**, $R_2$ cannot represent a methylene radical,
- when $R_1$ represents a radical of the general formula **($Z_0$)** and $R_{12}$ represents a hydrogen atom, $R_2$ cannot represent the following radicals :
  $>CH-O-CH_3$, $>CH-O-CH_2CH_3$, $>CH-O-CH_2CH_2CH_3$, $>CH-O-CH_2CH_2-O-CH_2CH_3$, $>CH-O-CH_2CH_2-O-CH_2CH_2CH_2CH_3$
- when $R_1$ represents a radical of the general formula **($Z_0$)**, $R_{12}$ represents a hydrogen atom and $R_2$ represents a radical of the general formula **($Y_1$)** in which $n = 1$ and $R_4$ represents a carbonyl radical, $R_5$ cannot represent a methyl, isopropyl or phenyl radical,

- when $R_1$ represents a radical of the general formula **($Z_0$)** and $R_{12}$ represents a hydrogen atom, $R_2$ cannot represent a carbonyl radical,

characterised in that :

either

- 3-cyano-2-(2-formylpyrrol-1-yl)thiophene, a compound of formula (II) :

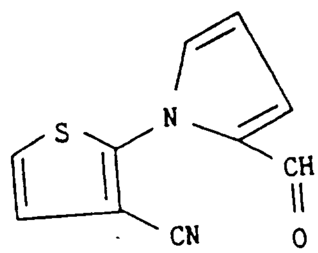

(II)

is reacted

**either**

- with a methylketone of the general formula (III) :

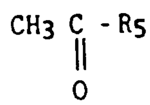

(III)

in which $R_5$ is as defined for compounds of the general formula (I), in the presence of a strong mineral base and hydrogen peroxide, to obtain compounds of the general formula **($I_A$)** :

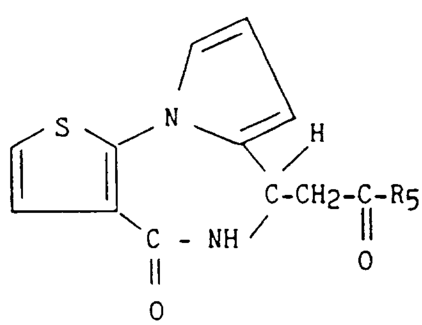

($I_A$)

in which $R_5$ is as defined for compounds of the general formula (I),

**which are then :**

**either**

subjected to the action of sodium borohydride, in solution in an alcoholic solvent, to yield compounds of the general formula **($I_B$)** :

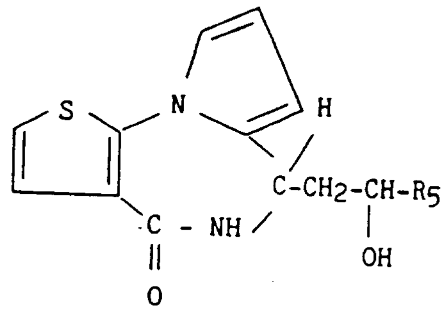

($I_B$)

in which $R_5$ is as defined for compounds of the general formula (I), which may optionally be subjected to the action of phosgene, in an aromatic organic solvent, with the application of heat, to form compounds of the general formula **($I_C$)**:

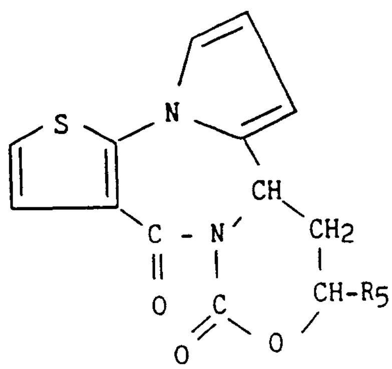

($I_C$)

in which $R_5$ is as defined for compounds of the general formula (I)
or reacted with a compound of the general formula (XI) :

$R_{13}$ - Cl (XI)

in which $R_{13}$ is as defined for compounds of the general formula (I), to obtain compounds of the general formula **($I_M$)** :

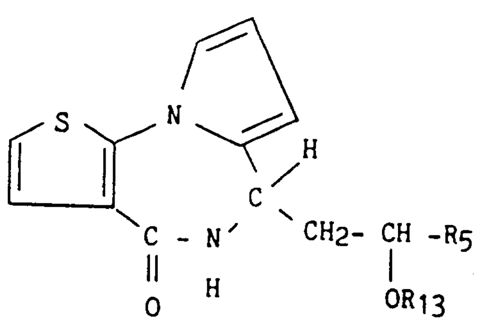

($I_M$)

in which $R_5$ and $R_{13}$ are as defined for compounds of the general formula (I),

<u>or</u>

condensed with a hydroxylamine compound of the general formula (IV) :

$H_2N$ - O - $R_6$ (IV)

in which $R_6$ is as defined for compounds of the general formula (I), to form compounds of the

general formula **($I_D$)** :

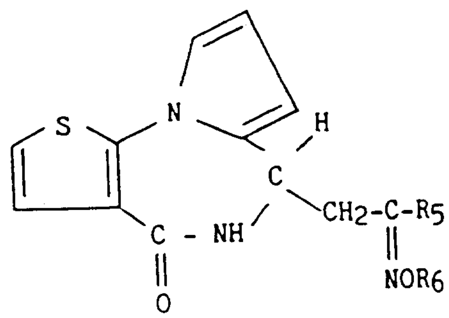

($I_D$)

in which $R_5$ and $R_6$ are as defined for compounds of the general formula (I),

**<u>or</u>**

condensed with a hydrazine compound of the general formula (XII) :

$H_2N$ - NH - $R_6$ (XII)

in which $R_6$ is as defined for compounds of the general formula (I), to obtain compounds of the general formula **($I_N$)**:

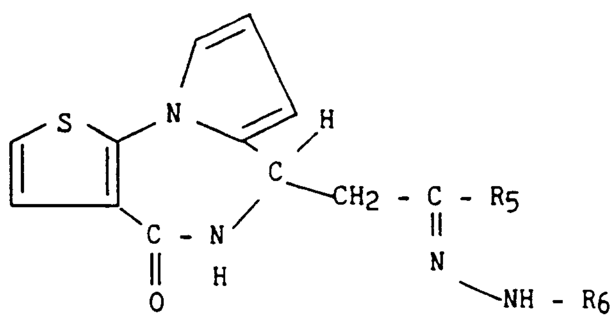

($I_N$)

in which $R_5$ and $R_6$ are as defined for compounds of the general formula (I),

**or**

with a primary alcohol of the general formula (V) :

$R_3OH$ (V)

in which $R_3$ is as defined for compounds of the general formula (I),

in the presence of a strong mineral base at a temperature of from 30° to 80°C, to obtain compounds of the general formula **($I_E$)** :

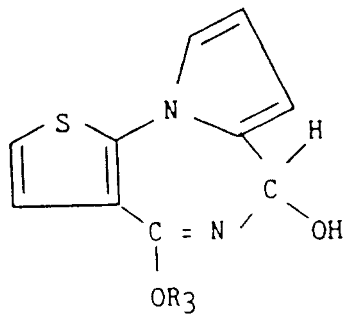

($I_E$)

in which $R_3$ is as defined for compounds of the general formula (I), which are then subjected to the action of potassium permanganate at room temperature to form compounds of the general formula **($I_F$)**,

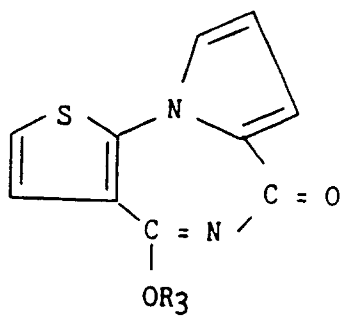

($I_F$)

in which $R_3$ is as defined for compounds of the general formula (I), which may optionally be subjected to the action of sodium borohydride to obtain compounds of the general formula **($I_G$)**,

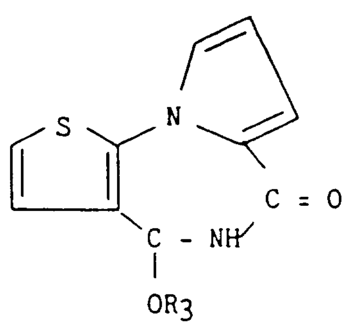

($I_G$)

in which $R_3$ is as defined for compounds of the general formula (I),

or

2-(2-formylpyrrol-1-yl)-3-thiophene carboxamide, a compound of formula (VI) :

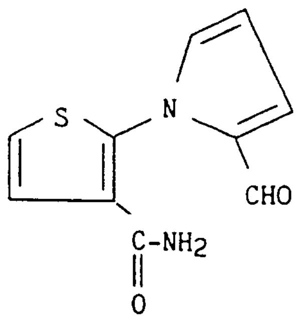

(VI)

is subjected

<u>either</u>

to the action of an amine of the general formula (VII) :

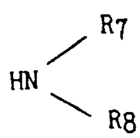

(VII)

in which $R_7$ and $R_8$ are as defined for compounds of the general formula (I), to obtain compounds of the general formula **($I_H$)** :

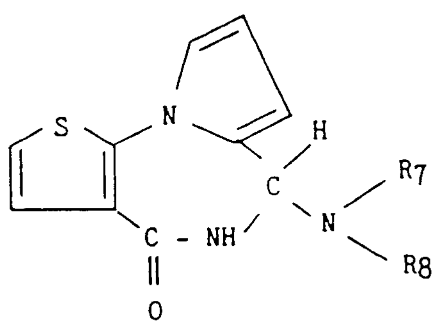

($I_H$)

in which $R_7$ and $R_8$ are as defined for compounds of the general formula (I), which may optionally be subjected, when $R_7$ and $R_8$ together with the nitrogen atom to which they are attached form a morpholino radical, to the action of sodium borohydride, to obtain the compound of the general formula (I) in which $R_2$ represents a methylene radical and $R_1$ represents a radical of the general formula **($Z_0$)** in which $R_{12}$ = H,

<u>or</u>

to the action of triethylamine, in the presence of water and at room temperature, to form the compound of the general formula (I) in which $R_2$ represents a hydroxymethylene radical and $R_1$ represents a radical of the formula **($Z_0$)** in which $R_{12}$ = H,

<u>which is then,</u>

<u>either</u>

reacted with an amine of the general formula (VII) to form a compound of the general formula **($I_H$)**

<u>or</u>

condensed with a compound of the general formula (VIII) :

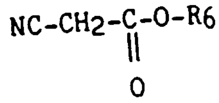

(VIII)

in which $R_6$ is as defined for compounds of the general formula (I), in the presence of triethylamine, to form a compound of the general formula **($I_I$)** :

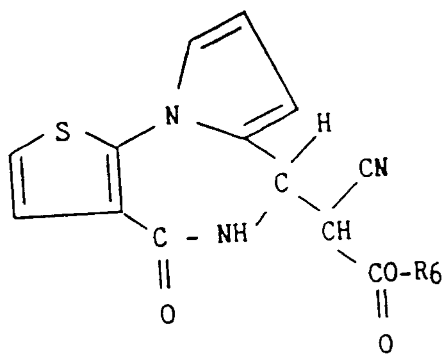

($I_I$)

in which $R_6$ is as defined for compounds of the general formula (I),

<u>or</u>

reacted with an alcohol of the general formula (IX)

$R_5OH$ (IX)

in which $R_5$ is as defined for compounds of the general formula (I), with the application of heat, to obtain a compound of the general formula **($I_J$)** :

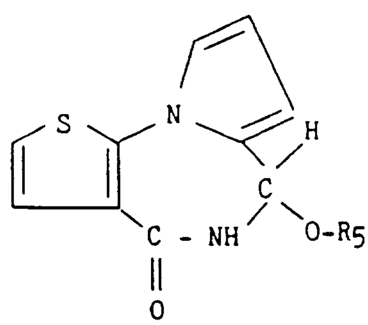

($I_J$)

in which $R_5$ is as defined for compounds of the general formula (I),

<u>or</u>

to the action of a compound of the general formula (X) :

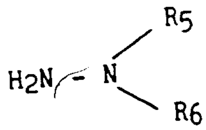

(X)

in which $R_5$ and $R_6$ are as defined for compounds of the general formula (I), to form a compound of the general formula **($I_K$)** :

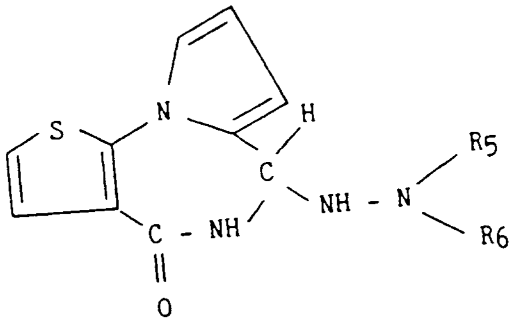

($I_K$)

in which $R_5$ and $R_6$ are as defined for compounds of the general formula (I),

<u>or</u>

to the action of an alcohol of the general formula (IX) to form a compound of the general formula **($I_J$)**,

<u>or</u>

to the action of a thiol of the general formula (XI) :

$R_5SH$ (XI)

in which $R_5$ is as defined for compounds of the general formula (I), at room temperature, to form a compound of the general formula **($I_L$)**,

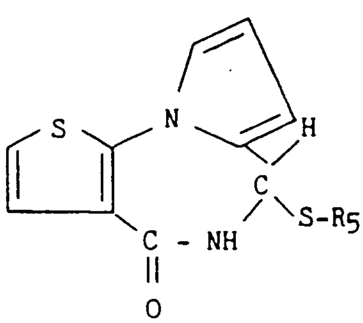

($I_L$)

in which $R_5$ is as defined for compounds of the general formula (I), which may optionally be subjected, when $R_5$ represents a carboxyalkyl radical, to the action of triethylamine and a chloroformate, then to the action of gaseous ammonia, to form the corresponding amide.

**2.** Process according to claim 1) for the preparation of compounds of the general formula **($I_A$)** :

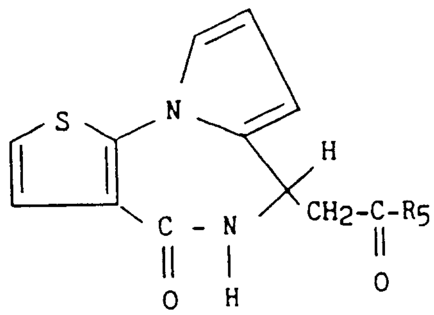

($I_A$)

in which $R_5$ is as defined in claim 1), as well as their isomers, diastereoisomers and enantiomers, isolated or in the form of a mixture.

**3.** Process according to claim 1) for the preparation of compounds of the general formula **($I_B$)** :

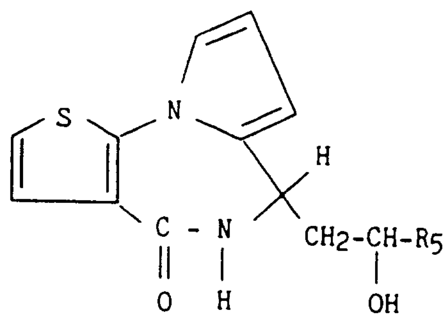

($I_B$)

in which $R_5$ is as defined in claim 1), as well as their isomers, diastereoisomers and enantiomers, isolated or in the form of a mixture.

**4.** Process according to claim 1) for the preparation of compounds of the general formula **($I_C$)** :

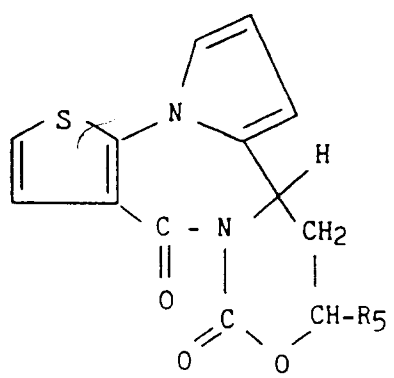

($I_C$)

in which $R_5$ is as defined in claim 1), as well as their isomers, enantiomers and diastereoisomers, isolated or in the form of a mixture.

**5.** Process according to claim 1) for the preparation of compounds of the general formula **($I_D$)** :

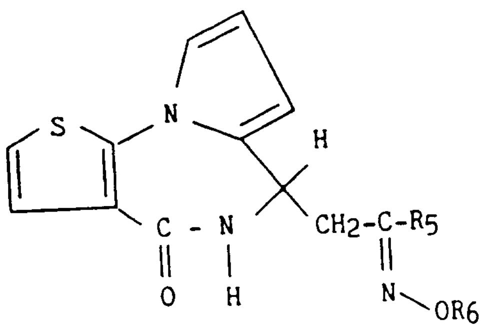

($I_D$)

in which $R_5$ and $R_6$ are as defined in claim 1), as well as their isomers, enantiomers and diastereoisomers, isolated or in the form of a mixture.

**6.** Process according to claim 1) for the preparation of compounds of the general formula **($I_E$)** :

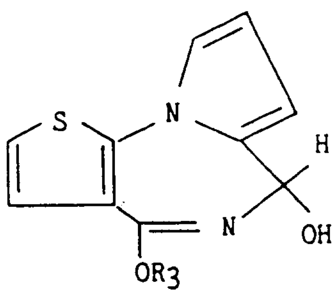

($I_E$)

in which $R_3$ is as defined in claim 1), as well as their isomers, enantiomers and diastereoisomers, isolated or in the form of a mixture.

**7.** Process according to claim 1) for the preparation of compounds of the general formula **($I_F$)** :

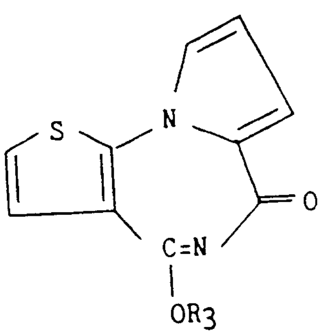

($I_F$)

in which $R_3$ is as defined in claim 1), as well as their isomers, diastereoisomers and enantiomers, isolated or in the form of a mixture.

**8.** Process according to claim 1) for the preparation of compounds of the general formula **($I_G$)** :

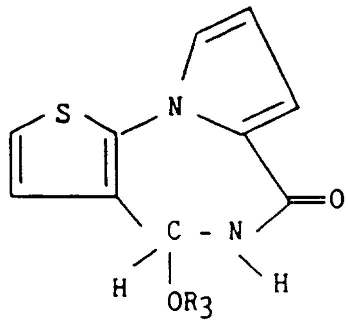

($I_G$)

in which $R_3$ is as defined in claim 1), as well as their isomers, diastereoisomers and enantiomers, isolated or in the form of a mixture.

**9.** Process according to claim 1) for the preparation of compounds of the general formula **($I_H$)** :

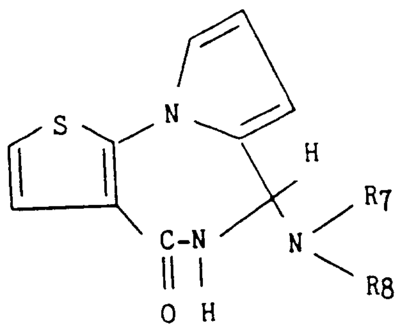

($I_H$)

in which $R_7$ and $R_8$ are as defined in claim 1), as well as their isomers, diastereoisomers and enantiomers, isolated or in the form of a mixture, and their possible addition salts with a pharmaceutically acceptable mineral or organic acid.

**10.** Process according to claim 1) for the preparation of compounds of the general formula **($I_J$)** :

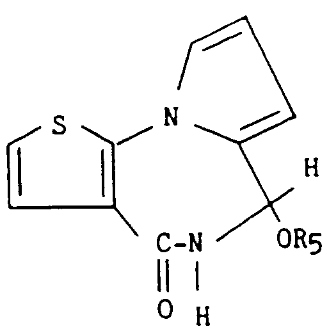

($I_J$)

in which $R_5$ is as defined in claim 1), as well as their isomers, diastereoisomers and enantiomers, isolated or in the form of a mixture.

**11.** Process according to claim 1) for the preparation of compounds of the general formula **($I_K$)** :

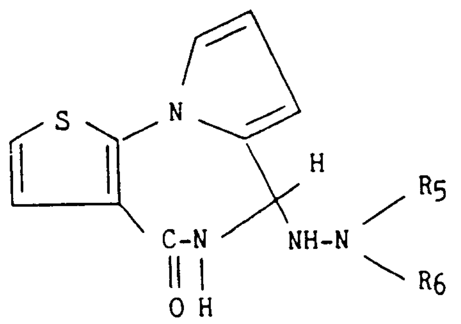

($I_K$)

in which $R_5$ and $R_6$ are as defined in claim 1), as well as their isomers, diastereoisomers and enantiomers, isolated or in the form of a mixture, and their possible addition salts with a pharmaceutically acceptable mineral or organic acid.

**12.** Process according to claim 1) for the preparation of compounds of the general formula **($I_L$)** :

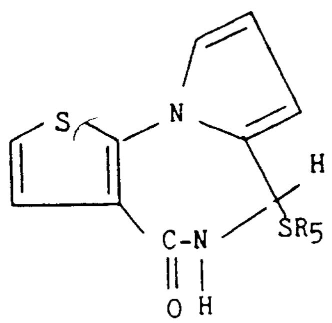

($I_L$)

in which $R_5$ is as defined in claim 1), as well as their isomers, diastereoisomers and enantiomers, isolated or in the form of a mixture.

**13.** Process according to claim 1) for the preparation of the compound 5,6-dihydro-6-(2-benzyloxypropyl)-4-oxo-4H-pyrrolo[1,2-a]thieno[3,2-f][1,4]diazepine, as well as its diastereoisomers, isolated or in the form of a mixture.

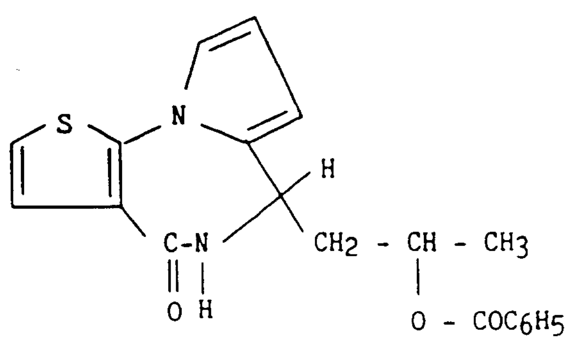

**14.** Process according to claim 1) for the preparation of the compound 5,6-dihydro-6-(2-hydrazono-1-pentyl)-4-oxo-4H-pyrrolo[1,2-a]thieno[3,2-f][1,4]diazepine, as well as its enantiomers, isolated or in the form of a mixture, and its addition salts with a pharmaceutically acceptable mineral or organic acid

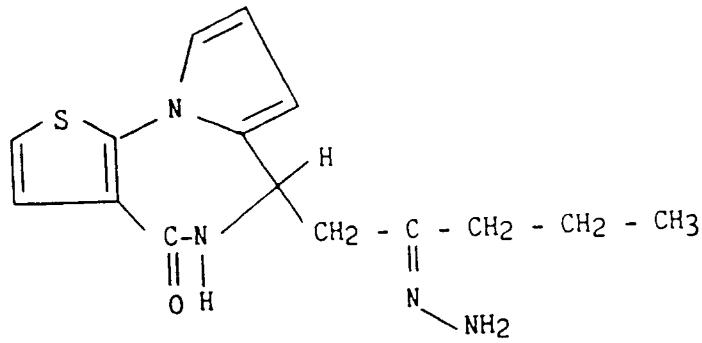

**15.** Process according to claims 1) and 10) for the preparation of the compound 5,6-dihydro-6-hydroxy-4-oxo-4H-pyrrolo[1,2-a]thieno[3,2-f][1,4]diazepine, as well as its enantiomers isolated or in the form of a mixture.

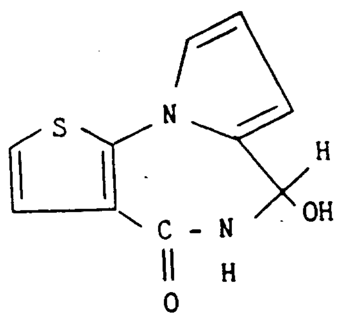

**16.** Process according to claim 1) for the preparation of the compound 5,6-dihydro-4-oxo-4H-pyrrolo[1,2-a]-thieno[3,2-f][1,4]diazepine

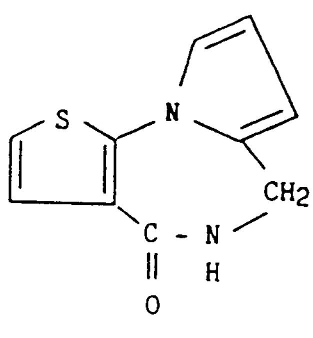

**17.** Process for the preparation of pharmaceutical compositions comprising as active ingredient a compound prepared according to any one of claims 1) to 16), alone or in combination with one or more pharmaceutically acceptable, inert, non-toxic excipients or carriers.

**18.** Process according to claim 17) for the preparation of pharmaceutical compositions presented in a form suitable especially for the treatment of cerebral vascular accidents, normal or pathological cerebral ageing, and ischaemic syndromes.

**19.** Process according to claim 17) for the preparation pharmaceutical compositions presented in a form suitable especially for the treatment of gastrointestinal disorders, disorders of the pancreas, of the gall

bladder, of the appetite and of the nervous system, and pain.

**20.** Process according to claim 17 for the preparation of pharmaceutical compositions presented in a form suitable especially for the treatment of hyperlipidaemia, hypertriglyceridaemia, hypercholesterolaemia, hyperglycaemia, hypertension, and associated disorders.

**Claims for the following Contracting State : GR**

**1.** Process for the preparation of compounds of the general formula (I) :

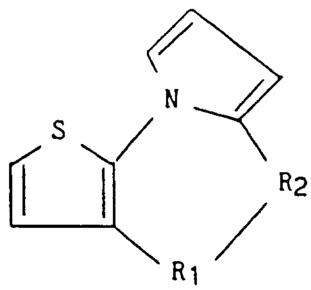

in which :

- $R_1$ represents a radical of the general formula **($Z_0$)**, **($Z_1$)**, **($Z_2$)**, **($Z_3$)** or **($Z_4$)**

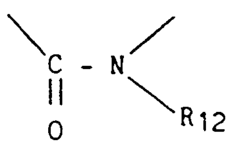

($Z_0$)

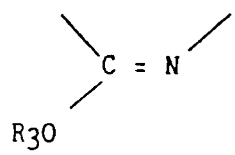

($Z_1$)

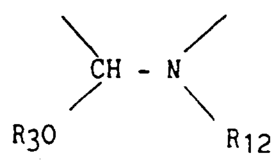

($Z_2$)

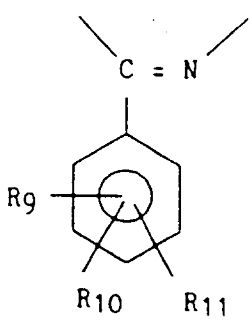

($Z_3$)

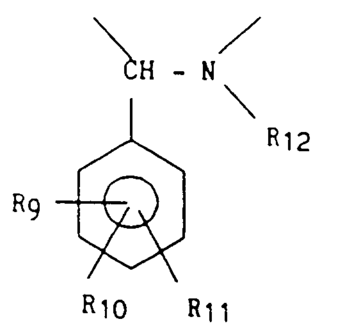

($Z_4$)

in which :

- $R_3$ represents a hydrogen atom, or a straight-chain or branched alkyl radical having from 1 to 6 carbon atoms which is saturated or contains a double bond,
- $R_9$, $R_{10}$ and $R_{11}$, which are the same or different, each represents a hydrogen atom, a halogen atom or an alkyl radical having from 1 to 6 carbon atoms,
- $R_{12}$ represents a hydrogen atom or, together with $R_2$ and the nitrogen atom to which they are attached, forms a radical of the general formula **(W)** :

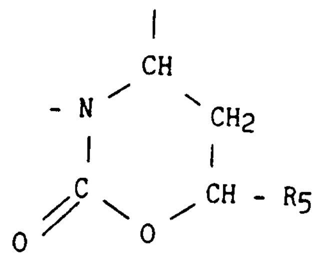

(W)

- $R_2$ represents a methylene radical, a hydroxymethylene radical, a carbonyl radical, a radical of the general formula **($Y_1$)**, **($Y_2$)**, **($Y_3$)** or **($Y_4$)** :

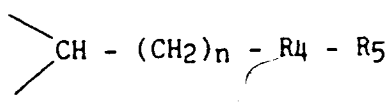

($Y_1$),

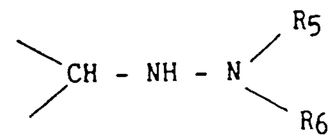

($Y_2$)

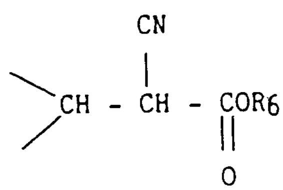

($Y_3$)

($Y_4$)

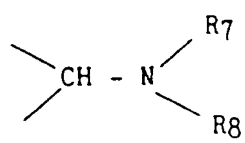

or, together with $R_{12}$ and the nitrogen atom to which they are attached, forms a radical of the general formula **(W)** :

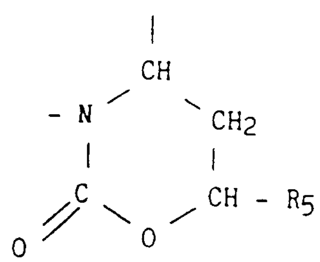

(W)

in which formulae :

- $R_4$ represents an oxygen atom, a sulphur atom, a carbonyl radical, a radical of the general formula **($X_1$)**, **($X_2$)**, **($X_3$)** or **($X_4$)** :

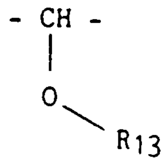 $(X_1)$ 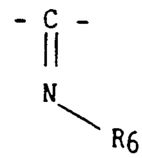 $(X_2)$

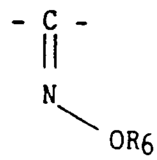 $(X_3)$ 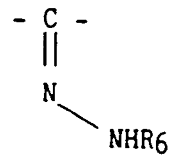 $(X_4)$

- n is from 0 to 4 inclusive,
- $R_5$ represents a hydrogen atom, a straight or branched alkyl chain containing from 1 to 10 carbon atoms which is optionally interrupted by one or more oxygen or sulphur atoms, a phenyl, benzoyl or aralkyl radical containing from 7 to 11 carbon atoms (optionally substituted at the aromatic nucleus by one or more halogen atoms, straight-chain or branched alkyl radicals containing from 1 to 6 carbon atoms, nitro radicals or straight-chain or branched alkoxy radicals containing from 1 to 6 carbon atoms), a straight-chain or branched carboxyalkyl radical containing from 2 to 7 carbon atoms, a straight-chain or branched alkoxycarbonylalkyl radical containing from 3 to 10 carbon atoms, a straight-chain or branched alkoxycarbonyl radical containing from 2 to 7 carbon atoms, a straight-chain or branched carbamoylalkyl radical containing from 2 to 7 carbon atoms, a cycloalkyl radical containing from 3 to 7 carbon atoms, an unsaturated cyclic system having from 5 to 7 apices and containing at least one hetero atom selected from nitrogen, sulphur and oxygen, a pyridinylcarbonyl radical, a pyrimidylcarbonyl radical, a Clofibroyl radical or a 6-hydroxy-2,5,7,8-tetramethylchroman-2-carbonyl radical,
- $R_6$ represents a hydrogen atom or a straight-chain or branched alkyl radical containing from 1 to 6 carbon atoms,
- $R_7$ and $R_8$, which are the same or different, each represents a hydrogen atom, a straight-chain or branched alkyl radical containing from 1 to 6 carbon atoms, a phenyl or phenylalkyl radical containing from 7 to 9 carbon atoms (optionally substituted at the aromatic nucleus by one or more halogen atoms or alkyl or alkoxy groups containing from 1 to 6 carbon atoms) or, together with the nitrogen atom to which they are attached, form a cyclic system having from 5 to 7 apices, which is saturated or unsaturated, contains 1 or 2 hetero atoms selected from nitrogen, oxygen and sulphur and is optionally substituted by an alkylcarbonyl or alkoxycarbonyl group containing from 2 to 5 carbon atoms,

  and
- $R_{13}$ represents a hydrogen atom, a straight-chain or branched alkylcarbonyl radical having from 2 to 6 carbon atoms, or a benzoyl radical,

and also their isomers, diastereoisomers and enantiomers, and their addition salts with a pharmaceutically acceptable mineral or organic acid,
with the provisos that :

- when $R_1$ represents a radical of the general formula **($Z_3$)**, $R_2$ cannot represent a methylene radical,
- when $R_1$ represents a radical of the general formula **($Z_0$)** and $R_{12}$ represents a hydrogen atom, $R_2$ cannot represent the following radicals :
  $>CH-O-CH_3$, $>CH-O-CH_2CH_3$, $>CH-O-CH_2CH_2CH_3$, $>CH-O-CH_2CH_2-O-CH_2CH_3$, $>CH-O-CH_2CH_2-O-CH_2CH_2CH_2CH_3$
- when $R_1$ represents a radical of the general formula **($Z_0$)**, $R_{12}$ represents a hydrogen atom and $R_2$ represents a radical of the general formula **($Y_1$)** in which n = 1 and $R_4$ represents a carbonyl radical, $R_5$ cannot represent a methyl, isopropyl or phenyl radical,

- when $R_1$ represents a radical of the general formula **($Z_0$)** and $R_{12}$ represents a hydrogen atom, $R_2$ cannot represent a carbonyl radical,

characterised in that :

**either**

- 3-cyano-2-(2-formylpyrrol-1-yl)thiophene, a compound of formula (II) :

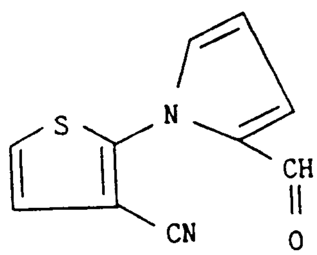

(II)

is reacted

**either**

- with a methylketone of the general formula (III) :

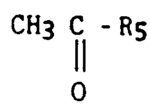

(III)

in which $R_5$ is as defined for compounds of the general formula (I), in the presence of a strong mineral base and hydrogen peroxide, to obtain compounds of the general formula **($I_A$)** :

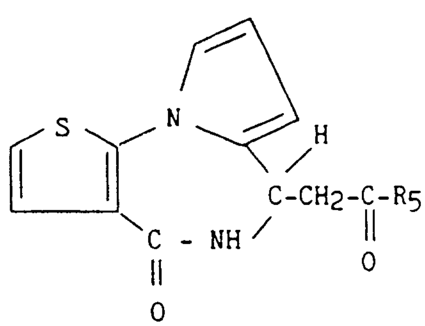

($I_A$)

in which $R_5$ is as defined for compounds of the general formula (I),

**which are then :**

**either**

subjected to the action of sodium borohydride, in solution in an alcoholic solvent, to yield compounds of the general formula **($I_B$)** :

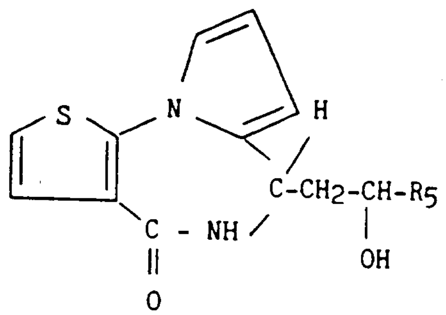

$(I_B)$

in which $R_5$ is as defined for compounds of the general formula (I), which may optionally be subjected to the action of phosgene, in an aromatic organic solvent, with the application of heat, to form compounds of the general formula **($I_C$)**:

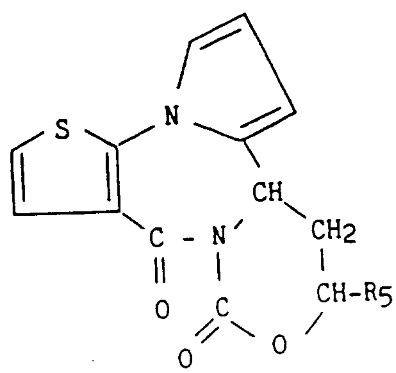

$(I_C)$

in which $R_5$ is as defined for compounds of the general formula (I)
or reacted with a compound of the general formula (XI) :

$R_{13}$ - Cl (XI)

in which $R_{13}$ is as defined for compounds of the general formula (I), to obtain compounds of the general formula **($I_M$)** :

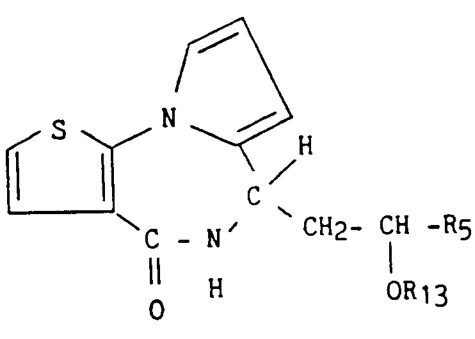

$(I_M)$

in which $R_5$ and $R_{13}$ are as defined for compounds of the general formula (I),

**or**

condensed with a hydroxylamine compound of the general formula (IV) :

$H_2N$ - O - $R_6$ (IV)

in which $R_6$ is as defined for compounds of the general formula (I), to form compounds of the general formula **($I_D$)** :

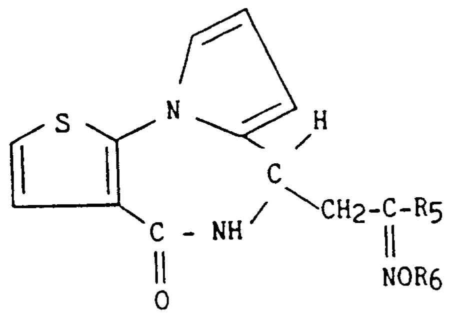

($I_D$)

in which $R_5$ and $R_6$ are as defined for compounds of the general formula (I),

<u>or</u>

condensed with a hydrazine compound of the general formula (XII) :

$H_2N$ - NH - $R_6$ (XII)

in which $R_6$ is as defined for compounds of the general formula (I), to obtain compounds of the general formula **($I_N$)**:

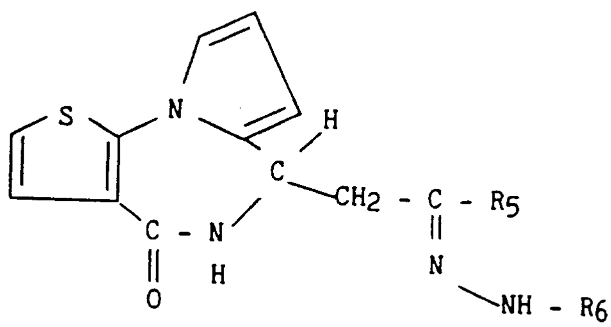

($I_N$)

in which $R_5$ and $R_6$ are as defined for compounds of the general formula (I),

<u>or</u>

with a primary alcohol of the general formula (V) :

$R_3OH$ (V)

in which $R_3$ is as defined for compounds of the general formula (I),

in the presence of a strong mineral base at a temperature of from 30° to 80°C, to obtain compounds of the general formula **($I_E$)** :

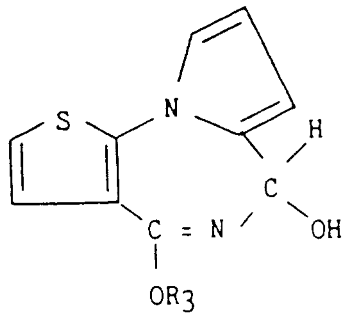

(I$_E$)

in which $R_3$ is as defined for compounds of the general formula (I), which are then subjected to the action of potassium permanganate at room temperature to form compounds of the general formula **(I$_F$)**,

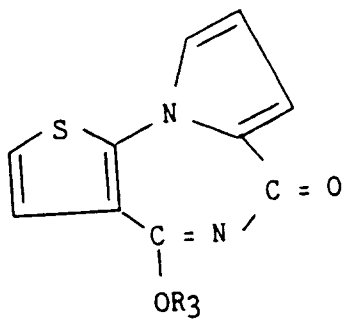

(I$_F$)

in which $R_3$ is as defined for compounds of the general formula (I), which may optionally be subjected to the action of sodium borohydride to obtain compounds of the general formula **(I$_G$)**,

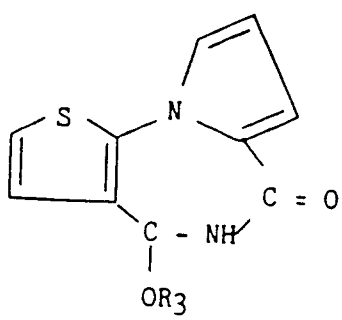

(I$_G$)

in which $R_3$ is as defined for compounds of the general formula (I),

or

2-(2-formylpyrrol-1-yl)-3-thiophene carboxamide, a compound of formula (VI) :

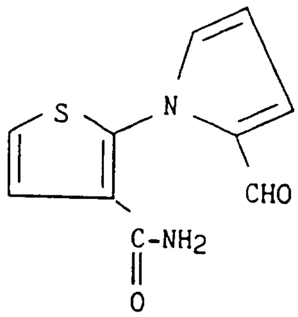

(VI)

is subjected

either

to the action of an amine of the general formula (VII) :

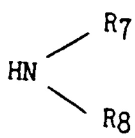

(VII)

in which $R_7$ and $R_8$ are as defined for compounds of the general formula (I), to obtain compounds of the general formula **($I_H$)** :

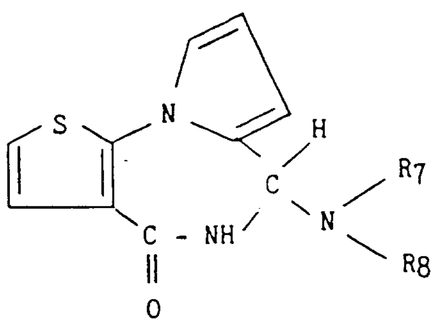

($I_H$)

in which $R_7$ and $R_8$ are as defined for compounds of the general formula (I), which may optionally be subjected, when $R_7$ and $R_8$ together with the nitrogen atom to which they are attached form a morpholino radical, to the action of sodium borohydride, to obtain the compound of the general formula (I) in which $R_2$ represents a methylene radical and $R_1$ represents a radical of the general formula **($Z_0$)** in which $R_{12}$ = H,

or

to the action of triethylamine, in the presence of water and at room temperature, to form the compound of the general formula (I) in which $R_2$ represents a hydroxymethylene radical and $R_1$ represents a radical of the formula **($Z_0$)** in which $R_{12}$ = H,

which is then,

either

reacted with an amine of the general formula (VII) to form a compound of the general formula **($I_H$)**

or

condensed with a compound of the general formula (VIII) :

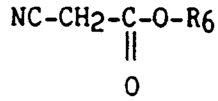

(VIII)

in which $R_6$ is as defined for compounds of the general formula (I), in the presence of triethylamine, to form a compound of the general formula **($I_I$)** :

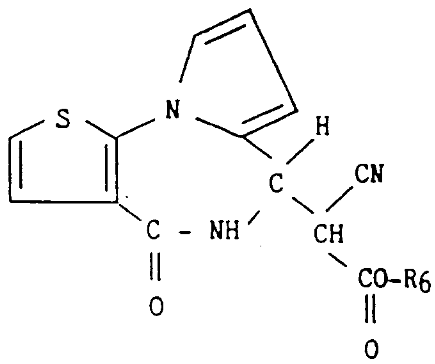

($I_I$)

in which $R_6$ is as defined for compounds of the general formula (I),

<u>or</u>

reacted with an alcohol of the general formula (IX)

$R_5OH$ (IX)

in which $R_5$ is as defined for compounds of the general formula (I), with the application of heat, to obtain a compound of the general formula **($I_J$)** :

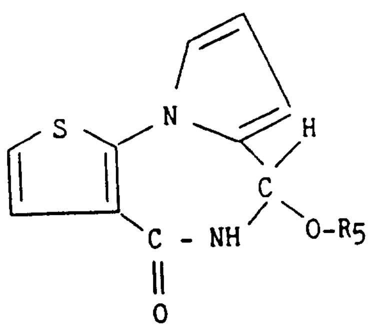

($I_J$)

in which $R_5$ is as defined for compounds of the general formula (I),

<u>or</u>

to the action of a compound of the general formula (X) :

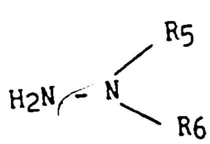

(X)

in which $R_5$ and $R_6$ are as defined for compounds of the general formula (I), to form a compound of the general formula **($I_K$)** :

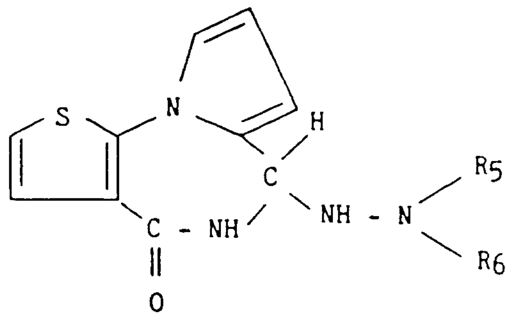

($I_K$)

in which $R_5$ and $R_6$ are as defined for compounds of the general formula (I),

<u>or</u>

to the action of an alcohol of the general formula (IX) to form a compound of the general formula **($I_J$)**,

<u>or</u>

to the action of a thiol of the general formula (XI) :

$R_5SH$ (XI)

in which $R_5$ is as defined for compounds of the general formula (I), at room temperature, to form a compound of the general formula **($I_L$)**,

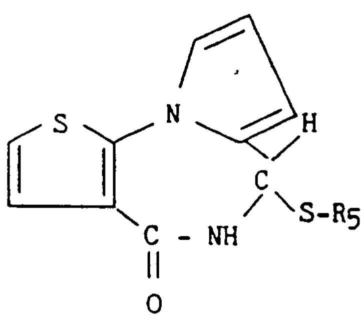

($I_L$)

in which $R_5$ is as defined for compounds of the general formula (I), which may optionally be subjected, when $R_5$ represents a carboxyalkyl radical, to the action of triethylamine and a chloroformate, then to the action of gaseous ammonia, to form the corresponding amide.

**2.** Process according to claim 1) for the preparation of compounds of the general formula **($I_A$)** :

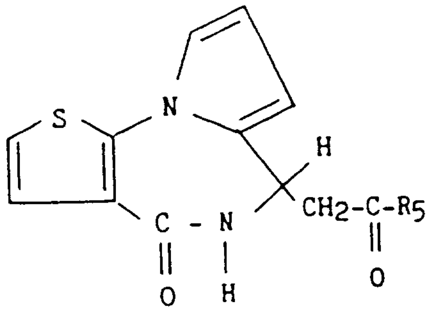

($I_A$)

in which $R_5$ is as defined in claim 1), as well as their isomers, diastereoisomers and enantiomers, isolated or in the form of a mixture.

**3.** Process according to claim 1) for the preparation of compounds of the general formula **($I_B$)** :

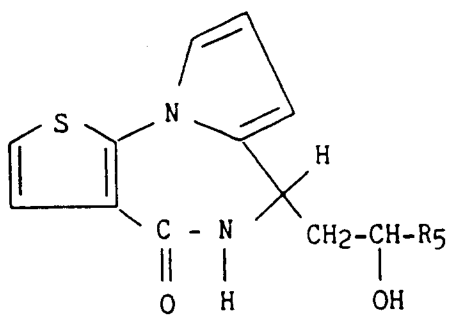

($I_B$)

in which $R_5$ is as defined in claim 1), as well as their isomers, diastereoisomers and enantiomers, isolated or in the form of a mixture.

**4.** Process according to claim 1) for the preparation of compounds of the general formula **($I_C$)** :

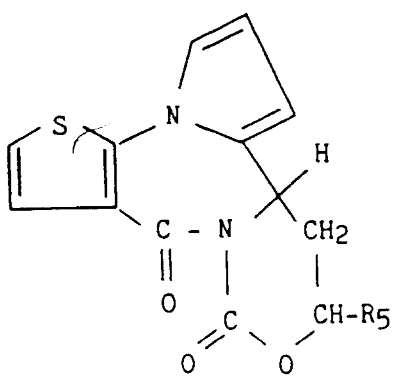

($I_C$)

in which $R_5$ is as defined in claim 1), as well as their isomers, enantiomers and diastereoisomers, isolated or in the form of a mixture.

**5.** Process according to claim 1) for the preparation of compounds of the general formula **($I_D$)** :

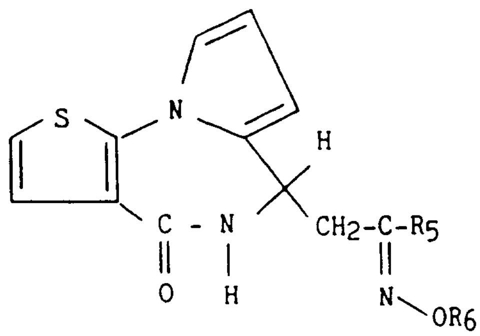

($I_D$)

in which $R_5$ and $R_6$ are as defined in claim 1), as well as their isomers, enantiomers and diastereoisomers, isolated or in the form of a mixture.

**6.** Process according to claim 1) for the preparation of compounds of the general formula **($I_E$)** :

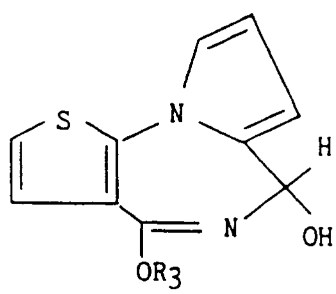

($I_E$)

in which $R_3$ is as defined in claim 1), as well as their isomers, enantiomers and diastereoisomers, isolated or in the form of a mixture.

**7.** Process according to claim 1) for the preparation of compounds of the general formula **($I_F$)** :

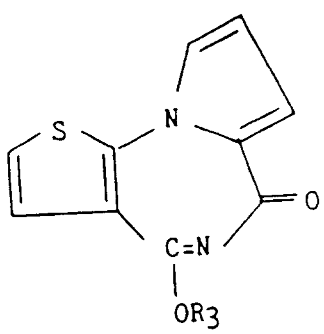

($I_F$)

in which $R_3$ is as defined in claim 1), as well as their isomers, diastereoisomers and enantiomers, isolated or in the form of a mixture.

**8.** Process according to claim 1) for the preparation of compounds of the general formula **($I_G$)** :

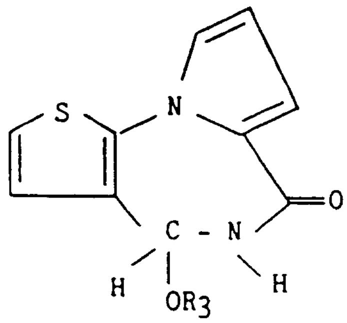

($I_G$)

in which $R_3$ is as defined in claim 1), as well as their isomers, diastereoisomers and enantiomers, isolated or in the form of a mixture.

**9.** Process according to claim 1) for the preparation of compounds of the general formula **($I_H$)** :

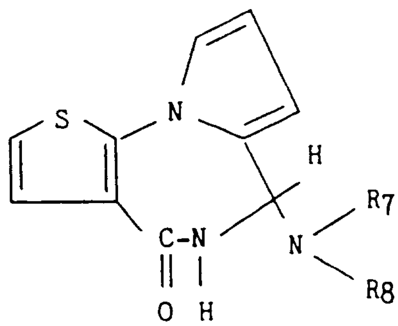

($I_H$)

in which $R_7$ and $R_8$ are as defined in claim 1), as well as their isomers, diastereoisomers and enantiomers, isolated or in the form of a mixture, and their possible addition salts with a pharmaceutically acceptable mineral or organic acid.

**10.** Process according to claim 1) for the preparation of compounds of the general formula **($I_J$)** :

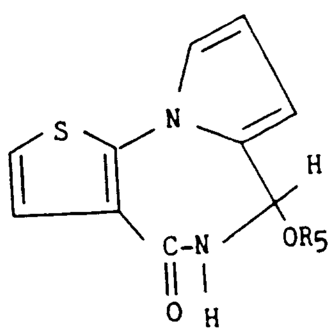

($I_J$)

in which $R_5$ is as defined in claim 1), as well as their isomers, diastereoisomers and enantiomers, isolated or in the form of a mixture.

**11.** Process according to claim 1) for the preparation of compounds of the general formula **($I_K$)** :

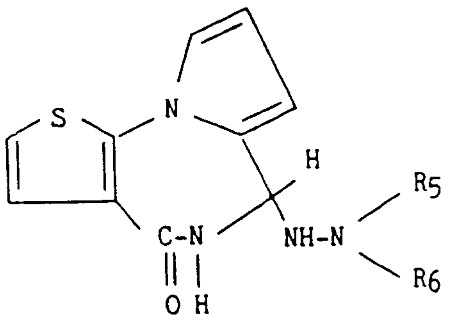

($I_K$)

in which $R_5$ and $R_6$ are as defined in claim 1), as well as their isomers, diastereoisomers and enantiomers, isolated or in the form of a mixture, and their possible addition salts with a pharmaceutically acceptable mineral or organic acid.

**12.** Process according to claim 1) for the preparation of compounds of the general formula **($I_L$)** :

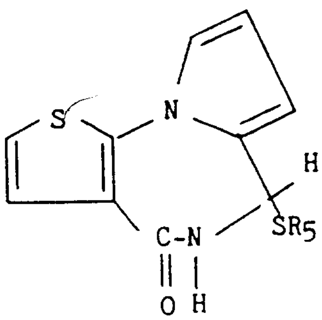

($I_L$)

in which $R_5$ is as defined in claim 1), as well as their isomers, diastereoisomers and enantiomers, isolated or in the form of a mixture.

**13.** Process according to claim 1) for the preparation of the compound 5,6-dihydro-6-(2-benzyloxypropyl)-4-oxo-4H-pyrrolo[1,2-a]thieno[3,2-f][1,4]diazepine, as well as its diastereoisomers, isolated or in the form of a mixture.

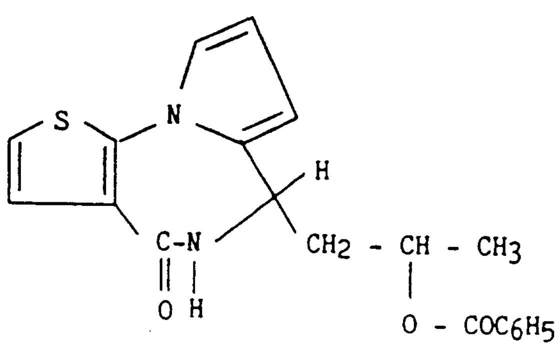

**14.** Process according to claim 1) for the preparation of the compound 5,6-dihydro-6-(2-hydrazono-1-pentyl)-4-oxo-4H-pyrrolo[1,2-a]thieno[3,2-f][1,4]diazepine, as well as its enantiomers, isolated or in the

form of a mixture, and its addition salts with a pharmaceutically acceptable mineral or organic acid

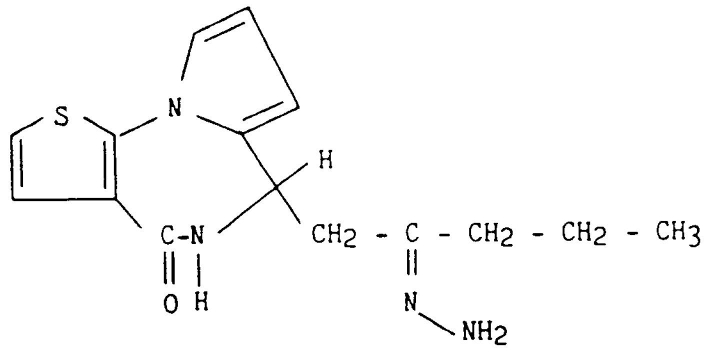

**15.** Process according to claims 1) and 10) for the preparation of the compound 5,6-dihydro-6-hydroxy-4-oxo-4H-pyrrolo[1,2-a]thieno[3,2-f][1,4]diazepine, as well as its enantiomers isolated or in the form of a mixture.

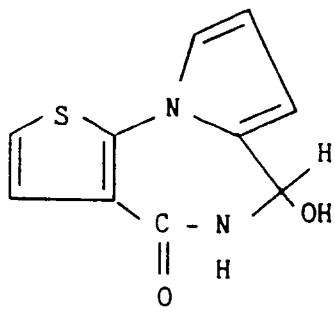

**16.** Process according to claim 1) for the preparation of the compound 5,6-dihydro-4-oxo-4H-pyrrolo[1,2-a]-thieno[3,2-f][1,4]diazepine

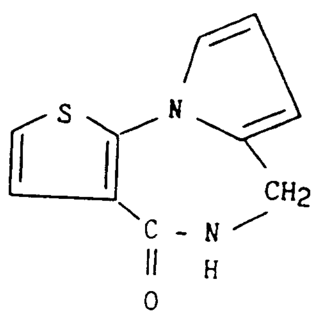

**17.** Process for the preparation of pharmaceutical compositions comprising as active ingredient a compound prepared according to any one of claims 1) to 16), alone or in combination with one or more pharmaceutically acceptable, inert, non-toxic excipients or carriers.

**18.** Process according to claim 17) for the preparation of pharmaceutical compositions presented in a form suitable especially for the treatment of cerebral vascular accidents, normal or pathological cerebral

ageing, and ischaemic syndromes.

**19.** Process according to claim 17) for the preparation pharmaceutical compositions presented in a form suitable especially for the treatment of gastrointestinal disorders, disorders of the pancreas, of the gall bladder, of the appetite and of the nervous system, and pain.

**20.** Process according to claim 17 for the preparation of pharmaceutical compositions presented in a form suitable especially for the treatment of hyperlipidaemia, hypertriglyceridaemia, hypercholesterolaemia, hyperglycaemia, hypertension, and associated disorders.

**Patentansprüche**
**Patentansprüche für folgende Vertragsstaaten : AT, BE, CH, DE, DK, FR, GB, IT, LI, LU, NL, SE**

**1.** Verbindungen der allgemeinen Formel (I):

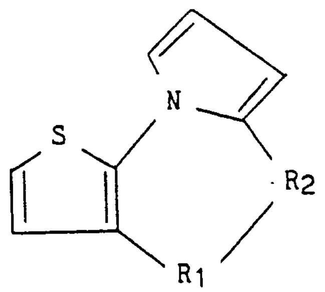

(I)

in der:

- $R_1$ eine Gruppe der allgemeinen Formel ($Z_0$), ($Z_1$), ($Z_2$), ($Z_3$) oder ($Z_4$)

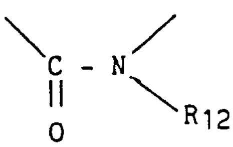

($Z_0$)

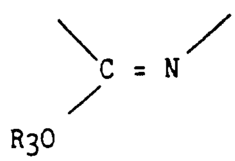

($Z_1$)

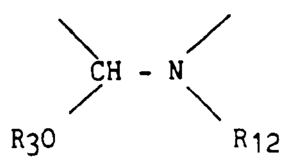

($Z_2$)

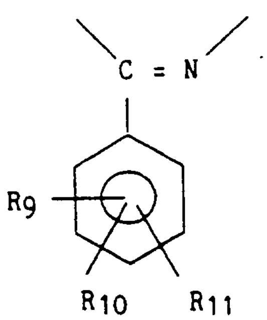

($Z_3$)

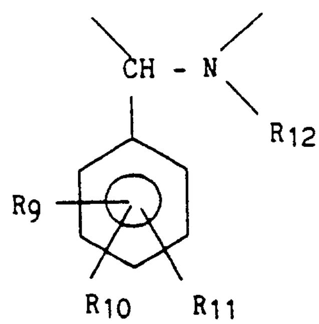

($Z_4$)

worin

- $R_3$ ein Wasserstoffatom, eine geradkettige oder verzweigte Alkylgruppe mit 1 bis 6 Kohlenstoffatomen, die gesättigt sein oder eine Doppelbindung aufweisen kann,
- $R_9$, $R_{10}$, $R_{11}$, die gleichartig oder verschieden sein können, jeweils ein Wasserstoffatom, ein Halogenatom oder eine Alkylgruppe mit 1 bis 6 Kohlenstoffatomen,
- $R_{12}$ ein Wasserstoffatom oder gemeinsam mit $R_2$ und dem Stickstoffatom, an die sie gebunden ist, eine Gruppe der allgemeinen Formel (W):

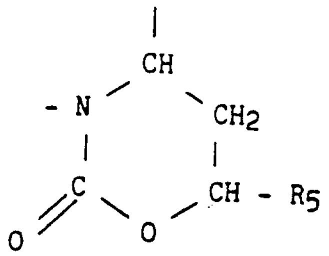

(W)

darstellen,

und

- $R_2$ eine Methylengruppe, eine Hydroxymethylengruppe, eine Carbonylgruppe, eine Gruppe der allgemeinen Formel ($Y_1$), ($Y_2$), ($Y_3$) oder ($Y_4$):

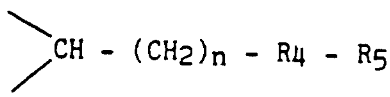

($Y_1$)

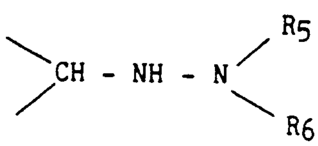

($Y_2$)

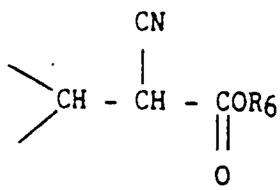

($Y_3$)

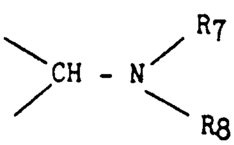

($Y_4$)

oder gemeinsam mit $R_{12}$ und dem Stickstoffatom, an die sie gebunden ist, eine Gruppe der allgemeinen Formel (W):

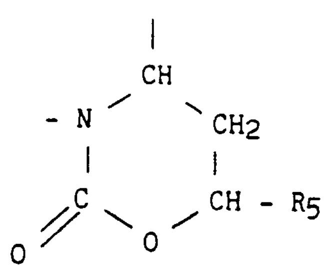

(W)

bedeuten, in welchen Formeln:

- $R_4$ ein Sauerstoffatom oder ein Schwefelatom, eine Carbonylgruppe, eine Gruppe der allgemeinen Formel ($X_1$), ($X_2$), ($X_3$) oder ($X_4$):

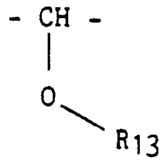 ($X_1$)

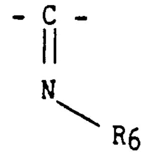 ($X_2$)

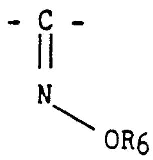 ($X_3$)

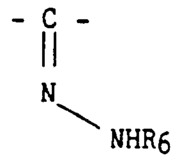 ($X_4$)

- n eine Zahl zwischen 0 und 4 einschließlich,
- $R_5$ ein Wasserstoffatom, eine geradkettige oder verzweigte Alkylkette mit 1 bis 10 Kohlenstoffatomen, die gegebenenfalls durch ein oder mehrere Sauerstoff- oder Schwefelatome unterbrochen ist, eine Phenyl-, Benzoyl- oder Arylkylgruppe mit 7 bis 10 Kohlenstoffatomen (die gegebenenfalls am aromatischen Kern durch ein oder mehrere Halogenatome, geradkettige oder verzweigte Alkylgruppen mit 1 bis 6 Kohlenstoffatomen, Nitrogruppen oder geradkettige oder verzweigte Alkoxygruppen mit 1 bis 6 Kohlenstoffatomen substituiert sind), eine geradkettige oder verzweigte Carboxyalkylgruppe mit 2 bis 7 Kohlenstoffatomen, eine geradkettige oder verzweigte Alkoxycarbonylalkylgruppe mit 3 bis 10 Kohlenstoffatomen, eine geradkettige oder verzweigte Alkoxycarbonylgruppe mit 2 bis 7 Kohlenstoffatomen, eine geradkettige oder verzweigte Carbamoylalkylgruppe mit 2 bis 7 Kohlenstoffatomen, eine Cycloalkylgruppe mit 3 bis 7 Kohlenstoffatomen, ein ungesättigtes cyclisches System mit 5 bis 7 Kettengliedern, das mindestens ein Heteroatom ausgewählt aus Stickstoff, Schwefel und Sauerstoff umfaßt, eine Pyridinylcarbonylgruppe, eine Pyrimidylcarbonylgruppe, eine Clofibroylgruppe oder eine 6-Hydroxy-2,5,7,8-tetramethyl-chroman-2-carbonylgruppe,
- $R_6$ ein Wasserstoffatom oder eine geradkettige oder verzweigte Alkylgruppe mit 1 bis 6 Kohlenstoffatomen,
- $R_7$ und $R_8$, die gleichartig oder verschieden sein können, jeweils ein Wasserstoffatom, eine geradkettige oder verzweigte Alkylgruppe mit 1 bis 6 Kohlenstoffatomen, eine Phenylgruppe oder Phenylalkylgruppe mit 7 bis 9 Kohlenstoffatomen (die gegebenenfalls am aromatischen Kern durch ein oder mehrere Halogenatome oder Alkyl- oder Alkoxygruppen mit 1 bis 6 Kohlenstoffatomen substituiert sind) oder gemeinsam mit dem Stickstoffatom, an das sie gebunden sind, ein gesättigtes oder ungesättigtes, cyclisches System mit 5 bis 7 Kettengliedern, welches 1 bis 2 Heteroatome ausgewählt aus Stickstoff, Sauerstoff und Schwefel umfaßt und gegebenenfalls durch eine Alkylcarbonyl- oder Alkoxycarbonylgruppe mit 2 bis 5 Kohlenstoffatmen substituiert ist,
- $R_{13}$ ein Wasserstoffatom, eine geradkettige oder verzweigte Alkylcarbonylgruppe mit 2 bis 6 Kohlenstoffatomen oder eine Benzoylgruppe bedeuten,

- deren Isomeren, Diastereoisomeren, Enantiomeren und
- deren Additionssalze mit einer anorganischen oder organischen, pharmazeutisch annehmbaren Säure,

mit den Maßgaben, daß:

- wenn $R_1$ eine Gruppe der allgemeinen Formel ($Z_3$) bedeutet, $R_2$ keine Methylengruppe darstellt,
- wenn $R_1$ eine Gruppe der allgemeinen Formel ($Z_0$) und $R_{12}$ ein Wasserstoffatom darstellen, $R_2$ nicht die folgenden Gruppen bedeuten kann:
  $>CH-O-CH_3$, $>CH-O-CH_2CH_3$, $>CH-O-CH_2CH_2CH_3$, $>CH-O-CH_2CH_2-O-CH_2CH_3$, $>CH-O-CH_2CH_2-O-CH_2CH_2CH_2CH_3$
- wenn $R_1$ eine Gruppe der allgemeinen Formel ($Z_0$), $R_{12}$ ein Wasserstoffatom und $R_2$ eine Gruppe der allgemeinen Formel ($Y_1$) darstellen, worin n = 1 und $R_4$ eine Carbonylgruppe bedeuten, $R_5$ keine Methylgruppe, Isopropylgruppe oder Phenylgruppe darstellt,

- wenn $R_1$ eine Gruppe der allgemeinen Formel ($Z_0$) und $R_{12}$ ein Wasserstoffatom darstellen, $R_2$ keine Carbonylgruppe bedeutet.

**2.** Verbindungen nach Anspruch 1 der allgemeinen Formel ($I_A$):

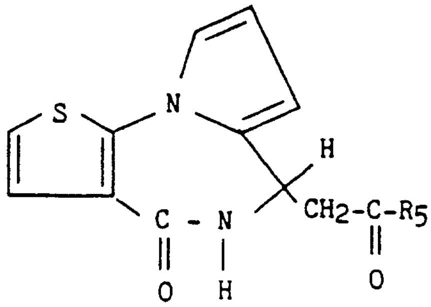

($I_A$)

in der $R_5$ die in Anspruch 1 angegebenen Bedeutungen besitzt, sowie deren Isomeren, Diastereoisomeren und Enantiomeren in isolierter Form oder in Form einer Mischung.

**3.** Verbindungen nach Anspruch 1 der allgemeinen Formel ($I_B$):

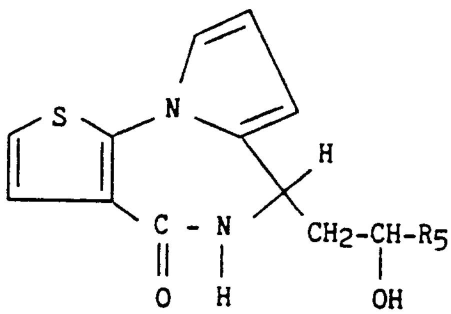

($I_B$)

in der $R_5$ die in Anspruch 1 angegebenen Bedeutungen besitzt, sowie deren Isomeren, Diastereoisomeren und Enantiomeren in isolierter Form oder in Form einer Mischung.

**4.** Verbindungen nach Anspruch 1 der allgemeinen Formel ($I_C$):

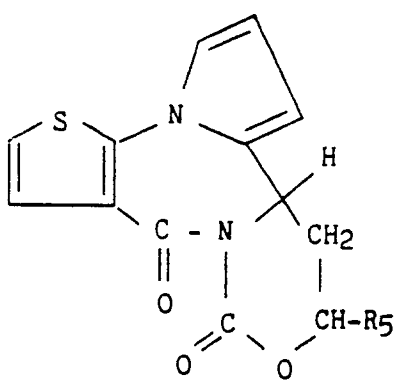

($I_C$)

in der $R_5$ die in Anspruch 1 angegebenen Bedeutungen besitzt, sowie deren Isomeren, Diastereoisomeren und Enantiomeren in isolierter Form oder in Form einer Mischung.

5. Verbindungen nach Anspruch 1 der allgemeinen Formel ($I_D$):

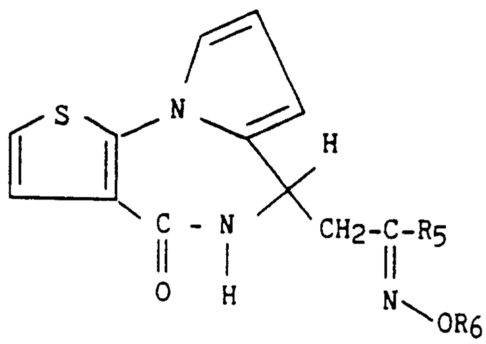

($I_D$)

in der $R_5$ und $R_6$ die in Anspruch 1 angegebenen Bedeutungen besitzen, sowie deren Isomeren Enantiomeren und Diastereoisomeren in isolierter Form oder in Form einer Mischung.

6. Verbindungen nach Anspruch 1 der allgemeinen Formel ($I_E$):

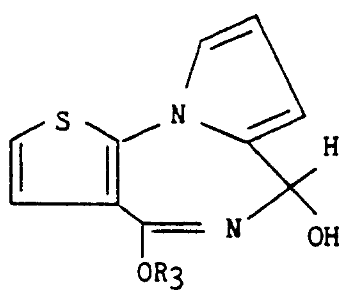

($I_E$)

in der $R_3$ die in Anspruch 1 angegebenen Bedeutungen besitzt, sowie deren Isomeren, Diastereoisomeren und Enantiomeren in isolierter Form oder in Form einer Mischung.

7. Verbindungen nach Anspruch 1 der allgemeinen Formel ($I_F$):

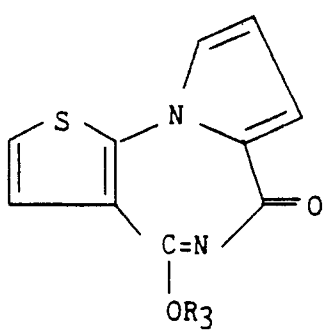

($I_F$)

in der $R_3$ die in Anspruch 1 angegebenen Bedeutungen besitzt, sowie deren Isomeren, Diastereoisomeren und Enantiomeren in isolierter Form oder in Form einer Mischung.

**8.** Verbindungen nach Anspruch 1 der allgemeinen Formel ($I_G$):

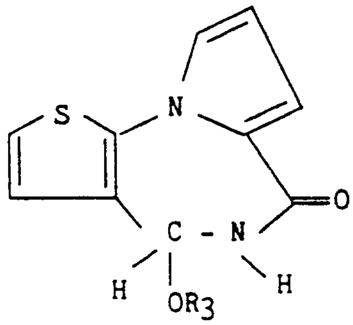

($I_G$)

in der $R_3$ die in Anspruch 1 angegebenen Bedeutungen besitzt, sowie deren Isomeren, Diastereoisomeren und Enantiomeren in isolierter Form oder in Form einer Mischung.

**9.** Verbindungen nach Anspruch 1 der allgemeinen Formel ($I_H$):

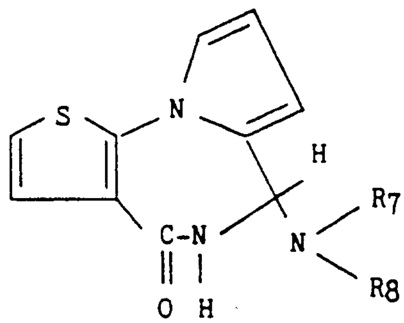

($I_H$)

in der $R_7$ und $R_8$ die in Anspruch 1 angegebenen Bedeutungen besitzen, sowie deren Isomeren, Diastereoisomeren und Enantiomeren in isolierter Form oder in Form einer Mischung und deren mögliche Salze mit einer anorganischen oder organischen, pharmazeutisch annehmbaren Säure.

**10.** Verbindungen nach Anspruch 1 der allgemeinen Formel ($I_J$):

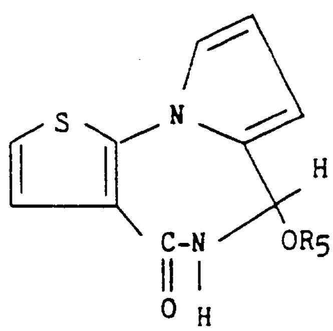

($I_J$)

in der $R_5$ die in Anspruch 1 angegebenen Bedeutungen besitzt, sowie deren Isomeren, Diastereoisomeren und Enantiomeren in isolierter Form oder in Form einer Mischung.

**11.** Verbindungen nach Anspruch 1 der allgemeinen Formel ($I_K$):

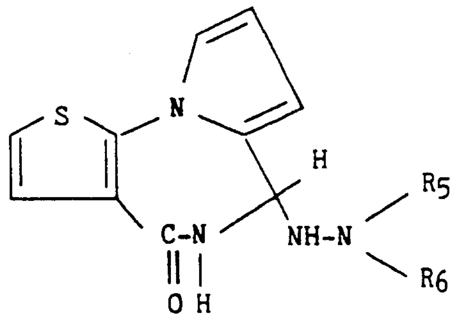

($I_K$)

in der $R_5$ und $R_6$ die in Anspruch 1 angegebenen Bedeutungen besitzen, sowie deren Isomeren. Diastereoisomeren und Enantiomeren in isolierter Form oder in Form einer Mischung und deren mögliche Salze mit einer anorganischen oder organischen, pharmazeutisch annehmbaren Saure.

**12.** Verbindungen nach Anspruch 1 der allgemeinen Formel ($I_L$):

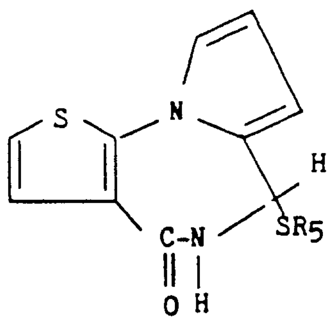

($I_L$)

in der $R_5$ die in Anspruch 1 angegebenen Bedeutungen besitzen, sowie deren Isomeren, Diastereoisomeren und Enantiomeren in isolierter Form oder in Form einer Mischung.

**13.** Verbindung nach Anspruch 1, nämlich 5,6-Dihydro-6-(2-benzyloxypropyl)-4-oxo-4H-pyrrolo[1,2-a]thieno-[3,2-f][1,4]diazepin

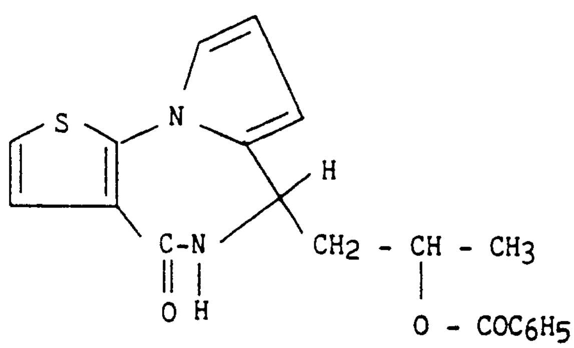

sowie dessen Diastereoisomeren in isolierter Form oder in Form einer Mischung.

**14.** Verbindung nach Anspruch 1, nämlich 5,6-Dihydro-6-(2-hydrazono-1-pentyl)-4-oxo-4H-pyrrolo[1,2-a]-thieno[3,2-f][1,4]diazepin

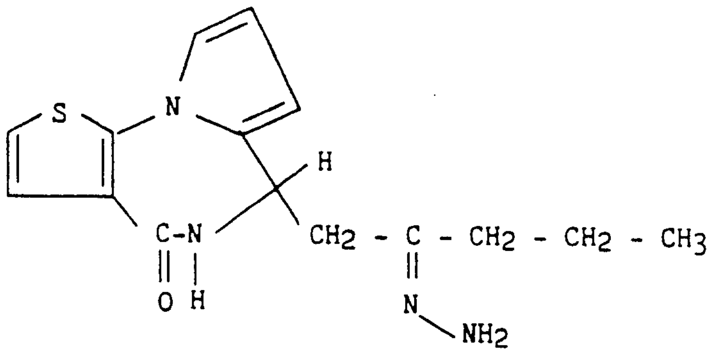

und dessen Enantiomeren in isolierter Form oder in Form einer Mischung sowie dessen Additionssalze mit einer anorganischen oder organischen, pharmazeutisch annehmbaren Säure.

**15.** Verbindung nach den Ansprüchen 1 und 10, nämlich 5,6-Dihydro-6-hydroxy-4-oxo-4H-pyrrolo[1,2-a]-thieno[3,2-f][1,4]diazepin

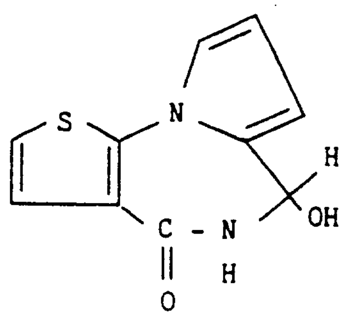

sowie dessen Enantiomeren in isolierter Form oder in Form einer Mischung.

**16.** Verbindung nach Anspruch 1, nämlich 5,6-Dihydro-4-oxo-4H-pyrrolo[1,2-a]thieno[3,2-f][1,4]diazepin

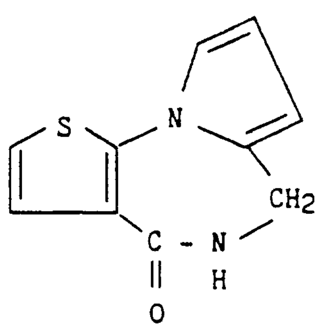

**17.** Verfahren zur Herstellung der Verbindungen der allgemeinen Formel (I), **dadurch gekennzeichnet**, daß man:

entweder

- 3-Cyano-2-(2-formyl-pyrrol-1-yl)-thiophen der Formel (II):

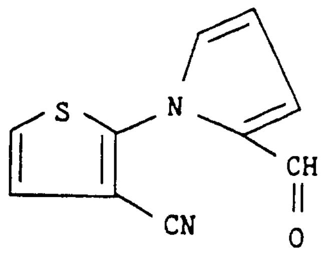

(II)

**entweder**

- mit einem Methylketon der allgemeinen Formel (III):

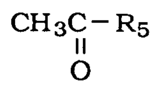

(III)

in der $R_5$ die bezüglich der Derivate der allgemeinen Formel (I) angegebenen Bedeutungen besitzt, in Gegenwart einer starken anorganischen Base und von Wasserstoffperoxid umsetzt, so daß man die Verbindungen der allgemeinen Formel ($I_A$) erhält:

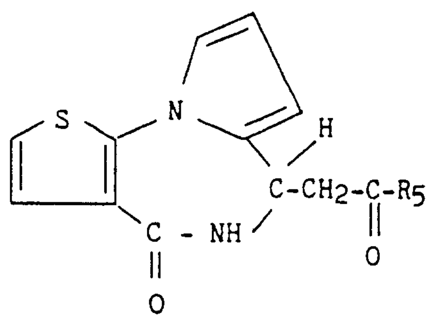

($I_A$)

in der $R_5$ die bezüglich der Derivate der allgemeinen Formel (I) angegebenen Bedeutungen besitzt, welche anschließend:

entweder

der Einwirkung von Natriumborhydrid in Lösung in einem alkoholischen Lösungsmittel unterworfen werden, so daß man die Verbindungen der allgemeinen Formel ($I_B$) erhält:

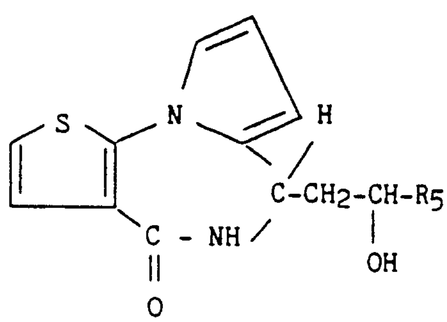

($I_B$)

in der $R_5$ die bezüglich der Derivate der allgemeinen Formel (I) angegebenen Bedeutungen besitzt, welche man gegebenenfalls der Einwirkung von Phosgen in einem aromatischen organischen Lösungsmittel in der Wärme unterwerfen kann zur Bildung der Verbindungen der allgemeinen Formel ($I_C$):

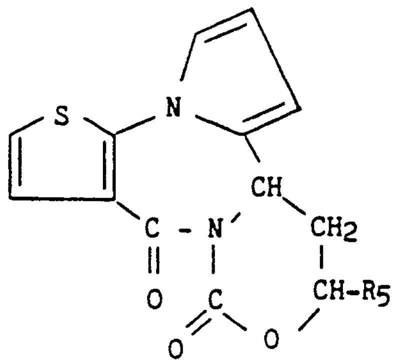

(Ic)

in der $R_5$ die bezüglich der Verbindungen der allgemeinen Formel (I) angegebenen Bedeutungen besitzt,
oder welche man mit einer Verbindung der allgemeinen Formel (XI):

$R_{13}$-Cl (XI)

in der $R_{13}$ die bezüglich der Verbindungen der allgemeinen Formel (I) angegebenen Bedeutungen besitzt, umsetzen kann, so daß man die Verbindungen der allgemeinen Formel ($I_M$) erhält:

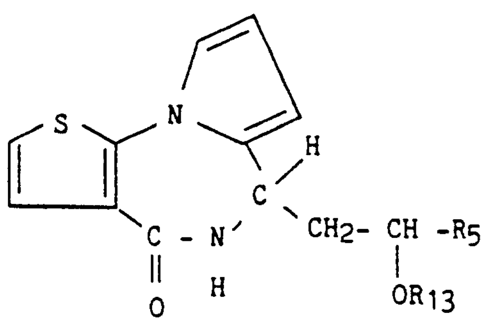

($I_M$)

in der $R_5$ und $R_{13}$ die bezüglich der Verbindungen der allgemeinen Formel (I) angegebenen Bedeutungen besitzen,
oder
mit einem Hydroxylaminderivat der allgemeinen Formel (IV):

$H_2N$ - O - $R_6$ (IV)

in der $R_6$ die bezüglich der Derivate der allgemeinen Formel (I) angegebenen Bedeutungen besitzt, kondensiert zur Bildung der Verbindungen der allgemeinen Formel ($I_D$):

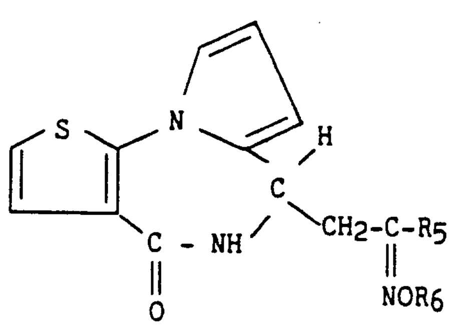

($I_D$)

in der $R_5$ und $R_6$ die bezüglich der Derivate der allgemeinen Formel (I) angegebenen Bedeutungen besitzen,

oder

mit einem Hydrazinderivat der allgemeinen Formel (XII):

$H_2N - NH - R_6$ (XII)

in der $R_6$ die bezüglich der Verbindungen der allgemeinen Formel (I) angegebenen Bedeutungen besitzt, kondensiert werden, so daß man die Verbindungen der allgemeinen Formel ($I_N$) erhält:

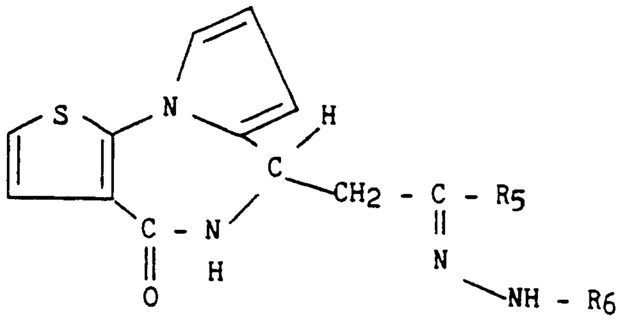

($I_N$)

in der $R_5$ und $R_6$ die bezüglich der Verbindungen der allgemeinen Formel (I) angegebenen Bedeutungen besitzen,

**oder**

mit einem primären Alkohol der allgemeinen Formel (V):

$R_3OH$ (V)

in der $R_3$ die bezüglich der Verbindungen der allgemeinen Formel (I) angegebenen Bedeutungen besitzt,

in Gegenwart einer starken anorganischen Base bei einer Temperatur zwischen 30° - 80°C umsetzt, so daß man die Verbindungen der allgemeinen Formel ($I_E$) erhält:

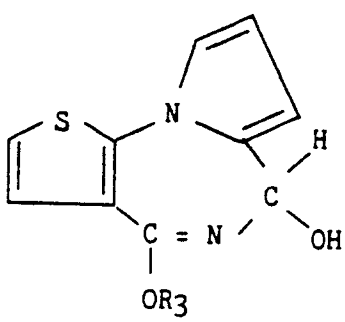

($I_E$)

in der $R_3$ die bezüglich der Verbindungen der allgemeinen Formel (I) angegebenen Bedeutungen besitzt, welche man anschließend der Einwirkung von Kaliumpermanganat bei Raumtemperatur unterwirft zur Bildung der Verbindungen der allgemeinen Formel ($I_F$):

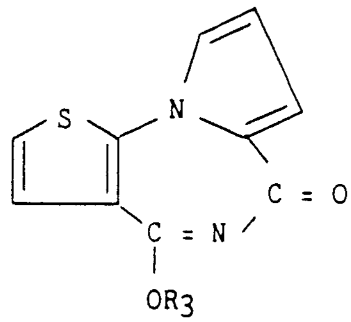

(IF)

in der $R_3$ die bezüglich der Verbindungen der allgemeinen Formel (I) angegebenen Bedeutungen besitzt, die man gegebenenfalls der Einwirkung von Natriumborhydrid unterwerfen kann zur Bildung der Verbindungen der allgemeinen Formel ($I_G$):

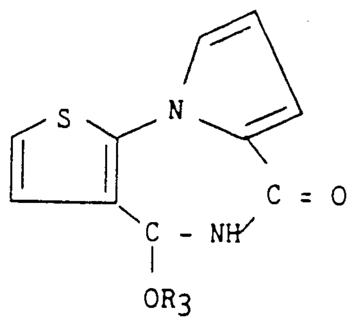

(IG)

in der $R_3$ die bezüglich der Verbindungen der allgemeinen Formel (I) angegebenen Bedeutungen besitzt,

oder

2-(2-Formyl-pyrrol-1-yl)-3-thiophen-carboxamid der Formel (VI):

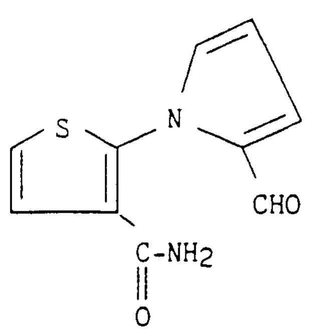

(VI)

**entweder**

der Einwirkung eines Amins der allgemeinen Formel (VII):

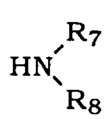

(VII)

in der $R_7$ und $R_8$ die bezüglich der Verbindungen der allgemeinen Formel (I) angegebenen Bedeutungen besitzen, unterwirft, so daß man die Verbindungen der allgemeinen Formel ($I_H$) erhält:

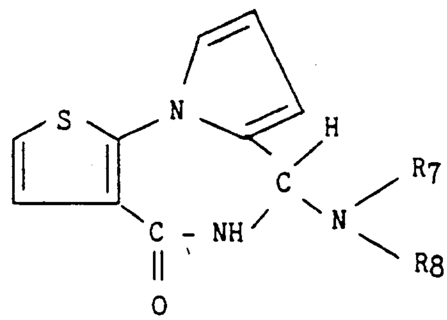

($I_H$)

in der $R_7$ und $R_8$ die bezüglich der Verbindungen der allgemeinen Formel (I) angegebenen Bedeutungen besitzen, welche man gegebenenfalls, wenn $R_7$ und $R_8$ gemeinsam mit dem Stickstoffatom, an das sie gebunden sind, eine Morpholinogruppe bilden, der Einwirkung von Natriumborhydrid unterwerfen kann zur Bildung der Verbindung der allgemeinen Formel (I), in der $R_2$ eine Methylengruppe und $R_1$ eine Gruppe der allgemeinen Formel ($Z_0$), worin $R_{12}$ = H darstellt, bedeuten,

oder

der Einwirkung von Triethylamin in Gegenwart von Wasser und bei Raumtemperatur unterwirft zur Bildung der Verbindung der allgemeinen Formel (I), in der $R_2$ eine Hydroxymethylengruppe und $R_1$ eine Gruppe der Formel ($Z_0$), worin $R_{12}$ = H darstellt, bedeuten,

welche man anschließend

entweder

mit einem Amin der allgemeinen Formel (VII) umsetzt zur Bildung der Verbindungen der allgemeinen Formel ($I_H$),

oder

mit einer Verbindung der allgemeinen Formel (VIII):

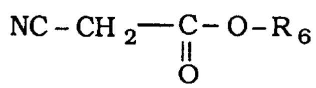

(VIII)

in der $R_6$ die bezüglich der Verbindungen der allgemeinen Formel (I) angegebenen Bedeutungen besitzt, in Gegenwart von Triethylamin kondensiert zur Bildung der Verbindungen der allgemeinen Formel ($I_I$):

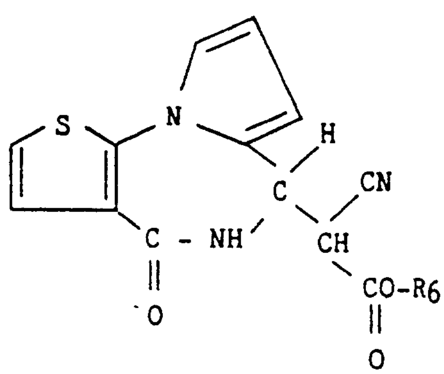

($I_I$)

in der $R_6$ die bezüglich der Verbindungen der allgemeinen Formel (I) angegebenen Bedeutungen besitzt,

oder

mit einem Alkohol der allgemeinen Formel (IX)

$R_5OH$ (IX)

in der $R_5$ die bezüglich der Verbindungen der Verbindungend er allgemeinen Formel (I) angegebenen Bedeutungen besitzt, in der Wärme umsetzt zur Bildung der Verbindungen der allgemeinen Formel ($I_J$):

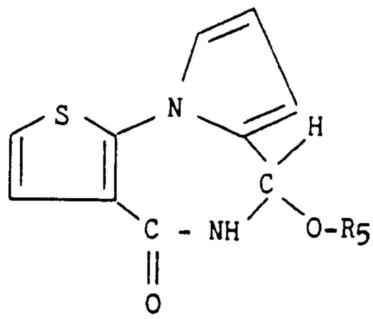

($I_J$)

in der $R_5$ die bezüglich der Derivate der allgemeinen Formel (I) angegebenen Bedeutungen besitzt,

**oder**

der Einwirkung einer Verbindung der allgemeinen Formel (X):

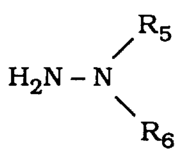

(X)

in der $R_5$ und $R_6$ die bezüglich der Verbindungen der allgemeinen Formel (I) angegebenen Bedeutungen besitzen, unterwirft zur Bildung der Verbindungen der allgemeinen Formel ($I_K$):

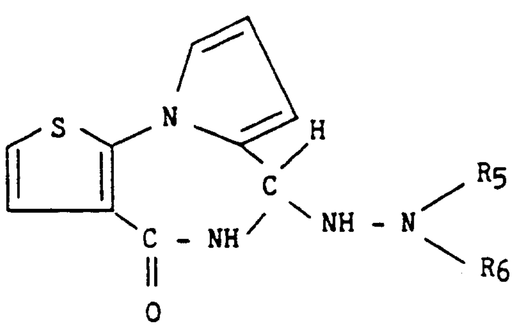

($I_K$)

in der $R_5$ und $R_6$ die bezüglich der Verbindungen der allgemeinen Formel (I) angegebenen Bedeutungen besitzen,

**oder**

der Einwirkung eines Alkohols der allgemeinen Formel (IX) unterwirft zur Bildung der Verbindungen der allgemeinen Formel ($I_J$),

**oder**

der Einwirkung eines Thiols der allgemeinen Formel (XI):

$R_5SH$ (XI)

in der $R_5$ die bezüglich der Verbindungen der allgemeinen Formel (I) angegebenen Bedeutungen besitzt, bei Raumtemperatur unterwirft zur Bildung der Verbindungen der allgemeinen Formel ($I_L$):

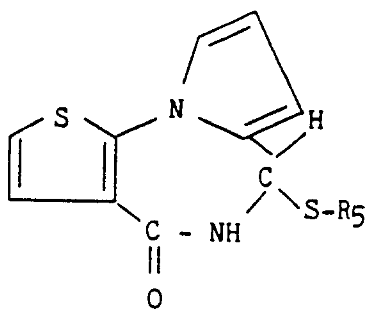

($I_L$)

in der $R_5$ die bezüglich der Verbindungen der allgemeinen Formel (I) angegebenen Bedeutungen besitzt, welche man gegebenenfalls, wenn $R_5$ eine Carboxyalkylgruppe darstellt, der Einwirkung von Triethylamin oder eines Chlorameisensäureesters und dann der Einwirkung von gasförmigem Ammoniak unterziehen kann zur Bildung der entsprechenden Amide,
wobei es sich bei versteht, daß die Verbindungen der allgemeinen Formel ($I_A$), ($I_B$), ($I_C$), ($I_D$), ($I_E$), ($I_F$), ($I_G$), ($I_H$), ($I_I$), ($I_J$), ($I_K$), ($I_L$), ($I_M$) und ($I_N$) Gegenstand der Erfindung sind und den Verbindungen der allgemeinen Formel (I) angehören.

**18.** Pharmazeutische Zubereitungen enthaltend als Wirkstoff eine Verbindung nach den Ansprüchen 1 bis 16 allein oder in Kombination mit einem oder mehreren inerten, nichttoxischen, pharmazeutisch annehmbaren Trägermaterialien oder Bindemitteln.

**19.** Pharmazeutische Zubereitungen nach Anspruch 18, die in einer Form vorliegen, die insbesondere zur Behandlung von Hirngefäßunfällen, des normalen oder pathologischen Alterns des Gehirns und von ischämischen Syndromen geeignet ist.

**20.** Pharmazeutische Zubereitungen nach Anspruch 18, die in einer Form vorliegen, die insbesondere zur Behandlung von Störungen des Magen-Darm-Trakts, des Pankreas, der Gallenblase, des Appetis, des Zentralnervensystems sowie von Schmerzen geeignet ist.

**21.** Pharmazeutische Zubereitungen nach Anspruch 18, die in einer Form vorliegen, die insbesondere zur Behandlung von Hyperlipidämie, Hypertriglyceridämie, Hypercholesterolämie, Hyperglykämie, der Hypertension und von damit verknüpften Erkrankungen geeignet ist.

**Patentansprüche für folgenden Vertragsstaat : ES**

**1.** Verfahren zur Herstellung von Verbindungen der allgemeinen Formel (I):

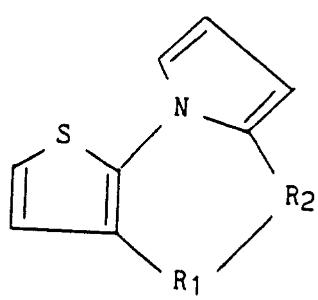

(I)

in der:

- $R_1$ eine Gruppe der allgemeinen Formel ($Z_0$), ($Z_1$), ($Z_2$), ($Z_3$) oder ($Z_4$)

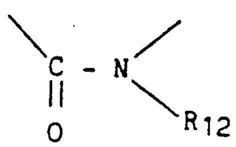 ($Z_0$) 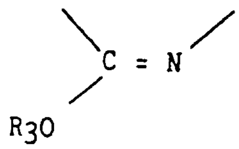 ($Z_1$) 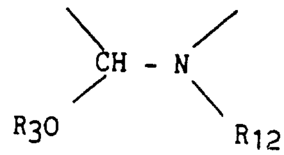 ($Z_2$)

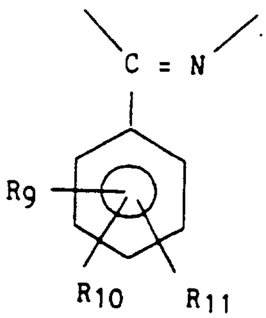 ($Z_3$) 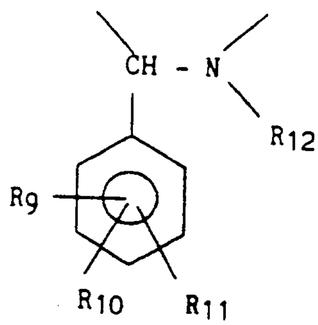 ($Z_4$)

worin

- $R_3$ ein Wasserstoffatom, eine geradkettige oder verzweigte Alkylgruppe mit 1 bis 6 Kohlenstoffatomen, die gesättigt sein oder eine Doppelbindung aufweisen kann,
- $R_9$, $R_{10}$, $R_{11}$, die gleichartig oder verschieden sein können, jeweils ein Wasserstoffatom, ein Halogenatom oder eine Alkylgruppe mit 1 bis 6 Kohlenstoffatomen,
- $R_{12}$ ein Wasserstoffatom oder gemeinsam mit $R_2$ und dem Stickstoffatom, an die sie gebunden ist, eine Gruppe der allgemeinen Formel (W):

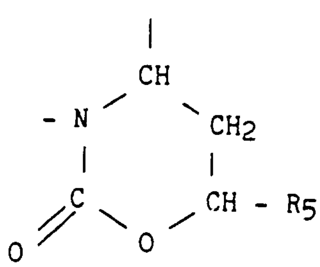 (W)

darstellen,

und

- $R_2$ eine Methylengruppe, eine Hydroxymethylengruppe, eine Carbonylgruppe, eine Gruppe der allgemeinen Formel ($Y_1$), ($Y_2$), ($Y_3$) oder ($Y_4$):

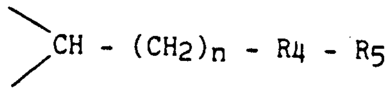 (Y1)

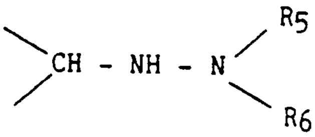 (Y2)

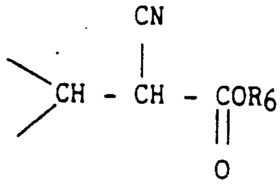 (Y3)

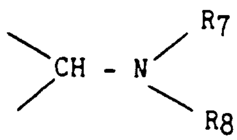 (Y4)

oder gemeinsam mit $R_{12}$ und dem Stickstoffatom, an die sie gebunden ist, eine Gruppe der allgemeinen Formel (W):

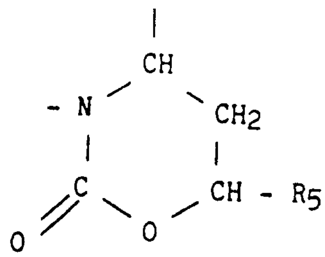 (W)

bedeuten, in welchen Formeln:

- $R_4$ ein Sauerstoffatom oder ein Schwefelatom, eine Carbonylgruppe, eine Gruppe der allgemeinen Formel ($X_1$), ($X_2$), ($X_3$) oder ($X_4$):

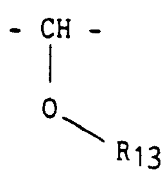 (X1)

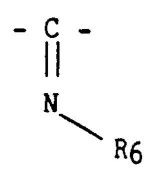 (X2)

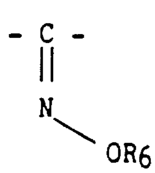 (X3)

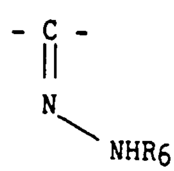 (X4)

- n eine Zahl zwischen 0 und 4 einschließlich,
- $R_5$ ein Wasserstoffatom, eine geradkettige oder verzweigte Alkylkette mit 1 bis 10 Kohlenstoffatomen, die gegebenenfalls durch ein oder mehrere Sauerstoff- oder Schwefelatome unterbrochen ist, eine Phenyl-, Benzoyl- oder Arylkylgruppe mit 7 bis 10 Kohlenstoffatomen (die gegebenenfalls am aromatischen Kern durch ein oder mehrere Halogenatome, geradkettige oder verzweigte Alkylgruppen mit 1 bis 6 Kohlenstoffatomen, Nitrogruppen oder geradkettige oder verzweigte Alkoxygruppen mit 1 bis 6 Kohlenstoffatomen substituiert sind), eine geradkettige oder verzweigte Carboxyalkylgruppe mit 2 bis 7 Kohlenstoffatomen, eine geradkettige oder verzweigte Alkoxycarbonylalkylgruppe mit 3 bis 10 Kohlenstoffatomen, eine geradkettige oder verzweigte Alkoxycarbonylgruppe mit 2 bis 7 Kohlenstoffatomen, eine geradkettige oder verzweigte Carbamoylalkylgruppe mit 2 bis 7 Kohlenstoffatomen, eine Cycloalkylgruppe mit 3 bis 7 Kohlenstoffatomen, ein ungesättigtes cyclisches System mit 5 bis 7 Kettengliedern, das mindestens ein Heteroatom ausgewählt aus Stickstoff, Schwefel und Sauerstoff umfaßt, eine Pyridinylcarbonylgruppe, eine Pyrimidylcarbonylgruppe, eine Clofibroylgruppe oder eine 6-Hydroxy-2,5,7,8-tetramethyl-chroman-2-carbonylgruppe,
- $R_6$ ein Wasserstoffatom oder eine geradkettige oder verzweigte Alkylgruppe mit 1 bis 6 Kohlenstoffatomen,
- $R_7$ und $R_8$, die gleichartig oder verschieden sein können, jeweils ein Wasserstoffatom, eine geradkettige oder verzweigte Alkylgruppe mit 1 bis 6 Kohlenstoffatomen, eine Phenylgruppe oder Phenylalkylgruppe mit 7 bis 9 Kohlenstoffatomen (die gegebenenfalls am aromatischen Kern durch ein oder mehrere Halogenatome oder Alkyl- oder Alkoxygruppen mit 1 bis 6 Kohlenstoffatomen substituiert sind) oder gemeinsam mit dem Stickstoffatom, an das sie gebunden sind, ein gesättigtes oder ungesättigter, cyclisches System mit 5 bis 7 Kettengliedern, welches 1 bis 2 Heteroatome ausgewählt aus Stickstoff, Sauerstoff und Schwefel umfaßt und gegebenenfalls durch eine Alkylcarbonyl- oder Alkoxycarbonylgruppe mit 2 bis 5 Kohlenstoffatmen substituiert ist,
- $R_{13}$ ein Wasserstoffatom, eine geradkettige oder verzweigte Alkylcarbonylgruppe mit 2 bis 6 Kohlenstoffatomen oder eine Benzoylgruppe bedeuten,

sowie von deren Isomeren, Diastereoisomeren, Enantiomeren und deren Additionssalzen mit einer anorganischen oder organischen, pharmazeutisch annehmbaren Säure,
mit den Maßgaben, daß:

- wenn $R_1$ eine Gruppe der allgemeinen Formel ($Z_3$) bedeutet, $R_2$ keine Methylengruppe darstellt,
- wenn $R_1$ eine Gruppe der allgemeinen Formel ($Z_0$) und $R_{12}$ ein Wasserstoffatom darstellen, $R_2$ nicht die folgenden Gruppen bedeuten kann:
  $>CH-O-CH_3$, $>CH-O-CH_2CH_3$, $>CH-O-CH_2CH_2CH_3$, $>CH-O-CH_2CH_2-O-CH_2CH_3$, $>CH-O-CH_2CH_2-O-CH_2CH_2CH_2CH_3$
- wenn $R_1$ eine Gruppe der allgemeinen Formel ($Z_0$), $R_{12}$ ein Wasserstoffatom und $R_2$ eine Gruppe der allgemeinen Formel ($Y_1$) darstellen, worin n = 1 und $R_4$ eine Carbonylgruppe bedeuten, $R_5$ keine Methylgruppe, Isopropylgruppe oder Phenylgruppe darstellt,
- wenn $R_1$ eine Gruppe der allgemeinen Formel ($Z_0$) und $R_{12}$ ein Wasserstoffatom darstellen, $R_2$ keine Carbonylgruppe bedeutet,

**dadurch gekennzeichnet**, daß man:

entweder

- 3-Cyano-2-(2-formyl-pyrrol-1-yl)-thiophen der Formel (II):

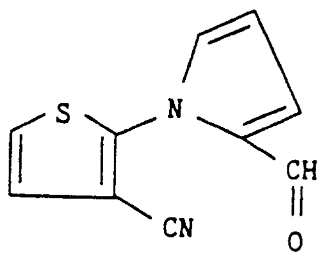

(II)

<u>**entweder**</u>

- mit einem Methylketon der allgemeinen Formel (III):

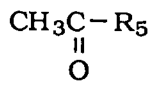

(III)

in der $R_5$ die bezüglich der Derivate der allgemeinen Formel (I) angegebenen Bedeutungen besitzt, in Gegenwart einer starken anorganischen Base und von Wasserstoffperoxid umsetzt, so daß man die Verbindungen der allgemeinen Formel ($I_A$) erhält:

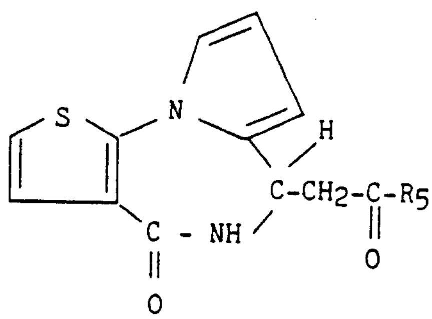

($I_A$)

in der $R_5$ die bezüglich der Derivate der allgemeinen Formel (I) angegebenen Bedeutungen besitzt,

<u>welche anschließend:</u>

<u>entweder</u>

der Einwirkung von Natriumborhydrid in Lösung in einem alkoholischen Lösungsmittel unterworfen werden, so daß man die Verbindungen der allgemeinen Formel ($I_B$) erhält:

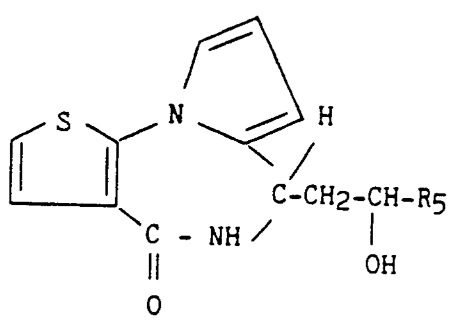

($I_B$)

in der $R_5$ die bezüglich der Derivate der allgemeinen Formel (I) angegebenen Bedeutungen besitzt, welche man gegebenenfalls der Einwirkung von Phosgen in einem aromatischen organischen Lösungsmittel in der Wärme unterwerfen kann zur Bildung der Verbindungen der allgemeinen Formel ($I_C$):

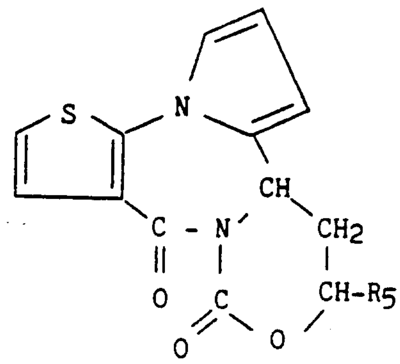

($I_C$)

in der $R_5$ die bezüglich der Verbindungen der allgemeinen Formel (I) angegebenen Bedeutungen besitzt,

oder welche man mit einer Verbindung der allgemeinen Formel (XI):

$R_{13}$-Cl (XI)

in der $R_{13}$ die bezüglich der Verbindungen der allgemeinen Formel (I) angegebenen Bedeutungen besitzt, umsetzen kann, so daß man die Verbindungen der allgemeinen Formel ($I_M$) erhält:

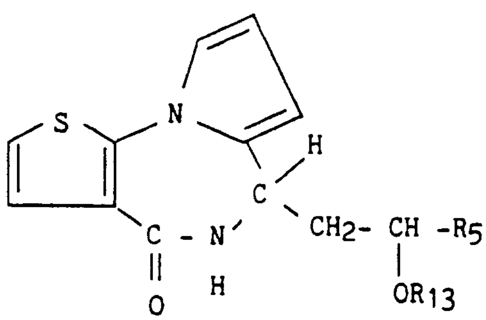

($I_M$)

in der $R_5$ und $R_{13}$ die bezüglich der Verbindungen der allgemeinen Formel (I) angegebenen Bedeutungen besitzen,

oder

mit einem Hydroxylaminderivat der allgemeinen Formel (IV):

$H_2N - O - R_6$ (IV)

in der $R_6$ die bezüglich der Derivate der allgemeinen Formel (I) angegebenen Bedeutungen besitzt, kondensiert zur Bildung der Verbindungen der allgemeinen Formel ($I_D$):

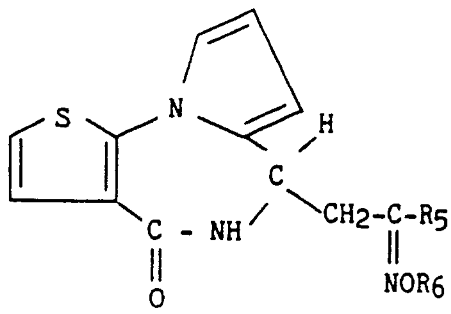

(I_D)

in der $R_5$ und $R_6$ die bezüglich der Derivate der allgemeinen Formel (I) angegebenen Bedeutungen besitzen,

oder

mit einem Hydrazinderivat der allgemeinen Formel (XII):

$H_2N - NH - R_6$ (XII)

in der $R_6$ die bezüglich der Verbindungen der allgemeinen Formel (I) angegebenen Bedeutungen besitzt, kondensiert werden, so daß man die Verbindungen der allgemeinen Formel ($I_N$) erhält:

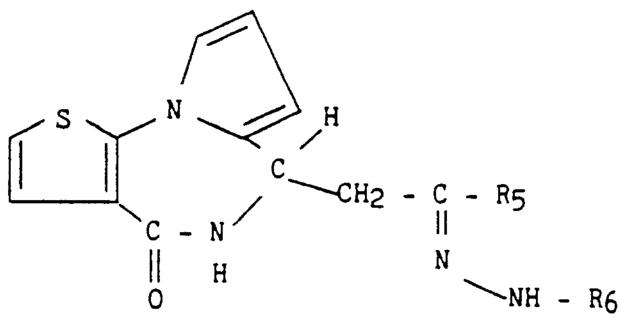

($I_N$)

in der $R_5$ und $R_6$ die bezüglich der Verbindungen der allgemeinen Formel (I) angegebenen Bedeutungen besitzen,

**oder**

mit einem primären Alkohol der allgemeinen Formel (V):

$R_3OH$ (V)

in der $R_3$ die bezüglich der Verbindungen der allgemeinen Formel (I) angegebenen Bedeutungen besitzt,

in Gegenwart einerstarken anorganischen Base bei einer Temperaturzwischen 30° - 80°C umsetzt, so daß man die Verbindungen der allgemeinen Formel ($I_E$) erhält:

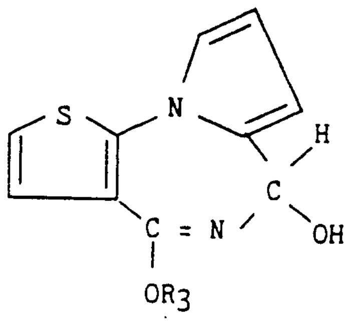

($I_E$)

in der $R_3$ die bezüglich der Verbindungen der allgemeinen Formel (I) angegebenen Bedeutungen besitzt, welche man anschließend der Einwirkung von Kaliumpermanganat bei Raumtemperatur unterwirft zur Bildung der Verbindungen der allgemeinen Formel ($I_F$):

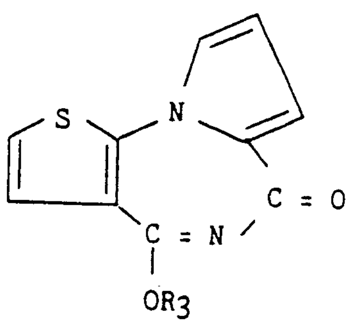

($I_F$)

in der $R_3$ die bezüglich der Verbindungen der allgemeinen Formel (I) angegebenen Bedeutungen besitzt, die man gegebenenfalls der Einwirkung von Natriumborhydrid unterwerfen kann zur Bildung der Verbindungen der allgemeinen Formel ($I_G$):

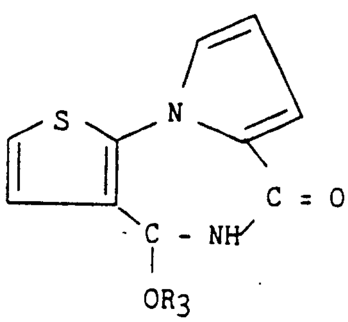

($I_G$)

in der $R_3$ die bezüglich der Verbindungen der allgemeinen Formel (I) angegebenen Bedeutungen besitzt.

**oder**

2-(2-Formyl-pyrrol-1-yl)-3-thiophen-carboxamid der Formel (VI):

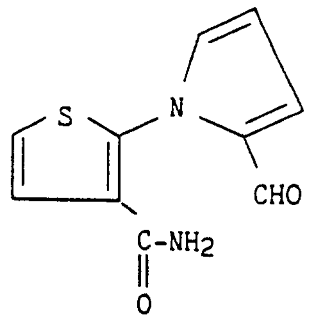

(VI)

entweder

der Einwirkung eines Amins der allgemeinen Formel (VII):

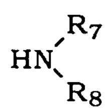

(VII)

in der $R_7$ und $R_8$ die bezüglich der Verbindungen der allgemeinen Formel (I) angegebenen Bedeutungen besitzen, unterwirft, so daß man die Verbindungen der allgemeinen Formel ($I_H$) erhält:

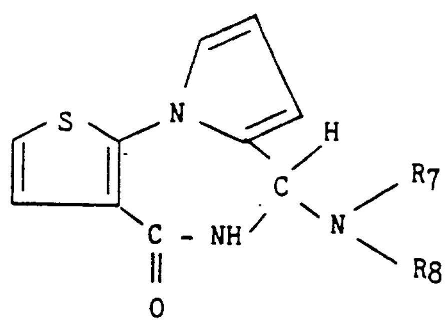

($I_H$)

in der $R_7$ und $R_8$ die bezüglich der Verbindungen der allgemeinen Formel (I) angegebenen Bedeutungen besitzen, welche man gegebenenfalls, wenn $R_7$ und $R_8$ gemeinsam mit dem Stickstoffatom, an das sie gebunden sind, eine Morpholinogruppe bilden, der Einwirkung von Natriumborhydrid unterwerfen kann zur Bildung der Verbindung der allgemeinen Formel (I), in der $R_2$ eine Methylengruppe und $R_1$ eine Gruppe der allgemeinen Formel ($Z_0$), worin $R_{12}$ = H darstellt, bedeuten,

oder

der Einwirkung von Triethylamin in Gegenwart von Wasser und bei Raumtemperatur unterwirft zur Bildung der Verbindung der allgemeinen Formel (I), in der $R_2$ eine Hydroxymethylengruppe und $R_1$ eine Gruppe der Formel ($Z_0$), worin $R_{12}$ = H darstellt, bedeuten,

welche man anschließend

entweder

mit einem Amin der allgemeinen Formel (VII) umsetzt zur Bildung der Verbindungen der allgemeinen Formel ($I_H$),

oder

mit einer Verbindung der allgemeinen Formel (VIII):

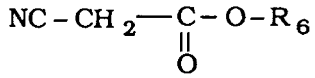

(VIII)

in der $R_6$ die bezüglich der Verbindungen der allgemeinen Formel (I) angegebenen Bedeutungen besitzt, in Gegenwart von Triethylamin kondensiert zur Bildung der Verbindungen der allgemeinen Formel ($I_I$):

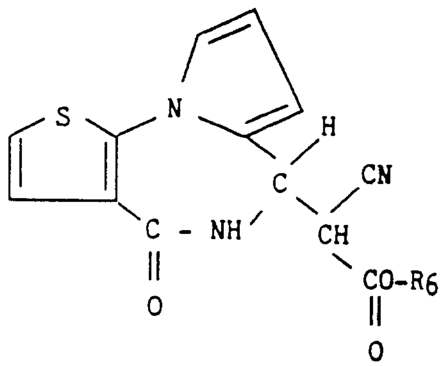

($I_I$)

in der $R_6$ die bezüglich der Verbindungen der allgemeinen Formel (I) angegebenen Bedeutungen besitzt,
oder
mit einem Alkohol der allgemeinen Formel (IX)

$R_5OH$ (IX)

in der $R_5$ die bezüglich der Verbindungen der Verbindungend er allgemeinen Formel (I) angegebenen Bedeutungen besitzt, in der Wärme umsetzt zur Bildung der Verbindungen der allgemeinen Formel ($I_J$):

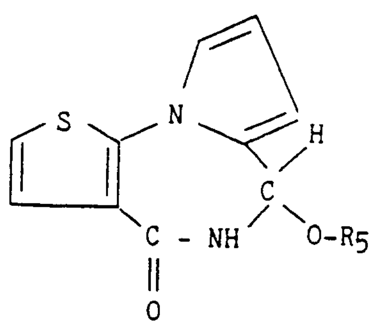

($I_J$)

in der $R_5$ die bezüglich der Derivate der allgemeinen Formel (I) angegebenen Bedeutungen besitzt,
**oder**
der Einwirkung elner Verbindung der allgemeinen Formel (X):

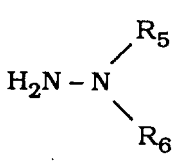

(X)

in der $R_5$ und $R_6$ die bezüglich der Verbindungen der allgemeinen Formel (I) angegebenen Bedeutungen besitzen, unterwirft zur Bildung der Verbindungen der allgemeinen Formel ($I_K$):

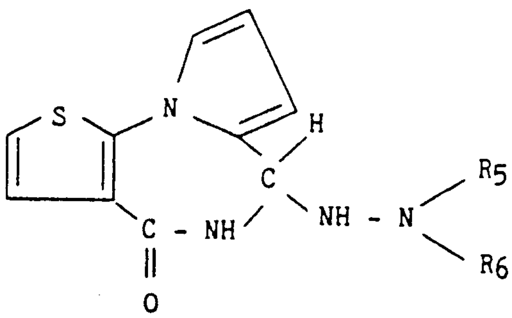

($I_K$)

in der $R_5$ und $R_6$ die bezüglich der Verbindungen der allgemeinen Formel (I) angegebenen Bedeutungen besitzen,

**oder**

der Einwirkung eines Alkohols der allgemeinen Formel (IX) unterwirft zur Bildung der Verbindungen der allgemeinen Formel ($I_J$),

**oder**

der Einwirkung eines Thiols der allgemeinen Formel (XI):

$R_5SH$ (XI)

in der $R_5$ die bezüglich der Verbindungen der allgemeinen Formel (I) angegebenen Bedeutungen besitzt, bei Raumtemperatur unterwirft zur Bildung der Verbindungen der allgemeinen Formel ($I_L$):

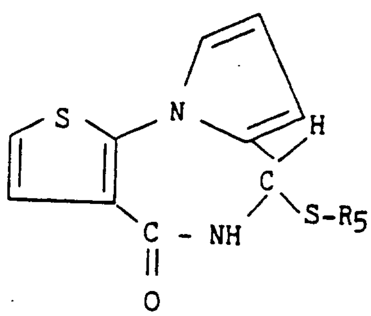

($I_L$)

in der $R_5$ die bezüglich der Verbindungen der allgemeinen Formel (I) angegebenen Bedeutungen besitzt, welche man gegebenenfalls, wenn $R_5$ eine Carboxyalkylgruppe darstellt, der Einwirkung von Triethylamin oder eines Chlorameisensäureesters und dann der Einwirkung von gasförmigem Ammoniak unterziehen kann zur Bildung der entsprechenden Amide.

**2.** Verfahren nach Anspruch 1 zur Herstellung von Verbindungen der allgemeinen Formel ($I_A$):

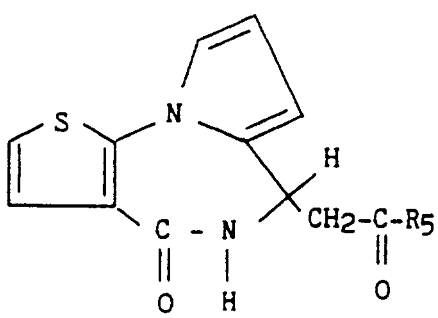

($I_A$)

in der $R_5$ die in Anspruch 1 angegebenen Bedeutungen besitzt, sowie von deren Isomeren, Diastereoisomeren und Enantiomeren in isolierter Form oder in Form einer Mischung.

**3.** Verfahren nach Anspruch 1 zur Herstellung von Verbindungen der allgemeinen Formel ($I_B$):

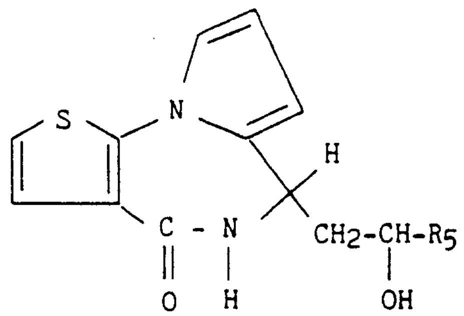

($I_B$)

in der $R_5$ die in Anspruch 1 angegebenen Bedeutungen besitzt, sowie von deren Isomeren, Diastereoisomeren und Enantiomeren in isolierter Form oder in Form einer Mischung.

**4.** Verfahren nach Anspruch 1 zur Herstellung von Verbindungen der allgemeinen Formel ($I_C$):

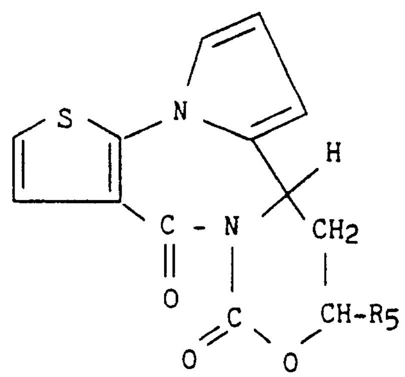

($I_C$)

in der $R_5$ die in Anspruch 1 angegebenen Bedeutungen besitzt, sowie von deren Isomeren, Enantiomeren und Diastereoisomeren in isolierter Form oder in Form einer Mischung.

**5.** Verfahren nach Anspruch 1 zur Herstellung von Verbindungen der allgemeinen Formel ($I_D$):

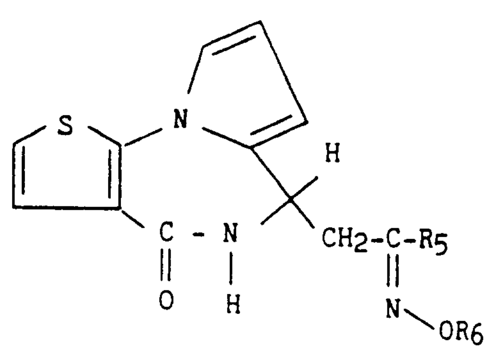

($I_D$)

in der $R_5$ und $R_6$ die in Anspruch 1 angegebenen Bedeutungen besitzt, sowie von deren Isomeren, Enantiomeren und Diastereoisomeren in isolierter Form oder in Form einer Mischung.

**6.** Verfahren nach Anspruch 1 zur Herstellung von Verbindungen der allgemeinen Formel ($I_E$):

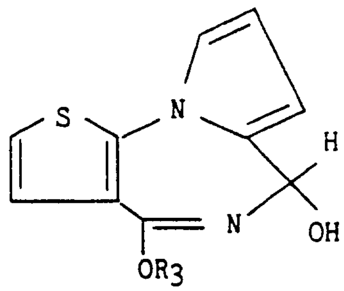

($I_E$)

in der $R_3$ die in Anspruch 1 angegebenen Bedeutungen besitzt, sowie von deren Isomeren, Enantiomeren und Diastereoisomeren in isolierter Form oder in Form einer Mischung.

**7.** Verfahren nach Anspruch 1 zur Herstellung von Verbindungen der allgemeinen Formel ($I_F$):

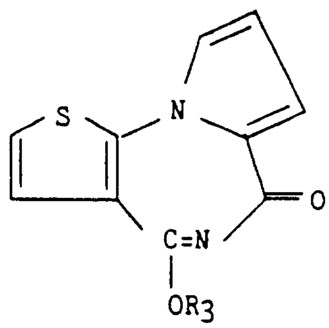

($I_F$)

in der $R_3$ die in Anspruch 1 angegebenen Bedeutungen besitzt, sowie von deren Isomeren, Diastereoisomeren und Enantiomeren in isolierter Form oder in Form einer Mischung.

**8.** Verfahren nach Anspruch 1 zur Herstellung von Verbindungen der allgemeinen Formel ($I_G$):

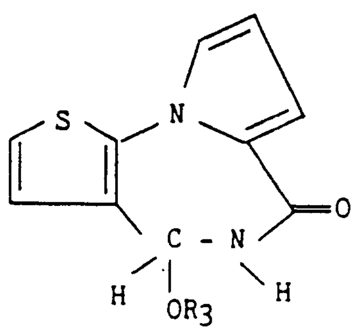

($I_G$)

in der $R_3$ die in Anspruch 1 angegebenen Bedeutungen besitzt, sowie von deren Isomeren, Diastereoisomeren und Enantiomeren in isolierter Form oder in Form einer Mischung.

**9.** Verfahren nach Anspruch 1 zur Herstellung von Verbindungen der allgemeinen Formel ($I_H$):

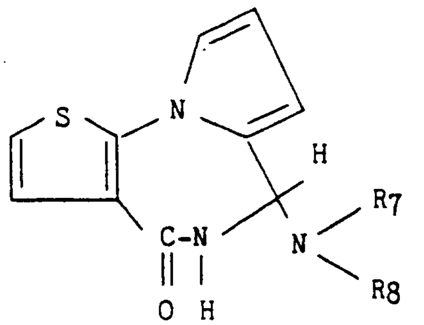

($I_H$)

in der $R_7$ und $R_8$ die in Anspruch 1 angegebenen Bedeutungen besitzt, sowie von deren Isomeren, Diastereoisomeren und Enantiomeren in isolierter Form oder in Form einer Mischung und von ihren gegebenenfalls möglichen Additionssalzen mit einer anorganischen oder organischen, pharmazeutisch annehmbaren Säure.

**10.** Verfahren nach Anspruch 1 zur Herstellung von Verbindungen der allgemeinen Formel ($I_J$):

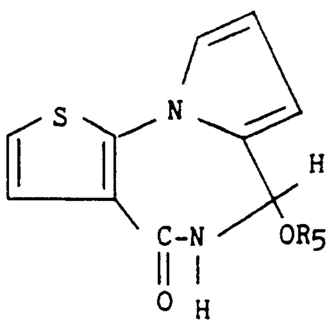

($I_J$)

in der $R_5$ die in Anspruch 1 angegebenen Bedeutungen besitzt, sowie von deren Isomeren, Diastereoisomeren und Enantiomeren in isolierter Form oder in Form einer Mischung.

**11.** Verfahren nach Anspruch 1 zur Herstellung von Verbindungen der allgemeinen Formel ($I_K$):

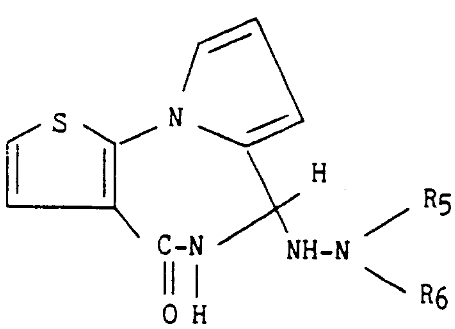

($I_K$)

in der $R_5$ und $R_6$ die in Anspruch 1 angegebenen Bedeutungen besitzt, sowie von deren Isomeren, Diastereoisomeren und Enantiomeren in isolierter Form oder in Form einer Mischung und von ihren gegebenenfalls möglichen Additionssalzen mit einer anorganischen oder organischen, pharmazeutisch annehmbaren Säure.

**12.** Verfahren nach Anspruch 1 zur Herstellung von Verbindungen der allgemeinen Formel ($I_L$):

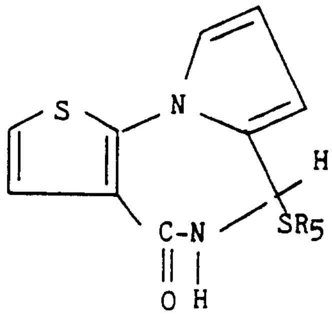

($I_L$)

in der $R_5$ die in Anspruch 1 angegebenen Bedeutungen besitzt, sowie von deren Isomeren, Diastereoisomeren und Enantiomeren in isolierter Form oder in Form einer Mischung.

**13.** Verfahren nach Anspruch 1 zur Herstellung von 5,6-Dihydro-6-(2-benzyloxypropyl)-4-oxo-4H-pyrrolo-[1,2-a]thieno[3,2-f][1,4]diazepin

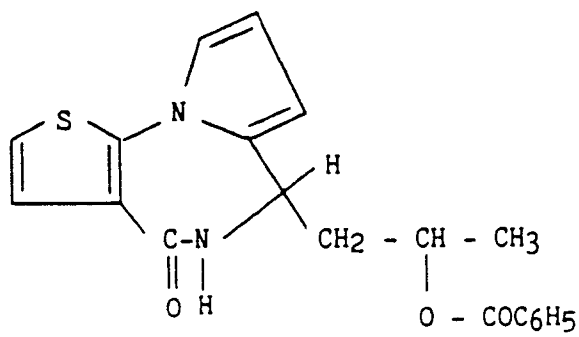

sowie von dessen Diastereoisomeren in isolierter Form oder in Form einer Mischung.

**14.** Verfahren nach Anspruch 1 zur Herstellung von 5,6-Dihydro-6-(2-hydrazono-1-pentyl)-4-oxo-4H-pyrrolo-[1,2-a]thieno[3,2-f][1,4]diazepin

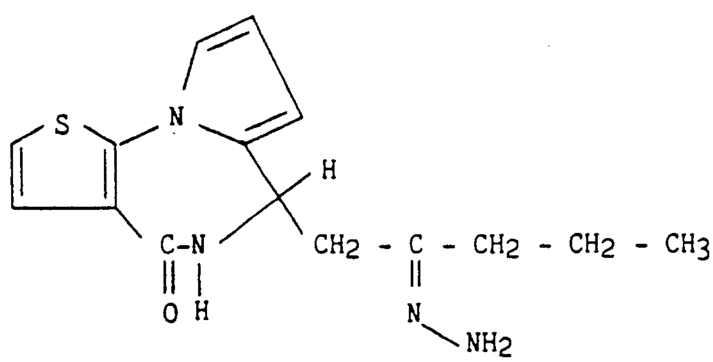

sowie von dessen Enantiomeren in isolierter Form oderin Form einer Mischung und von dessen Additionssalzen mit einer anorganischen oder organischen, pharmazeutisch annehmbaren Säure.

**15.** Verfahren nach den Ansprüchen 1 und 10 zur Herstellung von 5,6-Dihydro-6-hydroxy-4-oxo-4H-pyrrolo-[1,2-a]thieno[2,3-f][1,4]diazepin

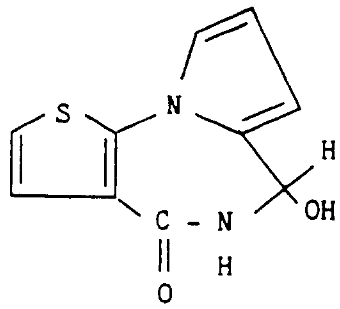

sowie von dessen Enantiomeren in isolierter Form oder in Form einer Mischung.

**16.** Verfahren nach Anspruch 1 zur Herstellung von 5,6-Dihydro-4-oxo-4H-pyrrolo[1,2-a]thieno[3,2-f][1,4]-diazepin

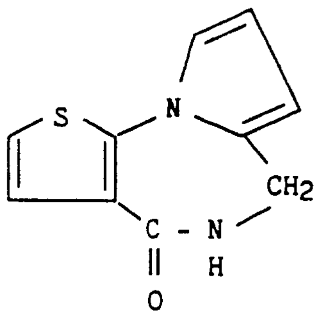

**17.** Verfahren zur Herstellung von pharmazeutischen Zubereitungen enthaltend als Wirkstoff eine Verbindung hergestellt nach den Ansprüchen 1 bis 16 allein oder in Kombination mit einem oder mehreren inerten, nichttoxischen, pharmazeutisch annehmbaren Trägermaterialien oder Bindemitteln.

**18.** Verfahren nach Anspruch 17 zur Herstellung von pharmazeutischen Zubereitungen, die in einer Form vorliegen, die insbesondere zur Behandlung von Gehirngefäßunfällen, des normalen oder pathologischen Alterns des Gehirns und von ischämischen Syndromen geeignet ist.

**19.** Verfahren nach Anspruch 17 zur Herstellung von pharmazeutischen Zubereitungen, die in einer Form vorliegen, die insbesondere zur Behandlung von Störungen des Magen-Darm-Trakts, des Pankreas, der Gallenblase, des Appetits, des Zentralnervensystems sowie von Schmerzen geeignet ist.

**20.** Verfahren nach Anspruch 17 zur Herstellung von pharmazeutischen Zubereitungen, die in einer Form vorliegen, die insbesondere zur Behandlung von Hyperlipidämie, Hypertriglyceridämie, Hypercholesterolämie, Hyperglykämie, der Hypertension und von damit verknüpften Krankheiten geeignet ist.

**Patentansprüche für folgenden Vertragsstaat : GR**

1. Verfahren zur Herstellung von Verbindungen der allgemeinen Formel (I);

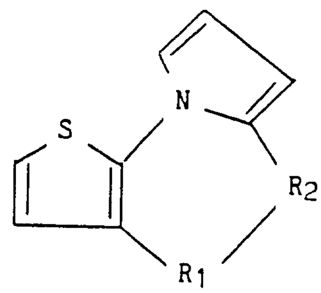

(I)

in der:

- $R_1$ eine Gruppe der allgemeinen Formel ($Z_0$), ($Z_1$), ($Z_2$), ($Z_3$) oder ($Z_4$)

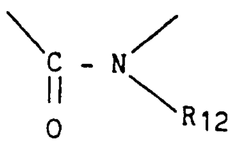

($Z_0$)

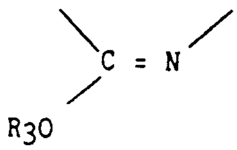

($Z_1$)

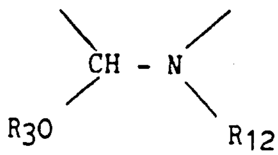

($Z_2$)

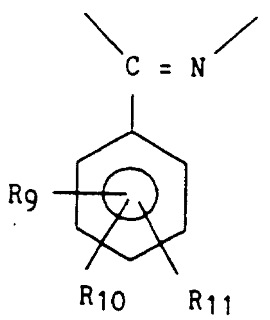

($Z_3$)

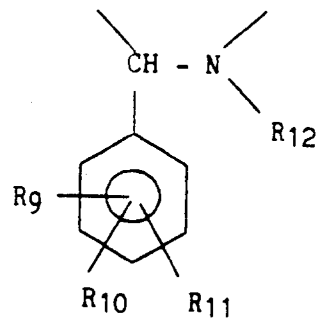

($Z_4$)

worin

- $R_3$ ein Wasserstoffatom, eine geradkettige oder verzweigte Alkylgruppe mit 1 bis 6 Kohlenstoffatomen, die gesättigt sein oder eine Doppelbindung aufweisen kann,
- $R_9$, $R_{10}$, $R_{11}$, die gleichartig oder verschieden sein können, jeweils ein Wasserstoffatom, ein Halogenatom oder eine Alkylgruppe mit 1 bis 6 Kohlenstoffatomen,
- $R_{12}$ ein Wasserstoffatom oder gemeinsam mit $R_2$ und dem Stickstoffatom, an die sie gebunden ist, eine Gruppe der allgemeinen Formel (W):

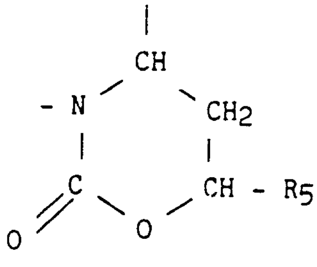

(W)

darstellen.
und

- $R_2$ eine Methylengruppe, eine Hydroxymethylengruppe, eine Carbonylgruppe, eine Gruppe der allgemeinen Formel ($Y_1$), ($Y_2$), ($Y_3$) oder ($Y_4$):

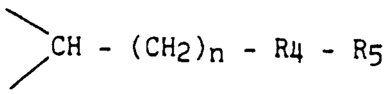

($Y_1$)

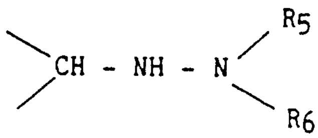

($Y_2$)

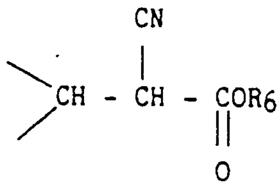

($Y_3$)

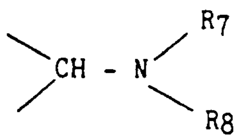

($Y_4$)

oder gemeinsam mit $R_{12}$ und dem Stickstoffatom, an die sie gebunden ist, eine Gruppe der allgemeinen Formel (W):

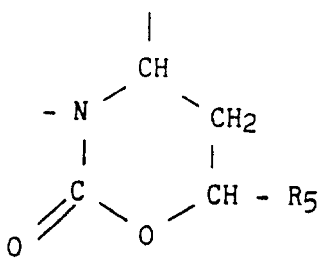

(W)

bedeuten, in welchen Formeln:

- $R_4$ ein Sauerstoffatom oder ein Schwefelatom, eine Carbonylgruppe, eine Gruppe der allgemeinen Formel ($X_1$), ($X_2$), ($X_3$) oder ($X_4$):

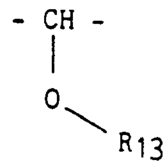
(X1)

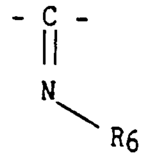
(X2)

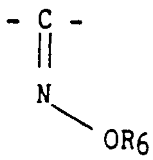
(X3)

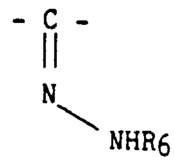
(X4)

- n eine Zahl zwischen 0 und 4 einschließlich,
- $R_5$ ein Wasserstoffatom, eine geradkettige oder verzweigte Alkylkette mit 1 bis 10 Kohlenstoffatomen, die gegebenenfalls durch ein oder mehrere Sauerstoff- oder Schwefelatome unterbrochen ist, eine Phenyl-, Benzoyl- oder Arylkylgruppe mit 7 bis 10 Kohlenstoffatomen (die gegebenenfalls am aromatischen Kern durch ein oder mehrere Halogenatome, geradkettige oder verzweigte Alkylgruppen mit 1 bis 6 Kohlenstoffatomen, Nitrogruppen oder geradkettige oder verzweigte Alkoxygruppen mit 1 bis 6 Kohlenstoffatomen substituiert sind), eine geradkettige oder verzweigte Carboxyalkylgruppe mit 2 bis 7 Kohlenstoffatomen, eine geradkettige oder verzweigte Alkoxycarbonylalkylgruppe mit 3 bis 10 Kohlenstoffatomen, eine geradkettige oder verzweigte Alkoxycarbonylgruppe mit 2 bis 7 Kohlenstoffatomen, eine geradkettige oder verzweigte Carbamoylalkylgruppe mit 2 bis 7 Kohlenstoffatomen, eine Cycloalkylgruppe mit 3 bis 7 Kohlenstoffatomen, ein ungesättigtes cyclisches System mit 5 bis 7 Kettengliedern, das mindestens ein Heteroatom ausgewählt aus Stickstoff, Schwefel und Sauerstoff umfaßt, eine Pyridinylcarbonylgruppe, eine Pyrimidylcarbonylgruppe, eine Clofibroylgruppe oder eine 6-Hydroxy-2,5,7,8-tetramethyl-chroman-2-carbonylgruppe,
- $R_6$ ein Wasserstoffatom oder eine geradkettige oder verzweigte Alkylgruppe mit 1 bis 6 Kohlenstoffatomen,
- $R_7$ und $R_8$, die gleichartig oder verschieden sein können, jeweils ein Wasserstoffatom, eine geradkettige oder verzweigte Alkylgruppe mit 1 bis 6 Kohlenstoffatomen, eine Phenylgruppe oder Phenylalkylgruppe mit 7 bis 9 Kohlenstoffatomen (die gegebenenfalls am aromatischen Kern durch ein oder mehrere Halogenatome oder Alkyl- oder Alkoxygruppen mit 1 bis 6 Kohlenstoffatomen substituiert sind) oder gemeinsam mit dem Stickstoffatom, an das sie gebunden sind, ein gesättigtes oder ungesättigter, cyclisches System mit 5 bis 7 Kettengliedern, welches 1 bis 2 Heteroatome ausgewählt aus Stickstoff, Sauerstoff und Schwefel umfaßt und gegebenenfalls durch eine Alkylcarbonyl- oder Alkoxycarbonylgruppe mit 2 bis 5 Kohlenstoffatmen substituiert ist,
- $R_{13}$ ein Wasserstoffatom, eine geradkettige oder verzweigte Alkylcarbonylgruppe mit 2 bis 6 Kohlenstoffatomen oder eine Benzoylgruppe bedeuten,

sowie von deren Isomeren, Diastereoisomeren, Enantiomeren und deren Additionssalzen mit einer anorganischen oder organischen, pharmazeutisch annehmbaren Säure,
mit den Maßgaben, daß:

- wenn $R_1$ eine Gruppe der allgemeinen Formel ($Z_3$) bedeutet, $R_2$ keine Methylengruppe darstellt.
- wenn $R_1$ eine Gruppe der allgemeinen Formel ($Z_0$) und $R_{12}$ ein Wasserstoffatom darstellen, $R_2$ nicht die folgenden Gruppen bedeuten kann:
  $>CH-O-CH_3$, $>CH-O-CH_2CH_3$, $>CH-O-CH_2CH_2CH_3$, $>CH-O-CH_2CH_2-O-CH_2CH_3$, $>CH-O-CH_2CH_2-O-CH_2CH_2CH_2CH_3$
- wenn $R_1$ eine Gruppe der allgemeinen Formel ($Z_0$), $R_{12}$ ein Wasserstoffatom und $R_2$ eine Gruppe der allgemeinen Formel ($Y_1$) darstellen, worin n = 1 und $R_4$ eine Carbonylgruppe bedeuten, $R_5$ keine Methylgruppe, Isopropylgruppe oder Phenylgruppe darstellt,
- wenn $R_1$ eine Gruppe der allgemeinen Formel ($Z_0$) und $R_{12}$ ein Wasserstoffatom darstellen, $R_2$ keine Carbonylgruppe bedeutet,

**dadurch gekennzeichnet**, daß man:

entweder

- 3-Cyano-2-(2-formyl-pyrrol-1-yl)-thiophen der Formel (II):

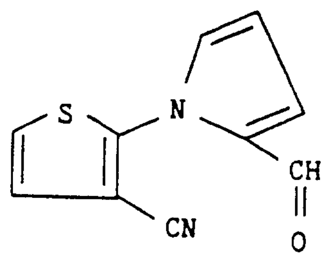

(II)

entweder

- mit einem Methylketon der allgemeinen Formel (III):

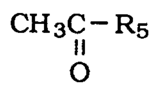

(III)

in der $R_5$ die bezüglich der Derivate der allgemeinen Formel (I) angegebenen Bedeutungen besitzt, in Gegenwart einer starken anorganischen Base und von Wasserstoffperoxid umsetzt, so daß man die Verbindungen der allgemeinen Formel ($I_A$) erhält:

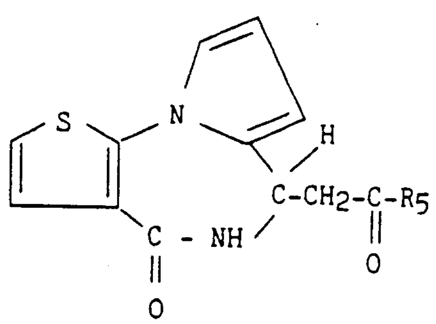

($I_A$)

in der $R_5$ die bezüglich der Derivate der allgemeinen Formel (I) angegebenen Bedeutungen besitzt,

welche anschließend:

entweder

der Einwirkung von Natriumborhydrid in Lösung in einem alkoholischen Lösungsmittel unterworfen werden, so daß man die Verbindungen der allgemeinen Formel ($I_B$) erhält:

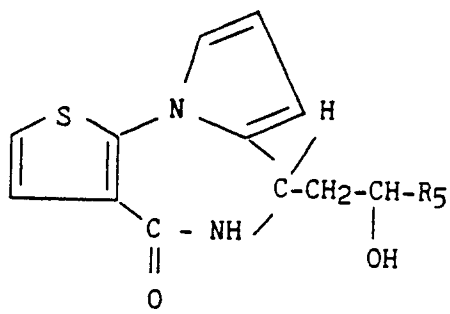

(IB)

in der $R_5$ die bezüglich der Derivate der allgemeinen Formel (I) angegebenen Bedeutungen besitzt, welche man gegebenenfalls der Einwirkung von Phosgen in einem aromatischen organischen Lösungsmittel in der Wärme unterwerfen kann zur Bildung der Verbindungen der allgemeinen Formel ($I_C$):

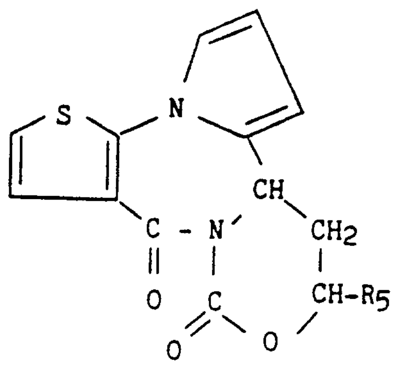

(IC)

in der $R_5$ die bezüglich der Verbindungen der allgemeinen Formel (I) angegebenen Bedeutungen besitzt,
oder welche man mit einer Verbindung der allgemeinen Formel (XI):

$R_{13}$-Cl (XI)

in der $R_{13}$ die bezüglich der Verbindungen der allgemeinen Formel (I) angegebenen Bedeutungen besitzt, umsetzen kann, so daß man die Verbindungen der allgemeinen Formel ($I_M$) erhält:

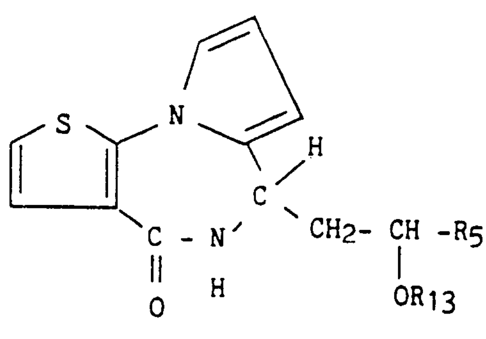

(IM)

in der $R_5$ und $R_{13}$ die bezüglich der Verbindungen der allgemeinen Formel (I) angegebenen Bedeutungen besitzen,
oder
mit einem Hydroxylaminderivat der allgemeinen Formel (IV):

$H_2N$ - O - $R_6$ (IV)

in der $R_6$ die bezüglich der Derivate der allgemeinen Formel (I) angegebenen Bedeutungen besitzt,

kondensiert zur Bildung der Verbindungen der allgemeinen Formel ($I_D$):

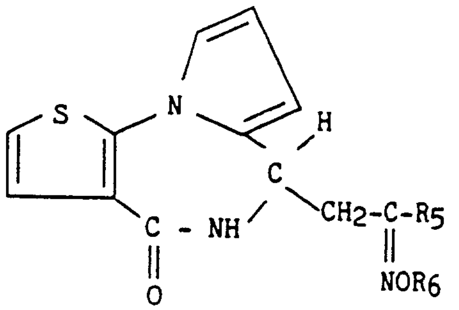

($I_D$)

in der $R_5$ und $R_6$ die bezüglich der Derivate der allgemeinen Formel (I) angegebenen Bedeutungen besitzen,

oder

mit einem Hydrazinderivat der allgemeinen Formel (XII):

$H_2N - NH - R_6$ (XII)

in der $R_6$ die bezüglich der Verbindungen der allgemeinen Formel (I) angegebenen Bedeutungen besitzt, kondensiert werden, so daß man die Verbindungen der allgemeinen Formel ($I_N$) erhält:

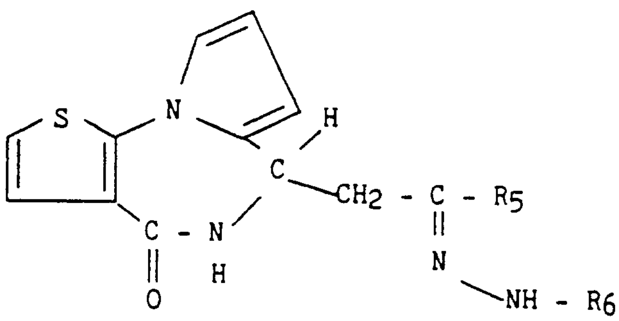

($I_N$)

in der $R_5$ und $R_6$ die bezüglich der Verbindungen der allgemeinen Formel (I) angegebenen Bedeutungen besitzen,

**oder**

mit einem primären Alkohol der allgemeinen Formel (V):

$R_3OH$ (V)

in der $R_3$ die bezüglich der Verbindungen der allgemeinen Formel (I) angegebenen Bedeutungen besitzt,

in Gegenwart einerstarken anorganischen Base bei einer Temperatur zwischen 30° - 80°C umsetzt, so daß man die Verbindungen der allgemeinen Formel ($I_E$) erhält:

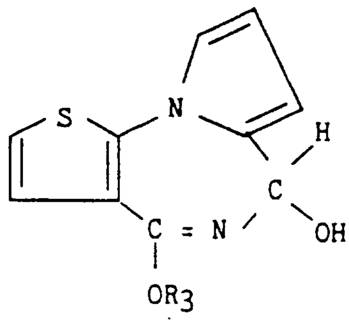

($I_E$)

in der $R_3$ die bezüglich der Verbindungen der allgemeinen Formel (I) angegebenen Bedeutungen besitzt, welche man anschließend der Einwirkung von Kaliumpermanganat bei Raumtemperatur unterwirft zur Bildung der Verbindungen der allgemeinen Formel ($I_F$):

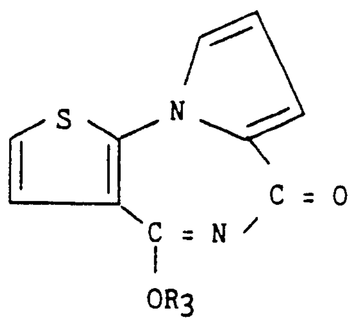

($I_F$)

in der $R_3$ die bezüglich der Verbindungen der allgemeinen Formel (I) angegebenen Bedeutungen besitzt, die man gegebenenfalls der Einwirkung von Natriumborhydrid unterwerfen kann zur Bildung der Verbindungen der allgemeinen Formel ($I_G$):

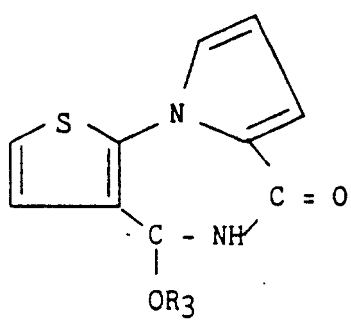

($I_G$)

in der $R_3$ die bezüglich der Verbindungen der allgemeinen Formel (I) angegebenen Bedeutungen besitzt,

oder

2-(2-Formyl-pyrrol-1-yl)-3-thiophen-carboxamid der Formel (VI):

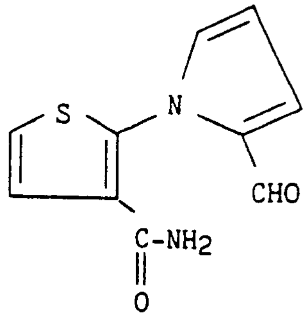

(VI)

**entweder**

der Einwirkung eines Amins der allgemeinen Formel (VII):

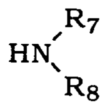

(VII)

in der $R_7$ und $R_8$ die bezüglich der Verbindungen der allgemeinen Formel (I) angegebenen Bedeutungen besitzen, unterwirft, so daß man die Verbindungen der allgemeinen Formel ($I_H$) erhält:

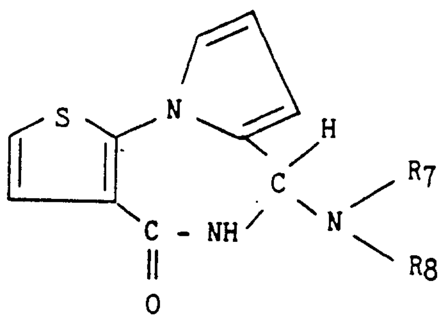

($I_H$)

in der $R_7$ und $R_8$ die bezüglich der Verbindungen der allgemeinen Formel (I) angegebenen Bedeutungen besitzen, welche man gegebenenfalls, wenn $R_7$ und $R_8$ gemeinsam mit dem Stickstoffatom, an das sie gebunden sind, eine Morpholinogruppe bilden, der Einwirkung von Natriumborhydrid unterwerfen kann zur Bildung der Verbindung der allgemeinen Formel (I), in der $R_2$ eine Methylengruppe und $R_1$ eine Gruppe der allgemeinen Formel ($Z_0$), worin $R_{12}$ = H darstellt, bedeuten,

oder

der Einwirkung von Triethylamin in Gegenwart von Wasser und bei Raumtemperatur unterwirft zur Bildung der Verbindung der allgemeinen Formel (I), in der $R_2$ eine Hydroxymethylengruppe und $R_1$ eine Gruppe der Formel ($Z_0$), worin $R_{12}$ = H darstellt, bedeuten.

welche man anschließend

entweder

mit einem Amin der allgemeinen Formel (VII) umsetzt zur Bildung der Verbindungen der allgemeinen Formel ($I_H$),

oder

mit einer Verbindung der allgemeinen Formel (VIII):

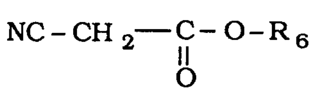

(VIII)

in der $R_6$ die bezüglich der Verbindungen der allgemeinen Formel (I) angegebenen Bedeutungen besitzt, in Gegenwart von Triethylamin kondensiert zur Bildung der Verbindungen der allgemeinen Formel ($I_I$):

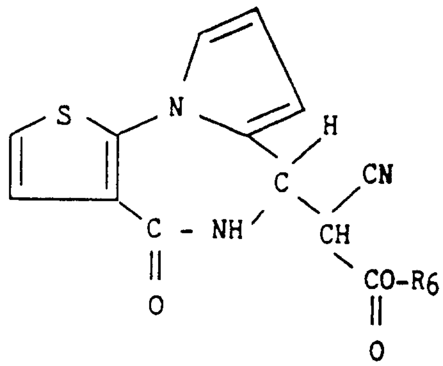

($I_I$)

in der $R_6$ die bezüglich der Verbindungen der allgemeinen Formel (I) angegebenen Bedeutungen besitzt,

oder

mit einem Alkohol der allgemeinen Formel (IX)

$R_5OH$ (IX)

in der $R_5$ die bezüglich der Verbindungen der Verbindungend er allgemeinen Formel (I) angegebenen Bedeutungen besitzt, in der Wärme umsetzt zur Bildung der Verbindungen der allgemeinen Formel ($I_J$):

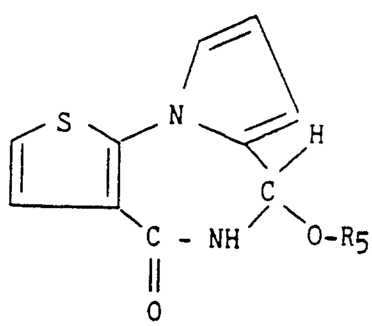

($I_J$)

in der $R_5$ die bezüglich der Derivate der allgemeinen Formel (I) angegebenen Bedeutungen besitzt,

**oder**

der Einwirkung einer Verbindung der allgemeinen Formel (X):

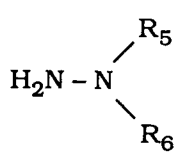

(X)

in der $R_5$ und $R_6$ die bezüglich der Verbindungen der allgemeinen Formel (I) angegebenen Bedeutungen besitzen, unterwirft zur Bildung der Verbindungen der allgemeinen Formel ($I_K$):

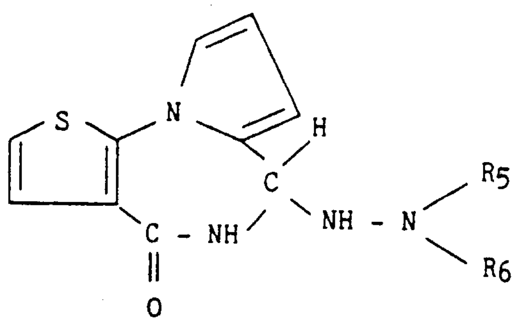

$(I_K)$

in der $R_5$ und $R_6$ die bezüglich der Verbindungen der allgemeinen Formel (I) angegebenen Bedeutungen besitzen,

**oder**

der Einwirkung eines Alkohols der allgemeinen Formel (IX) unterwirft zur Bildung der Verbindungen der allgemeinen Formel ($I_J$),

**oder**

der Einwirkung eines Thiols der allgemeinen Formel (XI):

$R_5SH$ (XI)

in der $R_5$ die bezüglich der Verbindungen der allgemeinen Formel (I) angegebenen Bedeutungen besitzt, bei Raumtemperatur unterwirft zur Bildung der Verbindungen der allgemeinen Formel ($I_L$):

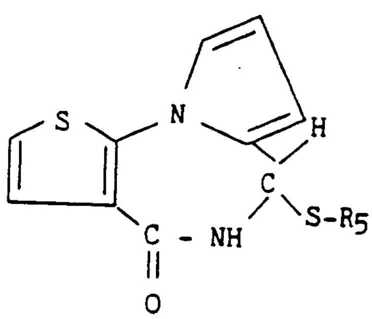

$(I_L)$

in der $R_5$ die bezüglich der Verbindungen der allgemeinen Formel (I) angegebenen Bedeutungen besitzt, welche man gegebenenfalls, wenn $R_5$ eine Carboxyalkylgruppe darstellt, der Einwirkung von Triethylamin oder eines Chlorameisensäureesters und dann der Einwirkung von gasförmigem Ammoniak unterziehen kann zur Bildung der entsprechenden Amide.

2. Verfahren nach Anspruch 1 zur Herstellung von Verbindungen der allgemeinen Formel ($I_A$):

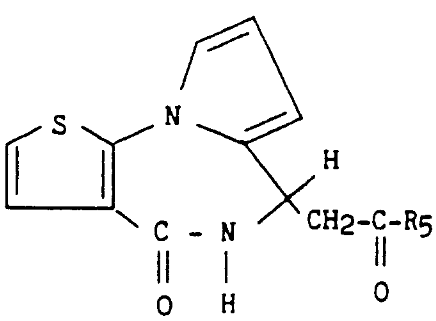

$(I_A)$

in der $R_5$ die in Anspruch 1 angegebenen Bedeutungen besitzt, sowie von deren Isomeren, Diastereoisomeren und Enantiomeren in isolierter Form oder in Form einer Mischung.

**3.** Verfahren nach Anspruch 1 zur Herstellung von Verbindungen der allgemeinen Formel ($I_B$):

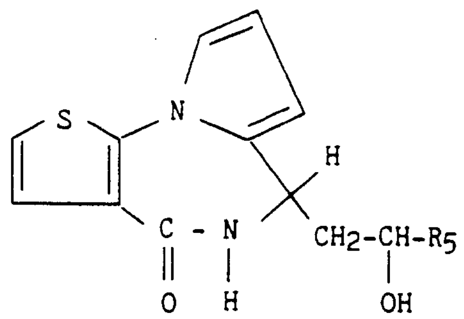

($I_B$)

in der $R_5$ die in Anspruch 1 angegebenen Bedeutungen besitzt, sowie von deren Isomeren, Diastereoisomeren und Enantiomeren in isolierter Form oder in Form einer Mischung.

**4.** Verfahren nach Anspruch 1 zur Herstellung von Verbindungen der allgemeinen Formel ($I_C$):

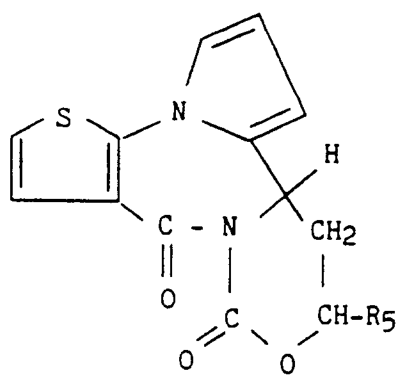

($I_C$)

in der $R_5$ die in Anspruch 1 angegebenen Bedeutungen besitzt, sowie von deren Isomeren, Enantiomeren und Diastereoisomeren in isolierter Form oder in Form einer Mischung.

**5.** Verfahren nach Anspruch 1 zur Herstellung von Verbindungen der allgemeinen Formel ($I_D$):

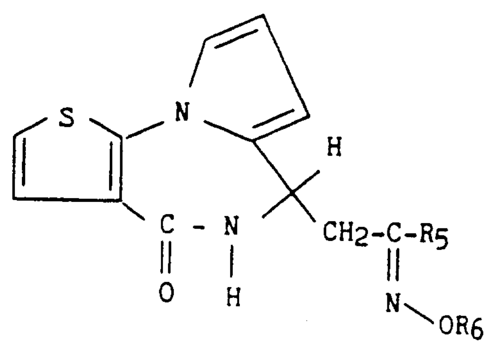

($I_D$)

in der $R_5$ und $R_6$ die in Anspruch 1 angegebenen Bedeutungen besitzt, sowie von deren Isomeren, Enantiomeren und Diastereoisomeren in isolierter Form oder in Form einer Mischung.

**6.** Verfahren nach Anspruch 1 zur Herstellung von Verbindungen der allgemeinen Formel ($I_E$):

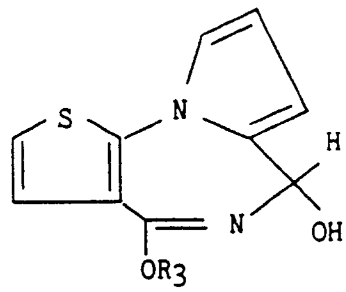

($I_E$)

in der $R_3$ die in Anspruch 1 angegebenen Bedeutungen besitzt, sowie von deren Isomeren, Enantiomeren und Diastereoisomeren in isolierter Form oder in Form einer Mischung.

**7.** Verfahren nach Anspruch 1 zur Herstellung von Verbindungen der allgemeinen Formel ($I_F$):

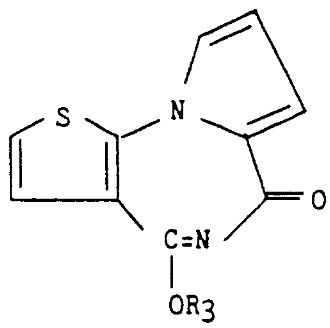

($I_F$)

in der $R_3$ die in Anspruch 1 angegebenen Bedeutungen besitzt, sowie von deren Isomeren, Diastereoisomeren und Enantiomeren in isolierter Form oder in Form einer Mischung.

**8.** Verfahren nach Anspruch 1 zur Herstellung von Verbindungen der allgemeinen Formel ($I_G$):

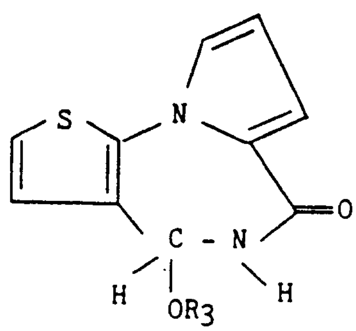

($I_G$)

in der $R_3$ die in Anspruch 1 angegebenen Bedeutungen besitzt, sowie von deren Isomeren, Diastereoisomeren und Enantiomeren in isolierter Form oder in Form einer Mischung.

**9.** Verfahren nach Anspruch 1 zur Herstellung von Verbindungen der allgemeinen Formel ($I_H$):

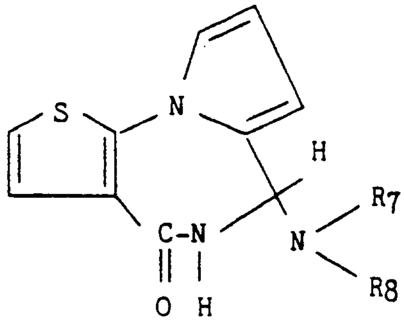

($I_H$)

in der $R_7$ und $R_8$ die in Anspruch 1 angegebenen Bedeutungen besitzt, sowie von deren Isomeren, Diastereoisomeren und Enantiomeren in isolierter Form oder in Form einer Mischung und von ihren gegebenenfalls möglichen Additionssalzen mit einer anorganischen oder organischen, pharmazeutisch annehmbaren Säure.

**10.** Verfahren nach Anspruch 1 zur Herstellung von Verbindungen der allgemeinen Formel ($I_J$):

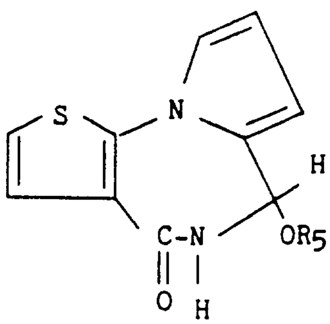

($I_J$)

in der $R_5$ die in Anspruch 1 angegebenen Bedeutungen besitzt, sowie von deren Isomeren, Diastereoisomeren und Enantiomeren in isolierter Form oder in Form einer Mischung.

**11.** Verfahren nach Anspruch 1 zur Herstellung von Verbindungen der allgemeinen Formel ($I_K$):

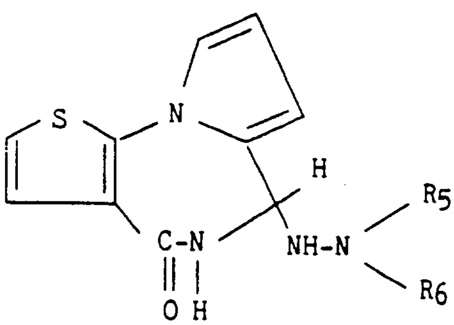

($I_K$)

in der $R_5$ und $R_6$ die in Anspruch 1 angegebenen Bedeutungen besitzt, sowie von deren Isomeren, Diastereoisomeren und Enantiomeren in isolierter Form oder in Form einer Mischung und von ihren gegebenenfalls möglichen Additionssalzen mit einer anorganischen oder organischen, pharmazeutisch annehmbaren Säure.

**12.** Verfahren nach Anspruch 1 zur Herstellung von Verbindungen der allgemeinen Formel ($I_L$):

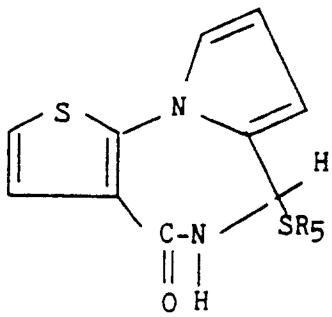

($I_L$)

in der $R_5$ die in Anspruch 1 angegebenen Bedeutungen besitzt, sowie von deren Isomeren, Diastereoisomeren und Enantiomeren in isolierter Form oder in Form einer Mischung.

**13.** Verfahren nach Anspruch 1 zur Herstellung von 5,6-Dihydro-6-(2-benzyloxypropyl)-4-oxo-4H-pyrrolo-[1,2-a]thieno[3,2-f][1,4]diazepin

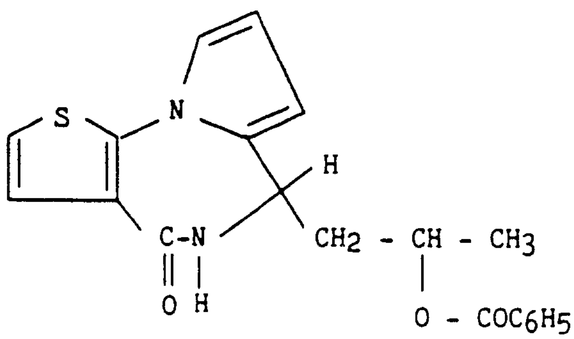

sowie von dessen Diastereoisomeren in isolierter Form oder in Form einer Mischung.

**14.** Verfahren nach Anspruch 1 zur Herstellung von 5,6-Dihydro-6-(2-hydrazono-1-pentyl)-4-oxo-4H-pyrrolo-[1,2-a]thieno[3,2-f][1,4]diazepin

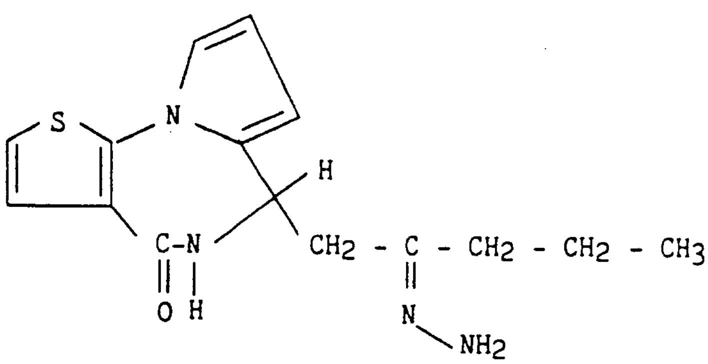

sowie von dessen Enantiomeren in isolierter Form oderin Form einer Mischung und von dessen Additionssalzen mit einer anorganischen oder organischen, pharmazeutisch annehmbaren Säure.

**15.** Verfahren nach den Ansprüchen 1 und 10 zur Herstellung von 5,6-Dihydro-6-hydroxy-4-oxo-4H-pyrrolo-[1,2-a]thieno[2,3-f][1,4]diazepin

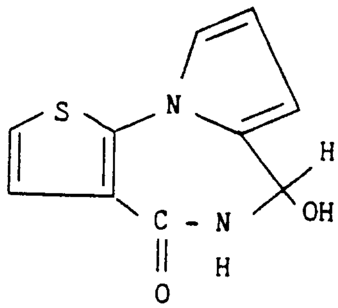

sowie von dessen Enantiomeren in isolierter Form oder in Form einer Mischung.

**16.** Verfahren nach Anspruch 1 zur Herstellung von 5,6-Dihydro-4-oxo-4H-pyrrolo[1,2-a]thieno[3,2-f][1,4]-diazepin

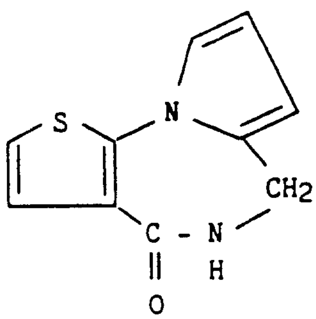

**17.** Verfahren zur Herstellung von pharmazeutischen Zubereitungen enthaltend als Wirkstoff eine Verbindung hergestellt nach den Ansprüchen 1 bis 16 allein oder in Kombination mit einem oder mehreren inerten, nichttoxischen, pharmazeutisch annehmbaren Trägermaterialien oder Bindemitteln.

**18.** Verfahren nach Anspruch 17 zur Herstellung von pharmazeutischen Zubereitungen, die in einer Form vorliegen, die insbesondere zur Behandlung von Gehirngefäßunfällen, des normalen oder pathologischen Alterns des Gehirns und von ischämischen Syndromen geeignet ist.

**19.** Verfahren nach Anspruch 17 zur Herstellung von pharmazeutischen Zubereitungen, die in einer Form vorliegen, die insbesondere zur Behandlung von Störungen des Magen-Darm-Trakts, des Pankreas, der Gallenblase, des Appetits, des Zentralnervensystems sowie von Schmerzen geeignet ist.

**20.** Verfahren nach Anspruch 17 zur Herstellung von pharmazeutischen Zubereitungen, die in einer Form vorliegen, die insbesondere zur Behandlung von Hyperlipidämie, Hypertriglyceridämie, Hypercholesterolämie, Hyperglykämie, der Hypertension und von damit verknüpften Krankheiten geeignet ist.